# EUROPEAN PATENT SPECIFICATION

(11) **EP 3 130 595 B1**
(45) Date of publication and mention of the grant of the patent: **17.10.2018**
(21) Application number: 16183831.3
(22) Date of filing: 11.08.2016
(51) Int. Cl.: C07F 15/00, C09K 11/06, H01L 51/50, H05B 33/14, H01L 51/00

(54) **ORGANOMETALLIC COMPOUND, ORGANIC LIGHT-EMITTING DEVICE INCLUDING THE ORGANOMETALLIC COMPOUND, AND DIAGNOSIS COMPOSITION INCLUDING THE ORGANOMETALLIC COMPOUND**
ORGANOMETALLISCHE VERBINDUNG, ORGANISCHE LICHTEMITTIERENDE VORRICHTUNG MIT DER ORGANOMETALLISCHEN VERBINDUNG UND DIAGNOSEZUSAMMENSETZUNG MIT DER ORGANOMETALLISCHEN VERBINDUNG
COMPOSÉ ORGANOMÉTALLIQUE, COMPOSITION CONTENANT LE COMPOSÉ ORGANOMÉTALLIQUE ET DISPOSITIF ÉLECTROLUMINESCENT ORGANIQUE COMPRENANT CE COMPOSÉ OU CETTE COMPOSITION ORGANOMÉTALLIQUE

(30) Priority: 13.08.2015 KR 20150114549
(43) Date of publication of application: 15.02.2017
(73) Proprietor: Samsung Electronics Co., Ltd., Suwon-si, Gyeonggi-do 16677 (KR)
(72) Inventor: LEE, Jiyoun, 16678 Gyeonggi-do (KR); KWAK, Yoonhyun, 16678 Gyeonggi-do (KR); PARK, Bumwoo, 16678 Gyeonggi-do (KR); LEE, Sunyoung, 16678 Gyeonggi-do (KR); LEE, Jungin, 16678 Gyeonggi-do (KR); PARK, Youngjae, 16678 Gyeonggi-do (KR)
(74) Representative: Scheuermann, Erik

(56) References cited:
- EP-A1- 2 873 711
- DE-A1-102011 001 007
- US-A1- 2013 193 428
- US-A1- 2014 142 080
- QINDE LUI ET AL: "New red-orange phosphorescent/electrolumine scent cycloplatinated complex of 2, 6-bis(2'-indolyl) pyridine", JOURNAL OF THE CHEMICAL SOCIETY, DALTON TRANSACTIONS, CHEMICAL SOCIETY. LETCHWORTH, GB, no. 16, 18 July 2002 (2002-07-18), pages 3234-3240, XP002975889, ISSN: 1472-7773
- ZHILINA EKATERINA F ET AL: "Neutral tetranuclear Cu(II) complex of 2,6-di(5-trifluoromethylpyrazol-3-yl)pyrid ine: Synthesis, characterization and its transformation with selected aza-ligands", POLYHEDRON, PERGAMON PRESS, OXFORD, GB, vol. 53, 1 February 2013 (2013-02-01), pages 122-131, XP028996262, ISSN: 0277-5387, DOI: 10.1016/J.POLY.2013.01.021
- WEN-LI JIA ET AL: "Novel Phosphorescent Cyclometalated Organotin(IV) and Organolead(IV) Complexes of 2,6-Bis(2'-indolyl)pyridine and 2,6-Bis[2'-(7-azaindolyl)]pyridine", ORGANOMETALLICS, vol. 22, no. 20, 1 September 2003 (2003-09-01), pages 4070-4078, XP055326165, US ISSN: 0276-7333, DOI: 10.1021/om030404a
- YONGBO ZHOU ET AL: "Mononuclear, dinuclear, hexanuclear, and one-dimensional polymeric silver complexes having ligand-supported and unsupported argentophilic interactions stabilized by pincer-like 2,6-bis(5-pyrazolyl)pyridine ligands", DALTON TRANSACTIONS: THE INTERNATIONAL JOURNAL FOR INORGANIC, ORGANOMETALLIC AND BIOINORGANIC CHEMISTRY, no. 11, 1 January 2008 (2008-01-01), page 1444, XP055321043, GB ISSN: 1477-9226, DOI: 10.1039/b710916d
- NOBUYUKI KOMINE ET AL: "Probing the Steric and Electronic Characteristics of a New Bis-Pyrrolide Pincer Ligand", INORGANIC CHEMISTRY, vol. 53, no. 3, 3 February 2014 (2014-02-03), pages 1361-1369, XP055321042, EASTON, US ISSN: 0020-1669, DOI: 10.1021/ic402120r
- ANZHELA GALSTYAN ET AL: "Correlating the Structural and Photo-physical Features of Pincer Luminophores and Monodentate Ancillary Ligands in Pt II Phosphors", EUROPEAN JOURNAL OF INORGANIC CHEMISTRY., vol. 2015, no. 36, 21 October 2015 (2015-10-21), pages 5822-5831, XP055321041, DE ISSN: 1434-1948, DOI: 10.1002/ejic.201500949
- Kang-Wei Wang ET AL: "Mono- versus Dinuclear Pt(II) 6-(5-Trifluoromethyl-Pyrazol-3-yl)-2,2'-Bi pyridine Complexes: Synthesis, Characterization, and Remarkable Difference in Luminescent Properties", INORGANIC CHEMISTRY, vol. 49, no. 4, 15 February 2010 (2010-02-15), pages 1372-1383, XP055409510, EASTON, US ISSN: 0020-1669, DOI: 10.1021/ic9011313

## Description

### FIELD OF THE INVENTION

One or more aspects of example embodiments of the present disclosure are related to an organometallic compound, an organic light-emitting device including the organometallic compound, and composition for use in diagnosis including the organometallic compound.

### BACKGROUND OF THE INVENTION

Organic light-emitting devices are self-emission devices, have better characteristics than conventional devices in terms of a viewing angle, a response time, and brightness, a driving voltage, and a response speed, and produce full-color images.

In an example, an organic light-emitting device includes an anode, a cathode, and an organic layer between the anode and the cathode, wherein the organic layer includes an emission layer. A hole transport region may be between the anode and the emission layer, and an electron transport region may be between the emission layer and the cathode. Holes provided from the anode may move toward the emission layer through the hole transport region, and electrons provided from the cathode may move toward the emission layer through the electron transport region. The holes and the electrons recombine in the emission layer to produce excitons. These excitons transition from an excited state to a ground state, thereby generating light.

DE102011001007 A1 discloses platinum(II) complexes comprising a dianionic tridentate nitrogen, nitrogen, carbon ligand or a dianionic tridentate nitrogen, nitrogen, nitrogen ligand and a monodentate neutral ligand, useful as triplet emitter in organic LED.

Wang et al. ((Inorg. Chem. 2010, 49, 1372-1383) have investigated the luminescent properties of mono- and dinuclear Pt(II) 6-(5-trifluoromethyl-pyrazol-3-yl)-2,2'-bipyridine complexes.

Meanwhile, luminescent compounds may be used to monitor, sense, or detect a biological material, such as a cell protein. Examples of such luminescent compounds include a phosphorescent luminescent compound.

### SUMMARY OF THE INVENTION

One or more embodiments include an organometallic compound, an organic light-emitting device including the organometallic compound, and a for use in diagnosis including the organometallic compound.

Additional aspects will be set forth in part in the description which follows and, in part, will be apparent from the description, or may be learned by practice of the presented embodiments.

According to one or more embodiments, an organometallic compound is represented by Formula 1 below:

<Formula 1> M(L₁)(L₂)

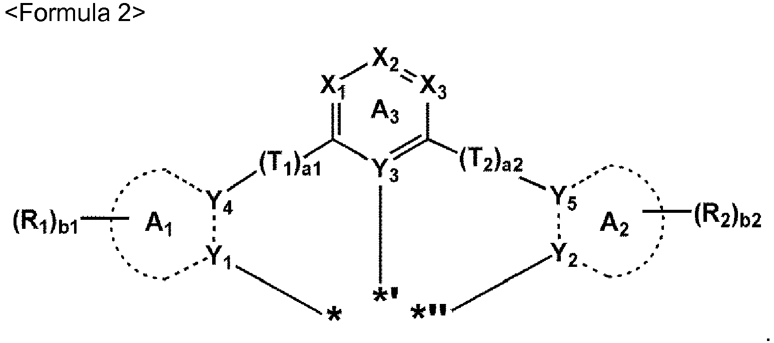

In Formulae 1 and 2,
M may be ruthenium (Ru), rhodium (Rh), palladium (Pd), silver (Ag), rhenium (Re), platinum (Pt) or gold (Au),
L₁ may be selected from tridentate ligands represented by Formula 2,
L₂ may be selected from monodentate organic ligands,
*, *', and *" in Formula 2 each indicate a binding site to M in Formula 1,
Y₁ to Y₃ may each be nitrogen (N),
Y₄ and Y₅ may each be carbon (C),
A bond between Y₁ and Y₄ may be a single bond or a double bond, and a bond between Y₂ and Y₅ may be a single bond or a double bond,
One selected from a bond between Y₁ and M, a bond between Y₂ and M, and a bond between ligand L₂ and M may be a coordinate bond, and the other two may be a covalent bond,
A bond between Y₃ and M may be a coordinate bond,
rings A₁ and A₂ may each independently be selected from a pyrrole ring, a pyrazole ring, and an imidazole ring, whereby at least one selected from rings A₁ and A₂ is a pyrazole ring,
X₁ may be N or C(R₃), X₂ may be N or C(R₄), X₃ may be N or C(R₅), two or more selected from R₃ to R₅ may be optionally connected to each other to form a substituted or unsubstituted C₅-C₃₀ carbocyclic group or a substituted or unsubstituted C₂-C₃₀ heterocyclic group, and ring A₃ may have two or less nitrogen atoms as a ring-forming atom,
T₁ and T₂ may each independently be selected from a single bond, *-O-*', *-S-*', *-C(R₆)(R₇)-*', *-C(R₆)=*', *=C(R₆)-*', *-C(R₆)=C(R₇)-*', *-C(=O)-*', *-C(=S)-*', *-C≡C-*', *-N(R₆)-*', and *-Si(R₆)(R₇)-*', and R₆ and R₇ may be optionally connected to each other to form a substituted or unsubstituted C₅-C₃₀ carbocyclic group or a substituted or unsubstituted C₂-C₃₀ heterocyclic group,
a1 and a2 may each independently be an integer selected from 1 to 3,
R₁ to R₇ may each independently be selected from hydrogen, deuterium, -F, -CI, -Br, -I, -SF₅, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a substituted or unsubstituted C₁-C₆₀ alkyl group, a substituted or unsubstituted C₂-C₆₀ alkenyl group, a substituted or unsubstituted C₂-C₆₀ alkynyl group, a substituted or unsubstituted C₁-C₆₀ alkoxy group, a substituted or unsubstituted C₃-C₁₀ cycloalkyl group, a substituted or unsubstituted C₁-C₁₀ heterocycloalkyl group, a substituted or unsubstituted C₃-C₁₀ cycloalkenyl group, a substituted or unsubstituted C₁-C₁₀ heterocycloalkenyl group, a substituted or unsubstituted C₆-C₆₀ aryl group, a substituted or unsubstituted C₆-C₆₀ aryloxy group, a substituted or unsubstituted C₆-C₆₀ arylthio group, a substituted or unsubstituted C₁-C₆₀ heteroaryl group, a substituted or unsubstituted monovalent non-aromatic condensed polycyclic group, a substituted or unsubstituted monovalent non-aromatic condensed heteropolycyclic group, -N(Q₁)(Q₂), -Si(Q₃)(Q₄)(Q₅), -B(Q₆)(Q₇), and -P(=O)(Q₈)(Q₉),
b1 and b2 may each independently be an integer selected from 0 to 3, wherein, when b1 is two or more, two or more R₁(s) may be identical to or different from each other, and when b2 is two or more, two or more R₂(s) may be identical to or different from each other,
two selected from R₁(s) in the number of b1 may be optionally connected to each other to form a substituted or unsubstituted C₅-C₃₀ carbocyclic group or a substituted or unsubstituted C₂-C₃₀ heterocyclic group,
two selected from R₂(s) in the number of b2 may be optionally connected to form a substituted or unsubstituted C₅-C₃₀ carbocyclic group or a substituted or unsubstituted C₂-C₃₀ heterocyclic group,
a moiety represented by 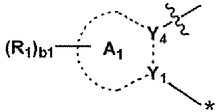 in Formula 2 may not be 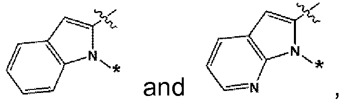 and a moiety represented by 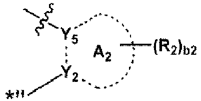 in Formula 2 may not be 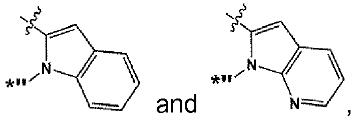 at least one substituent selected from a substituent(s) of the substituted C₅-C₃₀ carbocyclic group, the substituted C₂-C₃₀ heterocyclic group, the substituted C₁-C₆₀ alkyl group, the substituted C₂-C₆₀ alkenyl group, the substituted C₂-C₆₀ alkynyl group, the substituted C₁-C₆₀ alkoxy group, the substituted C₃-C₁₀ cycloalkyl group, the substituted C₁-C₁₀ heterocycloalkyl group, the substituted C₃-C₁₀ cycloalkenyl group, the substituted C₁-C₁₀ heterocycloalkenyl group, the substituted C₆-C₆₀ aryl group, the substituted C₆-C₆₀ aryloxy group, the substituted C₆-C₆₀ arylthio group, the substituted C₁-C₆₀ heteroaryl group, the substituted monovalent non-aromatic condensed polycyclic group, and the substituted monovalent non-aromatic condensed heteropolycyclic group may be selected from:
deuterium, -F, -CI, -Br, -I, -CD₃, -CD₂H, -CDH₂, -CF₃, -CF₂H, -CFH₂, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a C₁-C₆₀ alkyl group, a C₂-C₆₀ alkenyl group, a C₂-C₆₀ alkynyl group, and a C₁-C₆₀ alkoxy group;
a C₁-C₆₀ alkyl group, a C₂-C₆₀ alkenyl group, a C₂-C₆₀ alkynyl group, and a C₁-C₆₀ alkoxy group, each substituted with at least one selected from deuterium, -F, -CI, -Br, -I, -CD₃, -CD₂H, -CDH₂, -CF₃, -CF₂H, -CFH₂, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a C₃-C₁₀ cycloalkyl group, a C₁-C₁₀ heterocycloalkyl group, a C₃-C₁₀ cycloalkenyl group, a C₁-C₁₀ heterocycloalkenyl group, a C₆-C₆₀ aryl group, a C₆-C₆₀ aryloxy group, a C₆-C₆₀ arylthio group, a C₁-C₆₀ heteroaryl group, a monovalent non-aromatic condensed polycyclic group, a monovalent non-aromatic condensed heteropolycyclic group, -N(Q₁₁)(Q₁₂), -Si(Q₁₃)(Q₁₄)(Q₁₅), -B(Q₁₆)(Q₁₇), and -P(=O)(Q₁₈)(Q₁₉);
a C₃-C₁₀ cycloalkyl group, a C₁-C₁₀ heterocycloalkyl group, a C₃-C₁₀ cycloalkenyl group, a C₁-C₁₀ heterocycloalkenyl group, a C₆-C₆₀ aryl group, a C₆-C₆₀ aryloxy group, a C₆-C₆₀ arylthio group, a C₁-C₆₀ heteroaryl group, a monovalent non-aromatic condensed polycyclic group, and a monovalent non-aromatic condensed heteropolycyclic group;
a C₃-C₁₀ cycloalkyl group, a C₁-C₁₀ heterocycloalkyl group, a C₃-C₁₀ cycloalkenyl group, a C₁-C₁₀ heterocycloalkenyl group, a C₆-C₆₀ aryl group, a C₆-C₆₀ aryloxy group, a C₆-C₆₀ arylthio group, a C₁-C₆₀ heteroaryl group, a monovalent non-aromatic condensed polycyclic group, and a monovalent non-aromatic condensed heteropolycyclic group, each substituted with at least one selected from deuterium, -F, -CI, -Br, -I, -CD₃, -CD₂H, -CDH₂, -CF₃, -CF₂H, -CFH₂, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a C₁-C₆₀ alkyl group, a C₂-C₆₀ alkenyl group, a C₂-C₆₀ alkynyl group, a C₁-C₆₀ alkoxy group, a C₃-C₁₀ cycloalkyl group, a C₁-C₁₀ heterocycloalkyl group, a C₃-C₁₀ cycloalkenyl group, a C₁-C₁₀ heterocycloalkenyl group, a C₆-C₆₀ aryl group, a C₆-C₆₀ aryloxy group, a C₆-C₆₀ arylthio group, a C₁-C₆₀ heteroaryl group, a monovalent non-aromatic condensed polycyclic group, a monovalent non-aromatic condensed heteropolycyclic group, -N(Q₂₁)(Q₂₂), -Si(Q₂₃)(Q₂₄)(Q₂₅), -B(Q₂₆)(Q₂₇), and -P(=O)(Q₂₈)(Q₂₉); and
-N(Q₃₁)(Q₃₂), -Si(Q₃₃)(Q₃₄)(Q₃₅), -B(Q₃₆)(Q₃₇), and -P(=O)(Q₃₈)(Q₃₉),
wherein Q₁ to Q₉, Q₁₁ to Q₁₉, Q₂₁ to Q₂₉, and Q₃₁ to Q₃₉ may each independently be selected from hydrogen, deuterium, -F, -Cl, -Br, -I, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a C₁-C₆₀ alkyl group, a C₂-C₆₀ alkenyl group, a C₂-C₆₀ alkynyl group, a C₁-C₆₀ alkoxy group, a C₃-C₁₀ cycloalkyl group, a C₁-C₁₀ heterocycloalkyl group, a C₃-C₁₀ cycloalkenyl group, a C₁-C₁₀ heterocycloalkenyl group, a C₆-C₆₀ aryl group, a C₆-C₆₀ aryl group substituted with at least one selected from a C₁-C₆₀ alkyl group and a C₆-C₆₀ aryl group, a C₆-C₆₀ aryloxy group, a C₆-C₆₀ arylthio group, a C₁-C₆₀ heteroaryl group, a monovalent non-aromatic condensed polycyclic group, and a monovalent non-aromatic condensed heteropolycyclic group.

In one or more embodiments, an organic light-emitting device includes: a first electrode; a second electrode; and an organic layer between the first electrode and the second electrode, the organic layer including an emission layer, wherein the organic layer includes one or more selected from the organometallic compounds.

The organometallic compounds may act as a dopant in the emission layer.

In one or more embodiments, a composition for use in diagnosis includes one or more selected from the organometallic compounds represented by Formula 1. Diagnosis in the sense of the invention refers to both *in vivo* and *in vitro* diagnosis.

### BRIEF DESCRIPTION OF THE DRAWINGS

These and/or other aspects will become apparent and more readily appreciated from the following description of the embodiments, taken in conjunction with the accompanying drawing in which:

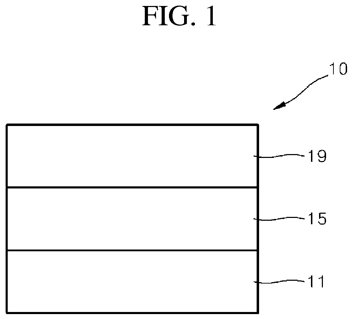

FIG. 1 is a schematic cross-sectional view of an organic light-emitting device according to an embodiment.

### DETAILED DESCRIPTION OF THE EMBODIMENTS

An organometallic compound according to an embodiment may be represented by Formula 1 below:

<Formula 1> M(L₁)(L₂),

M in Formula 1 may be ruthenium (Ru), rhodium (Rh), palladium (Pd), platinum (Pt) or gold (Au). For example, M in Formula 1 may be platinum (Pt).

L₁ in Formula 1 may be selected from tridentate ligands represented by Formula 2, L₂ may be selected from monodentate organic ligands, and *, *', and *" in Formula 2 each indicate a binding site to M in Formula 1. 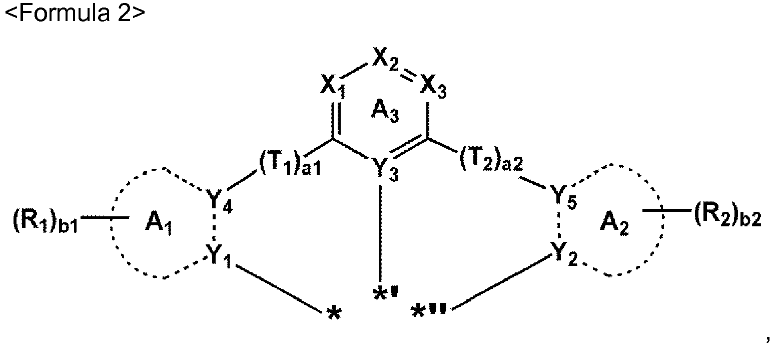 wherein, in Formula 2, Y₁ to Y₃ may be nitrogen (N), Y₄ and Y₅ may be carbon (C), a bond between Y₁ and Y₄ may be a single bond or a double bond, and a bond between Y₂ and Y₅ may be a single bond or a double bond.

In Formula 2, one selected from a bond between Y₁ and M, a bond between Y₂ and M, and a bond between ligand L₂ and M may be a coordinate bond, and the other two may be a covalent bond. Also, a bond between Y₃ and M may be a coordinate bond. Therefore, the organometallic compound represented by Formula 1 is in a neutral state having no electric charge.

In one or more embodiments, in Formulae 1 and 2, a bond between Y₁ and M and a bond between Y₂ and M may be a covalent bond, and a bond between Y₃ and M and a bond between ligand L₂ and M may be a coordinate bond.

In one or more embodiments, in Formulae 1 and 2, a bond between Y₁ and M and a bond between ligand L₂ and M may be a covalent bond, and a bond between Y₃ and M and a bond between Y₂ and M may be a coordinate bond.

Rings A₁ and A₂ in Formula 2 may each independently be selected from a pyrrole ring, a pyrazole ring, and an imidazole ring, wherein at least one selected from rings A₁ and A₂ is a pyrazole ring. Accordingly, rings A₁ and A₂ may include one or two nitrogen atoms as a ring-forming atom.

In Formula 2, X₁ may be N or C(R₃), X₂ may be N or C(R₄), and X₃ may be N or C(R₅). Two or more selected from R₃ to R₅ may be optionally connected to each other to form a substituted or unsubstituted C₅-C₃₀ carbocyclic group or a substituted or unsubstituted C₂-C₃₀ heterocyclic group (for example, a substituted or unsubstituted cyclopentadiene group, a substituted or unsubstituted cyclopentane group, a substituted or unsubstituted cyclohexane group, a substituted or unsubstituted adamantane group, a substituted or unsubstituted bicyclo[2.2.1]heptane group, a substituted or unsubstituted benzene group, a substituted or unsubstituted pyridine group, a substituted or unsubstituted pyrimidine group, a substituted or unsubstituted pyrazine group, a substituted or unsubstituted pyridazine group, a substituted or unsubstituted naphthalene group, a substituted or unsubstituted anthracene group, a substituted or unsubstituted tetracene group, a substituted or unsubstituted phenanthrene group, a substituted or unsubstituted dihydronaphthalene group, a substituted or unsubstituted phenalene group, a substituted or unsubstituted benzothiophene group, a substituted or unsubstituted benzofuran group, a substituted or unsubstituted indene group, a substituted or unsubstituted indole group, and the like). Descriptions for a susbtituent of the substituted C₅-C₃₀ carbocyclic group and the substituted C₂-C₃₀ heterocyclic group are same as descriptions for R₁ in this disclosure.

Ring A₃ in Formula 2 may have one or two nitrogen atoms as a ring-forming atom.

In one or more embodiments, a moiety represented by 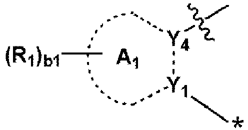 in Formula 2 and a moiety represented by 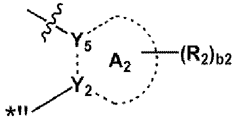 in Formula 2 may be identical to each other.

In one or more embodiments, a moiety represented by 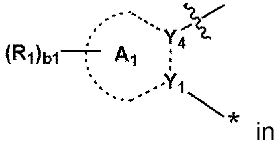 Formula 2 and a moiety represented by 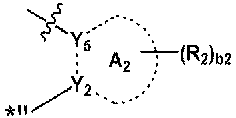 in Formula 2 may be different from each other.

In one or more embodiments, at least one selected from rings A₁ and A₂ in Formula 2 may be pyrazol ring.

In one or more embodiments, both rings A₁ and A₂ in Formula 2 may be a pyrazol ring, but embodiments of the present disclosure are not limited thereto.

T₁ and T₂ in Formula 2 may each independently be selected from a single bond, *-O-*', *-S-*', *-C(R₆)(R₇)-*', *-C(R₆)=*', *=C(R₆)-*', *-C(R₆)=C(R₇)-*', *-C(=O)-*', *-C(=S)-*', *-C≡C-*', *-N(R₆)-*', and *-Si(R₆)(R₇)-*'. R₆ and R₇ may be optionally connected to each other to form a substituted or unsubstituted C₅-C₃₀ carbocyclic group or a substituted or unsubstituted C₂-C₃₀ heterocyclic group (for example, a substituted or unsubstituted cyclopentane group, a substituted or unsubstituted cyclohexane group, a substituted or unsubstituted benzene group, a substituted or unsubstituted naphthalene group, and the like). R₆ and R₇ are the same as described below.

a1 and a2 in Formula 1 may each independently be an integer selected from 1 to 3. a1 indicates the number of T₁(s), wherein, when a1 is two or more, two or more T₁(s) may be identical to or different from each other. a2 indicates the number of T₂(s), wherein, when a2 is two or more, two or more T₂(s) may be identical to or different from each other. For example, a1 and a2 may each independently be 1 or 2.

In one or more embodiments, T₁ and T₂ in Formula 1 may each independently be selected from a single bond, *-O-*', *-S-*', *-C(R₆)(R₇)-*', *-C(R₆)=*', *=C(R₆)-*', *-C(R₆)=C(R₇)-*', *-C(=O)-*', *-C(=S)-*', *-C≡C-*', *-N(R₆)-*', and a group represented by any of Formulae 11-1 to 11-4, and a1 and a2 may each independently be 1 or 2:

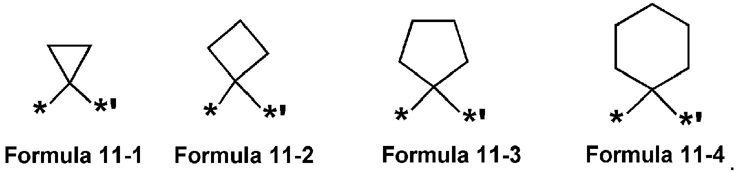

In one or more embodiments, T₁ and T₂ in Formula 1 may be a single bond, but embodiments of the present disclosure are not limited thereto.

A moiety represented by 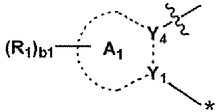 in Formula 2 may not be 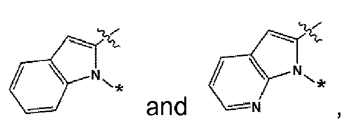 and a moiety represented by 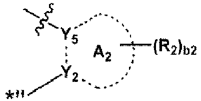 in Formula 2 may not be

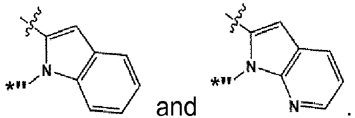

For example, a moiety represented by 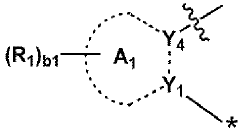 in Formula 2 may be represented by one selected from Formulae 3-1 to 3-16 and 3-31 to 3-70,
a moiety represented by 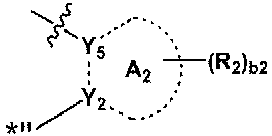 in Formula 2 may be represented by one selected from Formulae 4-1 to 4-16 and 4-31 to 4-70, and
a moiety represented by 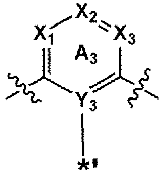 in Formula 2 may be represented by Formulae 5-1 to 5-47:

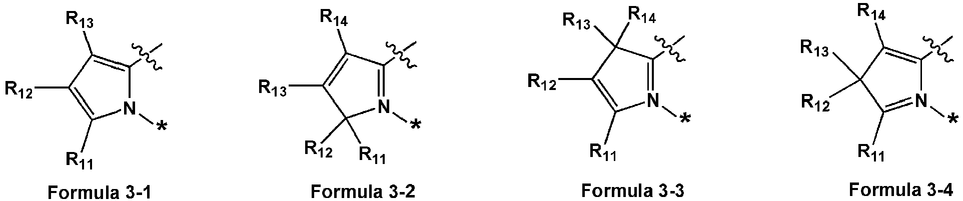

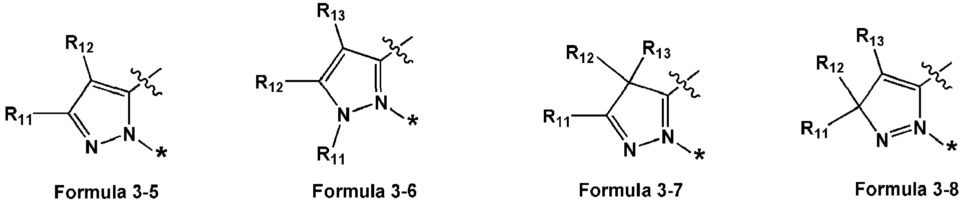

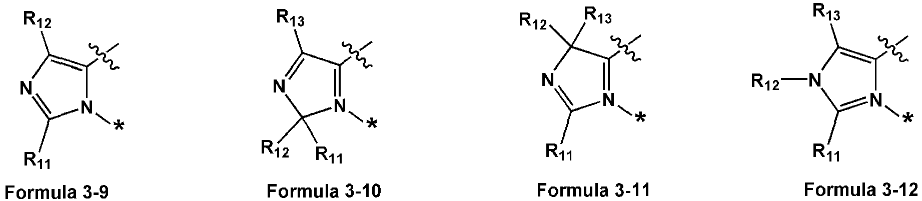

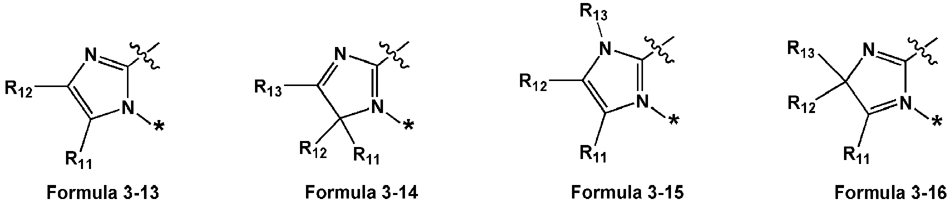

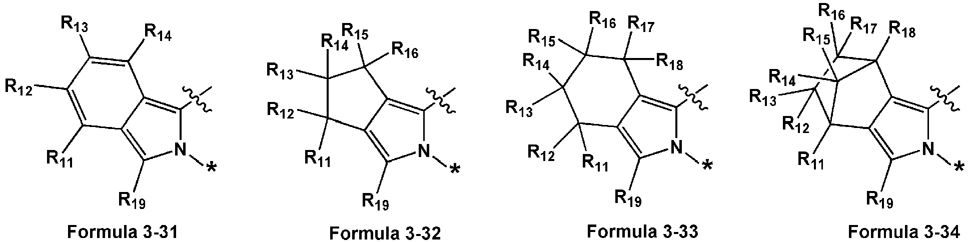

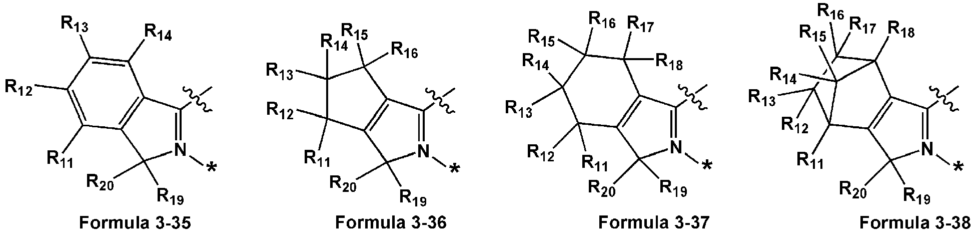

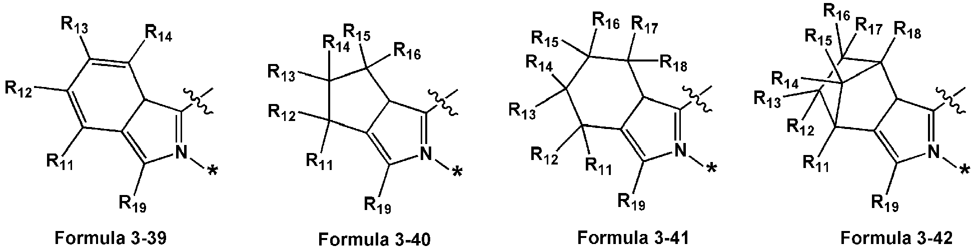

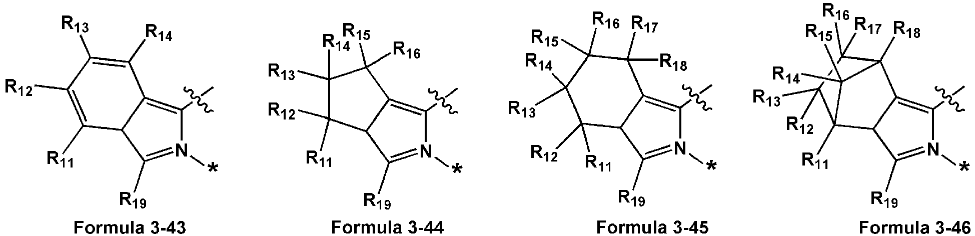

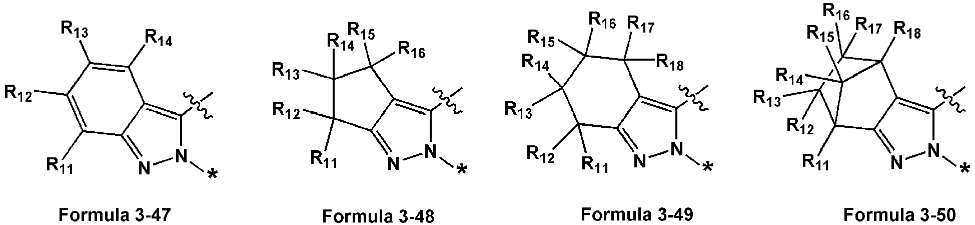

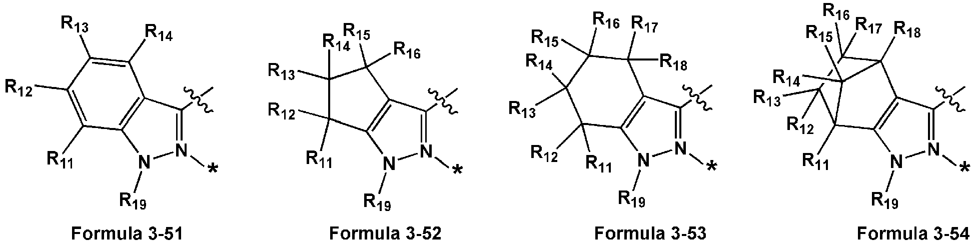

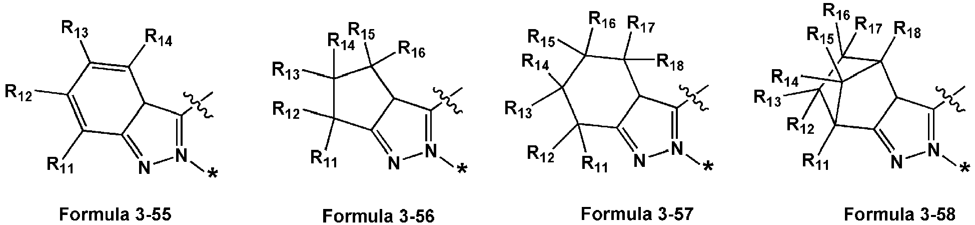

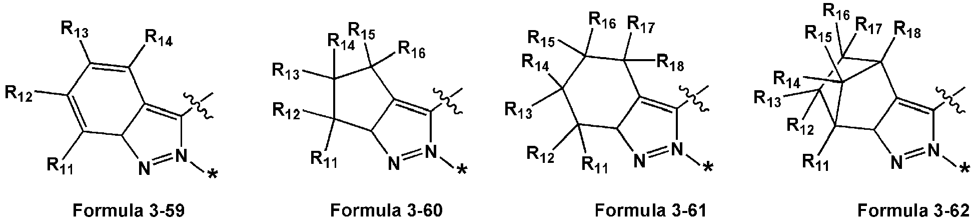

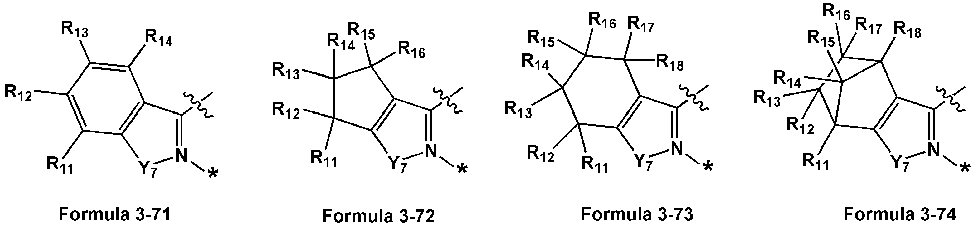

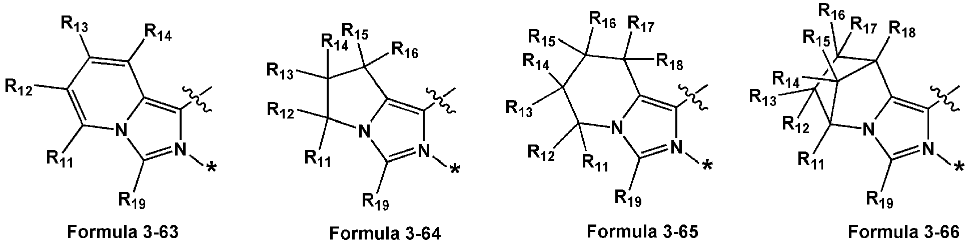

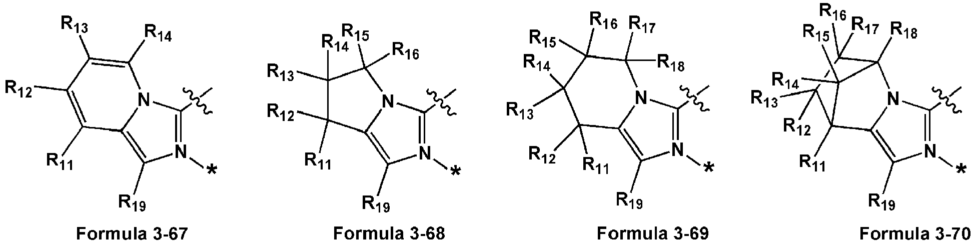

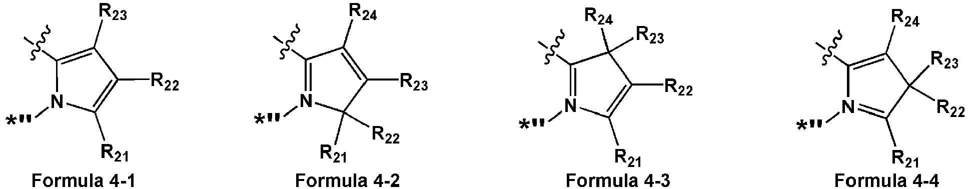

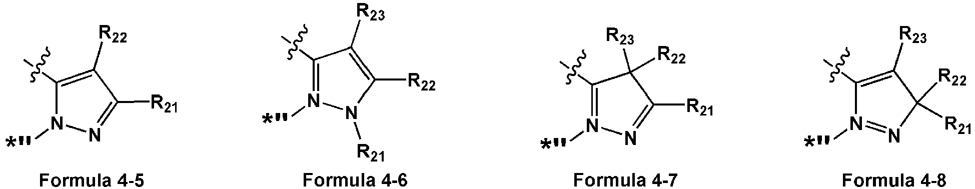

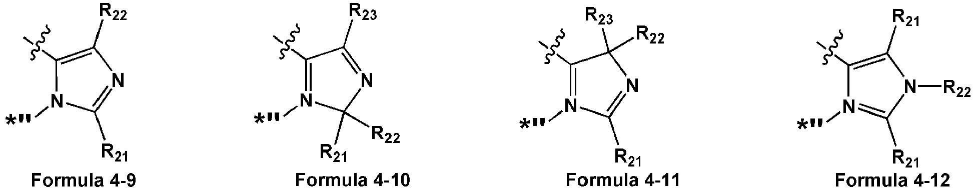

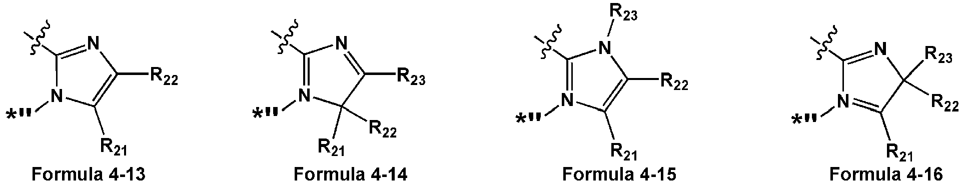

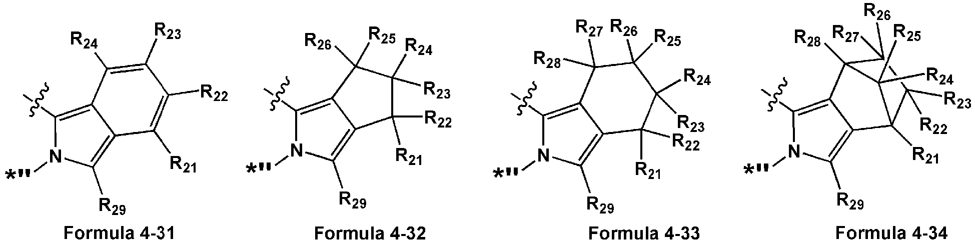

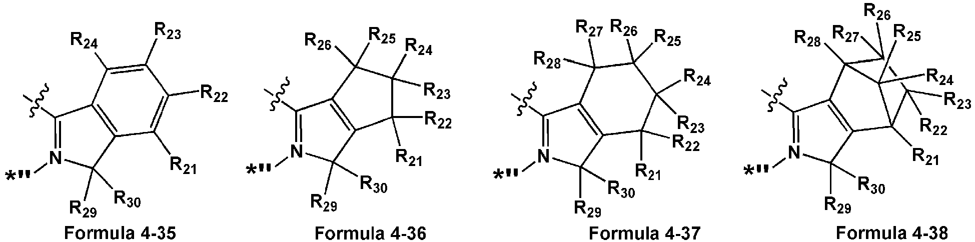

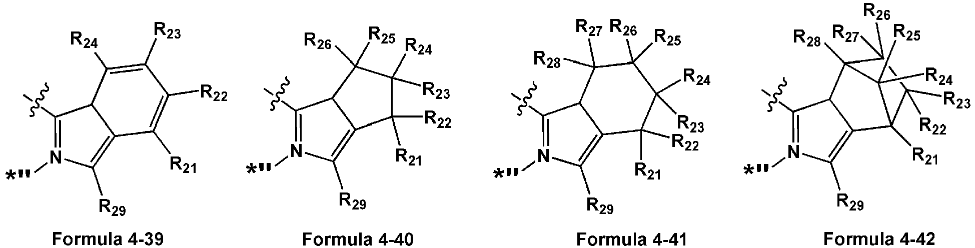

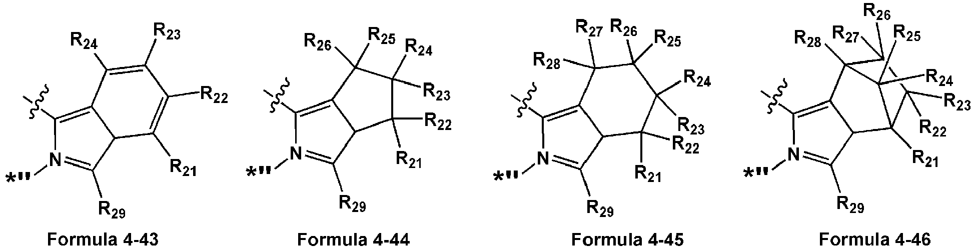

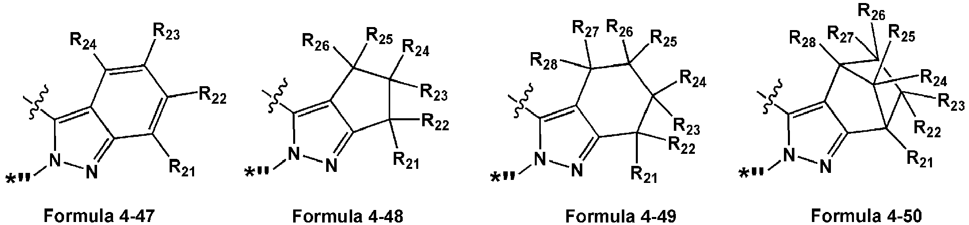

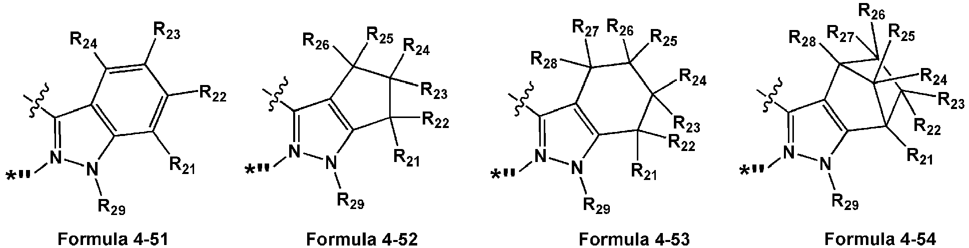

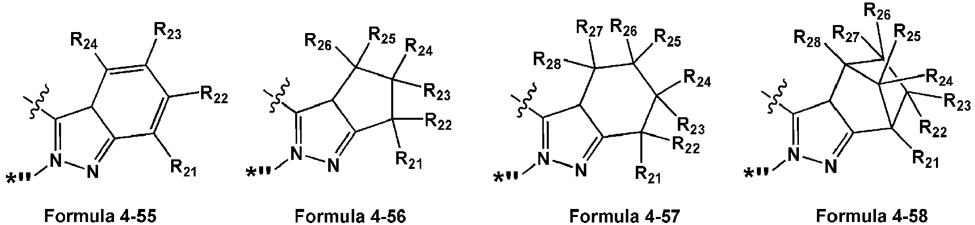

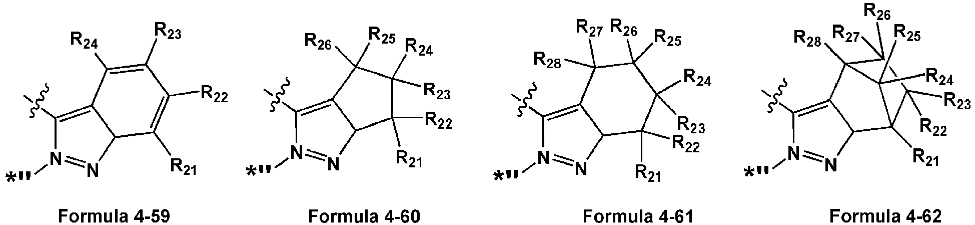

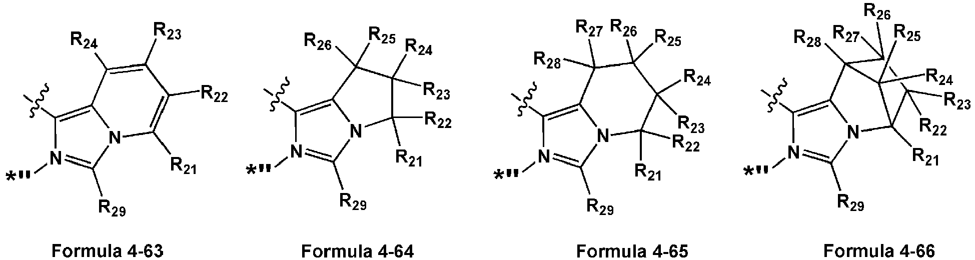

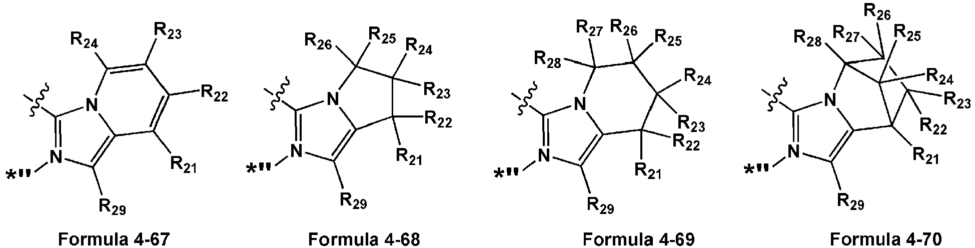

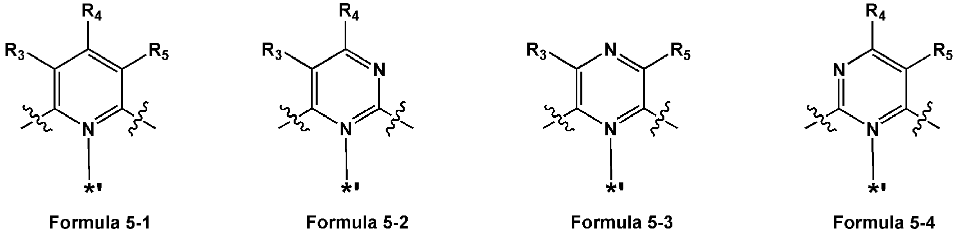

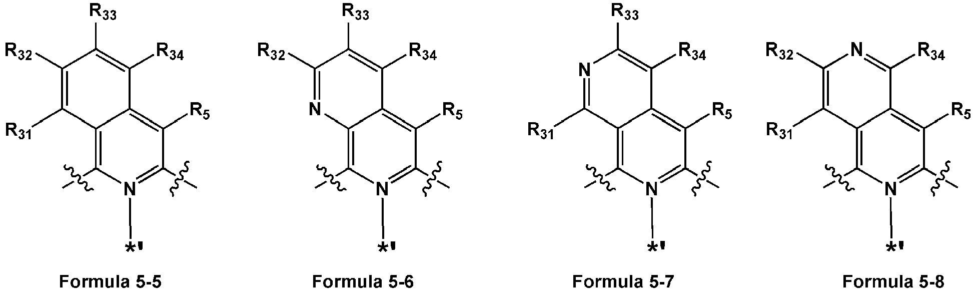

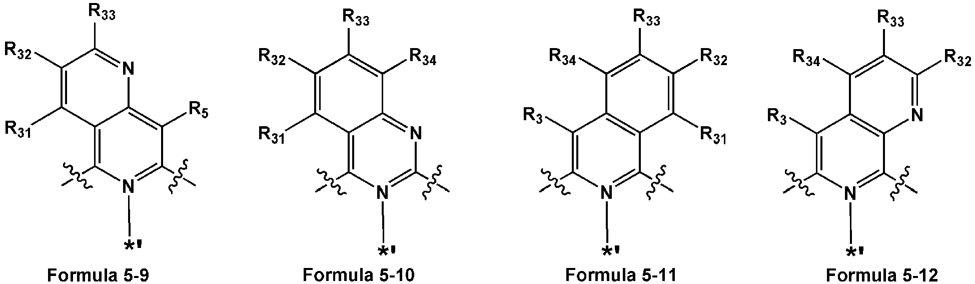

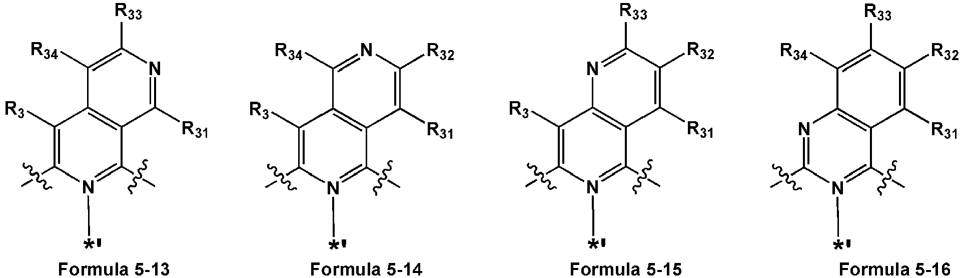

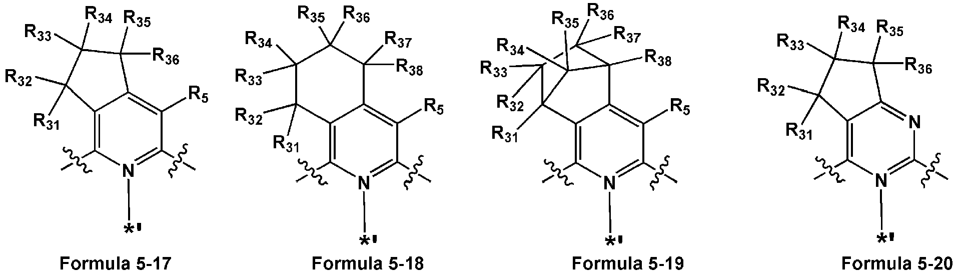

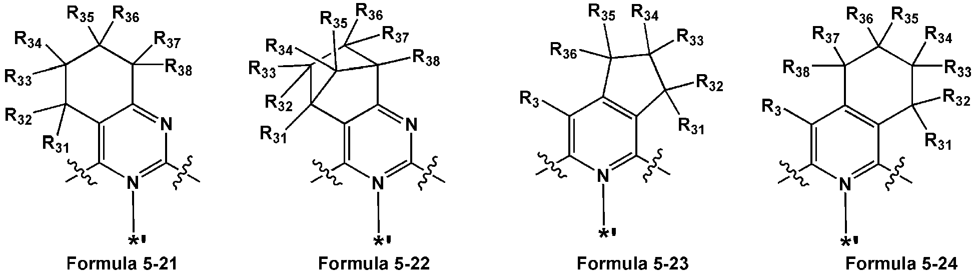

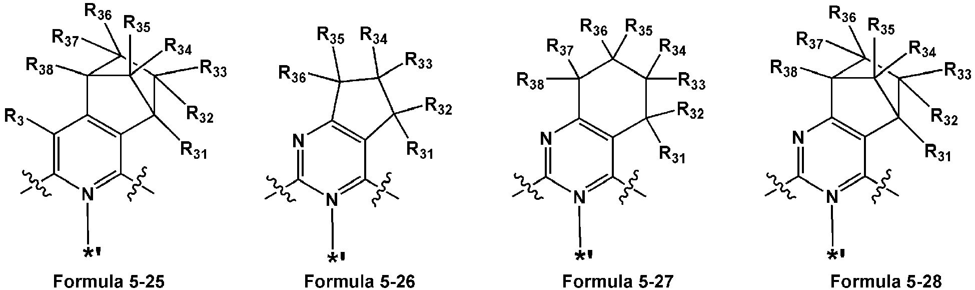

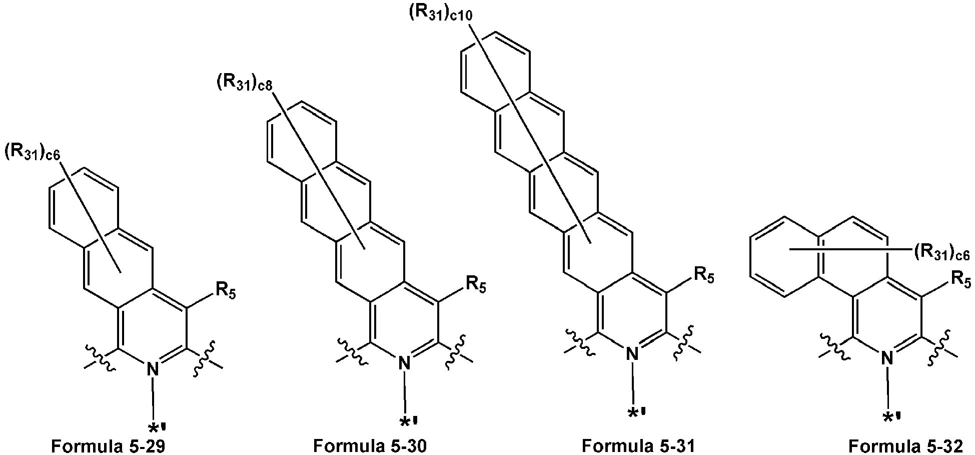

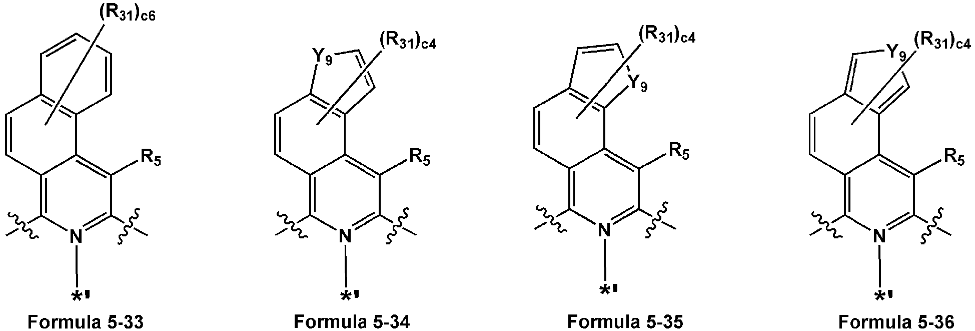

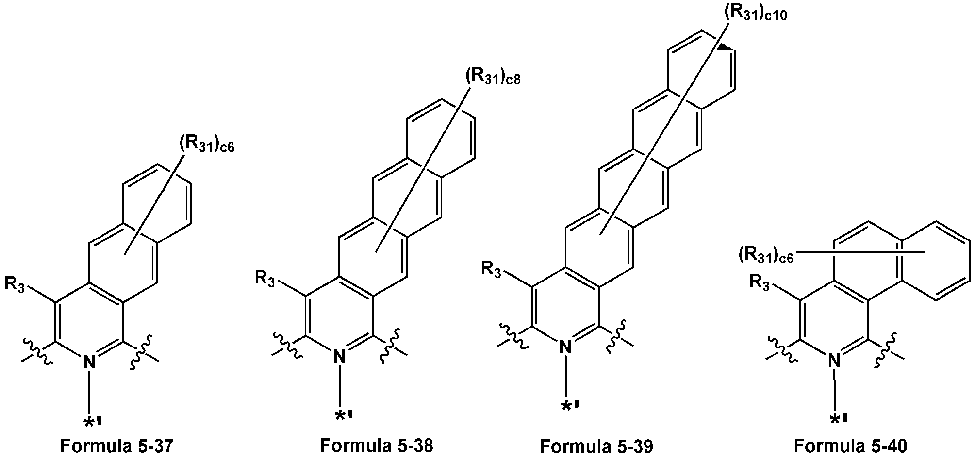

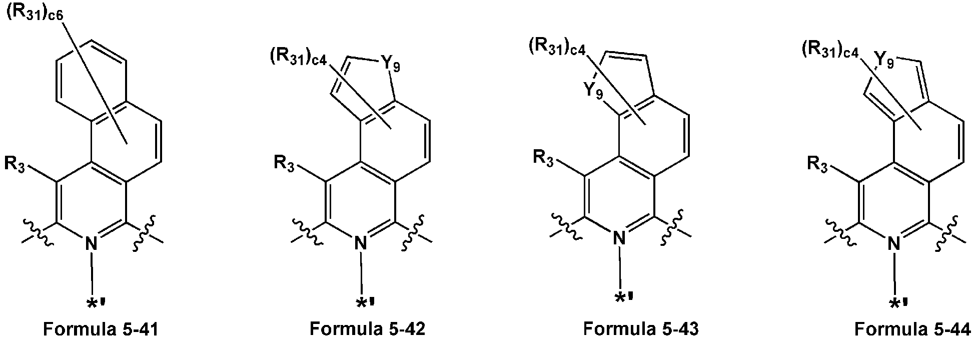

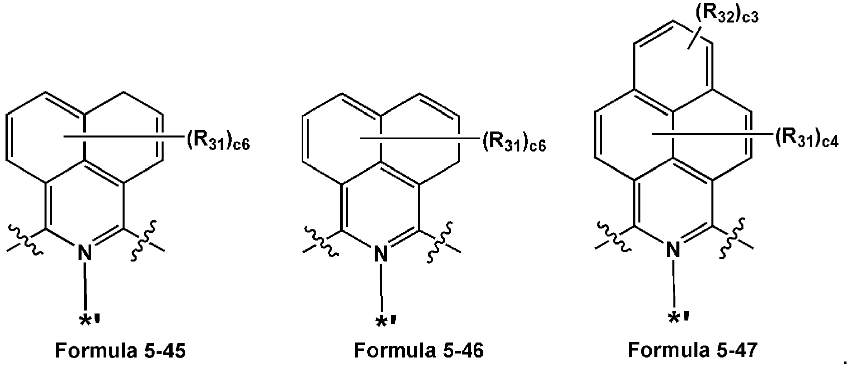

In Formulae 3-1 to 3-25, 3-31 to 3-74, 4-1 to 4-25, 4-31 to 4-74, and 5-1 to 5-47,
Y₇ and Y₈ may each independently be O or S,
Y₉ may be O, S or N(R₃₉),
R₃ to R₅ are the same as described above,
R₁₁ to R₂₀ are each independently the same as described above in connection with R₁,
R₂₁ to R₃₀ are each independently the same as described above in connection with R₂,
R₃₁ to R₃₉ are each independently the same as described above in connection with R₃,
c3 may be an integer selected from 0 to 3,
c4 may be an integer selected from 0 to 4,
c6 may be an integer selected from 0 to 6,
c8 may be an integer selected from 0 to 8,
c10 may be an integer selected from 0 to 10, and
*, *', and *" each indicate a binding site to M in Formula 1.
R₃ to R₅ and R₁₁ to R₃₉ in Formulae 3-1 to 3-25, 3-31 to 3-74, 4-1 to 4-25, 4-31 to 4-74, and 5-1 to 5-47 are the same as described below.
R₁ to R₇ and R₁₁ to R₃₉ may each independently be selected from hydrogen, deuterium, -F, -Cl, -Br, -I, -SF₅, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a substituted or unsubstituted C₁-C₆₀ alkyl group, a substituted or unsubstituted C₂-C₆₀ alkenyl group, a substituted or unsubstituted C₂-C₆₀ alkynyl group, a substituted or unsubstituted C₁-C₆₀ alkoxy group, a substituted or unsubstituted C₃-C₁₀ cycloalkyl group, a substituted or unsubstituted C₁-C₁₀ heterocycloalkyl group, a substituted or unsubstituted C₃-C₁₀ cycloalkenyl group, a substituted or unsubstituted C₁-C₁₀ heterocycloalkenyl group, a substituted or unsubstituted C₆-C₆₀ aryl group, a substituted or unsubstituted C₆-C₆₀ aryloxy group, a substituted or unsubstituted C₆-C₆₀ arylthio group, a substituted or unsubstituted C₁-C₆₀ heteroaryl group, a substituted or unsubstituted monovalent non-aromatic condensed polycyclic group, a substituted or unsubstituted monovalent non-aromatic condensed heteropolycyclic group, -N(Q₁)(Q₂), -Si(Q₃)(Q₄)(Q₅), -B(Q₆)(Q₇), and -P(=O)(Q₈)(Q₉).

In one or more embodiments, R₁ to R₇ and R₁₁ to R₃₉ may each independently be selected from:
hydrogen, deuterium, -F, -Cl, -Br, -I, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, -SF₅, a C₁-C₂₀ alkyl group, and a C₁-C₂₀ alkoxy group;
a C₁-C₂₀ alkyl group and a C₁-C₂₀ alkoxy group, each substituted with at least one selected from deuterium, -F, -Cl, -Br, -I, -CD₃, -CD₂H, -CDH₂, -CF₃, -CF₂H, -CFH₂, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a C₁-C₁₀ alkyl group, a cyclopentyl group, a cyclohexyl group, a cycloheptyl group, a cyclooctyl group, an adamantanyl group, a norbornanyl group, a norbornenyl group, a cyclopentenyl group, a cyclohexenyl group, a cycloheptenyl group, a phenyl group, a naphthyl group, a pyridinyl group, and a pyrimidinyl group;
a cyclopentyl group, a cyclohexyl group, a cycloheptyl group, a cyclooctyl group, an adamantanyl group, a norbornanyl group, a norbornenyl group, a cyclopentenyl group, a cyclohexenyl group, a cycloheptenyl group, a phenyl group, a naphthyl group, a fluorenyl group, a phenanthrenyl group, an anthracenyl group, a fluoranthenyl group, a triphenylenyl group, a pyrenyl group, a chrysenyl group, a pyrrolyl group, a thiophenyl group, a furanyl group, an imidazolyl group, a pyrazolyl group, a thiazolyl group, an isothiazolyl group, an oxazolyl group, an isoxazolyl group, a pyridinyl group, a pyrazinyl group, a pyrimidinyl group, a pyridazinyl group, an isoindolyl group, an indolyl group, an indazolyl group, a purinyl group, a quinolinyl group, an isoquinolinyl group, a benzoquinolinyl group, a quinoxalinyl group, a quinazolinyl group, a cinnolinyl group, a carbazolyl group, a phenanthrolinyl group, a benzimidazolyl group, a benzofuranyl group, a benzothiophenyl group, an isobenzothiazolyl group, a benzoxazolyl group, an isobenzoxazolyl group, a triazolyl group, a tetrazolyl group, an oxadiazolyl group, a triazinyl group, a dibenzofuranyl group, a dibenzothiophenyl group, a benzocarbazolyl group, a dibenzocarbazolyl group, an imidazopyridinyl group, and an imidazopyrimidinyl group;
a cyclopentyl group, a cyclohexyl group, a cycloheptyl group, a cyclooctyl group, an adamantanyl group, a norbornanyl group, a norbornenyl group, a cyclopentenyl group, a cyclohexenyl group, a cycloheptenyl group, a phenyl group, a naphthyl group, a fluorenyl group, a phenanthrenyl group, an anthracenyl group, a fluoranthenyl group, a triphenylenyl group, a pyrenyl group, a chrysenyl group, a pyrrolyl group, a thiophenyl group, a furanyl group, an imidazolyl group, a pyrazolyl group, a thiazolyl group, an isothiazolyl group, an oxazolyl group, an isoxazolyl group, a pyridinyl group, a pyrazinyl group, a pyrimidinyl group, a pyridazinyl group, an isoindolyl group, an indolyl group, an indazolyl group, a purinyl group, a quinolinyl group, an isoquinolinyl group, a benzoquinolinyl group, a quinoxalinyl group, a quinazolinyl group, a cinnolinyl group, a carbazolyl group, a phenanthrolinyl group, a benzimidazolyl group, a benzofuranyl group, a benzothiophenyl group, an isobenzothiazolyl group, a benzoxazolyl group, an isobenzoxazolyl group, a triazolyl group, a tetrazolyl group, an oxadiazolyl group, a triazinyl group, a dibenzofuranyl group, a dibenzothiophenyl group, a benzocarbazolyl group, a dibenzocarbazolyl group, an imidazopyridinyl group, and an imidazopyrimidinyl group, each substituted with at least one selected from deuterium, -F, -Cl, -Br, -I, -CD₃, -CD₂H, -CDH₂, -CF₃, -CF₂H, -CFH₂, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a C₁-C₂₀ alkyl group, a C₁-C₂₀ alkoxy group, a cyclopentyl group, a cyclohexyl group, a cycloheptyl group, a cyclooctyl group, an adamantanyl group, a norbornanyl group, a norbornenyl group, a cyclopentenyl group, a cyclohexenyl group, a cycloheptenyl group, a phenyl group, a naphthyl group, a fluorenyl group, a phenanthrenyl group, an anthracenyl group, a fluoranthenyl group, a triphenylenyl group, a pyrenyl group, a chrysenyl group, a pyrrolyl group, a thiophenyl group, a furanyl group, an imidazolyl group, a pyrazolyl group, a thiazolyl group, an isothiazolyl group, an oxazolyl group, an isoxazolyl group, a pyridinyl group, a pyrazinyl group, a pyrimidinyl group, a pyridazinyl group, an isoindolyl group, an indolyl group, an indazolyl group, a purinyl group, a quinolinyl group, an isoquinolinyl group, a benzoquinolinyl group, a quinoxalinyl group, a quinazolinyl group, a cinnolinyl group, a carbazolyl group, a phenanthrolinyl group, a benzimidazolyl group, a benzofuranyl group, a benzothiophenyl group, an isobenzothiazolyl group, a benzoxazolyl group, an isobenzoxazolyl group, a triazolyl group, a tetrazolyl group, an oxadiazolyl group, a triazinyl group, a dibenzofuranyl group, a dibenzothiophenyl group, a benzocarbazolyl group, a dibenzocarbazolyl group, an imidazopyridinyl group, and an imidazopyrimidinyl group; and
-N(Q₁)(Q₂), -Si(Q₃)(Q₄)(Q₅), -B(Q₆)(Q₇), and -P(=O)(Q₈)(Q₉),
wherein Q₁ to Q₉ may each independently be selected from:
   -CH₃, -CD₃, -CD₂H, -CDH₂, -CH₂CH₃, -CH₂CD₃, -CH₂CD₂H, -CH₂CDH₂, -CHDCH₃, -CHDCD₂H, -CHDCDH₂, -CHDCD₃, -CD₂CD₃, -CD₂CD₂H, and -CD₂CDH₂;
   an n-propyl group, an isopropyl group, an n-butyl group, an isobutyl group, a sec-butyl group, a tert-butyl group, an n-pentyl group, an isopentyl group, a sec-pentyl group, a tert-pentyl group, a phenyl group, and a naphthyl group; and
   an n-propyl group, an iso-propyl group, an n-butyl group, an isobutyl group, a sec-butyl group, a tert-butyl group, an n-pentyl group, an isopentyl group, a sec-pentyl group, a tert-pentyl group, a phenyl group, and a naphthyl group, each substituted with at least one selected from deuterium, a C₁-C₁₀ alkyl group, and a phenyl group.

For example, R₁ to R₇ and R₁₁ to R₃₉ may each independently be selected from:
hydrogen, deuterium, -F, a cyano group, a nitro group, -SF₅, a methyl group, an ethyl group, an n-propyl group, an iso-propyl group, an n-butyl group, an isobutyl group, a sec-butyl group, a tert-butyl group, an n-pentyl group, an isopentyl group, a sec-pentyl group, a tert-pentyl group, an n-hexyl group, an iso-hexyl group, a sec-hexyl group, a tert-hexyl group, an n-heptyl group, an iso-heptyl group, a sec-heptyl group, a tert-heptyl group, an n-octyl group, an iso-octyl group, a sec-octyl group, a tert-octyl group, an n-nonyl group, an iso-nonyl group, a sec-nonyl group, a tert-nonyl group, an n-decyl group, an iso-decyl group, a sec-decyl group, a tert-decyl group, a methoxy group, an ethoxy group, a propoxy group, a butoxy group, a pentoxy group, a cyclopentyl group, a cyclohexyl group, a cycloheptyl group, a cyclooctyl group, an adamantanyl group, a norbornanyl group, a norbornenyl group, a cyclopentenyl group, a cyclohexenyl group, a cycloheptenyl group, a phenyl group, a naphthyl group, a pyridinyl group, a pyrimidinyl group, a dibenzofuranyl group, and a dibenzothiophenyl group;
a methyl group, an ethyl group, an n-propyl group, an iso-propyl group, an n-butyl group, an isobutyl group, a sec-butyl group, a tert-butyl group, an n-pentyl group, an isopentyl group, a sec-pentyl group, a tert-pentyl group, an n-hexyl group, an iso-hexyl group, a sec-hexyl group, a tert-hexyl group, an n-heptyl group, an iso-heptyl group, a sec-heptyl group, a tert-heptyl group, an n-octyl group, an iso-octyl group, a sec-octyl group, a tert-octyl group, an n-nonyl group, an iso-nonyl group, a sec-nonyl group, a tert-nonyl group, an n-decyl group, an iso-decyl group, a sec-decyl group, a tert-decyl group, a methoxy group, an ethoxy group, a propoxy group, a butoxy group, a pentoxy group, a cyclopentyl group, a cyclohexyl group, a cycloheptyl group, a cyclooctyl group, an adamantanyl group, a norbornanyl group, a norbornenyl group, a cyclopentenyl group, a cyclohexenyl group, a cycloheptenyl group, a phenyl group, a naphthyl group, a pyridinyl group, a pyrimidinyl group, a dibenzofuranyl group, and a dibenzothiophenyl group, each substituted with at least one selected from deuterium, -F, -CD₃, -CD₂H, -CDH₂, -CF₃, -CF₂H, -CFH₂, a cyano group, a nitro group, a C₁-C₁₀ alkyl group, a C₁-C₁₀ alkoxy group, a cyclopentyl group, a cyclohexyl group, a cycloheptyl group, a cyclooctyl group, an adamantanyl group, a norbornanyl group, a norbornenyl group, a cyclopentenyl group, a cyclohexenyl group, a cycloheptenyl group, a phenyl group, a naphthyl group, a pyridinyl group, a pyrimidinyl group, a dibenzofuranyl group, and a dibenzothiophenyl group; and
-N(Q₁)(Q₂), -Si(Q₃)(Q₄)(Q₅), -B(Q₆)(Q₇), and -P(=O)(Q₈)(Q₉),
wherein Q₁ to Q₉ may each independently be selected from:
   -CH₃, -CD₃, -CD₂H, -CDH₂, -CH₂CH₃, -CH₂CD₃, -CH₂CD₂H, -CH₂CDH₂, -CHDCH₃, -CHDCD₂H, -CHDCDH₂, -CHDCD₃, -CD₂CD₃, -CD₂CD₂H, and -CD₂CDH₂;
   an n-propyl group, an isopropyl group, an n-butyl group, an isobutyl group, a sec-butyl group, a tert-butyl group, an n-pentyl group, an isopentyl group, a sec-pentyl group, a tert-pentyl group, a phenyl group, and a naphthyl group; and
   an n-propyl group, an iso-propyl group, an n-butyl group, an isobutyl group, a sec-butyl group, a tert-butyl group, an n-pentyl group, an isopentyl group, a sec-pentyl group, a tert-pentyl group, a phenyl group, and a naphthyl group, each substituted with at least one selected from deuterium, a C₁-C₁₀ alkyl group, and a phenyl group.

In one or more embodiments, R₁ to R₇ and R₁₁ to R₃₉ may each independently be selected from hydrogen, deuterium, -F, a cyano group, a nitro group, -SF₅, -CH₃, -CD₃, -CD₂H, -CDH₂, -CF₃, -CF₂H, -CFH₂, a group represented by any of Formulae 9-1 to 9-19, a group represented by any of Formulae 10-1 to 10-38, and -Si(Q₃)(Q₄)(Q₅) (descriptions for Q₃ to Q₅ are the same as described in this disclosure), but embodiments of the present disclosure are not limited thereto: 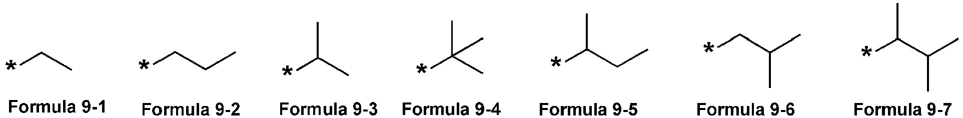 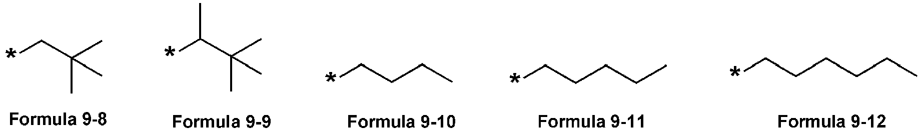 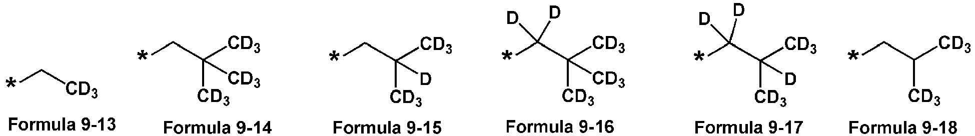 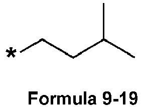 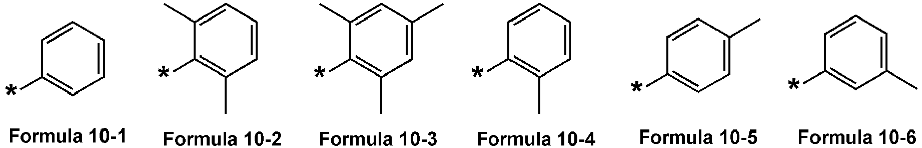 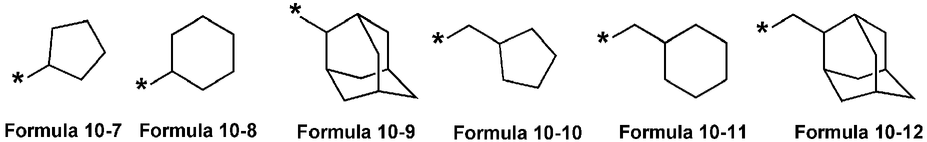 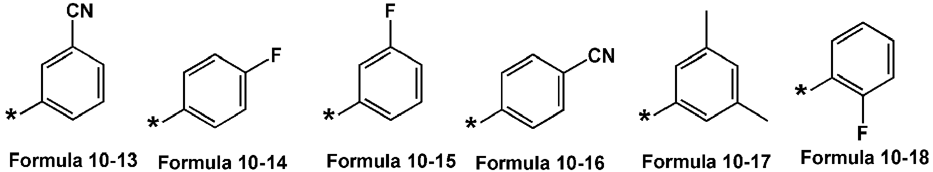 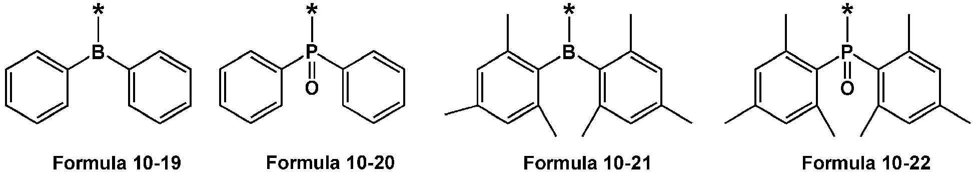 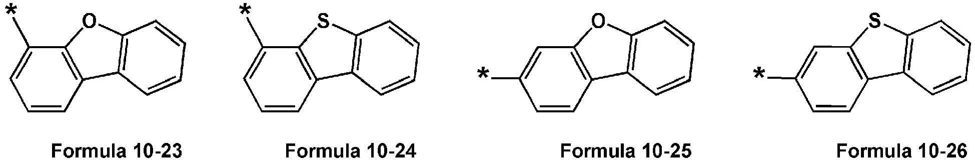 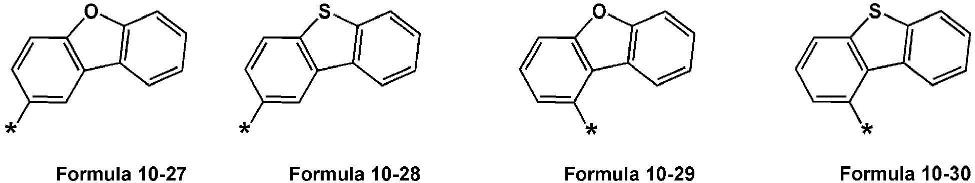 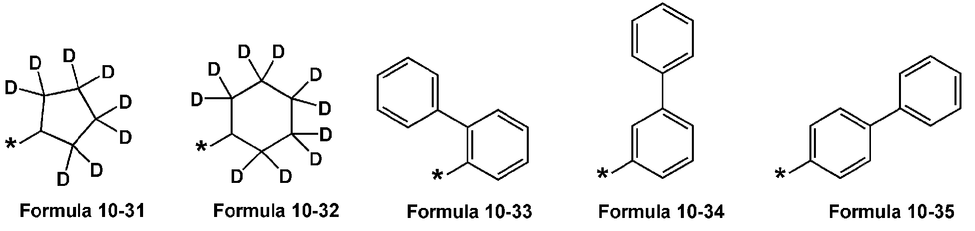 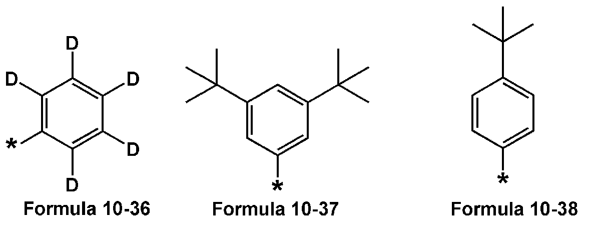 * in Formulae 9-1 to 9-19 and 10-1 to 10-38 indicates a binding site to a neighboring atom.

b1 and b2 in Formula 2 may each independently be an integer selected from 0 to 3, wherein, when b1 is two or more, two or more R₁(s) may be identical to or different from each other, and when b2 is two or more, two or more R₂(s) may be identical to or different from each other.

Two selected from R₁(s) in the number of b1 in Formula 2 may be optionally connected to each other to form a substituted or unsubstituted C₅-C₃₀ carbocyclic group or a substituted or unsubstituted C₂-C₃₀ heterocyclic group (for example, a substituted or unsubstituted cyclopentane group, a substituted or unsubstituted cyclohexane group, a substituted or unsubstituted adamantane group, a substituted or unsubstituted bicyclo[2.2.1]heptane group, a substituted or unsubstituted benzene group, a substituted or unsubstituted pyridine group, a substituted or unsubstituted pyrimidine group, a substituted or unsubstituted pyrazine group, a substituted or unsubstituted pyridazine group, a substituted or unsubstituted naphthalene group, and the like). Descriptions for a susbtituent of the substituted C₅-C₃₀ carbocyclic group and the substituted C₂-C₃₀ heterocyclic group are same as descriptions for R₁ in this disclosure.

Two selected from R₂(s) in the number of b2 in Formula 2 may be optionally connected to each other to form a substituted or unsubstituted C₅-C₃₀ carbocyclic group or a substituted or unsubstituted C₂-C₃₀ heterocyclic group (for example, a substituted or unsubstituted cyclopentane group, a substituted or unsubstituted cyclohexane group, a substituted or unsubstituted adamantane group, a substituted or unsubstituted bicyclo[2.2.1]heptane group, a substituted or unsubstituted benzene group, a substituted or unsubstituted pyridine group, a substituted or unsubstituted pyrimidine group, a substituted or unsubstituted pyrazine group, a substituted or unsubstituted pyridazine group, a substituted or unsubstituted naphthalene, and the like). Descriptions for a susbtituent of the substituted C₅-C₃₀ carbocyclic group and the substituted C₂-C₃₀ heterocyclic group are same as descriptions for R₁ in this disclosure.

In one or more embodiments, a moiety represented by 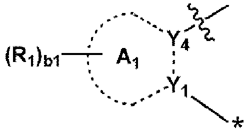 in Formula 2 may be represented by one selected from Formulae 3-5 to 3-8, and 3-47 to 3-62, and
a moiety represented by 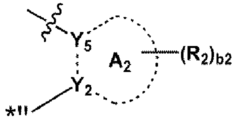 in Formula 2 may be represented by one selected from Formulae 4-5 to 4-8, and 4-47 to 4-62.

In one or more embodiments, a moiety represented by 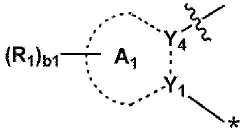 in Formula 2 may be represented by one selected from Formulae 3-1, 3-5, 3-9, 3-13, 3-31 to 3-34, and 3-47 to 3-50, and
a moiety represented by 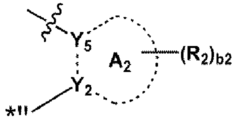 in Formula 2 may be represented by one selected from Formulae 4-1, 4-5, 4-9, 4-13, 4-31 to 4-34, and 4-47 to 4-50.

In one or more embodiments, a moiety represented by 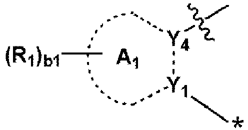 in Formula 2 may be represented by one selected from Formulae 3-1, 3-5, 3-9, 3-13, 3-31 to 3-34, and 3-47 to 3-50, and
a moiety represented by 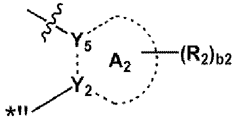 in Formula 2 may be represented by one selected from Formulae 4-2 to 4-4, 4-6 to 4-8, 4-10 to 4-12, 4-14 to 4-16, 4-35 to 4-46, and 4-51 to 4-70.

In one or more embodiments, a moiety represented by 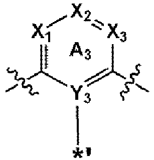 in Formula 2 may be represented by one selected from Formulae 5-1 to 5-28, 5-29, and 5-45.

In one or more embodiments,
a moiety represented by 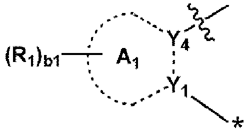 in Formula 2 may be represented by one selected from Formulae 3-5 and 3-47 to 3-50,
a moiety represented by 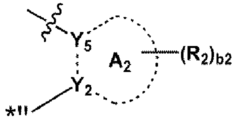 in Formula 2 may be represented by one selected from Formulae 4-1, 4-5, 4-31 to 4-34, and 4-47 to 4-50, and
a moiety represented by 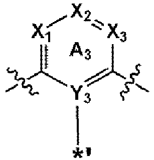 in Formula 2 may be represented by one selected from Formulae 5-1, 5-3 to 5-5, 5-7, 5-8, 5-29, and 5-45, but embodiments are not limited thereto.

For example, L₁ in Formula 1 may be selected from ligands represented by Formulae 2A-1 to 2E-1 and 2A-2 to 2E-2, but embodiments of the present disclosure are not limited thereto:

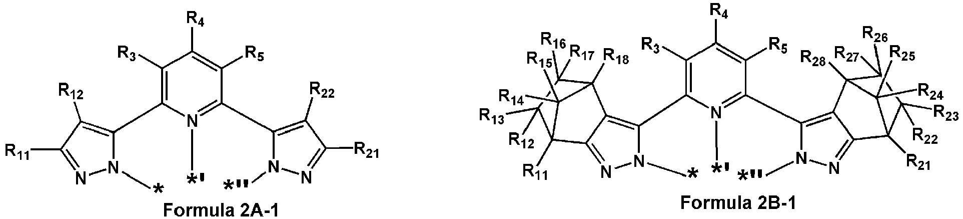

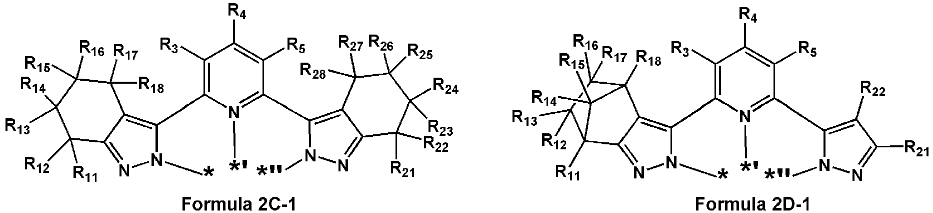

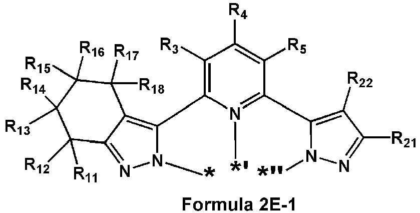

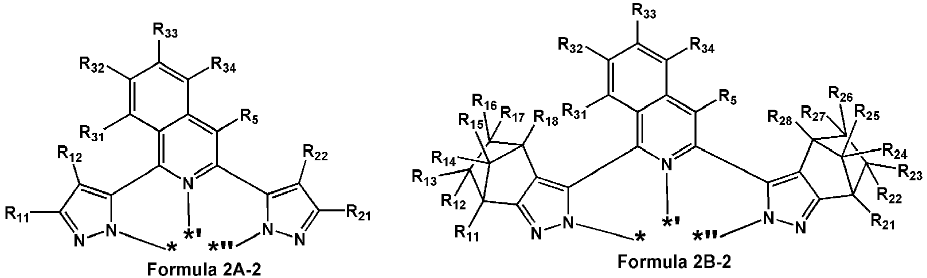

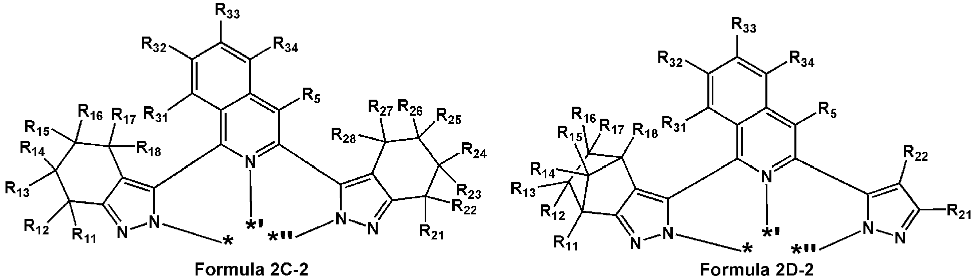

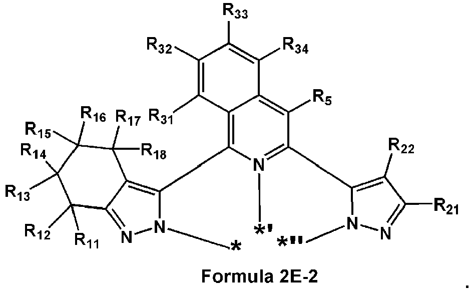

In Formulae 2A-1 to 2E-1 and 2A-2 to 2E-2, R₃ to R₅, R₁₁ to R₁₈, R₂₁ to R₂₈, and R₃₁ to R₃₄ are the same as described above, and *, *', and *" each indicate a binding site to M in Formula 1.

For example, R₃ to R₅, R₁₁ to R₁₈, R₂₁ to R₂₈, and R₃₁ to R₃₄ in Formulae 2A-1 to 2E-1 and 2A-2 to 2E-2 may each independently be selected from hydrogen, deuterium, -F, a cyano group, a nitro group, -SF₅, -CH₃, -CD₃, -CD₂H, -CDH₂, -CF₃, -CF₂H, -CFH₂, a group represented by any of Formulae 9-1 to 9-19, a group represented by any of Formulae 10-1 to 10-38, and -Si(Q₃)(Q₄)(Q₅) (descriptions for Q₃ to Q₅ are the same as described in this disclosure), but embodiments of the present disclosure are not limited thereto.

L₂ in Formula 1 may be selected from ligands represented by Formula 6-1:

*(̵T₃)ₐ₃-R₆₁ **Formula 6-1.**

In Formula 6-1,
T₃ may be selected from a single bond, *-O-*', *-S-*', *-C(R₆₂)(R₆₃)-*', *-C(R₆₂)=*', *=C(R₆₂)-*', *-C(R₆₂)=C(R₆₃)-*', *-C(=O)-*', *-C(=S)-*', *-C≡C-*', and *-N(R₆₂)-*',
a3 may be an integer selected from 1 to 5,
R₆₁ may each independently be selected from hydrogen, deuterium, -F, -CI, -Br, -I, -SF₅, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a substituted or unsubstituted C₁-C₆₀ alkyl group, a substituted or unsubstituted C₂-C₆₀ alkenyl group, a substituted or unsubstituted C₂-C₆₀ alkynyl group, a substituted or unsubstituted C₁-C₆₀ alkoxy group, a substituted or unsubstituted C₃-C₁₀ cycloalkyl group, a substituted or unsubstituted C₁-C₁₀ heterocycloalkyl group, a substituted or unsubstituted C₃-C₁₀ cycloalkenyl group, a substituted or unsubstituted C₁-C₁₀ heterocycloalkenyl group, a substituted or unsubstituted C₆-C₆₀ aryl group, a substituted or unsubstituted C₆-C₆₀ aryloxy group, a substituted or unsubstituted C₆-C₆₀ arylthio group, a substituted or unsubstituted C₁-C₆₀ heteroaryl group, a substituted or unsubstituted monovalent non-aromatic condensed polycyclic group, a substituted or unsubstituted monovalent non-aromatic condensed heteropolycyclic group, -N(Q₁)(Q₂), -Si(Q₃)(Q₄)(Q₅), -B(Q₆)(Q₇), -P(=O)(Q₈)(Q₉), and -P(Q₄₁)(Q₄₂)(Q₄₃),
Q₁ to Q₉ and Q₄₁ to Q₄₃ may each independently be selected from:
-CH₃, -CD₃, -CD₂H, -CDH₂, -CH₂CH₃, -CH₂CD₃, -CH₂CD₂H, -CH₂CDH₂, -CHDCH₃, -CHDCD₂H, -CHDCDH₂, -CHDCD₃, -CD₂CD₃, -CD₂CD₂H, and -CD₂CDH₂;
an n-propyl group, an isopropyl group, an n-butyl group, an isobutyl group, a sec-butyl group, a tert-butyl group, an n-pentyl group, an isopentyl group, a sec-pentyl group, a tert-pentyl group, a phenyl group, and a naphthyl group; and
an n-propyl group, an iso-propyl group, an n-butyl group, an isobutyl group, a sec-butyl group, a tert-butyl group, an n-pentyl group, an isopentyl group, a sec-pentyl group, a tert-pentyl group, a phenyl group, and a naphthyl group, each substituted with at least one selected from deuterium, a C₁-C₁₀ alkyl group, and a phenyl group,
R₆₂ and R₆₃ may each independently be selected from:
hydrogen, deuterium, -F, a cyano group, a nitro group, -SF₅, a methyl group, an ethyl group, an n-propyl group, an iso-propyl group, an n-butyl group, an isobutyl group, a sec-butyl group, a tert-butyl group, an n-pentyl group, an isopentyl group, a sec-pentyl group, a tert-pentyl group, an n-hexyl group, an iso-hexyl group, a sec-hexyl group, a tert-hexyl group, an n-heptyl group, an iso-heptyl group, a sec-heptyl group, a tert-heptyl group, an n-octyl group, an iso-octyl group, a sec-octyl group, a tert-octyl group, an n-nonyl group, an iso-nonyl group, a sec-nonyl group, a tert-nonyl group, an n-decyl group, an iso-decyl group, a sec-decyl group, a tert-decyl group, a methoxy group, an ethoxy group, a propoxy group, a butoxy group, a pentoxy group, a cyclopentyl group, a cyclohexyl group, a cycloheptyl group, a cyclooctyl group, an adamantanyl group, a norbornanyl group, a norbornenyl group, a cyclopentenyl group, a cyclohexenyl group, a cycloheptenyl group, a phenyl group, a naphthyl group, a pyridinyl group, a pyrimidinyl group, a dibenzofuranyl group, and a dibenzothiophenyl group; and
a methyl group, an ethyl group, an n-propyl group, an iso-propyl group, an n-butyl group, an isobutyl group, a sec-butyl group, a tert-butyl group, an n-pentyl group, an isopentyl group, a sec-pentyl group, a tert-pentyl group, an n-hexyl group, an iso-hexyl group, a sec-hexyl group, a tert-hexyl group, an n-heptyl group, an iso-heptyl group, a sec-heptyl group, a tert-heptyl group, an n-octyl group, an iso-octyl group, a sec-octyl group, a tert-octyl group, an n-nonyl group, an iso-nonyl group, a sec-nonyl group, a tert-nonyl group, an n-decyl group, an iso-decyl group, a sec-decyl group, a tert-decyl group, a methoxy group, an ethoxy group, a propoxy group, a butoxy group, a pentoxy group, a cyclopentyl group, a cyclohexyl group, a cycloheptyl group, a cyclooctyl group, an adamantanyl group, a norbornanyl group, a norbornenyl group, a cyclopentenyl group, a cyclohexenyl group, a cycloheptenyl group, a phenyl group, a naphthyl group, a pyridinyl group, a pyrimidinyl group, a dibenzofuranyl group, and a dibenzothiophenyl group, each substituted with at least one selected from deuterium, -F, -CD₃, -CD₂H, -CDH₂, -CF₃, -CF₂H, -CFH₂, a cyano group, a nitro group, a C₁-C₁₀ alkyl group, a C₁-C₁₀ alkoxy group, a cyclopentyl group, a cyclohexyl group, a cycloheptyl group, a cyclooctyl group, an adamantanyl group, a norbornanyl group, a norbornenyl group, a cyclopentenyl group, a cyclohexenyl group, a cycloheptenyl group, a phenyl group, a naphthyl group, a pyridinyl group, a pyrimidinyl group, a dibenzofuranyl group, and a dibenzothiophenyl group,
R₆₂ and R₆₃ may be optionally connected to each other to form a substituted or unsubstituted C₅-C₃₀ carbocyclic group or a substituted or unsubstituted C₂-C₃₀ heterocyclic group, and
* indicates a binding site to M in Formula 1.

For example, R₆₁ may be selected from:
hydrogen, deuterium, -F, -CI, -Br, -I, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, -SF₅, a C₁-C₂₀ alkyl group, and a C₁-C₂₀ alkoxy group;
a C₁-C₂₀ alkyl group and a C₁-C₂₀ alkoxy group, each substituted with at least one selected from deuterium, -F, -CI, -Br, -I, -CD₃, -CD₂H, -CDH₂, -CF₃, -CF₂H, -CFH₂, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a c1-C₁₀ alkyl group, a cyclopentyl group, a cyclohexyl group, a cycloheptyl group, a cyclooctyl group, an adamantanyl group, a norbornanyl group, a norbornenyl group, a cyclopentenyl group, a cyclohexenyl group, a cycloheptenyl group, a phenyl group, a naphthyl group, a pyridinyl group, and a pyrimidinyl group;
a cyclopentyl group, a cyclohexyl group, a cycloheptyl group, a cyclooctyl group, an adamantanyl group, a norbornanyl group, a norbornenyl group, a cyclopentenyl group, a cyclohexenyl group, a cycloheptenyl group, a phenyl group, a naphthyl group, a fluorenyl group, a phenanthrenyl group, an anthracenyl group, a fluoranthenyl group, a triphenylenyl group, a pyrenyl group, a chrysenyl group, a pyrrolyl group, a thiophenyl group, a furanyl group, an imidazolyl group, a pyrazolyl group, a thiazolyl group, an isothiazolyl group, an oxazolyl group, an isoxazolyl group, a pyridinyl group, a pyrazinyl group, a pyrimidinyl group, a pyridazinyl group, an isoindolyl group, an indolyl group, an indazolyl group, a purinyl group, a quinolinyl group, an isoquinolinyl group, a benzoquinolinyl group, a quinoxalinyl group, a quinazolinyl group, a cinnolinyl group, a carbazolyl group, a phenanthrolinyl group, a benzimidazolyl group, a benzofuranyl group, a benzothiophenyl group, an isobenzothiazolyl group, a benzoxazolyl group, an isobenzoxazolyl group, a triazolyl group, a tetrazolyl group, an oxadiazolyl group, a triazinyl group, a dibenzofuranyl group, a dibenzothiophenyl group, a benzocarbazolyl group, a dibenzocarbazolyl group, an imidazopyridinyl group, and an imidazopyrimidinyl group;
a cyclopentyl group, a cyclohexyl group, a cycloheptyl group, a cyclooctyl group, an adamantanyl group, a norbornanyl group, a norbornenyl group, a cyclopentenyl group, a cyclohexenyl group, a cycloheptenyl group, a phenyl group, a naphthyl group, a fluorenyl group, a phenanthrenyl group, an anthracenyl group, a fluoranthenyl group, a triphenylenyl group, a pyrenyl group, a chrysenyl group, a pyrrolyl group, a thiophenyl group, a furanyl group, an imidazolyl group, a pyrazolyl group, a thiazolyl group, an isothiazolyl group, an oxazolyl group, an isoxazolyl group, a pyridinyl group, a pyrazinyl group, a pyrimidinyl group, a pyridazinyl group, an isoindolyl group, an indolyl group, an indazolyl group, a purinyl group, a quinolinyl group, an isoquinolinyl group, a benzoquinolinyl group, a quinoxalinyl group, a quinazolinyl group, a cinnolinyl group, a carbazolyl group, a phenanthrolinyl group, a benzimidazolyl group, a benzofuranyl group, a benzothiophenyl group, an isobenzothiazolyl group, a benzoxazolyl group, an isobenzoxazolyl group, a triazolyl group, a tetrazolyl group, an oxadiazolyl group, a triazinyl group, a dibenzofuranyl group, a dibenzothiophenyl group, a benzocarbazolyl group, a dibenzocarbazolyl group, an imidazopyridinyl group, and an imidazopyrimidinyl group, each substituted with at least one selected from deuterium, -F, -CI, -Br, -I, -CD₃, -CD₂H, -CDH₂, -CF₃, -CF₂H, -CFH₂, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a C₁-C₂₀ alkyl group, a C₁-C₂₀ alkoxy group, a cyclopentyl group, a cyclohexyl group, a cycloheptyl group, a cyclooctyl group, an adamantanyl group, a norbornanyl group, a norbornenyl group, a cyclopentenyl group, a cyclohexenyl group, a cycloheptenyl group, a phenyl group, a naphthyl group, a fluorenyl group, a phenanthrenyl group, an anthracenyl group, a fluoranthenyl group, a triphenylenyl group, a pyrenyl group, a chrysenyl group, a pyrrolyl group, a thiophenyl group, a furanyl group, an imidazolyl group, a pyrazolyl group, a thiazolyl group, an isothiazolyl group, an oxazolyl group, an isoxazolyl group, a pyridinyl group, a pyrazinyl group, a pyrimidinyl group, a pyridazinyl group, an isoindolyl group, an indolyl group, an indazolyl group, a purinyl group, a quinolinyl group, an isoquinolinyl group, a benzoquinolinyl group, a quinoxalinyl group, a quinazolinyl group, a cinnolinyl group, a carbazolyl group, a phenanthrolinyl group, a benzimidazolyl group, a benzofuranyl group, a benzothiophenyl group, an isobenzothiazolyl group, a benzoxazolyl group, an isobenzoxazolyl group, a triazolyl group, a tetrazolyl group, an oxadiazolyl group, a triazinyl group, a dibenzofuranyl group, a dibenzothiophenyl group, a benzocarbazolyl group, a dibenzocarbazolyl group, an imidazopyridinyl group, and an imidazopyrimidinyl group; and
-N(Q₁)(Q₂), -Si(Q₃)(Q₄)(Q₅), -B(Q₆)(Q₇), -P(=O)(Q₈)(Q₉), and P(Q₄₁)(Q₄₂)(Q₄₃),
wherein Q₁ to Q₉ and Q₄₁ to Q₄₃ may each independently be selected from:
   -CH₃, -CD₃, -CD₂H, -CDH₂, -CH₂CH₃, -CH₂CD₃, -CH₂CD₂H, -CH₂CDH₂, -CHDCH₃, -CHDCD₂H, -CHDCDH₂, -CHDCD₃, -CD₂CD₃, -CD₂CD₂H, and -CD₂CDH₂;
   an n-propyl group, an isopropyl group, an n-butyl group, an isobutyl group, a sec-butyl group, a tert-butyl group, an n-pentyl group, an isopentyl group, a sec-pentyl group, a tert-pentyl group, a phenyl group, and a naphthyl group; and
   an n-propyl group, an iso-propyl group, an n-butyl group, an isobutyl group, a sec-butyl group, a tert-butyl group, an n-pentyl group, an isopentyl group, a sec-pentyl group, a tert-pentyl group, a phenyl group, and a naphthyl group, each substituted with at least one selected from deuterium, a C₁-C₁₀ alkyl group, and a phenyl group,
but embodiments are not limited thereto.

In one or more embodiments, L₂ in Formula 1 may be selected from ligands represented by Formulae 12-1 to 12-5, but embodiments of the present disclosure are not limited thereto: 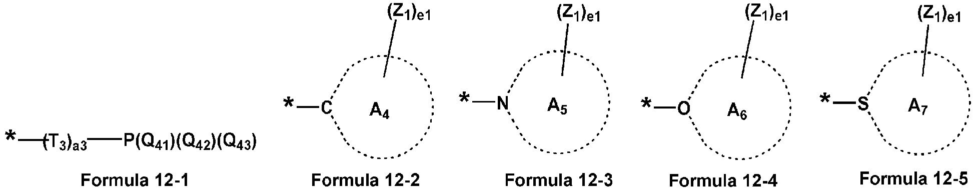 wherein, in Formulae 12-1 to 12-5,
T₃ may be selected from a single bond, *-O-*', *-S-*', *-C(R₆₂)(R₆₃)-*', *-C(R₆₂)=*', *=C(R₆₂)-*', *-C(R₆₂)=C(R₆₃)-*', *-C(=O)-*', *-C(=S)-*', *-C≡C-*', and *-N(R₆₂)-*',
R₆₂ and R₆₃ may each independently be selected from hydrogen, deuterium, -F, -CI, -Br, -I, -SF₅, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a C₁-C₂₀ alkyl group, a C₁-C₂₀ alkoxy group, a phenyl group, and a naphthyl group,
a3 may be an integer selected from 1 to 5,
ring A₄ is selected from a cyclopentene ring, a cyclohexene ring, cycloheptene ring, a benzene ring, an indene ring, a naphthalene ring, an azulene ring, a heptalene ring, an indacene ring, an acenaphthylene ring, a fluorene ring, a spiro-bifluorene ring, a benzofluorene ring, a dibenzofluorene ring, a phenalene ring, a phenanthrene ring, an anthracene ring, a fluoranthene ring, a triphenylene ring, a pyrene ring, a chrysene ring, a naphthacene ring, a picene ring, a perylene ring, a pentacene ring, a hexacene ring, a rubicene ring, a coronene ring, an ovalene ring, a pyrrole ring, a thiophene ring, a furan ring, an imidazole ring, a pyrazole ring, a thiazole ring, an isothiazole ring, an oxazole ring, an isoxazole ring, a pyridine ring, a pyrazine ring, a pyrimidine ring, a pyridazine ring, an iso-indole ring, an indole ring, an indazole ring, a purine ring, a quinoline ring, an isoquinoline ring, a benzoquinoline ring, a quinoxaline ring, a quinazoline ring, a cinnoline ring, a naphthyridine ring, a carbazole ring, a phenanthroline ring, a benzimidazole ring, a benzofuran ring, a benzothiophene ring, a benzothiazole ring, an iso-benzothiazole ring, a benzoxazole ring, an isobenzoxazole ring, a triazole ring, a tetrazole ring, an oxadiazole ring, a thiadiazol ring, a triazine ring, a dibenzofuran ring, a dibenzothiophene ring, a benzocarbazole ring, a dibenzocarbazole ring, an imidazopyridine ring, and an imidazopyrimidine ring,
ring A₅ is selected from a pyrrole ring, an imidazole ring, a pyrazole ring, a thiazole ring, an isothiazole ring, an oxazole ring, an isoxazole ring, a pyridine ring, a pyrazine ring, a pyrimidine ring, a pyridazine ring, an iso-indole ring, an indole ring, an indazole ring, a purine ring, a quinoline ring, an isoquinoline ring, a benzoquinoline ring, a quinoxaline ring, a quinazoline ring, a cinnoline ring, a naphthyridine ring, a carbazole ring, a phenanthroline ring, a benzimidazole ring, a benzofuran ring, a benzothiazole ring, an iso-benzothiazole ring, a benzoxazole ring, an isobenzoxazole ring, a triazole ring, a tetrazole ring, an oxadiazole ring, a thiadiazol ring, a triazine ring, a benzocarbazole ring, a dibenzocarbazole ring, an imidazopyridine ring, and an imidazopyrimidine ring,
ring A₆ is selected from a furan ring, an oxazole ring, an isoxazole ring, a benzofuran ring, a benzoxazole ring, an isobenzoxazole ring, an oxadiazole ring, and a dibenzofuran ring,
ring A₇ is selected from a thiophene ring, a thiazole ring, an isothiazole ring, a benzothiophene ring, a benzothiazole ring, an iso-benzothiazole ring, a thiadiazol ring, and a dibenzothiophene ring,
Z₁ may each independently be selected from:
   hydrogen, deuterium, -F, a cyano group, a nitro group, -SF₅, a methyl group, an ethyl group, an n-propyl group, an iso-propyl group, an n-butyl group, an isobutyl group, a sec-butyl group, a tert-butyl group, an n-pentyl group, an isopentyl group, a sec-pentyl group, a tert-pentyl group, an n-hexyl group, an iso-hexyl group, a sec-hexyl group, a tert-hexyl group, an n-heptyl group, an iso-heptyl group, a sec-heptyl group, a tert-heptyl group, an n-octyl group, an iso-octyl group, a sec-octyl group, a tert-octyl group, an n-nonyl group, an iso-nonyl group, a sec-nonyl group, a tert-nonyl group, an n-decyl group, an iso-decyl group, a sec-decyl group, a tert-decyl group, a methoxy group, an ethoxy group, a propoxy group, a butoxy group, a pentoxy group, a cyclopentyl group, a cyclohexyl group, a cycloheptyl group, a cyclooctyl group, an adamantanyl group, a norbornanyl group, a norbornenyl group, a cyclopentenyl group, a cyclohexenyl group, a cycloheptenyl group, a phenyl group, a naphthyl group, a pyridinyl group, a pyrimidinyl group, a dibenzofuranyl group, and a dibenzothiophenyl group;
   a methyl group, an ethyl group, an n-propyl group, an iso-propyl group, an n-butyl group, an isobutyl group, a sec-butyl group, a tert-butyl group, an n-pentyl group, an isopentyl group, a sec-pentyl group, a tert-pentyl group, an n-hexyl group, an iso-hexyl group, a sec-hexyl group, a tert-hexyl group, an n-heptyl group, an iso-heptyl group, a sec-heptyl group, a tert-heptyl group, an n-octyl group, an iso-octyl group, a sec-octyl group, a tert-octyl group, an n-nonyl group, an iso-nonyl group, a sec-nonyl group, a tert-nonyl group, an n-decyl group, an iso-decyl group, a sec-decyl group, a tert-decyl group, a methoxy group, an ethoxy group, a propoxy group, a butoxy group, a pentoxy group, a cyclopentyl group, a cyclohexyl group, a cycloheptyl group, a cyclooctyl group, an adamantanyl group, a norbornanyl group, a norbornenyl group, a cyclopentenyl group, a cyclohexenyl group, a cycloheptenyl group, a phenyl group, a naphthyl group, a pyridinyl group, a pyrimidinyl group, a dibenzofuranyl group, and a dibenzothiophenyl group, each substituted with at least one selected from deuterium, -F, -CD₃, -CD₂H, -CDH₂, -CF₃, -CF₂H, -CFH₂, a cyano group, a nitro group, a C₁-C₁₀ alkyl group, a C₁-C₁₀ alkoxy group, a cyclopentyl group, a cyclohexyl group, a cycloheptyl group, a cyclooctyl group, an adamantanyl group, a norbornanyl group, a norbornenyl group, a cyclopentenyl group, a cyclohexenyl group, a cycloheptenyl group, a phenyl group, a naphthyl group, a pyridinyl group, a pyrimidinyl group, a dibenzofuranyl group, and a dibenzothiophenyl group; and
   -N(Q₃₁)(Q₃₂), -Si(Q₃₃)(Q₃₄)(Q₃₅), -B(Q₃₆)(Q₃₇), and -P(=O)(Q₃₈)(Q₃₉),
wherein Q₃₁ to Q₃₉ may each independently be selected from:
   -CH₃, -CD₃, -CD₂H, -CDH₂, -CH₂CH₃, -CH₂CD₃, -CH₂CD₂H, -CH₂CDH₂, -CHDCH₃, -CHDCD₂H, -CHDCDH₂, -CHDCD₃, -CD₂CD₃, -CD₂CD₂H, and -CD₂CDH₂;
   an n-propyl group, an isopropyl group, an n-butyl group, an isobutyl group, a sec-butyl group, a tert-butyl group, an n-pentyl group, an isopentyl group, a sec-pentyl group, a tert-pentyl group, a phenyl group, and a naphthyl group; and
   an n-propyl group, an iso-propyl group, an n-butyl group, an isobutyl group, a sec-butyl group, a tert-butyl group, an n-pentyl group, an isopentyl group, a sec-pentyl group, a tert-pentyl group, a phenyl group, and a naphthyl group, each substituted with at least one selected from deuterium, a C₁-C₁₀ alkyl group, and a phenyl group,
neighboring two or more selected from a plurality of Z₁(s) may be optionally connected to form a substituted or unsubstituted C₅-C₃₀ carbocyclic group or a substituted or unsubstituted C₂-C₃₀ heterocyclic group,
e1 may be an integer selected from 0 to 8, and
* indicates a binding site to M in Formula 1.

For example, L₂ in Formula 1 may be selected from ligands represented by Formulae 13-1 to 13-47 and 14-1 to 14-28, but embodiments of the present disclosure are not limited thereto:

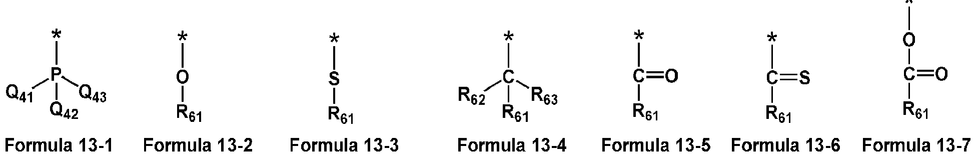

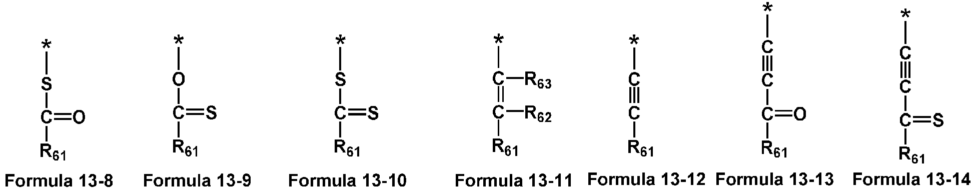

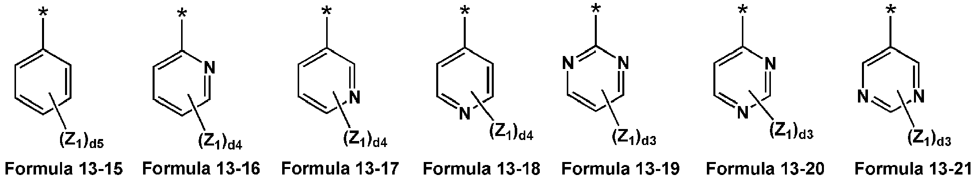

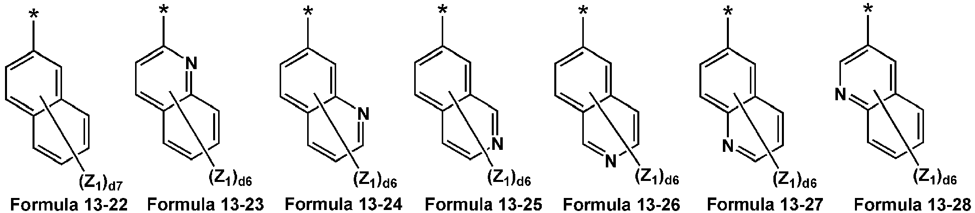

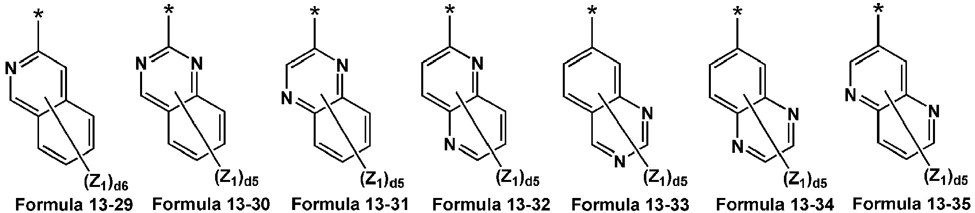

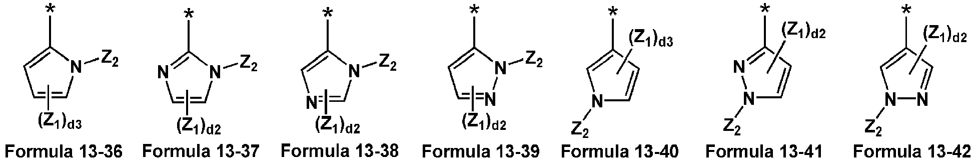

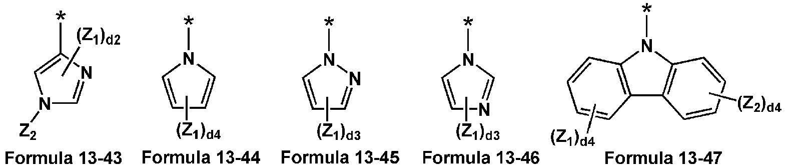

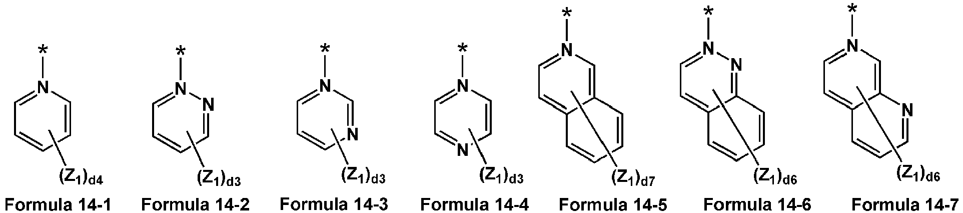

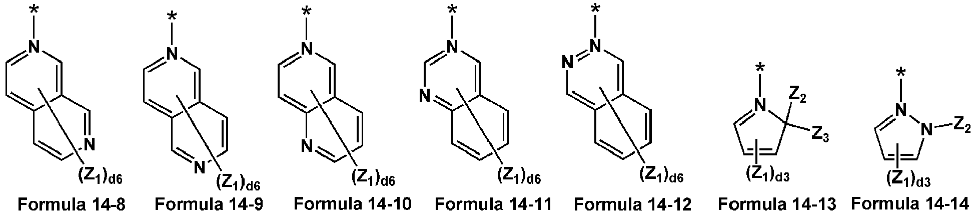

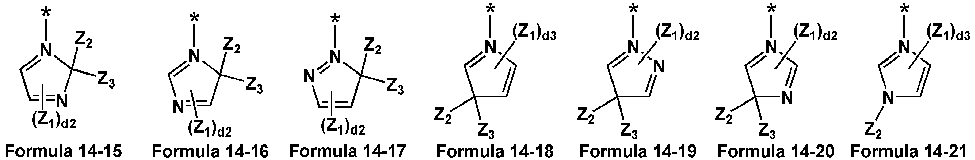

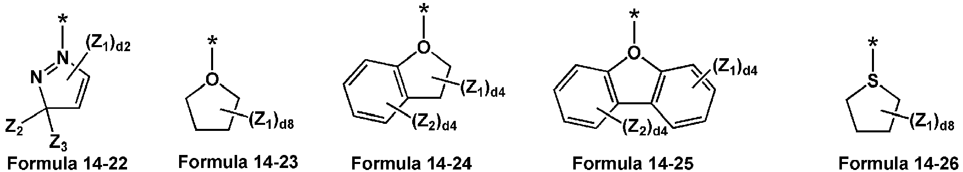

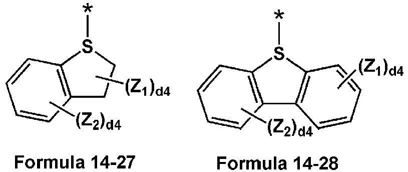

In Formulae 13-1 to 13-47 and 14-1 to 14-28,
R₆₁ may be selected from:
hydrogen, deuterium, -F, -Cl, -Br, -I, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, -SF₅, a C₁-C₂₀ alkyl group, and a C₁-C₂₀ alkoxy group;
a C₁-C₂₀ alkyl group and a C₁-C₂₀ alkoxy group, each substituted with at least one selected from deuterium, -F, -Cl, -Br, -I, -CD₃, -CD₂H, -CDH₂, -CF₃, -CF₂H, -CFH₂, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a C₁-C₁₀ alkyl group, a cyclopentyl group, a cyclohexyl group, a cycloheptyl group, a cyclooctyl group, an adamantanyl group, a norbornanyl group, a norbornenyl group, a cyclopentenyl group, a cyclohexenyl group, a cycloheptenyl group, a phenyl group, a naphthyl group, a pyridinyl group, and a pyrimidinyl group;
a cyclopentyl group, a cyclohexyl group, a cycloheptyl group, a cyclooctyl group, an adamantanyl group, a norbornanyl group, a norbornenyl group, a cyclopentenyl group, a cyclohexenyl group, a cycloheptenyl group, a phenyl group, a naphthyl group, a fluorenyl group, a phenanthrenyl group, an anthracenyl group, a fluoranthenyl group, a triphenylenyl group, a pyrenyl group, a chrysenyl group, a pyrrolyl group, a thiophenyl group, a furanyl group, an imidazolyl group, a pyrazolyl group, a thiazolyl group, an isothiazolyl group, an oxazolyl group, an isoxazolyl group, a pyridinyl group, a pyrazinyl group, a pyrimidinyl group, a pyridazinyl group, an isoindolyl group, an indolyl group, an indazolyl group, a purinyl group, a quinolinyl group, an isoquinolinyl group, a benzoquinolinyl group, a quinoxalinyl group, a quinazolinyl group, a cinnolinyl group, a carbazolyl group, a phenanthrolinyl group, a benzimidazolyl group, a benzofuranyl group, a benzothiophenyl group, an isobenzothiazolyl group, a benzoxazolyl group, an isobenzoxazolyl group, a triazolyl group, a tetrazolyl group, an oxadiazolyl group, a triazinyl group, a dibenzofuranyl group, a dibenzothiophenyl group, a benzocarbazolyl group, a dibenzocarbazolyl group, an imidazopyridinyl group, and an imidazopyrimidinyl group;
a cyclopentyl group, a cyclohexyl group, a cycloheptyl group, a cyclooctyl group, an adamantanyl group, a norbornanyl group, a norbornenyl group, a cyclopentenyl group, a cyclohexenyl group, a cycloheptenyl group, a phenyl group, a naphthyl group, a fluorenyl group, a phenanthrenyl group, an anthracenyl group, a fluoranthenyl group, a triphenylenyl group, a pyrenyl group, a chrysenyl group, a pyrrolyl group, a thiophenyl group, a furanyl group, an imidazolyl group, a pyrazolyl group, a thiazolyl group, an isothiazolyl group, an oxazolyl group, an isoxazolyl group, a pyridinyl group, a pyrazinyl group, a pyrimidinyl group, a pyridazinyl group, an isoindolyl group, an indolyl group, an indazolyl group, a purinyl group, a quinolinyl group, an isoquinolinyl group, a benzoquinolinyl group, a quinoxalinyl group, a quinazolinyl group, a cinnolinyl group, a carbazolyl group, a phenanthrolinyl group, a benzimidazolyl group, a benzofuranyl group, a benzothiophenyl group, an isobenzothiazolyl group, a benzoxazolyl group, an isobenzoxazolyl group, a triazolyl group, a tetrazolyl group, an oxadiazolyl group, a triazinyl group, a dibenzofuranyl group, a dibenzothiophenyl group, a benzocarbazolyl group, a dibenzocarbazolyl group, an imidazopyridinyl group, and an imidazopyrimidinyl group, each substituted with at least one selected from deuterium, -F, -Cl, -Br, -I, -CD₃, -CD₂H, -CDH₂, -CF₃, -CF₂H, -CFH₂, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a C₁-C₂₀ alkyl group, a C₁-C₂₀ alkoxy group, a cyclopentyl group, a cyclohexyl group, a cycloheptyl group, a cyclooctyl group, an adamantanyl group, a norbornanyl group, a norbornenyl group, a cyclopentenyl group, a cyclohexenyl group, a cycloheptenyl group, a phenyl group, a naphthyl group, a fluorenyl group, a phenanthrenyl group, an anthracenyl group, a fluoranthenyl group, a triphenylenyl group, a pyrenyl group, a chrysenyl group, a pyrrolyl group, a thiophenyl group, a furanyl group, an imidazolyl group, a pyrazolyl group, a thiazolyl group, an isothiazolyl group, an oxazolyl group, an isoxazolyl group, a pyridinyl group, a pyrazinyl group, a pyrimidinyl group, a pyridazinyl group, an isoindolyl group, an indolyl group, an indazolyl group, a purinyl group, a quinolinyl group, an isoquinolinyl group, a benzoquinolinyl group, a quinoxalinyl group, a quinazolinyl group, a cinnolinyl group, a carbazolyl group, a phenanthrolinyl group, a benzimidazolyl group, a benzofuranyl group, a benzothiophenyl group, an isobenzothiazolyl group, a benzoxazolyl group, an isobenzoxazolyl group, a triazolyl group, a tetrazolyl group, an oxadiazolyl group, a triazinyl group, a dibenzofuranyl group, a dibenzothiophenyl group, a benzocarbazolyl group, a dibenzocarbazolyl group, an imidazopyridinyl group, and an imidazopyrimidinyl group; and
-N(Q₁)(Q₂), -Si(Q₃)(Q₄)(Q₅), -B(Q₆)(Q₇), -P(=O)(Q₈)(Q₉), and P(Q₄₁)(Q₄₂)(Q₄₃),
Z₁ to Z₃ may each independently be selected from:
   hydrogen, deuterium, -F, a cyano group, a nitro group, -SF₅, a methyl group, an ethyl group, an n-propyl group, an iso-propyl group, an n-butyl group, an isobutyl group, a sec-butyl group, a tert-butyl group, an n-pentyl group, an isopentyl group, a sec-pentyl group, a tert-pentyl group, an n-hexyl group, an iso-hexyl group, a sec-hexyl group, a tert-hexyl group, an n-heptyl group, an iso-heptyl group, a sec-heptyl group, a tert-heptyl group, an n-octyl group, an iso-octyl group, a sec-octyl group, a tert-octyl group, an n-nonyl group, an iso-nonyl group, a sec-nonyl group, a tert-nonyl group, an n-decyl group, an iso-decyl group, a sec-decyl group, a tert-decyl group, a methoxy group, an ethoxy group, a propoxy group, a butoxy group, a pentoxy group, a cyclopentyl group, a cyclohexyl group, a cycloheptyl group, a cyclooctyl group, an adamantanyl group, a norbornanyl group, a norbornenyl group, a cyclopentenyl group, a cyclohexenyl group, a cycloheptenyl group, a phenyl group, a naphthyl group, a pyridinyl group, a pyrimidinyl group, a dibenzofuranyl group, and a dibenzothiophenyl group;
   a methyl group, an ethyl group, an n-propyl group, an iso-propyl group, an n-butyl group, an isobutyl group, a sec-butyl group, a tert-butyl group, an n-pentyl group, an isopentyl group, a sec-pentyl group, a tert-pentyl group, an n-hexyl group, an iso-hexyl group, a sec-hexyl group, a tert-hexyl group, an n-heptyl group, an iso-heptyl group, a sec-heptyl group, a tert-heptyl group, an n-octyl group, an iso-octyl group, a sec-octyl group, a tert-octyl group, an n-nonyl group, an iso-nonyl group, a sec-nonyl group, a tert-nonyl group, an n-decyl group, an iso-decyl group, a sec-decyl group, a tert-decyl group, a methoxy group, an ethoxy group, a propoxy group, a butoxy group, a pentoxy group, a cyclopentyl group, a cyclohexyl group, a cycloheptyl group, a cyclooctyl group, an adamantanyl group, a norbornanyl group, a norbornenyl group, a cyclopentenyl group, a cyclohexenyl group, a cycloheptenyl group, a phenyl group, a naphthyl group, a pyridinyl group, a pyrimidinyl group, a dibenzofuranyl group, and a dibenzothiophenyl group, each substituted with at least one selected from deuterium, -F, -CD₃, -CD₂H, -CDH₂, -CF₃, -CF₂H, -CFH₂, a cyano group, a nitro group, a C₁-C₁₀ alkyl group, a C₁-C₁₀ alkoxy group, a cyclopentyl group, a cyclohexyl group, a cycloheptyl group, a cyclooctyl group, an adamantanyl group, a norbornanyl group, a norbornenyl group, a cyclopentenyl group, a cyclohexenyl group, a cycloheptenyl group, a phenyl group, a naphthyl group, a pyridinyl group, a pyrimidinyl group, a dibenzofuranyl group, and a dibenzothiophenyl group; and
   -N(Q₃₁)(Q₃₂), -Si(Q₃₃)(Q₃₄)(Q₃₅), -B(Q₃₆)(Q₃₇), and -P(=O)(Q₃₈)(Q₃₉),
wherein Q₁ to Q₉, Q₃₁ to Q₃₉, and Q₄₁ to Q₄₃ may each independently be selected from:
   -CH₃, -CD₃, -CD₂H, -CDH₂, -CH₂CH₃, -CH₂CD₃, -CH₂CD₂H, -CH₂CDH₂, -CHDCH₃, -CHDCD₂H, -CHDCDH₂, -CHDCD₃, -CD₂CD₃, -CD₂CD₂H, and -CD₂CDH₂;
   an n-propyl group, an isopropyl group, an n-butyl group, an isobutyl group, a sec-butyl group, a tert-butyl group, an n-pentyl group, an isopentyl group, a sec-pentyl group, a tert-pentyl group, a phenyl group, and a naphthyl group; and
   an n-propyl group, an iso-propyl group, an n-butyl group, an isobutyl group, a sec-butyl group, a tert-butyl group, an n-pentyl group, an isopentyl group, a sec-pentyl group, a tert-pentyl group, a phenyl group, and a naphthyl group, each substituted with at least one selected from deuterium, a C₁-C₁₀ alkyl group, and a phenyl group,
d2 may be 1 or 2,
d3 may be an integer selected from 1 to 3,
d4 may be an integer selected from 1 to 4,
d5 may be an integer selected from 1 to 5,
d6 may be an integer selected from 1 to 6,
d7 may be an integer selected from 1 to 7,
d8 may be an integer selected from 1 to 8, and
* indicates a binding site to M in Formula 1.

The organometallic compound represented by Formula 1 may be one selected from Compounds 1 to 142, but embodiments of the present disclosure are not limited thereto:

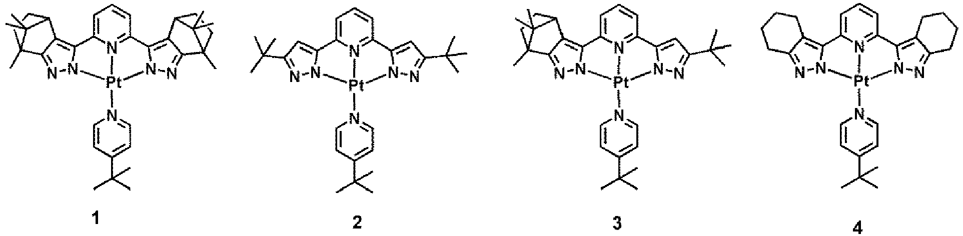

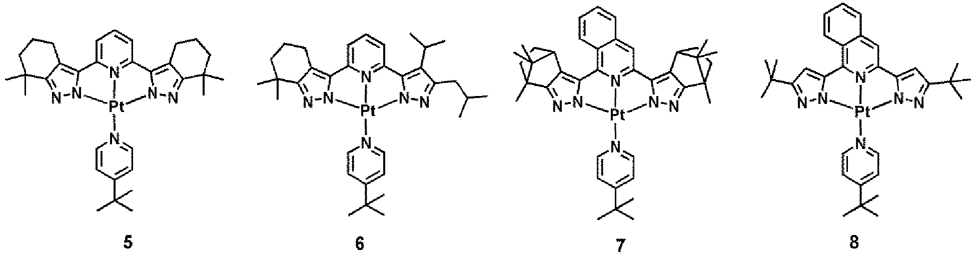

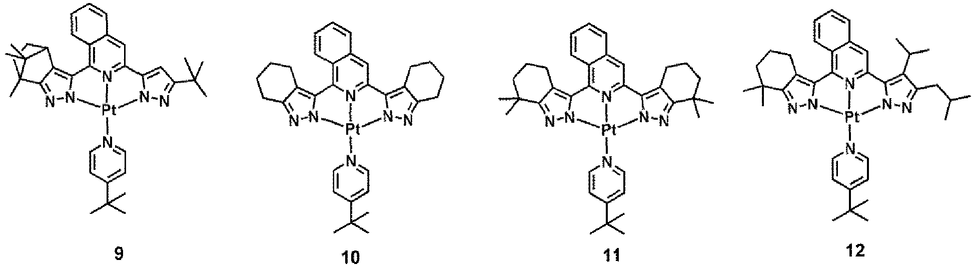

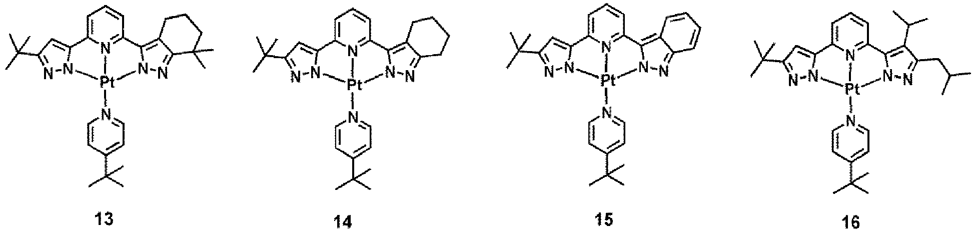

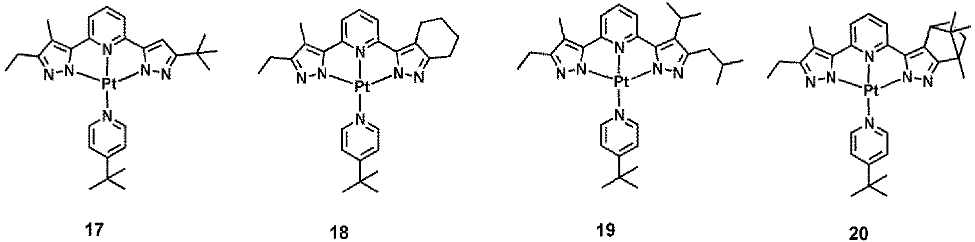

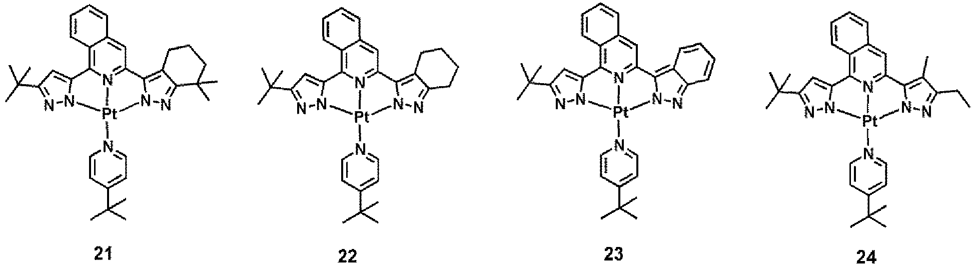

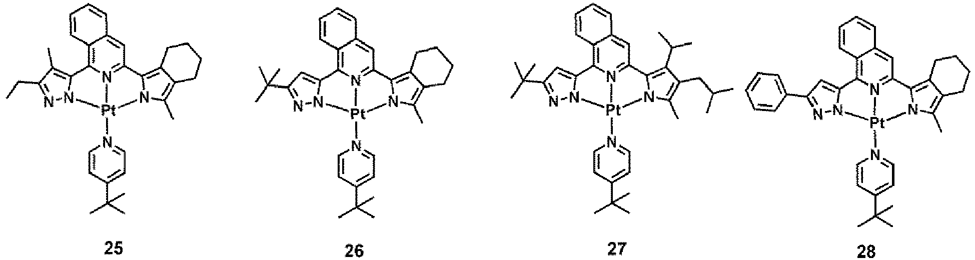

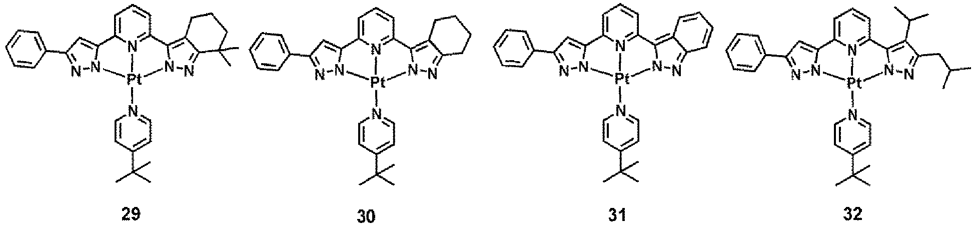

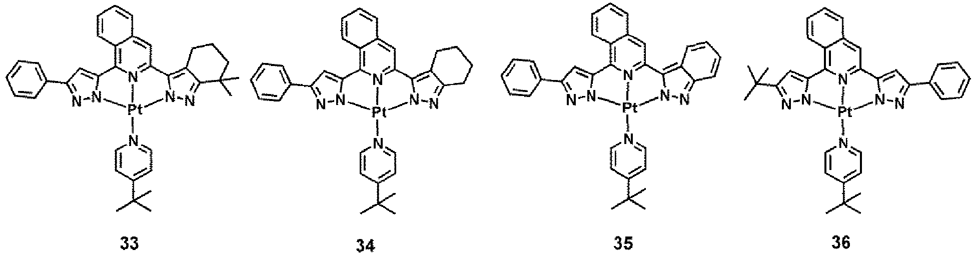

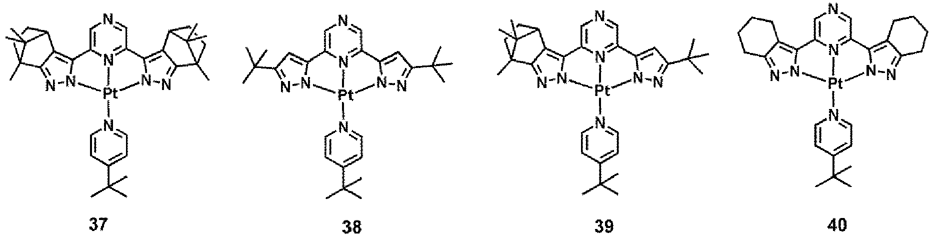

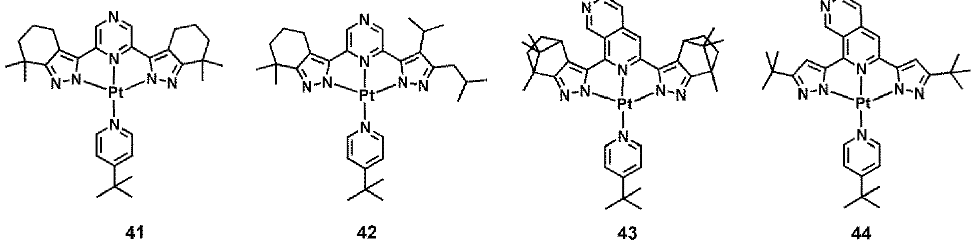

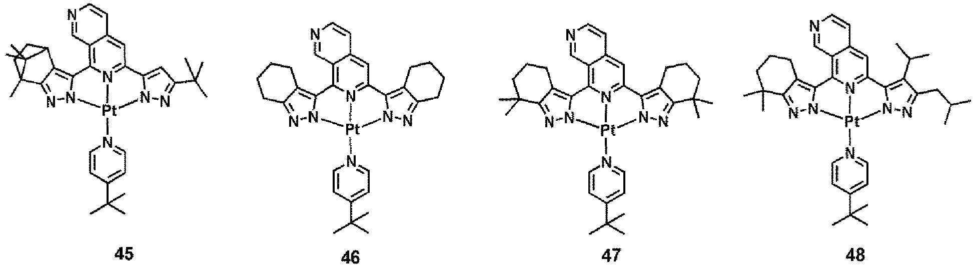

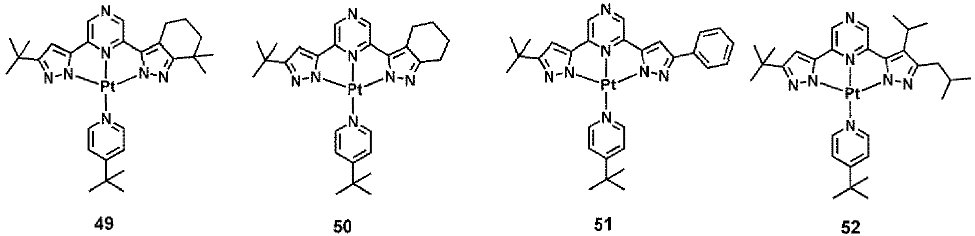

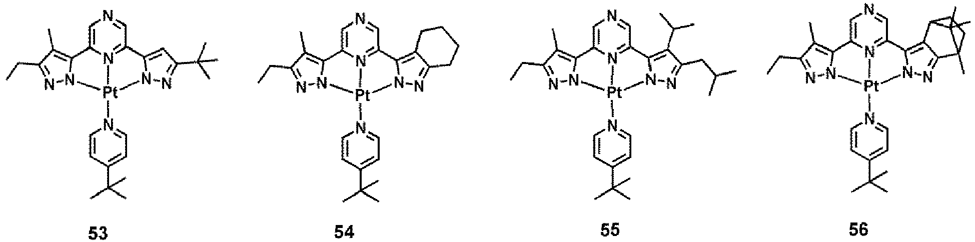

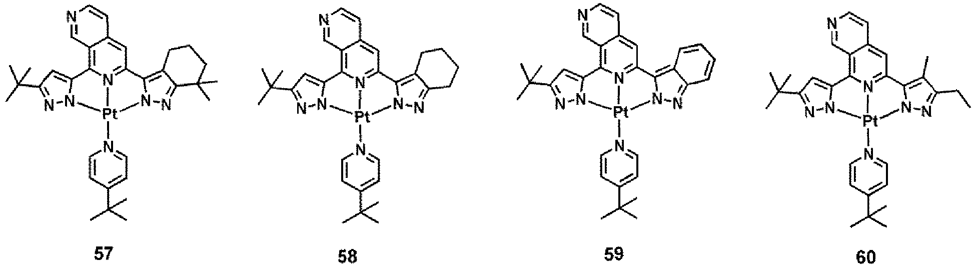

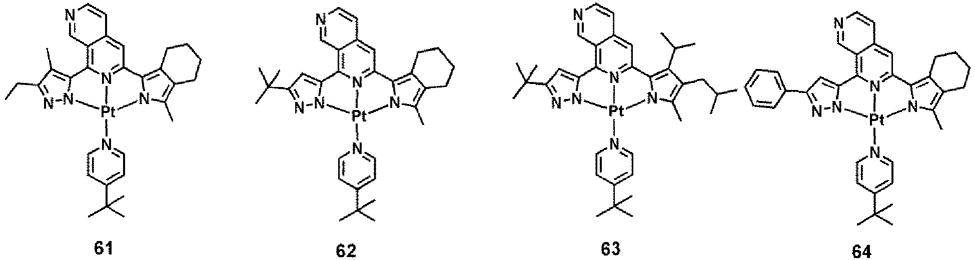

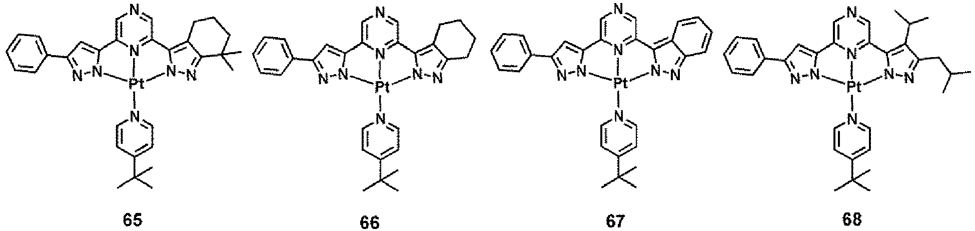

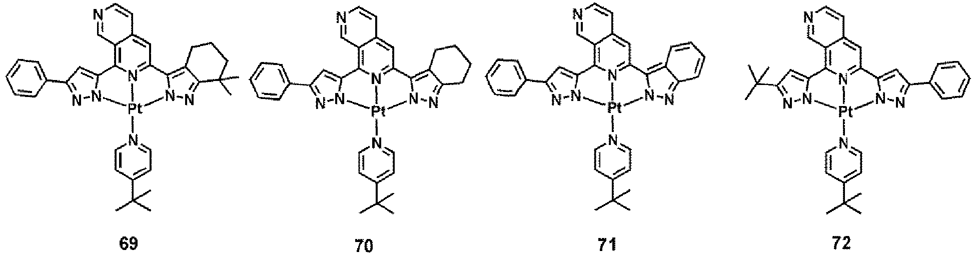

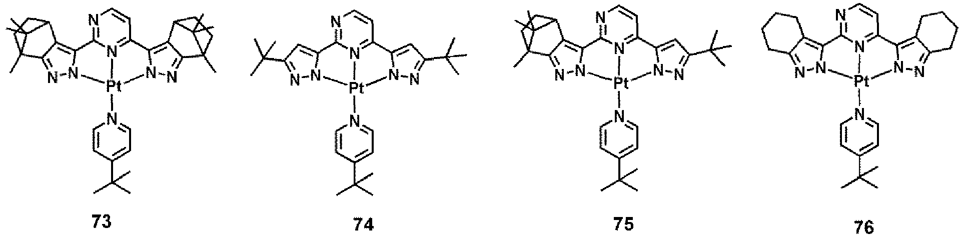

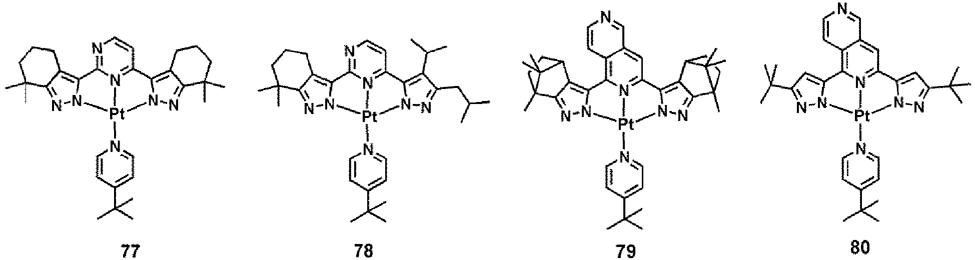

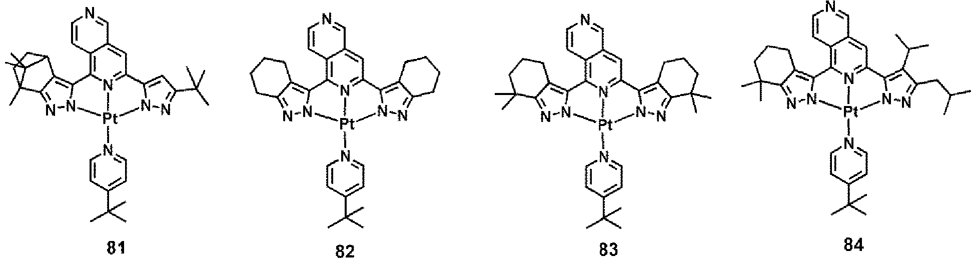

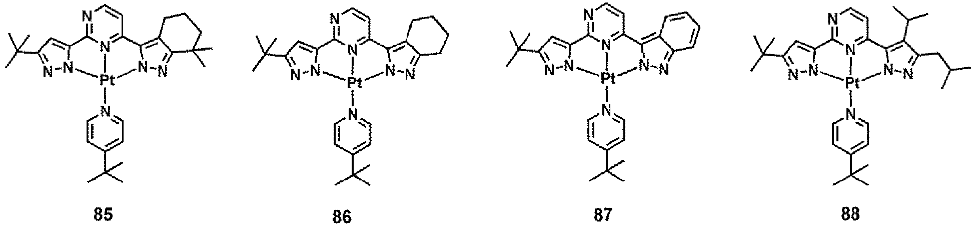

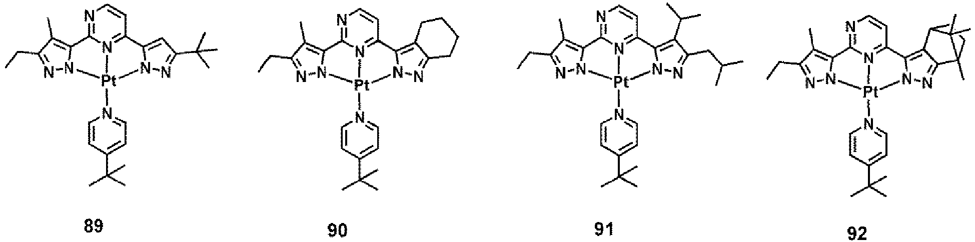

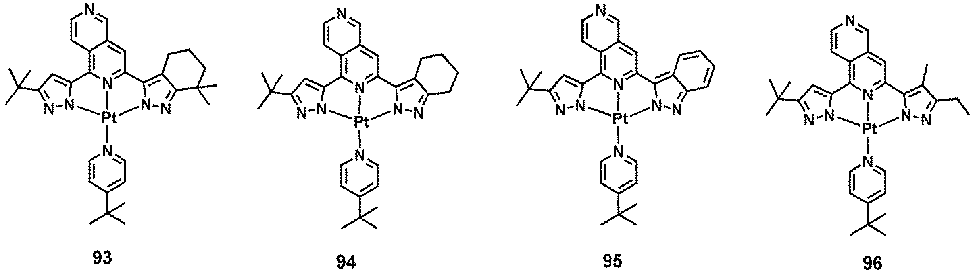

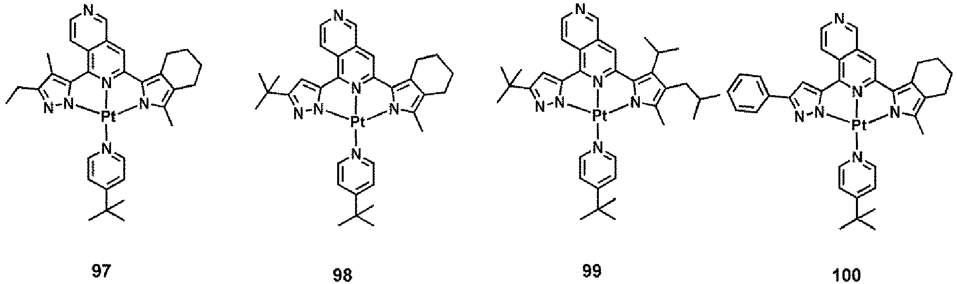

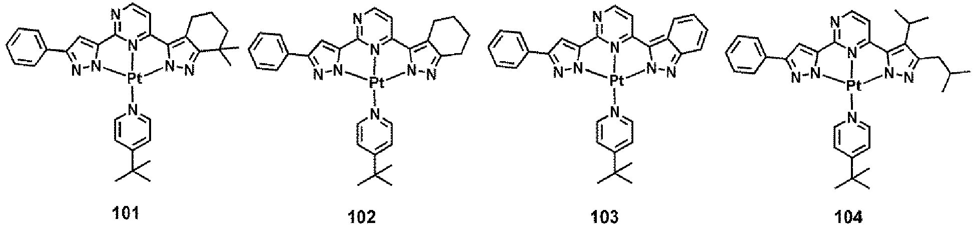

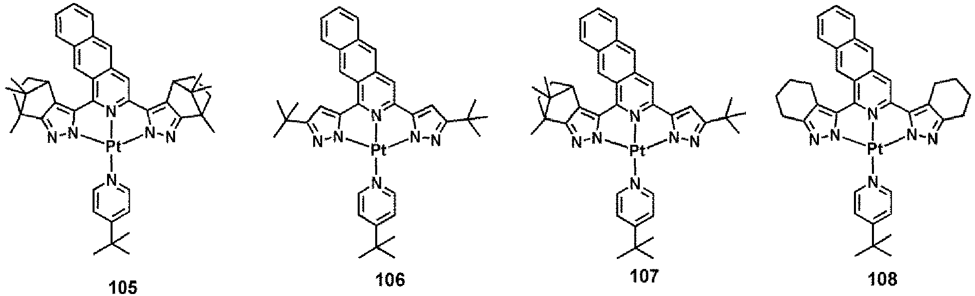

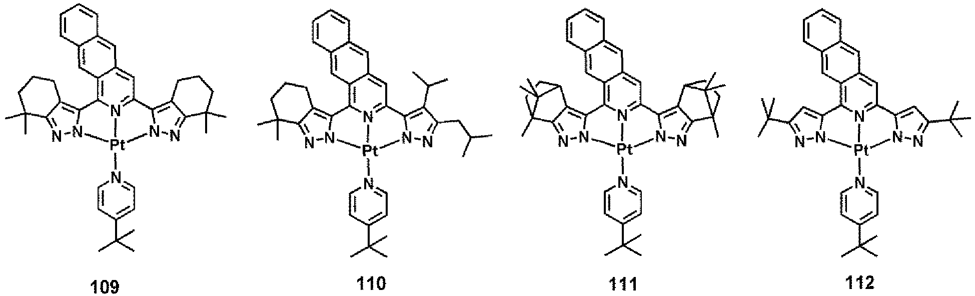

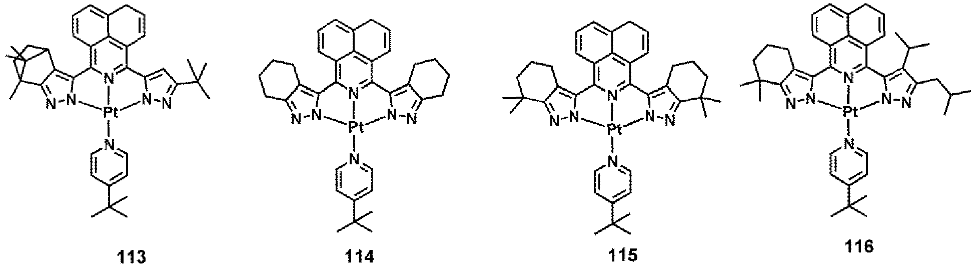

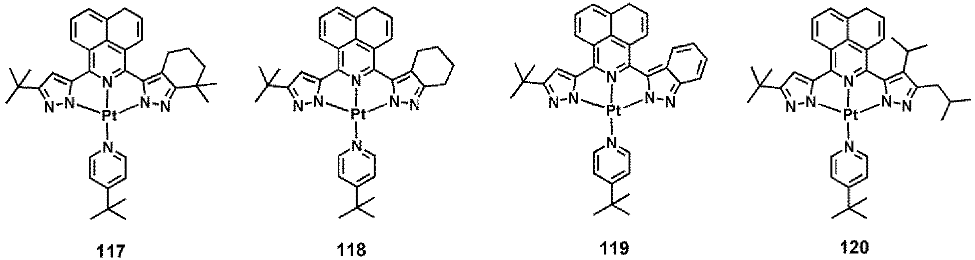

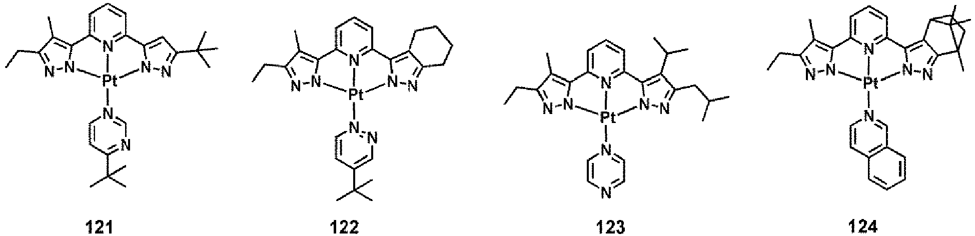

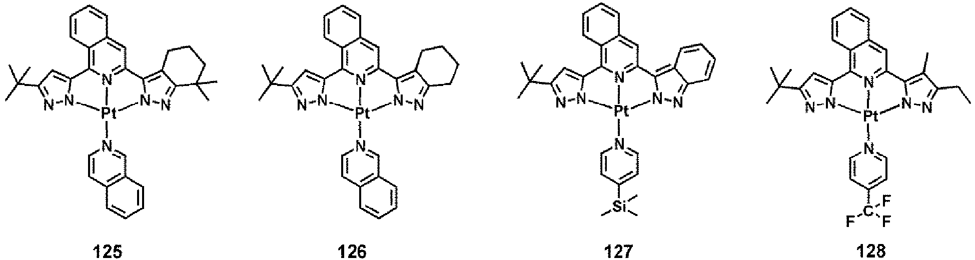

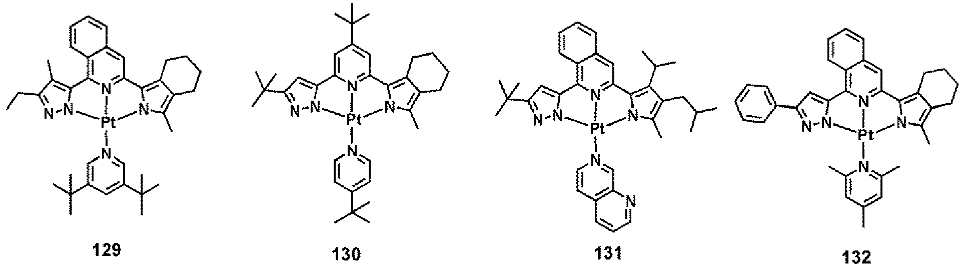

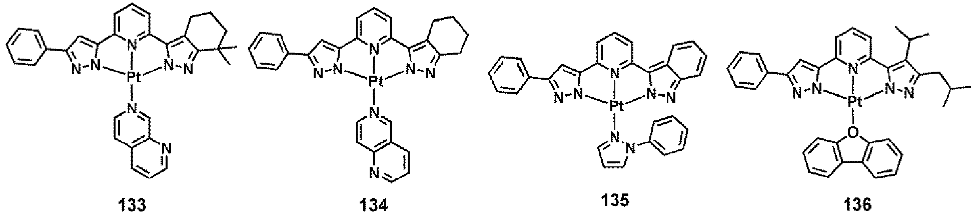

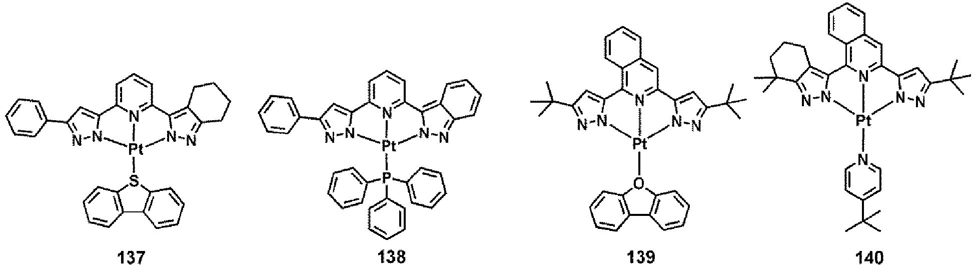

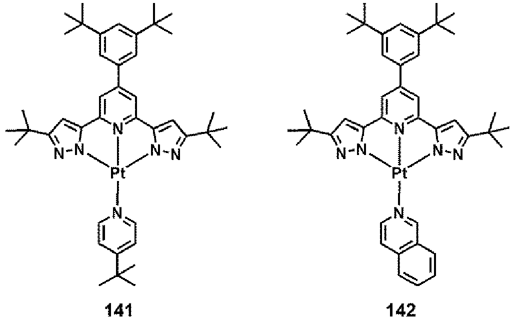

Ligand L₁ in the organometallic compound represented by Formula 1 may be selected from ligands represented by Formula 2, and rings A₁, A₂, and A₃ in Formula 2 may have one or two nitrogen atoms as a ring-forming atom. The organometallic compound represented by Formula 1 is easy in terms of energy transfer from a host, as compared with a compound (for example, see Compound A) that has the same structure as the organometallic compound represented by Formula 1, provided that rings A₁, A₂, and A₃ in Formula 2 have three or more nitrogen atoms. Therefore, an electronic device, for example, an organic light-emitting device, which includes the organometallic compound represented by Formula 1, may have high efficiency.

Also, Y₃ in Formula 2 may be nitrogen, and a bond between Y₃ in Formula 2 and M in Formula 1 may be a coordinate bond. Therefore, an electronic device, for example, an organic light-emitting device, which includes the organometallic compound represented by Formula 1, may have high luminescent efficiency, high power efficiency, high quantum efficiency, and a long lifespan.

For example, the highest occupied molecular orbital (HOMO), lowest unoccupied molecular orbital (LUMO), singlet (S₁) energy level, and triplet (T₁) energy level of Compounds 1, 2, 7, 8, 9, 10, 11, and 12 and Compounds A to C were evaluated by using a density functional theory (DFT) method of a Gaussian program (the structure was optimized at B3LYP, 6-31G(d,p) level). Results thereof are shown in Table 1.

**[Table 1]**

| Compound No. | HOMO (eV) | LUMO (eV) | S₁ energy level (eV) | T₁ energy level (eV) |
|---|---|---|---|---|
| 1 | -4.942 | -1.563 | 2.628 | 1.452 |
| 2 | -5.212 | -1.675 | 2.759 | 2.518 |
| 7 | -4.899 | -1.836 | 2.399 | 2.046 |
| 8 | -5.1 | -1.94 | 2.516 | 2.044 |
| 9 | -5.007 | -1.881 | 2.46 | 2.059 |
| 10 | -4.978 | -1.831 | 2.481 | 2.051 |
| 11 | -5.019 | -1.862 | 2.493 | 2.057 |
| 12 | -5.033 | -1.882 | 2.480 | 2.063 |
| A | -6.486 | -2.629 | 3.028 | 2.742 |
| B | -5.666 | -2.11 | 2.768 | 2.515 |
| C | -4.862 | -1.300 | 3.008 | 2.809 |

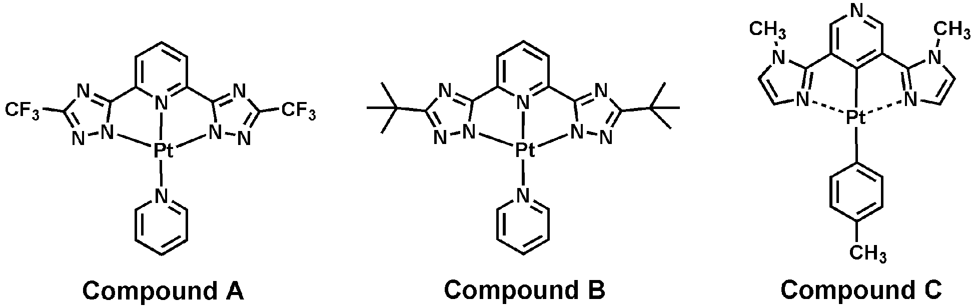

From Table 1, it is confirmed that the organometallic compound represented by Formula 1 has electric characteristics suitable for use in an electronic device, for example, a dopant of an organic light-emitting device. Although not limited by a specific theory, it is expected from Table 1 that, since the LUMO values of Compounds A and B are lower than the LUMO values of Compounds 1, 2, 7, 8, 9, 10, 11, and 12 (that is, the LUMO absolute values of Compounds A and B are greater than the LUMO absolute values of Compounds 1, 2, 7, 8, 9, 10, 11, and 12), the luminescent efficiency and lifespan of an electronic device, for example, an organic light-emitting device, which includes Compound A or B, is poorer than the luminescent efficiency and lifespan of an electronic device, for example, an organic light-emitting device, which includes one selected from Compounds 1, 2, 7, 8, 9, 10, 11, and 12. In addition, since the HOMO values of Compounds A and B are lower than the HOMO values of Compounds 1, 2, 7, 8, 9, 10, 11, and 12 (that is, the HOMO absolute values of Compounds A and B are greater than the HOMO absolute values of Compounds 1, 2, 7, 8, 9, 10, 11, and 12), it is expected that the energy transfer efficiency of Compound A and B, is poorer than the energy transfer efficiency of Compounds 1, 2, 7, 8, 9, 10, 11, and 12

Synthesis methods of the organometallic compound represented by Formula 1 may be recognizable by one of ordinary skill in the art by referring to Synthesis Examples provided below.

The organometallic compound represented by Formula 1 is suitable for use in an organic layer of an organic light-emitting device, for example, for use as a dopant in an emission layer of the organic layer. Thus, another aspect provides an organic light-emitting device that includes: a first electrode; a second electrode; and an organic layer between the first electrode and the second electrode and includes an emission layer and at least one of the organometallic compounds represented by Formula 1.

The organic light-emitting device may have, due to the inclusion of an organic layer including the organometallic compound represented by Formula 1, low driving voltage, high luminescent efficiency, high power efficiency, high quantum efficiency, long lifespan, and excellent color.

The organometallic compound of Formula 1 may be used between a pair of electrodes of an organic light-emitting device. For example, the organometallic compound represented by Formula 1 may be included in the emission layer. In this regard, the organometallic compound may act as a dopant, and the emission layer may further include a host (that is, an amount of the organometallic compound represented by Formula 1 is smaller than an amount of the host).

The expression that "(an organic layer) includes at least one of organometallic compounds" used herein may include a case in which "(an organic layer) includes identical organometallic compounds represented by Formula 1" and a case in which "(an organic layer) includes two or more different organometallic compounds represented by Formula 1".

For example, the organic layer may include only Compound 1 as the organometallic compound. In this regard, Compound 1 may exist only in the emission layer of the organic light-emitting device. In one or more embodiments, the organic layer may include, as the organometallic compound, Compound 1 and Compound 2. In this regard, Compound 1 and Compound 2 may exist in an identical layer (for example, Compound 1 and Compound 2 all may exist in an emission layer).

The first electrode may be an anode, which is a hole injection electrode, and the second electrode may be a cathode, which is an electron injection electrode; or the first electrode may be a cathode, which is an electron injection electrode, and the second electrode may be an anode, which is a hole injection electrode.

In one or more embodiments, in the organic light-emitting device, the first electrode is an anode, and the second electrode is a cathode, and the organic layer includes a hole transport region between the first electrode and the emission layer and an electron transport region between the emission layer and the second electrode, and the hole transport region includes at least one selected from a hole injection layer, a hole transport layer, and an electron blocking layer, and the electron transport region includes at least one selected from a hole blocking layer, an electron transport layer, and an electron injection layer.

The term "organic layer" used herein refers to a single layer and/or a plurality of layers between the first electrode and the second electrode of the organic light-emitting device. The "organic layer" may include, in addition to an organic compound, an organometallic complex including metal.

FIG. 1 is a schematic view of an organic light-emitting device 10 according to an embodiment. Hereinafter, the structure of an organic light-emitting device according to an embodiment and a method of manufacturing an organic light-emitting device according to an embodiment will be described in connection with FIG. 1. The organic light-emitting device 10 includes a first electrode 11, an organic layer 15, and a second electrode 19, which are sequentially stacked.

A substrate may be additionally disposed under the first electrode 11 or above the second electrode 19. For use as the substrate, any substrate that is used in general organic light-emitting devices may be used, and the substrate may be a glass substrate or a transparent plastic substrate, each having excellent mechanical strength, thermal stability, transparency, surface smoothness, ease of handling, and water-resistance.

The first electrode 11 may be formed by depositing or sputtering a material for forming the first electrode 11 on the substrate. The first electrode 11 may be an anode. The material for forming the first electrode 11 may be selected from materials with a high work function to facilitate hole injection. The first electrode 11 may be a reflective electrode, a semi-transmissive electrode, or a transmissive electrode. The material for forming the first electrode 11 may be, for example, indium tin oxide (ITO), indium zinc oxide (IZO), tin oxide (SnO₂), and zinc oxide (ZnO). In one or more embodiments, magnesium (Mg), aluminum (Al), aluminum-lithium (Al-Li), calcium (Ca), magnesium-indium (Mg-In), or magnesium-silver (Mg-Ag) may be used as the material for forming the first electrode 11.

The first electrode 11 may have a single-layered structure or a multi-layered structure including two or more layers. For example, the first electrode 11 may have a three-layered structure of ITO/Ag/ITO, but the structure of the first electrode 11 is not limited thereto.

The organic layer 15 is disposed on the first electrode 11.

The organic layer 15 may include a hole transport region, an emission layer, and an electron transport region.

The hole transport region may be disposed between the first electrode 11 and the emission layer.

The hole transport region may include at least one selected from a hole injection layer, a hole transport layer, an electron blocking layer, and a buffer layer.

The hole transport region may include only either a hole injection layer or a hole transport layer. In one or more embodiments, the hole transport region may have a structure of hole injection layer/hole transport layer or hole injection layer/hole transport layer/electron blocking layer, which are sequentially stacked in this stated order from the first electrode 11.

A hole injection layer may be formed on the first electrode 11 by using one or more suitable methods selected from vacuum deposition, spin coating, casting, or langmuir-blodgett (LB) deposition.

When a hole injection layer is formed by vacuum deposition, the deposition conditions may vary according to a material that is used to form the hole injection layer, and the structure and thermal characteristics of the hole injection layer. For example, the deposition conditions may include a deposition temperature of about 100 to about 500 °C, a vacuum pressure of about 10⁻⁸ to about 10⁻³ torr, and a deposition rate of about 0.001 to about 10 nm/s (about 0.01 to about 100 Å/sec). However, the deposition conditions are not limited thereto.

When the hole injection layer is formed using spin coating, coating conditions may vary according to the material used to form the hole injection layer, and the structure and thermal properties of the hole injection layer. For example, a coating speed may be from about 2000 rpm to about 5000 rpm, and a temperature at which a heat treatment is performed to remove a solvent after coating may be from about 80 °C to about 20 °C. However, the coating conditions are not limited thereto.

Conditions for forming a hole transport layer and an electron blocking layer may be understood by referring to conditions for forming the hole injection layer.

The hole transport region may include at least one selected from m-MTDATA, TDATA, 2-TNATA, NPB, β-NPB, TPD, Spiro-TPD, Spiro-NPB, methylated-NPB, TAPC, HMTPD, 4,4',4"-tris(N-carbazolyl)triphenylamine (TCTA), polyaniline/dodecylbenzenesulfonic acid (Pani/DBSA), poly(3,4-ethylenedioxythiophene)/poly-(4-styrenesulfonate) (PEDOT/PSS), polyaniline/camphor sulfonicacid (Pani/CSA), polyaniline/poly(4-styrenesulfonate) (Pani/PSS), a compound represented by Formula 201 below, and a compound represented by Formula 202:

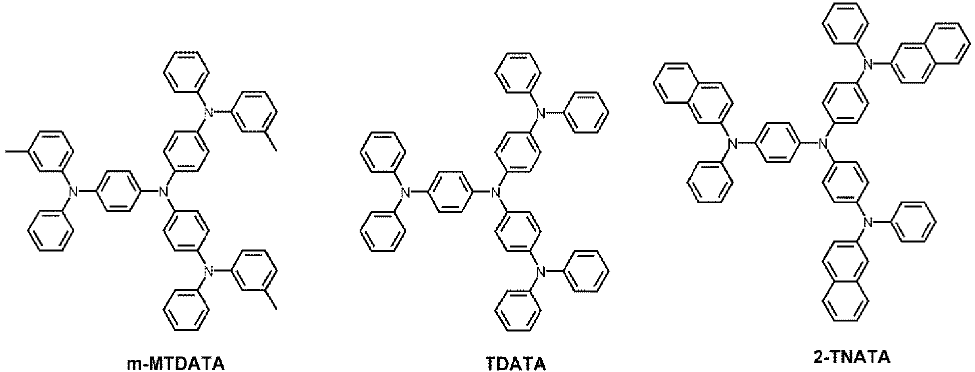

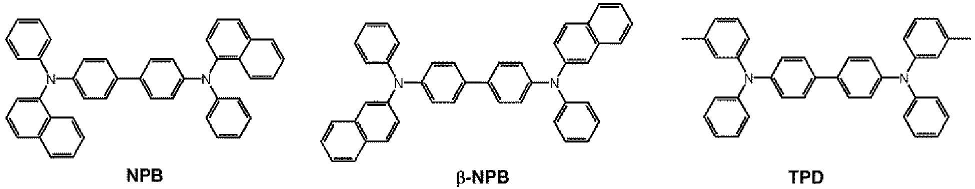

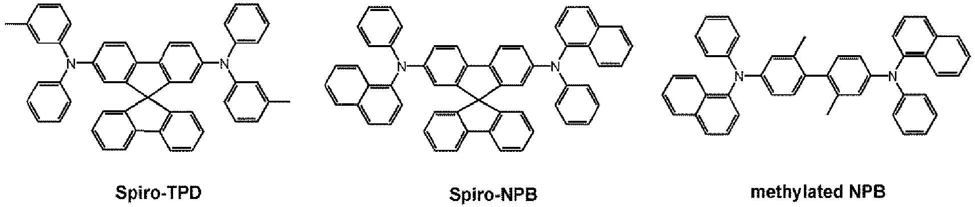

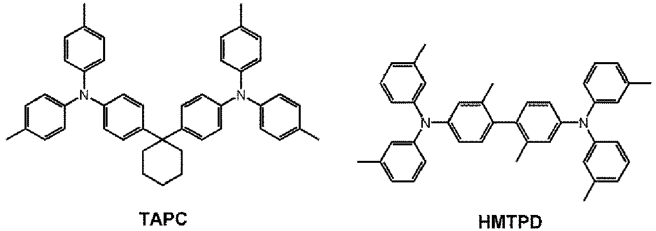

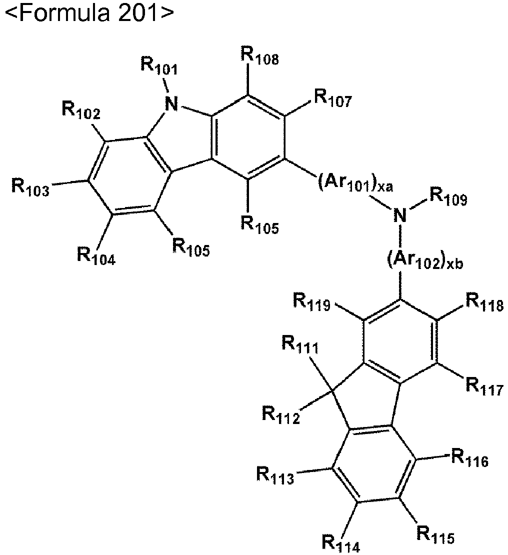

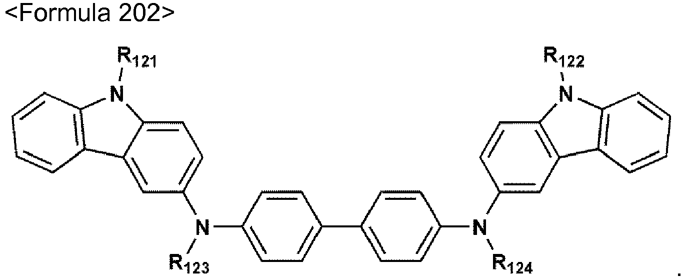

Ar₁₀₁ and Ar₁₀₂ in Formula 201 may each independently be selected from:
a phenylene group, a pentalenylene group, an indenylene group, a naphthylene group, an azulenylene group, a heptalenylene group, an acenaphthylene group, a fluorenylene group, a phenalenylene group, a phenanthrenylene group, an anthracenylene group, a fluoranthenylene group, a triphenylenylene group, a pyrenylene group, a chrysenylenylene group, a naphthacenylene group, a picenylene group, a perylenylene group, and a pentacenylene group; and
a phenylene group, a pentalenylene group, an indenylene group, a naphthylene group, an azulenylene group, a heptalenylene group, an acenaphthylene group, a fluorenylene group, a phenalenylene group, a phenanthrenylene group, an anthracenylene group, a fluoranthenylene group, a triphenylenylene group, a pyrenylene group, a chrysenylenylene group, a naphthacenylene group, a picenylene group, a perylenylene group, and a pentacenylene group, each substituted with at least one selected from deuterium, -F, -Cl, -Br, -I, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a C₁-C₆₀ alkyl group, a C₂-C₆₀ alkenyl group, a C₂-C₆₀ alkynyl group, a C₁-C₆₀ alkoxy group, a C₃-C₁₀ cycloalkyl group, a C₃-C₁₀ cycloalkenyl group, a C₁-C₁₀ heterocycloalkyl group, a C₁-C₁₀ heterocycloalkenyl group, a C₆-C₆₀ aryl group, a C₆-C₆₀ aryloxy group, a C₆-C₆₀ arythio group, a C₁-C₆₀ heteroaryl group, a monovalent non-aromatic condensed polycyclic group, and a monovalent non-aromatic condensed heteropolycyclic group.

In Formula 201, xa and xb may each independently be an integer selected from 0 to 5, or 0, 1, or 2. For example, xa is 1 and xb is 0, but xa and xb are not limited thereto.

R₁₀₁ to R₁₀₈, R₁₁₁ to R₁₁₉, and R₁₂₁ to R₁₂₄ in Formulae 201 and 202 may each independently be selected from:
hydrogen, deuterium, -F, -Cl, -Br, -I, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a C₁-C₁₀ alkyl group (for example, a methyl group, an ethyl group, a propyl group, a butyl group, a pentyl group, a hexyl group, and so on), or a C₁-C₁₀ alkoxy group (for example, a methoxy group, an ethoxy group, a propoxy group, a butoxy group, a pentoxy group, and so on);
a C₁-C₁₀ alkyl group or a C₁-C₁₀ alkoxy group, each substituted with at least one selected from deuterium, -F, -Cl, -Br, -I, a hydroxyl group, a cyano group, a nitro group, an amino group, an amidino group, a hydrazine group, a hydrazone group, a carboxylic acid group or a salt thereof, a sulfonic acid group or a salt thereof, and a phosphoric acid group or a salt thereof;
a phenyl group, a naphthyl group, an anthracenyl group, a fluorenyl group, and a pyrenyl group; and
a phenyl group, a naphthyl group, an anthracenyl group, a fluorenyl group, and a pyrenyl group, each substituted with at least one selected from deuterium, -F, -Cl, -Br, -I, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a C₁-C₁₀ alkyl group, and a C₁-C₁₀ alkoxy group, but they are not limited thereto.

R₁₀₉ in Formula 201 may be selected from:
a phenyl group, a naphthyl group, an anthracenyl group, and a pyridinyl group; and
a phenyl group, a naphthyl group, an anthracenyl group and a pyridinyl group, each substituted with at least one selected from deuterium, -F, -CI, -Br, -I, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a C₁-C₂₀ alkyl group, a C₁-C₂₀ alkoxy group, a phenyl group, a naphthyl group, an anthracenyl group, and a pyridinyl group.

In one or more embodiments, the compound represented by Formula 201 may be represented by Formula 201A, but embodiments of the present disclosure are not limited thereto:

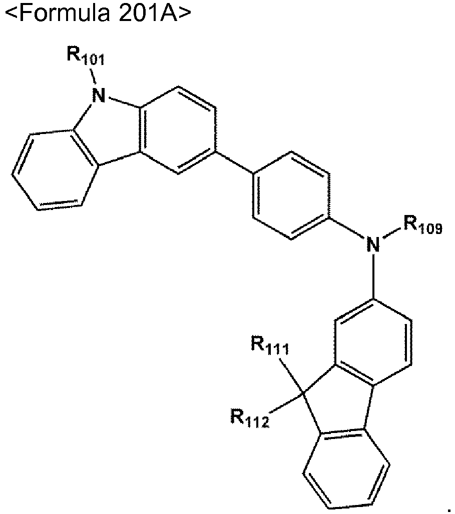

R₁₀₁, R₁₁₁, R₁₁₂, and R₁₀₉ in Formula 201A may be understood by referring to the description provided herein.

For example, the compound represented by Formula 201 and the compound represented by Formula 202 may include compounds HT1 to HT20 illustrated below, but embodiments of the present disclosure are not limited thereto.

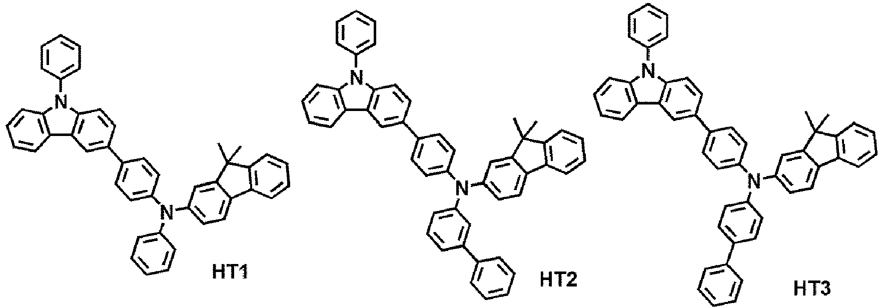

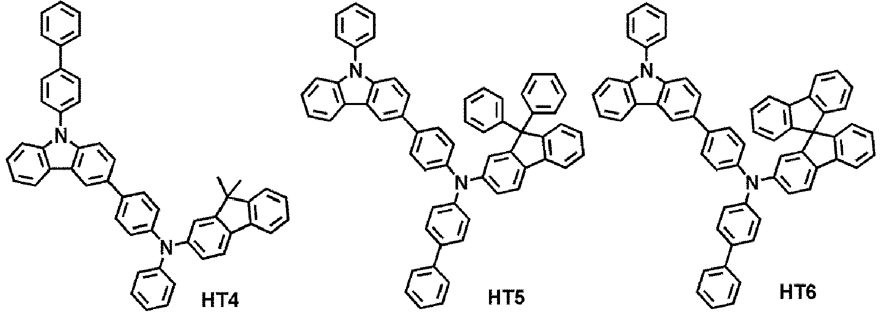

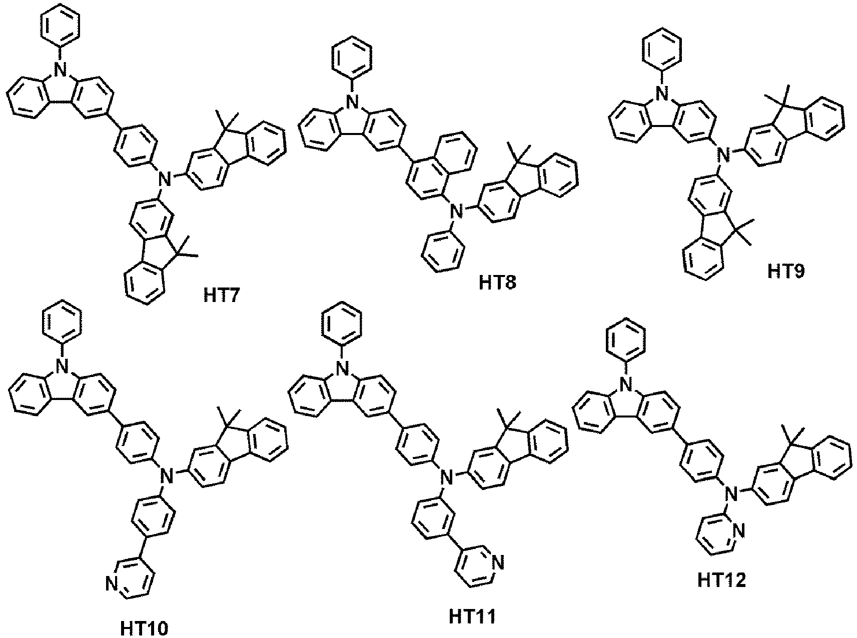

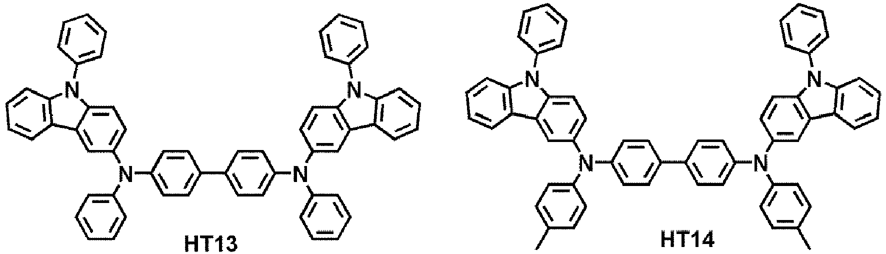

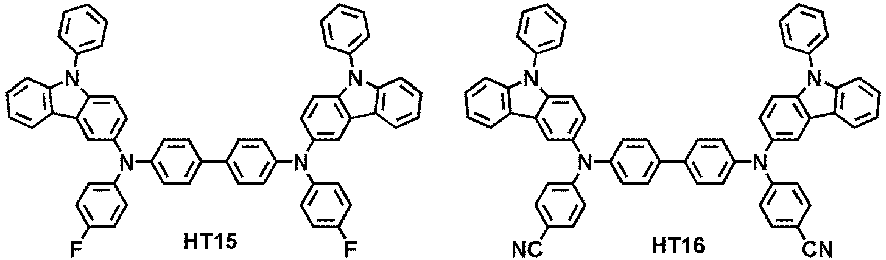

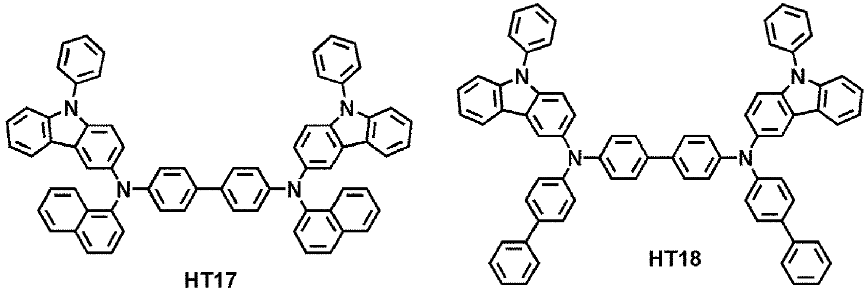

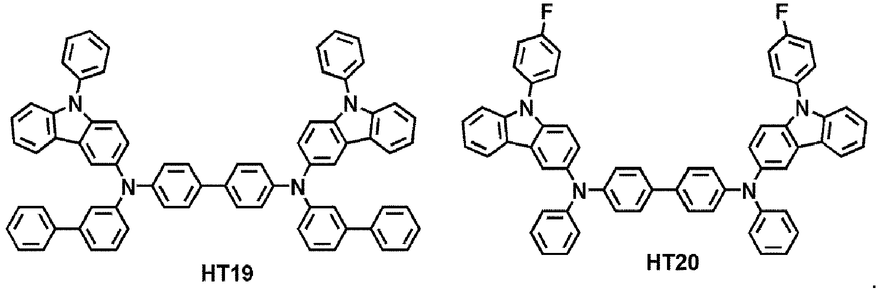

A thickness of the hole transport region may be in a range of about 10 nm to about 1,000 nm (about 100 Å to about 10,000 Å), for example, about 10 nm to about 100 nm (about 100 Å to about 1,000 Å). When the hole transport region includes a hole injection layer and a hole transport layer, the thickness of the hole injection layer may be in a range of about 10 nm to about 1,000nm (about 100 Å to about 10,000 Å), for example, about 10 nm to about 100 nm (about 100 Å to about 1,000 Å), and the thickness of the hole transport layer may be in a range of about 5 nm to about 200 nm (about 50 Å to about 2,000 Å), for example, about 10 nm to about 150 nm (about 100 Å to about 1,500 Å). When the thicknesses of the hole transport region, the hole injection layer, and the hole transport layer are within these ranges, satisfactory hole transporting characteristics may be obtained without a substantial increase in driving voltage.

The hole transport region may further include, in addition to these materials, a charge-generation material for the improvement of conductive properties. The charge-generation material may be homogeneously or non-homogeneously dispersed in the hole transport region.

The charge-generation material may be, for example, a p-dopant. The p-dopant may be one selected from a quinone derivative, a metal oxide, and a cyano group-containing compound, but embodiments are not limited thereto. Non-limiting examples of the p-dopant are a quinone derivative, such as tetracyanoquinonedimethane (TCNQ) or 2,3,5,6-tetrafluoro-tetracyano-1,4-benzo-quinonedimethane (F4-TCNQ); a metal oxide, such as a tungsten oxide or a molybdenum oxide; and a cyano group-containing compound, such as Compound HT-D1 below, but embodiments of the present disclosure are not limited thereto.

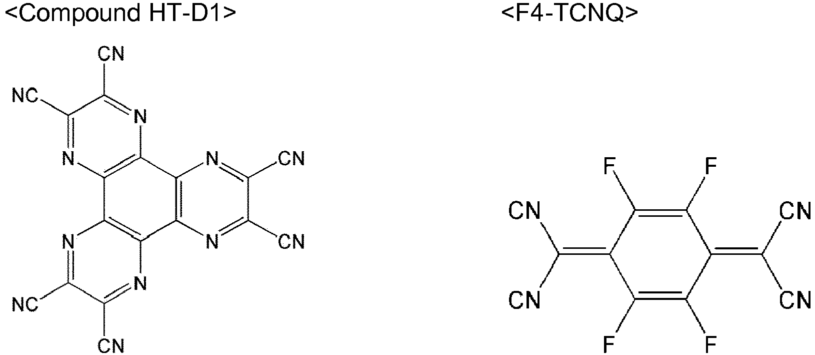

The hole transport region may include a buffer layer.

Also, the buffer layer may compensate for an optical resonance distance according to a wavelength of light emitted from the emission layer, and thus, the efficiency of a formed organic light-emitting device may be improved.

Then, an emission layer may be formed on the hole transport region by vacuum deposition, spin coating, casting, LB deposition, or the like. When the emission layer is formed by vacuum deposition or spin coating, the deposition or coating conditions may be similar to those applied to form the hole injection layer although the deposition or coating conditions may vary according to the material that is used to form the emission layer.

Meanwhile, when the hole transport region includes an electron blocking layer, a material for the electron blocking layer may be selected from materials for the hole transport region described above and materials for a host to be explained later. However, the material for the electron blocking layer is not limited thereto. For example, when the hole transport region includes an electron blocking layer, a material for the electron blocking layer may be mCP, which will be explained later.

The emission layer may include a host and a dopant, and the dopant may include the organometallic compound represented by Formula 1.

The host may include at least one selected from TPBi, TBADN, ADN (also referred to as "DNA"), CBP, CDBP, TCP, mCP, Compound H50, and Compound H51:

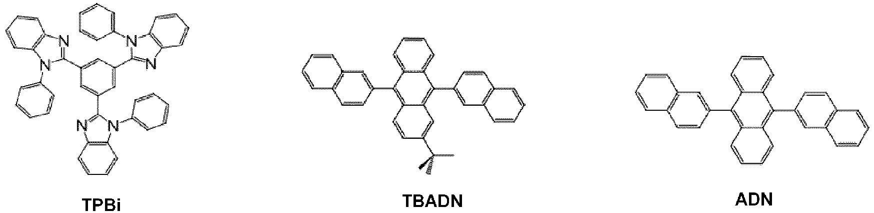

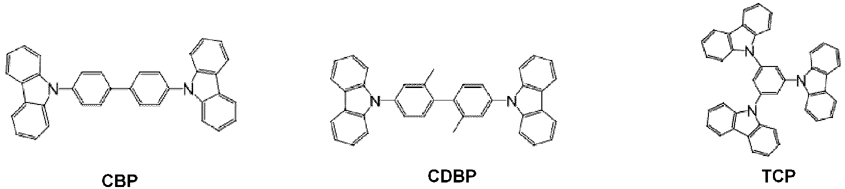

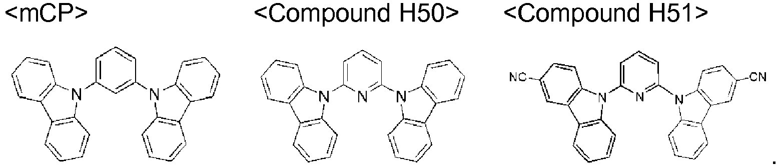

In one or more embodiments, the host may further include a compound represented by Formula 301.

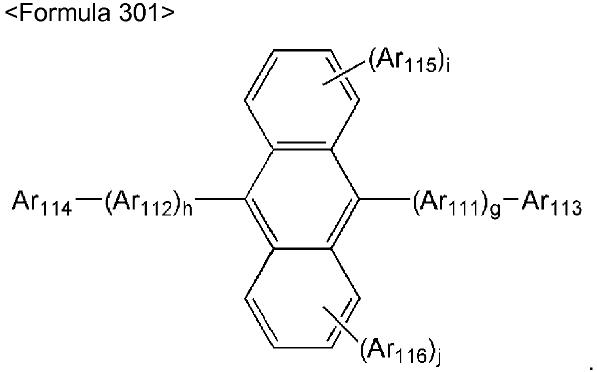

Ar₁₁₁ and Ar₁₁₂ in Formula 301 may each independently be selected from:
a phenylene group, a naphthylene group, a phenanthrenylene group, and a pyrenylene group; and
a phenylene group, a naphthylene group, a phenanthrenylene group, and a pyrenylene group, each substituted with at least one selected from a phenyl group, a naphthyl group, and an anthracenyl group.

Ar₁₁₃ to Ar₁₁₆ in Formula 301 may each independently be selected from:
a C₁-C₁₀ alkyl group, a phenyl group, a naphthyl group, a phenanthrenyl group, and a pyrenyl group; and
a phenyl group, a naphthyl group, a phenanthrenyl group, and a pyrenyl group, each substituted with at least one selected from a phenyl group, a naphthyl group, and an anthracenyl group.
g, h, i, and j in Formula 301 may each independently be an integer selected from 0 to 4, and may be, for example, 0, 1, or 2.

Ar₁₁₃ to Ar₁₁₆ in Formula 301 may each independently be selected from:
a C₁-C₁₀ alkyl group, substituted with at least one selected from a phenyl group, a naphthyl group, and an anthracenyl group;
a phenyl group, a naphthyl group, an anthracenyl group, a pyrenyl, a phenanthrenyl group, and a fluorenyl group;
a phenyl group, a naphthyl group, an anthracenyl group, a pyrenyl group, a phenanthrenyl group, and a fluorenyl group, each substituted with at least one selected from deuterium, -F, -Cl, -Br, -I, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a C₁-C₆₀ alkyl group, a C₂-C₆₀ alkenyl group, a C₂-C₆₀ alkynyl group, a C₁-C₆₀ alkoxy group, a phenyl group, a naphthyl group, an anthracenyl group, a pyrenyl group, a phenanthrenyl group, and a fluorenyl group; and 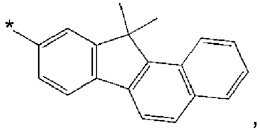 but embodiments are not limited thereto.

In one or more embodiments, the host may include a compound represented by Formula 302:

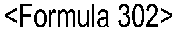

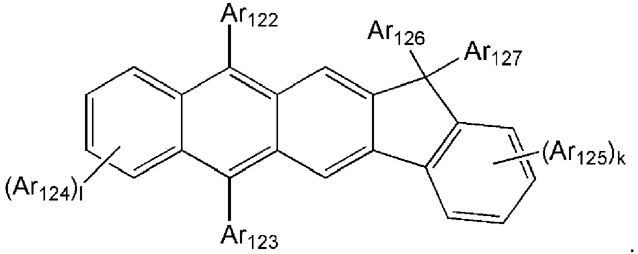

Ar₁₂₂ to Ar₁₂₅ in Formula 302 are the same as described in detail in connection with Ar₁₁₃ in Formula 301.

Ar₁₂₆ and Ar₁₂₇ in Formula 302 may each independently be a C₁-C₁₀ alkyl group (for example, a methyl group, an ethyl group, or a propyl group).

k and l in Formula 302 may each independently be an integer selected from 0 to 4. For example, k and l may be 0, 1, or 2.

The compound represented by Formula 301 and the compound represented by Formula 302 may include Compounds H1 to H42 illustrated below, but embodiments of the present disclosure are not limited thereto:

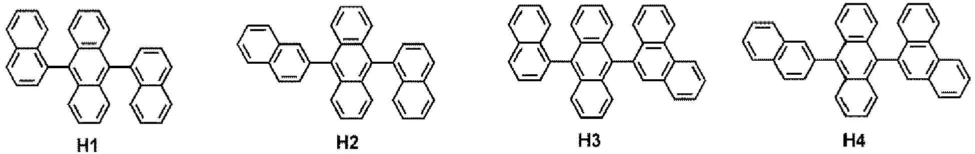

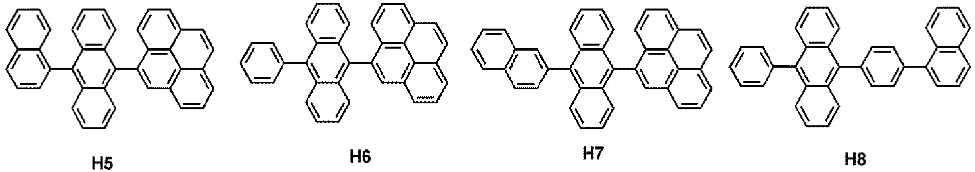

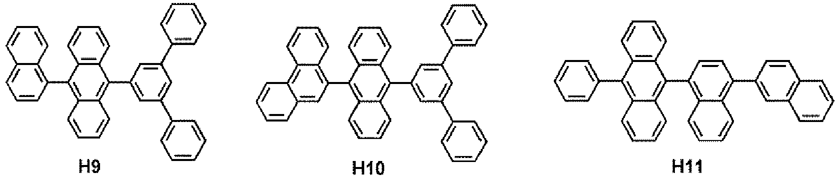

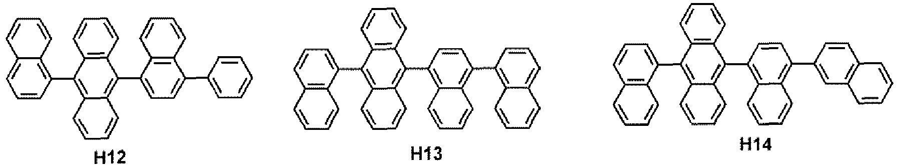

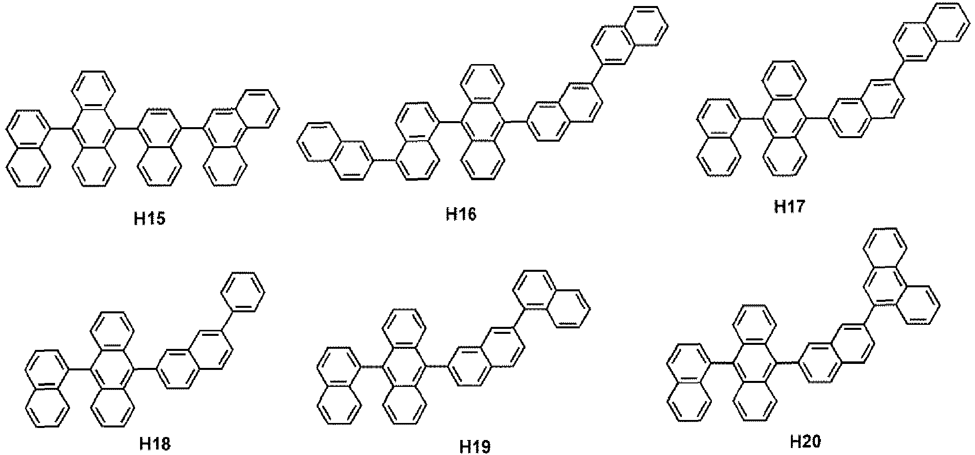

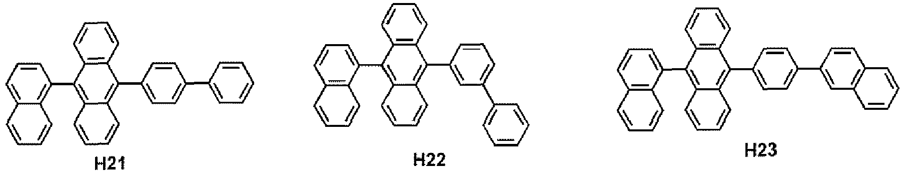

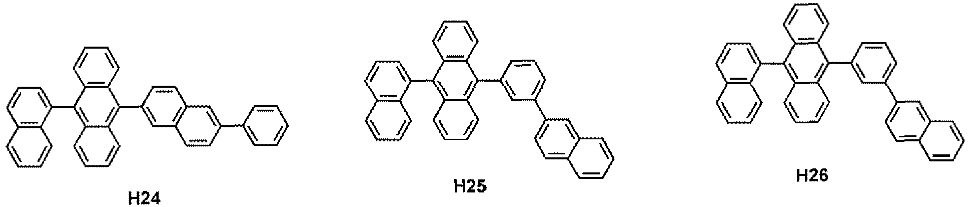

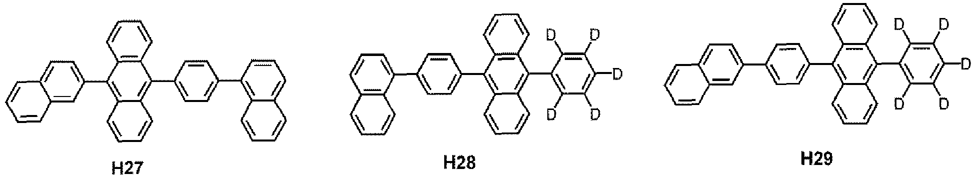

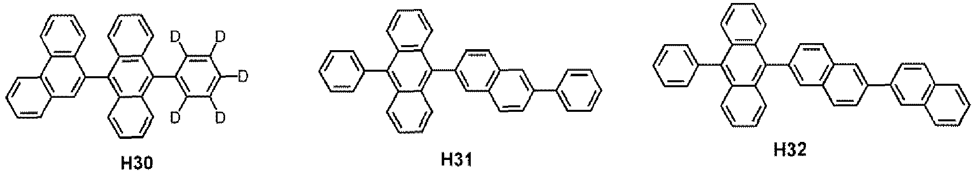

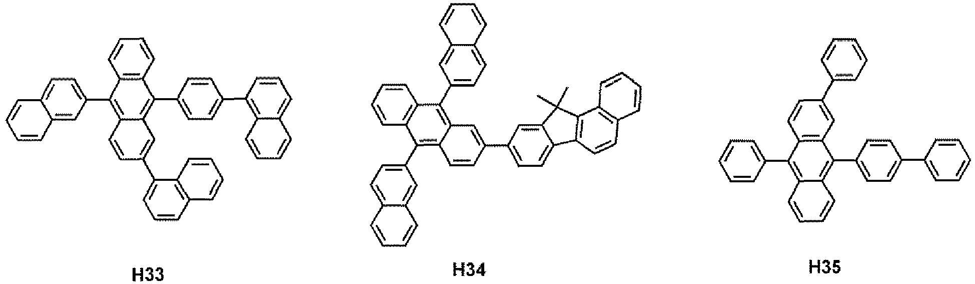

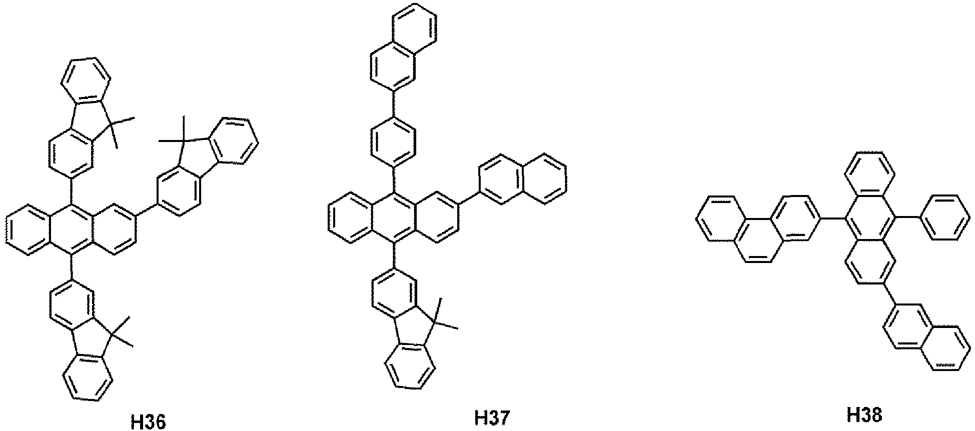

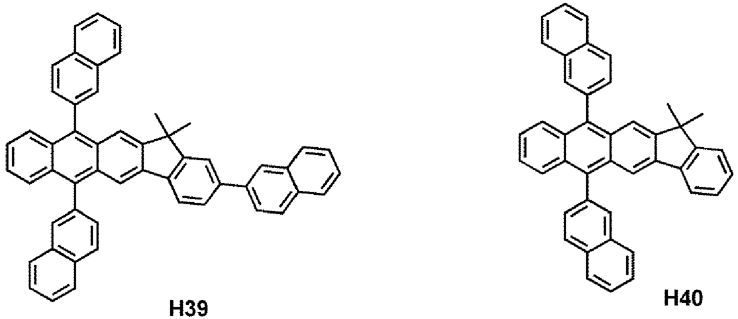

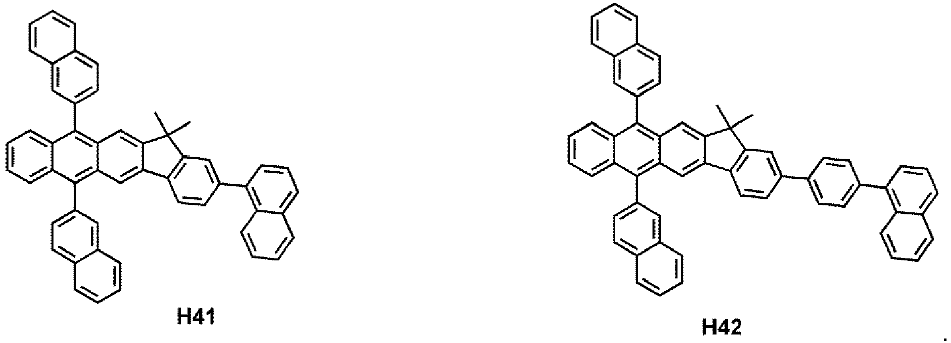

When the organic light-emitting device is a full-color organic light-emitting device, the emission layer may be patterned into a red emission layer, a green emission layer, and a blue emission layer. In one or more embodiments, due to a stack structure including a red emission layer, a green emission layer, and/or a blue emission layer, the emission layer may emit white light.

When the emission layer includes a host and a dopant, an amount of the dopant may be in a range of about 0.01 to about 15 parts by weight based on 100 parts by weight of the host, but embodiments of the present disclosure are not limited thereto.

A thickness of the emission layer may be in a range of about 10 nm to about 100 nm (about 100 Å to about 1,000 Å), for example, about 20 nm to about 60 nm (about 200 Å to about 600 Å). When the thickness of the emission layer is within this range, excellent light-emission characteristics may be obtained without a substantial increase in driving voltage.

Then, an electron transport region may be disposed on the emission layer.

The electron transport region may include at least one selected from a hole blocking layer, an electron transport layer, and an electron injection layer.

For example, the electron transport region may have a structure of hole blocking layer/electron transport layer/electron injection layer or a structure of electron transport layer/electron injection layer, but the structure of the electron transport region is not limited thereto. The electron transport layer may have a single-layered structure or a multi-layered structure including two or more different materials.

Conditions for forming the hole blocking layer, the electron transport layer, and the electron injection layer which constitute the electron transport region may be understood by referring to the conditions for forming the hole injection layer.

When the electron transport region includes a hole blocking layer, the hole blocking layer may include, for example, at least one of BCP, Bphen, and BAlq but embodiments of the present disclosure are not limited thereto.

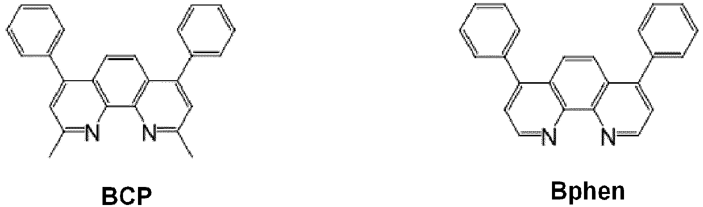

A thickness of the hole blocking layer may be in a range of about 2 nm to about 100 nm (about 20 Å to about 1,000 Å), for example, about 3 nm to about 30 nm (about 30 Å to about 300 Å). When the thickness of the hole blocking layer is within these ranges, the hole blocking layer may have improved hole blocking ability without a substantial increase in driving voltage.

The electron transport layer may further include at least one selected from BCP, Bphen, Alq₃, BAlq, TAZ, and NTAZ.

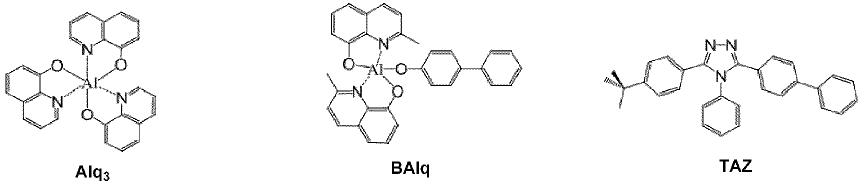

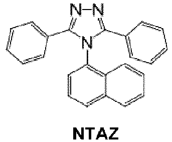

In one or more embodiments, the electron transport layer may include at least one of ET1 and ET2, but embodiments of the present disclosure are not limited thereto:

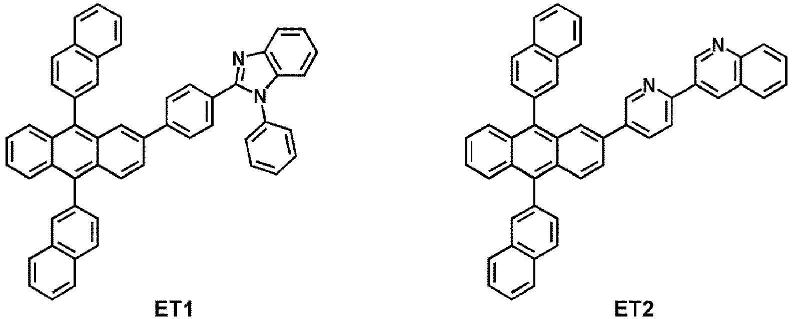

A thickness of the electron transport layer may be in a range of about 10 nm to about 100 nm (about 100 Å to about 1,000 Å), for example, about 15 nm to about 50 nm (about 150 Å to about 500 Å). When the thickness of the electron transport layer is within the range described above, the electron transport layer may have satisfactory electron transport characteristics without a substantial increase in driving voltage.

Also, the electron transport layer may further include, in addition to the materials described above, a metal-containing material.

The metal-containing material may include a Li complex. The Li complex may include, for example, Compound ET-D1 (lithium quinolate, LiQ) or ET-D2:

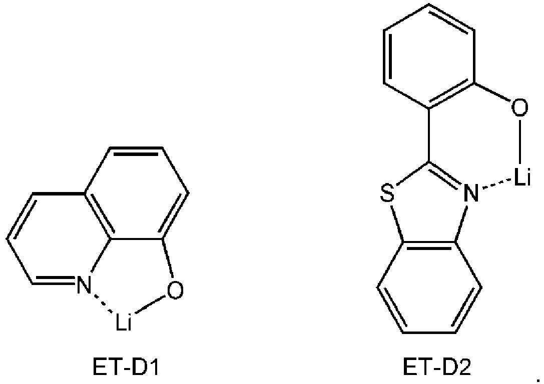

The electron transport region may include an electron injection layer (EIL) that promotes flow of electrons from the second electrode 19 thereinto.

The electron injection layer may include at least one selected from, LiF, NaCl, CsF, Li₂O, BaO, and LiQ.

A thickness of the electron injection layer may be in a range of about 0.1 nm to about 10 nm (about 1 Å to about 100 Å), for example, about 0.3 nm to about 9 nm (about 3 Å to about 90 Å). When the thickness of the electron injection layer is within the range described above, the electron injection layer may have satisfactory electron injection characteristics without a substantial increase in driving voltage.

The second electrode 19 is disposed on the organic layer 15. The second electrode 19 may be a cathode. A material for forming the second electrode 19 may be selected from metal, an alloy, an electrically conductive compound, and a combination thereof, which have a relatively low work function. For example, lithium (Li), magnesium (Mg), aluminum (Al), aluminum-lithium (Al-Li), calcium (Ca), magnesium-indium (Mg-In), or magnesium-silver (Mg-Ag) may be used as a material for forming the second electrode 19. In one or more embodiments, to manufacture a top emission type light-emitting device, a transmissive electrode formed using ITO or IZO may be used as the second electrode 19.

Hereinbefore, the organic light-emitting device has been described with reference to FIG. 1, but embodiments of the present disclosure are not limited thereto.

Another aspect of the present disclosure provides composition including at least one organometallic compound represented by Formula 1 for use in diagnosis. Diagnosis in the sense of the invention refers to both *in vivo* and *in vitro* diagnosis.

The organometallic compound represented by Formula 1 provides high luminescent efficiency. Accordingly, a composition for use in diagnosis including the organometallic compound may have high diagnosis efficiency.

The composition for diagnosis may be used in, for example, various diagnosis kits, diagnosis reagents, bio-sensors, or bio-markers.

The term C₁-C₆₀ alkyl group used herein refers to a linear or branched aliphatic saturated hydrocarbon monovalent group having 1 to 60 carbon atoms, and examples thereof include a methyl group, an ethyl group, a propyl group, an isobutyl group, a sec-butyl group, a tert-butyl group, a pentyl group, an iso-amyl group, and a hexyl group. The term "C₁-C₆₀ alkylene group" as used herein refers to a divalent group having the same structure as the C₁-C₆₀ alkyl group.

The term "C₁-C₆₀ alkoxy group" as used herein refers to a monovalent group represented by -OA₁₀₁ (wherein A₁₀₁ is the C₁-C₆₀ alkyl group), and non-limiting examples thereof include a methoxy group, an ethoxy group, and an isopropyloxy group.

The term "C₂-C₆₀ alkenyl group" as used herein refers to a hydrocarbon group formed by substituting at least one carbon-carbon double bond in the middle or at the terminus of the C₂-C₆₀ alkyl group, and examples thereof include an ethenyl group, a propenyl group, and a butenyl group. The term "C₂-C₆₀ alkenylene group" as used herein refers to a divalent group having the same structure as the C₂-C₆₀ alkenyl group.

The term "C₂-C₆₀ alkynyl group" as used herein refers to a hydrocarbon group formed by substituting at least one carbon-carbon triple bond in the middle or at the terminus of the C₂-C₆₀ alkyl group, and examples thereof include an ethynyl group, and a propynyl group. The term "C₂-C₆₀ alkynylene group" as used herein refers to a divalent group having the same structure as the C₂-C₆₀ alkynyl group.

The term "C₃-C₁₀ cycloalkyl group" as used herein refers to a monovalent saturated hydrocarbon monocyclic group having 3 to 10 carbon atoms, and non-limiting examples thereof include a cyclopropyl group, a cyclobutyl group, a cyclopentyl group, a cyclohexyl group, and a cycloheptyl group. The term "C₃-C₁₀ cycloalkylene group" as used herein refers to a divalent group having the same structure as the C₃-C₁₀ cycloalkyl group.

The term "C₁-C₁₀ heterocycloalkyl group" used herein refers to a monovalent saturated monocyclic group having at least one heteroatom selected from N, O, P, and S as a ring-forming atom and 1 to 10 carbon atoms, and examples thereof include a tetrahydrofuranyl group and a tetrahydrothiophenyl group. The term "C₁-C₁₀ heterocycloalkylene group" as used herein refers to a divalent group having the same structure as the C₁-C₁₀ heterocycloalkyl group.

The term "C₃-C₁₀ cycloalkenyl group" as used herein refers to a monovalent monocyclic group that has 3 to 10 carbon atoms and at least one carbon-carbon double bond in the ring thereof and does not have aromaticity, and non-limiting examples thereof include a cyclopentenyl group, a cyclohexenyl group, and a cycloheptenyl group. The term "C₃-C₁₀ cycloalkenylene group" as used herein refers to a divalent group having the same structure as the C₃-C₁₀ cycloalkenyl group.

The term "C₁-C₁₀ heterocycloalkenyl group" as used herein refers to a monovalent monocyclic group that has at least one heteroatom selected from N, O, P, and S as a ring-forming atom, 1 to 10 carbon atoms, and at least one carbon-carbon double bond in its ring. Non-limiting examples of the C₁-C₁₀ heterocycloalkenyl group include a 2,3-dihydrofuranyl group and a 2,3-dihydrothiophenyl group. The term "C₁-C₁₀ heterocycloalkenylene group" as used herein refers to a divalent group having the same structure as the C₁-C₁₀ heterocycloalkenyl group.

The term "C₆-C₆₀ aryl group" as used herein refers to a monovalent group having a carbocyclic aromatic system having 6 to 60 carbon atoms, and a C₆-C₆₀ arylene group used herein refers to a divalent group having a carbocyclic aromatic system having 6 to 60 carbon atoms. Non-limiting examples of the C₆-C₆₀ aryl group include a phenyl group, a naphthyl group, an anthracenyl group, a phenanthrenyl group, a pyrenyl group, and a chrysenyl group. When the C₆-C₆₀ aryl group and the C₆-C₆₀ arylene group each include two or more rings, the rings may be fused to each other.

The term "C₁-C₆₀ heteroaryl group" as used herein refers to a monovalent group having a carbocyclic aromatic system that has at least one heteroatom selected from N, O, P, and S as a ring-forming atom, and 1 to 60 carbon atoms. A C₁-C₆₀ heteroarylene group used herein refers to a divalent group having a heterocyclic aromatic system that has at least one heteroatom selected from N, O, P, and S as a ring-forming atom, and 1 to 60 carbon atoms. Non-limiting examples of the C₁-C₆₀ heteroaryl group include a pyridinyl group, a pyrimidinyl group, a pyrazinyl group, a pyridazinyl group, a triazinyl group, a quinolinyl group, and an isoquinolinyl group. When the C₁-C₆₀ heteroaryl group and the C₁-C₆₀ heteroarylene group each include two or more rings, the rings may be fused to each other.

The term "C₆-C₆₀ aryloxy group", as used herein indicates -OA₁₀₂ (wherein A₁₀₂ is the C₆-C₆₀ aryl group), and a C₆-C₆₀ arylthio group used herein indicates -SA₁₀₃ (wherein A₁₀₃ is the C₆-C₆₀ aryl group).

The term "monovalent non-aromatic condensed polycyclic group" as used herein refers to a monovalent group (for example, having 8 to 60 carbon atoms) that has two or more rings condensed to each other, only carbon atoms as a ring-forming atom, and non-aromaticity in the entire molecular structure. Examples of the monovalent non-aromatic condensed polycyclic group include a fluorenyl group. The term "divalent non-aromatic condensed polycyclic group", as used herein, refers to a divalent group having the same structure as the monovalent non-aromatic condensed polycyclic group.

The term "monovalent non-aromatic condensed heteropolycyclic group", as used herein refers to a monovalent group (for example, having 2 to 60 carbon atoms) that has two or more rings condensed to each other, has a heteroatom selected from N, O, P, and S, other than carbon atoms, as a ring-forming atom, and has non-aromaticity in the entire molecular structure. Non-limiting examples of the monovalent non-aromatic condensed heteropolycyclic group include a carbazolyl group. The term "divalent non-aromatic condensed heteropolycyclic group", as used herein, refers to a divalent group having the same structure as the monovalent non-aromatic condensed heteropolycyclic group.

At least one of substituents of the substituted C₅-C₃₀ carbocyclic group, the substituted C₂-C₃₀ heterocyclic group, the substituted C₁-C₆₀ alkyl group, the substituted C₂-C₆₀ alkenyl group, the substituted C₂-C₆₀ alkynyl group, the substituted C₁-C₆₀ alkoxy group, the substituted C₃-C₁₀ cycloalkyl group, the substituted C₁-C₁₀ heterocycloalkyl group, the substituted C₃-C₁₀ cycloalkenyl group, the substituted C₁-C₁₀ heterocycloalkenyl group, the substituted C₆-C₆₀ aryl group, the substituted C₆-C₆₀ aryloxy group, the substituted C₆-C₆₀ arylthio group, the substituted C₁-C₆₀ heteroaryl group, the substituted monovalent non-aromatic condensed polycyclic group, and the substituted monovalent non-aromatic condensed heteropolycyclic group may be selected from:
deuterium, -F, -Cl, -Br, -I, -CD₃, -CD₂H, -CDH₂, -CF₃, -CF₂H, -CFH₂, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a C₁-C₆₀ alkyl group, a C₂-C₆₀ alkenyl group, a C₂-C₆₀ alkynyl group, and a C₁-C₆₀ alkoxy group;
a C₁-C₆₀ alkyl group, a C₂-C₆₀ alkenyl group, a C₂-C₆₀ alkynyl group, and a C₁-C₆₀ alkoxy group, each substituted with at least one selected from deuterium, -F, -Cl, -Br, -I, -CD₃, -CD₂H, -CDH₂, -CF₃, -CF₂H, -CFH₂, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a C₃-C₁₀ cycloalkyl group, a C₁-C₁₀ heterocycloalkyl group, a C₃-C₁₀ cycloalkenyl group, a C₁-C₁₀ heterocycloalkenyl group, a C₆-C₆₀ aryl group, a C₆-C₆₀ aryloxy group, a C₆-C₆₀ arylthio group, a C₁-C₆₀ heteroaryl group, a monovalent non-aromatic condensed polycyclic group, a monovalent non-aromatic condensed heteropolycyclic group, -N(Q₁₁)(Q₁₂), -Si(Q₁₃)(Q₁₄)(Q₁₅), -B(Q₁₆)(Q₁₇), and -P(=O)(Q₁₈)(Q₁₉);
a C₃-C₁₀ cycloalkyl group, a C₁-C₁₀ heterocycloalkyl group, a C₃-C₁₀ cycloalkenyl group, a C₁-C₁₀ heterocycloalkenyl group, a C₆-C₆₀ aryl group, a C₆-C₆₀ aryloxy group, a C₆-C₆₀ arylthio group, a C₁-C₆₀ heteroaryl group, a monovalent non-aromatic condensed polycyclic group, and a monovalent non-aromatic condensed heteropolycyclic group;
a C₃-C₁₀ cycloalkyl group, a C₁-C₁₀ heterocycloalkyl group, a C₃-C₁₀ cycloalkenyl group, a C₁-C₁₀ heterocycloalkenyl group, a C₆-C₆₀ aryl group, a C₆-C₆₀ aryloxy group, a C₆-C₆₀ arylthio group, a C₁-C₆₀ heteroaryl group, a monovalent non-aromatic condensed polycyclic group, and a monovalent non-aromatic condensed heteropolycyclic group, each substituted with at least one selected from deuterium, -F, -Cl, -Br, -I, -CD₃, -CD₂H, -CDH₂, -CF₃, -CF₂H, -CFH₂, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a C₁-C₆₀ alkyl group, a C₂-C₆₀ alkenyl group, a C₂-C₆₀ alkynyl group, a C₁-C₆₀ alkoxy group, a C₃-C₁₀ cycloalkyl group, a C₁-C₁₀ heterocycloalkyl group, a C₃-C₁₀ cycloalkenyl group, a C₁-C₁₀ heterocycloalkenyl group, a C₆-C₆₀ aryl group, a C₆-C₆₀ aryloxy group, a C₆-C₆₀ arylthio group, a C₁-C₆₀ heteroaryl group, a monovalent non-aromatic condensed polycyclic group, a monovalent non-aromatic condensed heteropolycyclic group, -N(Q₂₁)(Q₂₂), -Si(Q₂₃)(Q₂₄)(Q₂₅), -B(Q₂₆)(Q₂₇), and -P(=O)(Q₂₈)(Q₂₉); and
-N(Q₃₁)(Q₃₂), -Si(Q₃₃)(Q₃₄)(Q₃₅), -B(Q₃₆)(Q₃₇), and -P(=O)(Q₃₈)(Q₃₉),
wherein Q₁ to Q₉, Q₁₁ to Q₁₉, Q₂₁ to Q₂₉, and Q₃₁ to Q₃₉ may each independently be selected from hydrogen, deuterium, -F, -Cl, -Br, -I, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a C₁-C₆₀ alkyl group, a C₂-C₆₀ alkenyl group, a C₂-C₆₀ alkynyl group, a C₁-C₆₀ alkoxy group, a C₃-C₁₀ cycloalkyl group, a C₁-C₁₀ heterocycloalkyl group, a C₃-C₁₀ cycloalkenyl group, a C₁-C₁₀ heterocycloalkenyl group, a C₆-C₆₀ aryl group, a C₆-C₆₀ aryl group that is substituted with at least one selected from a C₁-C₆₀ alkyl group and a C₆-C₆₀ aryl group, a C₆-C₆₀ aryloxy group, a C₆-C₆₀ arylthio group, a C₁-C₆₀ heteroaryl group, a monovalent non-aromatic condensed polycyclic group, and a monovalent non-aromatic condensed heteropolycyclic group.

Hereinafter, a compound and an organic light-emitting device according to embodiments are described in detail with reference to Synthesis Example and Examples. However, the organic light-emitting device is not limited thereto. The wording "B was used instead of A" used in describing Synthesis Examples refers to that an identical molar equivalent of B was used in place of A.

### [Example]

### Synthesis Example 1: Synthesis of Compound 11

### Synthesis of Intermediate A2

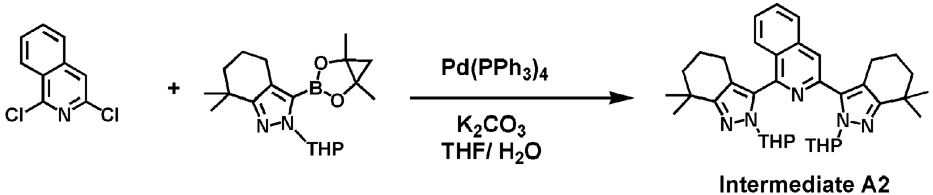

5 g (25.2 mmol) of 1,3-dichloroisoquinoline, 19.12 g (55.5 mmol) of 3-(1,5-dimethyl-2,4-dioxa-3-borabicyclo[3.1.0]hexan-3-yl)-7,7-dimethyl-2-(tetrahydro-2H-pyran-2-yl)-4,5,6,7-tetrahydro-2H-indazole, 2.042 g (1.8 mmol) of Pd(PPh₃)₄, and 6.979 g (50.5 mmol) of K₂CO₃ were mixed together with 60 mL of tetrahydrofuran (THF) and 30 mL of water (H₂O), and then, the mixed solution was stirred at a temperature of 75°C for 18 hours. The obtained solution was cooled to room temperature and then filtered. The filtrate was extracted using methylenechloride (MC). The organic phase was dried using anhydrous magnesium sulfate (MgSO₄) and filteed to obtain a filtrate. A residue obtained by evaporating the filtrate was purified by column chromatography using ethylacetate (EA):hexane = 10:90, to obtain 11.4 g (76%) of Intermediate A2.

### Synthesis of Intermediate A1

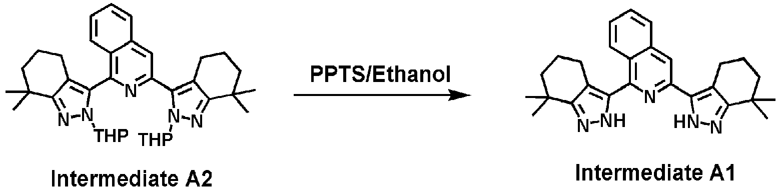

11.4 g (19.2 mmol) of Intermediate A2 and 0.48 g (1.92 mmol) of pyridinium *p*-toluenesulfonate (PPTS) were mixed together with 100 mL of ethyl alcohol (ethanol), and then, the mixed solution was stirred at a temperature of 78°C for 12 hours. The obtained solution was cooled to room temperature and then filtered. The filtrate was extracted using MC. The organic phase was dried using anhydrous magnesium sulfate (MgSO₄) and filtered to obtain a filtrate. A residue obtained by evaporating the filtrate was purified by column chromatography using EA:hexane:methanol = 24:75:1, to obtain 5.72 g (70%) of Intermediate A1.

### Synthesis of Compound 11

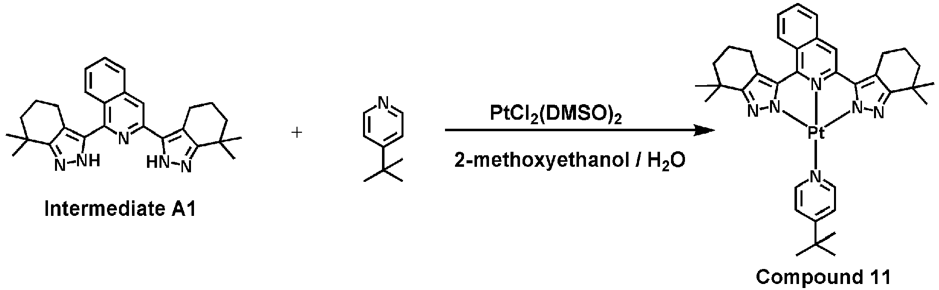

2 g (4.7 mmol) of Intermediate A1, 0.723 ml (4.9 mmol) of tert-butyl pyridine, and 1.98 g (4.7 mmol) of PtCl₂(DMSO)₂ were mixed together with 40 ml of 2-methoxyethanol and 20 ml of water, and then, the mixed solution was stirred at a temperature of 85°C for 24 hours. A reaction was performed thereon and the obtained solution was cooled. The resulting mixture was filtered to obtain a solid. The solid was washed with ethanol. Column chromatography was performed using ethanol:hexane = 20:80, to obtain 1.59 g (40%) of Compound 11. The prepared compound was confirmed by Mass and HPLC analysis.
HRMS(MALDI) calcd for C₃₆H₄₂N₆Pt: m/z 753.3119, Found: 753.3121

### Synthesis Example 2: Synthesis of Compound 8

### Synthesis of Intermediate B2

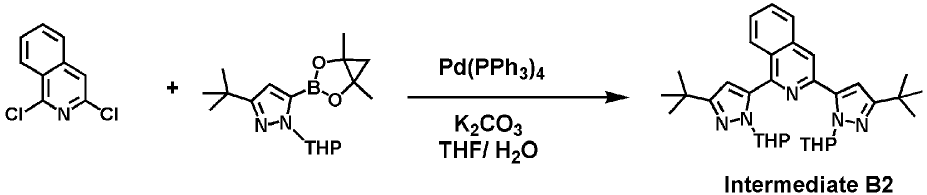

5 g (25.2 mmol) of 1,3-dichloroisoquinoline, 17.67 g (55.5 mmol) of 3-(tert-butyl)-5-(1,5-dimethyl-2,4-dioxa-3-borabicyclo[3.1.0]hexan-3-yl)-1-(tetrahydro-2H-pyran-2-yl)-1H-pyrazole, 2.042 g (1.8 mmol) of Pd(PPh₃)₄, and 6.979 g (50.5 mmol) of K₂CO₃ were mixed together with 60 mL of THF and 30 mL of water (H₂O), and then, the mixed solution was stirred at a temperature of 75°C for 18 hours. The obtained solution was cooled to room temperature and then filtered. The filtrate was extracted using MC. The organic phase was dried using anhydrous magnesium sulfate (MgSO₄) and filtered to obtain a filtrate. A residue obtained by evaporating the filtrate was purified by column chromatography using EA:hexane = 10:90, to obtain 9.7 g (71%) of Intermediate B2.

### Synthesis of Intermediate B1

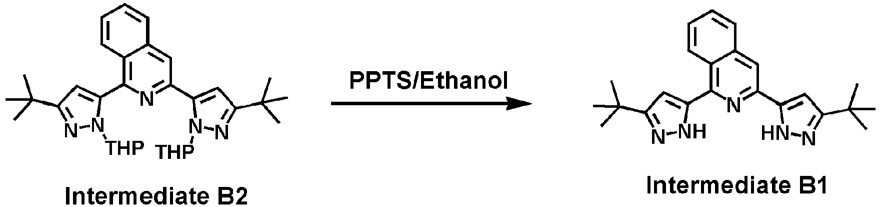

9.7 g (17.9 mmol) of Intermediate B2 and 0.45 g (1.79 mmol) of PPTS were mixed together with 100 mL of ethyl alcohol (ethanol), and then, the mixed solution was stirred at a temperature of 78°C for 12 hours. The obtained solution was cooled to room temperature and then filtered. The filtrate was extracted using MC. The organic phase was dried using anhydrous magnesium sulfate (MgSO₄) and filtered to obtain a filtrate. A residue obtained by evaporating the filtrate was purified by column chromatography using EA:hexane:methanol = 24:75:1, to obtain 4.55 g (68%) of Intermediate B1.

### Synthesis of Compound 8

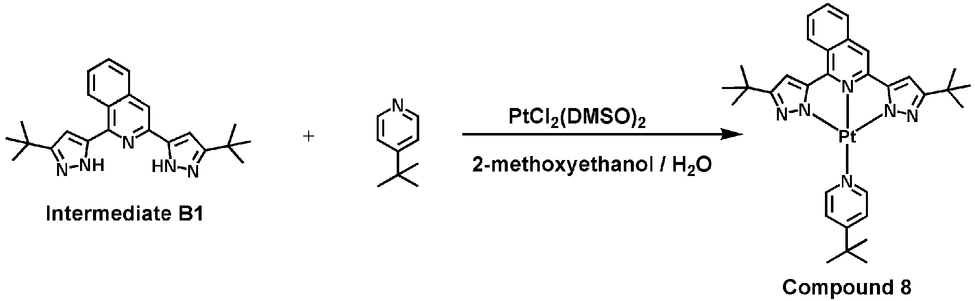

2 g (5.35 mmol) of Intermediate B1, 0.824 ml (5.6 mmol) of tert-butyl pyridine, and 2.26 g (5.35 mmol) of PtCl₂(DMSO)₂ were mixed together with 40 ml of 2-methoxyethanol and 20 ml of water, and then, the mixed solution was stirred at a temperature of 85°C for 24 hours. The obtained solution was cooled and filtered to obtain a solid. The solid was washed with ethanol and purified by column chromatography using ethanol:hexane = 20:80, to obtain 1.58 g (42%) of Compound 8. The obtained compound was confirmed by Mass and HPLC analysis.
HRMS(MALDI) calcd for C₃₂H₃₈N₆Pt: m/z 701.2806, Found: 701.2807

### Synthesis Example 3: Synthesis of Compound 139

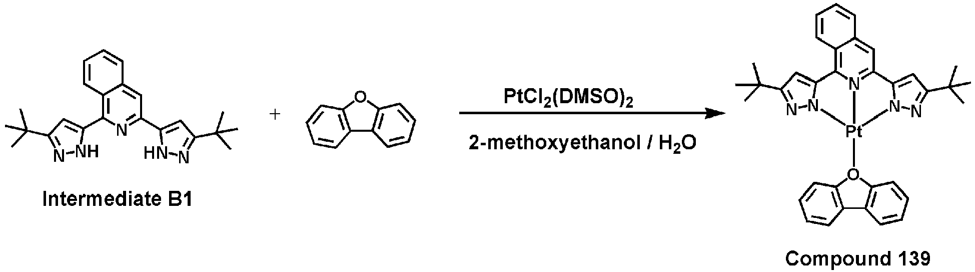

1.38 g (35%) of Compound 139 was obtained in the same manner as in Synthesis of Compound 11 of Synthesis Example 1, except that 2 g (5.35 mmol) of Intermediate B1 and 0.95 g (5.62 mmol) of dibenzofuran were used instead of Intermediate A1 and tert-butyl pyridine in synthesizing Compound 11. The obtained compound was confirmed by Mass and HPLC analysis.
HRMS(MALDI) calcd for C₃₅H₃₃N₅OPt : m/z 734.2333, Found: 734.2335

### Synthesis Example 4: Synthesis of Compound 140

### Synthesis of Intermediate C3

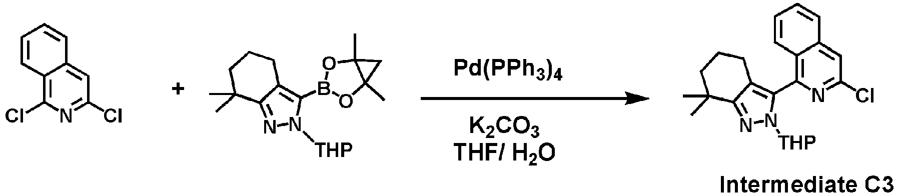

5 g (25.2 mmol) of 1,3-dichloroisoquinoline, 9.560 g (27.8 mmol) of 3-(1,5-dimethyl-2,4-dioxa-3-borabicyclo[3.1.0]hexan-3-yl)-7,7-dimethyl-2-(tetrahydro-2H-pyran-2-yl)-4,5,6,7-tetrahydro-2H-indazole, 2.042 g (1.8 mmol) of Pd(PPh₃)₄, and 6.979 g (50.5 mmol) of K₂CO₃ were mixed together with 60 mL of THF and 30 mL of water (H₂O), and then, the mixed solution was stirred at a temperature of 75°C for 18 hours. The obtained solution was cooled to room temperature and then filtered. The filtrate was extracted using MC. The organic phase was dried using anhydrous magnesium sulfate (MgSO₄) and filtered to obtain a filtrate. A residue obtained by evaporating the filtrate was purified by column chromatography under a condition of EA:hexane = 10:90, to obtain of 6.3 g (63%) of Intermediate C3.

### Synthesis of Intermediate C2

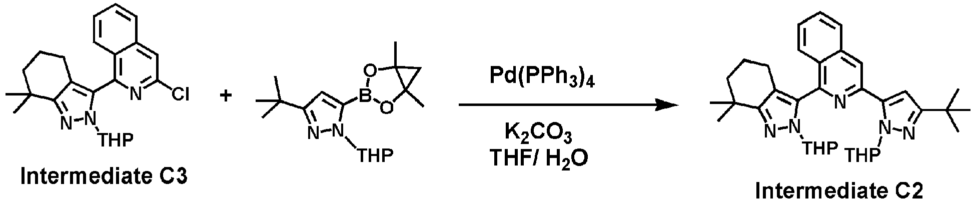

6.3 g (15.9 mmol) of Intermediate C3, 5.57 g (17.5 mmol) of 3-(tert-butyl)-5-(1,5-dimethyl-2,4-dioxa-3-borabicyclo[3.1.0]hexan-3-yl)-1-(tetrahydro-2H-pyran-2-yl)-1H-pyrazole, 1.29 g (1.11 mol) of Pd(PPh₃)₄, and 4.40 g (31.8 mmol) of K₂CO₃ were mixed together with 60 mL of THF and 30 mL of water (H₂O), and then, the mixed solution was stirred at a temperature of 75°C for 18 hours. The obtained solution was cooled to room temperature and then filtered. The filtrate was extracted using MC. The organic phase was dried using anhydrous magnesium sulfate (MgSO₄) and filtered to obtain a filtrate. A residue obtained by evaporating the filtrate was purified by column chromatography using EA:hexane = 10:90, to obtain 7.2 g (80%) of Intermediate C2.

### Synthesis of Intermediate C1

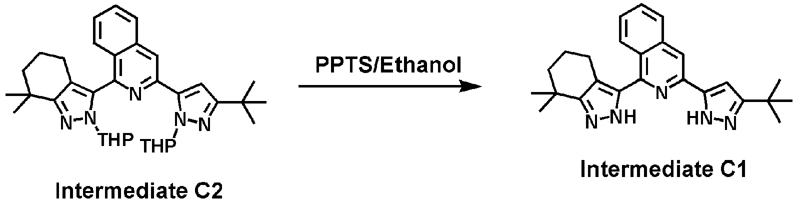

7.2 g (12.7 mmol) of Intermediate C2 and 0.32 g (1.27 mmol) of PPTS were mixed together with 100 mL of ethyl alcohol (ethanol), and then, the mixed solution was stirred at a temperature of 78°C for 12 hours. The obtained solution was cooled to room temperature and then filtered. The filtrate was extracted using MC. The organic phase was dried using anhydrous magnesium sulfate (MgSO₄) and filtered to obtain a filtrate. A residue obtained by evaporating the filtrate was purified by column chromatography using EA:hexane:methanol = 24:75:1, to obtain 3.69 g (73%) of Intermediate C1.

### Synthesis of Compound 140

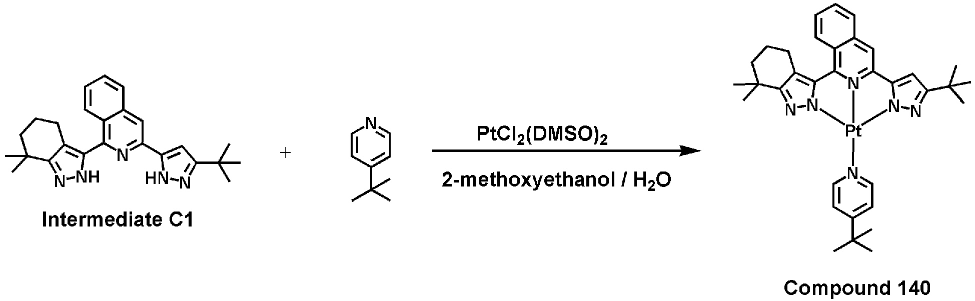

1.09 g (30%) of Compound 140 was obtained in the same manner in Synthesis of Compound 11 of Synthesis Example 1, except that 2.0 g (5.00 mmol) of Intermediate C1 was used instead of Intermediate A1 in synthesizing Compound 11. The obtained compound was confirmed by Mass and HPLC analysis.
HRMS(MALDI) calcd for C₃₄H₄₀N₆Pt: m/z 727.2962, Found: 727.2960

### Synthesis Example 5: Synthesis of Compound 5

### Synthesis of Intermediate D2

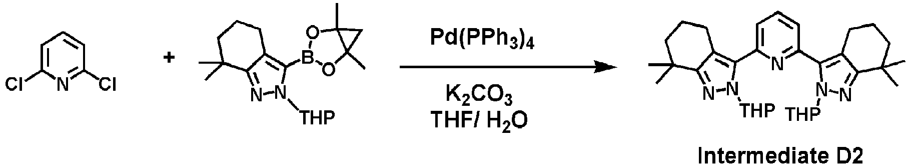

3 g (20.3 mmol) of 1,3-dichloropyridine, 15.35 g (44.6 mmol) of 3-(1,5-dimethyl-2,4-dioxa-3-borabicyclo[3.1.0]hexan-3-yl)-7,7-dimethyl-2-(tetrahydro-2H-pyran-2-yl)-4,5,6,7-tetrahydro-2H-indazole, 1.640 g (1.4 mmol) of Pd(PPh₃)₄, and 5.603 g (40.5 mmol) of K₂CO₃ were mixed together with 60 mL of THF and 30 mL of water (H₂O), and then, the mixed solution was stirred at a temperature of 75°C for 18 hours. The obtained solution was cooled to room temperature and then filtered. The filtrate was extracted using MC. The organic phase was dried using anhydrous magnesium sulfate (MgSO₄) and filtered to obtain a filtrate. A residue obtained by evaporating the filtrate was purified by column chromatography using EA:hexane = 10:90, to obtain 7.72 g (70%) of Intermediate D2.

### Synthesis of Intermediate D1

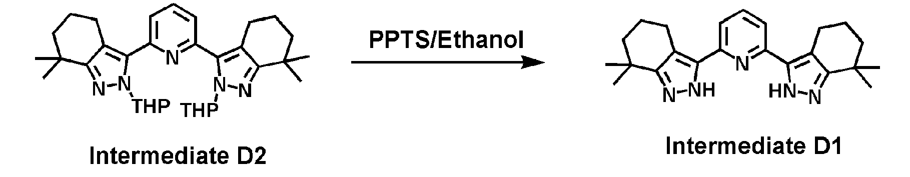

7.72 g (14.2 mmol) of Intermediate D2 and 0.15 g (1.42 mmol) of PPTS were mixed together with 100 mL of ethyl alcohol (ethanol), and then, the mixed solution was stirred at a temperature of 78°C for 12 hours. The obtained solution was cooled to room temperature and then filtered. The filtrate was extracted using MC. The organic phase was driedusing anhydrous magnesium sulfate (MgSO₄) and filtered to obtain a filtrate. A residue obtained by evaporating the filtrate was purified by column chromatography using EA:hexane:methanol = 24:75:1, to obtain 3.31 g (62%) of Intermediate D1.

### Synthesis of Compound 5

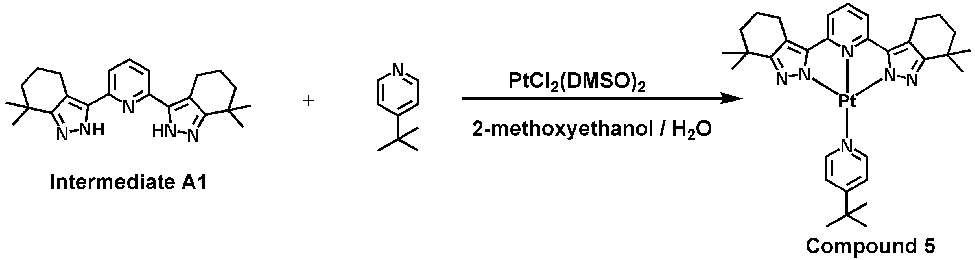

1.50 g (40%) of Compound 5 was obtained in the same manner as in Synthesis of Compound 11 of Synthesis Example 1, except that 2.0 g (5.33 mmol) of Intermediate D1 was used instead of Intermediate A1 in synthesizing Compound 11. The obtained compound was confirmed by Mass and HPLC analysis.
HRMS(MALDI) calcd for C₃₂H₄₀N₆Pt: m/z 703.2962, Found: 703.2963

### Synthesis Example 6: Synthesis of Compound 2

### Synthesis of Intermediate E2

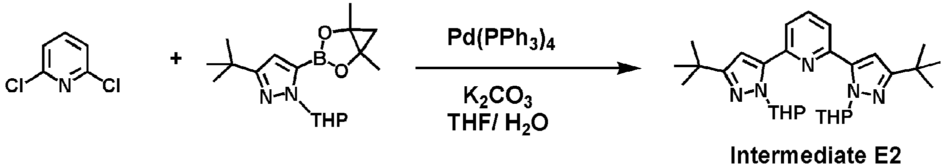

3 g (20.3 mmol) of 1,3-dichloropyridine, 14.18 g (44.6 mmol) of 3-(tert-butyl)-5-(1,5-dimethyl-2,4-dioxa-3-borabicyclo[3.1.0]hexan-3-yl)-1-(tetrahydro-2H-pyran-2-yl)-1H-pyrazole, 1.640 g (1.4 mmol) of Pd(PPh₃)₄, and 5.603 g (40.5 mmol) of K₂CO₃ were mixed together with 60 mL of THF and 30 mL of water (H₂O), and then, the mixed solution was stirred at a temperature of 75°C for 18 hours. The obtained solution was cooled to room temperature and then filtered. The filtrate was extracted using MC. The organic phase was dried using anhydrous magnesium sulfate (MgSO₄) and filtered to obtain a filtrate. A residue obtained by evaporating the filtrate was purified by column chromatography using EA:hexane = 10:90, to obtain 6.78 g (68%) of Intermediate E2.

### Synthesis of Intermediate E1

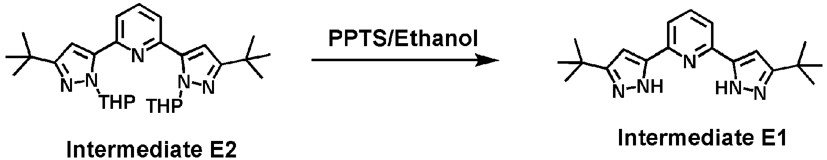

6.78 g (12.7 mmol) of Intermediate E2 and 0.32 g (1.27 mmol) of PPTS were mixed together with 100 mL of ethyl alcohol (ethanol), and then, the mixed solution was stirred at a temperature of 78°C for 12 hours. The obtained solution was cooled to room temperature and then filtered. The filtrate was extracted from the obtained product by using MC. The organic phase was dried using anhydrous magnesium sulfate (MgSO₄) and filtered to obtain a filtrate. A residue obtained by evaporating the filtrate was purified by column chromatography using EA:hexane:methanol = 24:75:1, to obtain 2.72 g (61%) of Intermediate E1.

### Synthesis of Compound 2

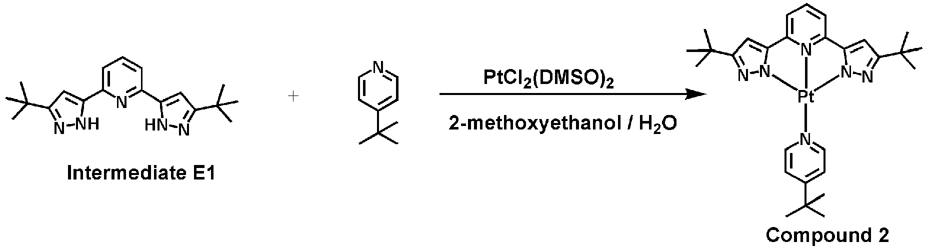

1.19 g (29%) of Compound 2 was obtained in the same manner as in Compound 11 of Synthesis Example 1, except that 2.0 g (6.18 mmol) of Intermediate E1 were used instead of Intermediate A1 in synthesizing Compound 11. The obtained compound was confirmed by Mass and HPLC analysis.
HRMS(MALDI) calcd for C₂₈H₃₆N₆Pt: m/z 651.2649, Found: 651.2650

### Synthesis Example 7 : Synthesis of Compound 141

### Synthesis of Intermediate F2

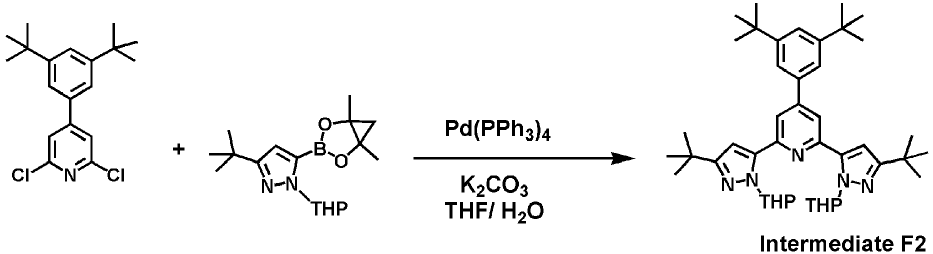

6.47 g (80%) of Intermediate F2 was obtained in the same manner as in Synthesis of Intermediate E2 of Synthesis Example 6, except that 4.0 g (11.6 mmol) of 2,6-dichloro-4-(3,5-di-tert-butylphenyl)pyridine was used instead of 3 g (20.3 mmol) of 1,3-dichloropyridine.

### Synthesis of Intermediate F1

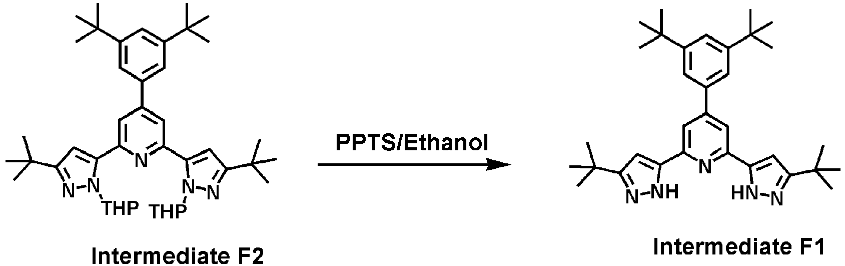

6.47 g (9.51 mmol) of Intermediate F2 and 0.24 g (0.95 mmol) of PPTS were mixed together with 100 mL of ethyl alcohol (ethanol), and then, the mixed solution was stirred at a temperature of 78°C for 12 hours. The obtained solution was cooled to room temperature and then filtered. The filtrate was extracted using MC. The organic phase was dried using anhydrous magnesium sulfate (MgSO₄) and filtered to obtain a filtrate. A residue obtained by evaporating the filtrate was purified by column chromatography using EA:hexane:methanol = 24:75:1, to obtain 4.09 g (84%) of Intermediate F1.

### Synthesis of Compound 141

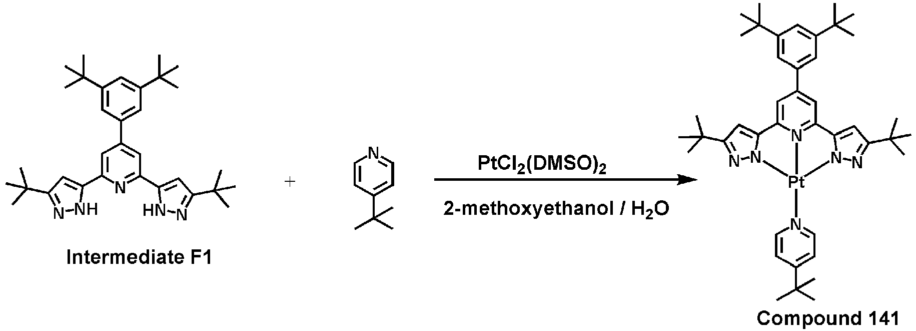

1.08 g (33%) of Compound 141 was obtained in the same manner as in Synthesis of Compound 11 of Synthesis Example 1, except that 2.0 g (3.91 mmol) of Intermediate F1 was used instead of Intermediate A1 in synthesizing Compound 11. The obtained compound was confirmed by Mass and HPLC analysis.
HRMS(MALDI) calcd for C₄₂H₅₆N₆Pt: m/z 839.4214, Found: 839.4212

### Synthesis Example 8 : Synthesis of Compound 142

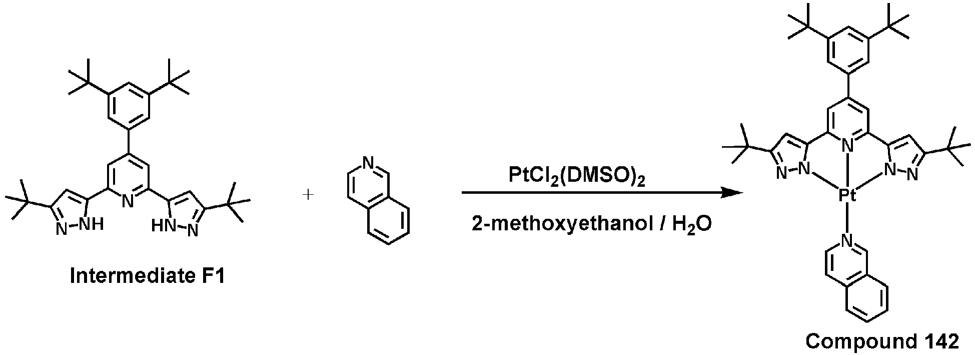

Compound 142 was obtained in the same manner as in Synthesis of Compound 141 of Synthesis Example 7, except that isoquinoline was used instead of tert-butyl pyridine in synthesizing Compound 141. The obtained compound was confirmed by Mass and HPLC analysis.
HRMS(MALDI) calcd for C₄₂H₅₀N₆Pt: m/z 833.3745, Found: 833.3746

### Example 1

An ITO glass substrate, on which an ITO electrode (anode) was deposited, was cut to a size of 50 mm x 50 mm x 0.5 mm, ultrasonically cleaned using acetone, isopropyl alcohol, and pure water each for 15 minutes, and then, exposed to irradiation of UV light for 30 minutes and ozone to clean.

Then, m-MTDATA was deposited on the ITO electrode (anode) at a deposition rate of 0.1 nm/sec (1 Å /sec) to form a hole injection layer having a thickness of 60 nm (600 Å), and α-NPD was deposited on the hole injection layer at a deposition rate of 0.1 nm/sec (1 Å/sec) to form a hole transport layer having a thickness of 25 nm (250 Å).

Compound 5 (as a dopant) and CBP (as a host) were respectively co-deposited on the hole transport layer at a deposition rate of 0.01 nm/sec (0.1 Å/sec) and a deposition rate of 0.1 nm/sec (1 Å/sec) to form an emission layer having a thickness of 40nm (400 Å).

BAlq was deposited on the emission layer at a deposition rate of 0.1 nm/sec (1 Å/sec) to form a hole blocking layer having a thickness of 5 nm (50 Å), and Alq₃ was deposited on the hole blocking layer to form an electron transport layer having a thickness of 30 nm (300 Å). Then, LiF was deposited on the electron transport layer to form an electron injection layer having a thickness of 1 nm (10 Å). Al was vacuum-deposited on the electron injection layer to form a second electrode (cathode) having a thickness of 120 nm (1,200 Å), thereby completing the manufacture of an organic light-emitting device having a structure of ITO/m-MTDATA (60 nm (600 Å))/α-NPD (25 nm (250 Å))/CBP+10% (Compound 5) (40 nm (400 Å))/BAlq (5 nm (50 Å))/Alq₃ (30 nm (300 Å))/LiF (1 nm (10 Å))/Al (120 nm (1,200 Å)).

### Examples 2 to 5 and Comparative Examples 1 and 2

Organic light-emitting devices were manufactured in the same manner as in Example 1, except that in forming an emission layer, for use as a dopant, corresponding compounds shown in Table 2 were used instead of Compound 5.

### Evaluation Example 1: Evaluation on characteristics of organic light-emitting devices.

The driving voltage, luminescent efficiency, power efficiency, color purity, quantum efficiency, and lifespan (T₉₅) of the organic light-emitting devices manufactured in Examples 1 to 5 and Comparative Examples 1 and 2 were evaluated. Results thereof are shown in Table 2. A current-voltage meter (Keithley 2400) and a luminance meter (Minolta Cs-1000A) were used as evaluation devices. The lifespan (T₉₅) (at 6000 nit) was evaluated as a period of time taken until the luminance was reduced to 95% of initial luminance.

**[Table 2]**

| | Dopant | Driving Voltage (V) | Luminescent Efficiency (cd/A) | Power Efficiency (lm/W) | CIEx | CIEy | Quantum Efficiency (%) | Lifespan (hr) (T₉₅) |
|---|---|---|---|---|---|---|---|---|
| Example 1 | Compound 5 | 5.1 | 47.7 | 29.3 | 0.362 | 0.601 | 17.1 | 180 |
| Example 2 | Compound 2 | 5.2 | 45.8 | 28.0 | 0.366 | 0.605 | 16.8 | 185 |
| Example 3 | Compound 141 | 5.5 | 49.9 | 31.0 | 0.360 | 0.600 | 18.8 | 230 |
| Example 4 | Compound 142 | 5.1 | 49.0 | 30.2 | 0.363 | 0.601 | 18.1 | 200 |
| Example 5 | Compound 140 | 5.2 | 48.8 | 29.9 | 0.362 | 0.599 | 18.3 | 190 |
| Comparative Example 1 | Compound A | 5.6 | 33.9 | 21.4 | 0.344 | 0.598 | 12.9 | 85 |
| Comparative Example 2 | Compound C | 5.2 | 34.3 | 22.5 | 0.368 | 0.604 | 13.7 | 100 |

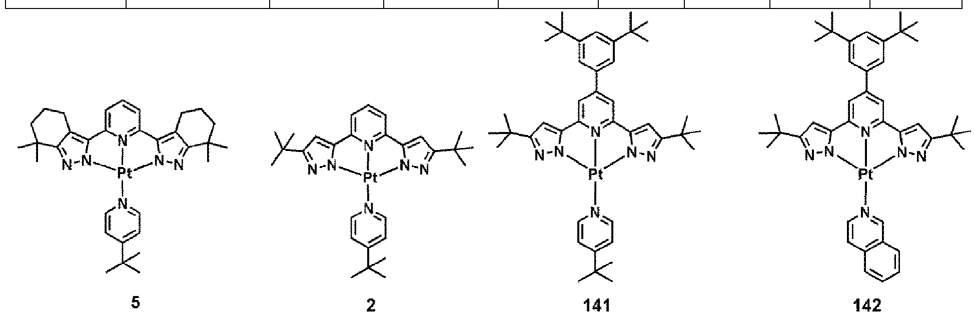

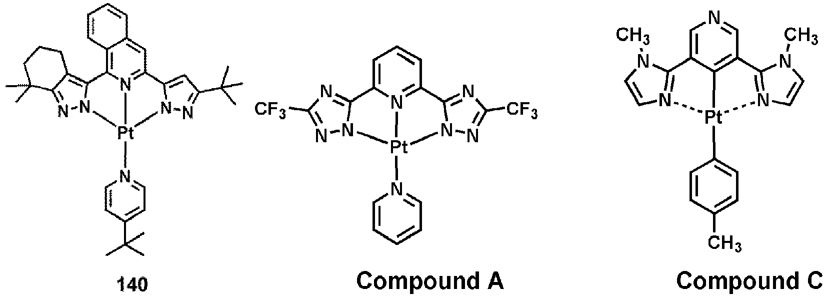

Referring to Table 2, it was confirmed that the organic light-emitting devices of Examples 1 to 5 had excellent driving voltage, luminescent efficiency, power efficiency, color purity, quantum efficiency, and lifespan characteristics, compared to those of the organic light-emitting devices of Comparative Examples 1 and 2.

Since an organometallic compound according to one or more embodiments has excellent electric characteristics and thermal stability, an organic light-emitting device including the organometallic compound may have excellent driving voltage, luminescent efficiency, power efficiency, color purity, quantum efficiency, and lifespan characteristics. In addition, since the organometallic compound has excellent phosphorescent luminescent characteristics, a composition for use in diagnosis having high diagnosis efficiency may be provided by using the organometallic compound.

It should be understood that embodiments described herein should be considered in a descriptive sense only and not for purposes of limitation. Descriptions of features or aspects within each embodiment should typically be considered as available for other similar features or aspects in other embodiments.

While one or more embodiments have been described with reference to the figures, it will be understood by those of ordinary skill in the art that various changes in form and details may be made therein without departing from the scope as defined by the following claims.

## Claims

1. An organometallic compound represented by Formula 1:
<Formula 1> M(L₁)(L₂) 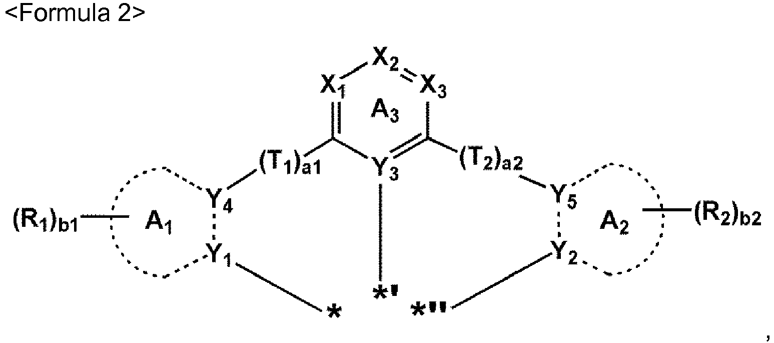
wherein, in Formulae 1 and 2,
M is ruthenium (Ru), rhodium (Rh), palladium (Pd), platinum (Pt) or gold (Au),
L₁ is selected from tridentate ligands represented by Formula 2,
L₂ is selected from monodentate organic ligands,
*, *', and *" in Formula 2 each indicate a binding site to M in Formula 1,
Y₁ to Y₃ are each independently nitrogen (N),
Y₄ and Y₅ are each independently carbon (C),
a bond between Y₁ and Y₄ is a single bond or a double bond, and a bond between Y₂ and Y₅ is a single bond or a double bond,
one selected from a bond between Y₁ and M, a bond between Y₂ and M, and a bond between ligand L₂ and M is a coordinate bond, and the other two are a covalent bond,
a bond between Y₃ and M is a coordinate bond,
rings A₁ and A₂ are each independently selected from a pyrrole ring, a pyrazole ring, and an imidazole ring, and at least one selected from rings A₁ and A₂ is a pyrazole ring
X₁ is N or C(R₃), X₂ is N or C(R₄), X₃ is N or C(R₅), two or more selected from R₃ to R₅ are optionally connected to each other to form a substituted or unsubstituted C₅-C₃₀ carbocyclic group or a substituted or unsubstituted C₂-C₃₀ heterocyclic group, and ring A₃ has two or less nitrogen atoms as a ring-forming atom,
T₁ and T₂ are each independently selected from a single bond, *-O-*', *-S-*', *-C(R₆)(R₇)-*', *-C(R₆)=*', *=C(R₆)-*', *-C(R₆)=C(R₇)-*', *-C(=O)-*', *-C(=S)-*', *-C=C-*', *-N(R₆)-*', and *-Si(R₆)(R₇)-*', and R₆ and R₇ are optionally connected to each other to form a substituted or unsubstituted C₅-C₃₀ carbocyclic group or a substituted or unsubstituted C₂-C₃₀ heterocyclic group,
a1 and a2 are each independently an integer selected from 1 to 3,
R₁ to R₇ are each independently selected from hydrogen, deuterium, -F, -Cl, -Br, -I, -SF₅, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a substituted or unsubstituted C₁-C₆₀ alkyl group, a substituted or unsubstituted C₂-C₆₀ alkenyl group, a substituted or unsubstituted C₂-C₆₀ alkynyl group, a substituted or unsubstituted C₁-C₆₀ alkoxy group, a substituted or unsubstituted C₃-C₁₀ cycloalkyl group, a substituted or unsubstituted C₁-C₁₀ heterocycloalkyl group, a substituted or unsubstituted C₃-C₁₀ cycloalkenyl group, a substituted or unsubstituted C₁-C₁₀ heterocycloalkenyl group, a substituted or unsubstituted C₆-C₆₀ aryl group, a substituted or unsubstituted C₆-C₆₀ aryloxy group, a substituted or unsubstituted C₆-C₆₀ arylthio group, a substituted or unsubstituted C₁-C₆₀ heteroaryl group, a substituted or unsubstituted monovalent non-aromatic condensed polycyclic group, a substituted or unsubstituted monovalent non-aromatic condensed heteropolycyclic group, -N(Q₁)(Q₂), -Si(Q₃)(Q₄)(Q₅), -B(Q₆)(Q₇), and -P(=O)(Q₈)(Q₉),
b1 and b2 are each independently an integer selected from 0 to 3, wherein, when b1 is two or more, two or more R₁(s) are identical to or different from each other, and when b2 is two or more, two or more R₂(s) are identical to or different from each other,
two selected from R₁(s) in the number of b1 are optionally connected to form a substituted or unsubstituted C₅-C₃₀ carbocyclic group or a substituted or unsubstituted C₂-C₃₀ heterocyclic group,
two selected from R₂(s) in the number of b2 are optionally connected to form a substituted or unsubstituted C₅-C₃₀ carbocyclic group or a substituted or unsubstituted C₂-C₃₀ heterocyclic group,
a moiety represented by 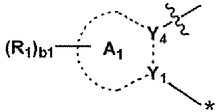 in Formula 2 is not 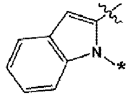 and 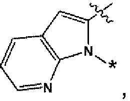 and a moiety represented by 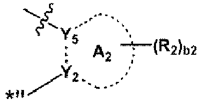 in Formula 2 is not 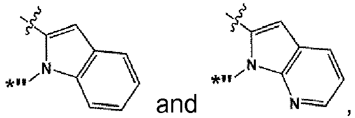 and
at least one substituent selected from a substituent(s) of the substituted C₅-C₃₀ carbocyclic group, the substituted C₂-C₃₀ heterocyclic group, the substituted C₁-C₆₀ alkyl group, the substituted C₂-C₆₀ alkenyl group, the substituted C₂-C₆₀ alkynyl group, the substituted C₁-C₆₀ alkoxy group, the substituted C₃-C₁₀ cycloalkyl group, the substituted C₁-C₁₀ heterocycloalkyl group, the substituted C₃-C₁₀ cycloalkenyl group, substituted C₁-C₁₀ heterocycloalkenyl group, the substituted C₆-C₆₀ aryl group, the substituted C₆-C₆₀ aryloxy group, the substituted C₆-C₆₀ arylthio group, the substituted C₁-C₆₀ heteroaryl group, the substituted monovalent non-aromatic condensed polycyclic group, and the substituted monovalent non-aromatic condensed heteropolycyclic group is selected from:
deuterium, -F, -Cl, -Br, -I, -CD₃, -CD₂H, -CDH₂, -CF₃, -CF₂H, -CFH₂, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a C₁-C₆₀ alkyl group, a C₂-C₆₀ alkenyl group, a C₂-C₆₀ alkynyl group, and a C₁-C₆₀ alkoxy group;
a C₁-C₆₀ alkyl group, a C₂-C₆₀ alkenyl group, a C₂-C₆₀ alkynyl group, and a C₁-C₆₀ alkoxy group, each substituted with at least one selected from deuterium, -F, -Cl, -Br, -I, -CD₃, -CD₂H, -CDH₂, -CF₃, -CF₂H, -CFH₂, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a C₃-C₁₀ cycloalkyl group, a C₁-C₁₀ heterocycloalkyl group, a C₃-C₁₀ cycloalkenyl group, a C₁-C₁₀ heterocycloalkenyl group, a C₆-C₆₀ aryl group, a C₆-C₆₀ aryloxy group, a C₆-C₆₀ arylthio group, a C₁-C₆₀ heteroaryl group, a monovalent non-aromatic condensed polycyclic group, a monovalent non-aromatic condensed heteropolycyclic group, -N(Q₁₁)(Q₁₂), -Si(Q₁₃)(Q₁₄)(Q₁₅), -B(Q₁₆)(Q₁₇), and -P(=O)(Q₁₈)(Q₁₉);
a C₃-C₁₀ cycloalkyl group, a C₁-C₁₀ heterocycloalkyl group, a C₃-C₁₀ cycloalkenyl group, a C₁-C₁₀ heterocycloalkenyl group, a C₆-C₆₀ aryl group, a C₆-C₆₀ aryloxy group, a C₆-C₆₀ arylthio group, a C₁-C₆₀ heteroaryl group, a monovalent non-aromatic condensed polycyclic group, and a monovalent non-aromatic condensed heteropolycyclic group;
a C₃-C₁₀ cycloalkyl group, a C₁-C₁₀ heterocycloalkyl group, a C₃-C₁₀ cycloalkenyl group, a C₁-C₁₀ heterocycloalkenyl group, a C₆-C₆₀ aryl group, a C₆-C₆₀ aryloxy group, a C₆-C₆₀ arylthio group, a C₁-C₆₀ heteroaryl group, a monovalent non-aromatic condensed polycyclic group, and a monovalent non-aromatic condensed heteropolycyclic group, each substituted with at least one selected from deuterium, -F, -Cl, -Br, -I, -CD₃, -CD₂H, -CDH₂, -CF₃, -CF₂H, -CFH₂, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a C₁-C₆₀ alkyl group, a C₂-C₆₀ alkenyl group, a C₂-C₆₀ alkynyl group, a C₁-C₆₀ alkoxy group, a C₃-C₁₀ cycloalkyl group, a C₁-C₁₀ heterocycloalkyl group, a C₃-C₁₀ cycloalkenyl group, a C₁-C₁₀ heterocycloalkenyl group, a C₆-C₆₀ aryl group, a C₆-C₆₀ aryloxy group, a C₆-C₆₀ arylthio group, a C₁-C₆₀ heteroaryl group, a monovalent non-aromatic condensed polycyclic group, a monovalent non-aromatic condensed heteropolycyclic group, -N(Q₂₁)(Q₂₂), -Si(Q₂₃)(Q₂₄)(Q₂₅), -B(Q₂₆)(Q₂₇), and -P(=O)(Q₂₈)(Q₂₉); and
-N(Q₃₁)(Q₃₂), -Si(Q₃₃)(Q₃₄)(Q₃₅), -B(Q₃₆)(Q₃₇), and -P(=O)(Q₃₈)(Q₃₉),
wherein Q₁ to Q₉, Q₁₁ to Q₁₉, Q₂₁ to Q₂₉, and Q₃₁ to Q₃₉ are each independently selected from hydrogen, deuterium, -F, -Cl, -Br, -I, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a C₁-C₆₀ alkyl group, a C₂-C₆₀ alkenyl group, a C₂-C₆₀ alkynyl group, a C₁-C₆₀ alkoxy group, a C₃-C₁₀ cycloalkyl group, a C₁-C₁₀ heterocycloalkyl group, a C₃-C₁₀ cycloalkenyl group, a C₁-C₁₀ heterocycloalkenyl group, a C₆-C₆₀ aryl group, a C₆-C₆₀ aryl group substituted with at least one selected from a C₁-C₆₀ alkyl group and a C₆-C₆₀ aryl group, a C₆-C₆₀ aryloxy group, a C₆-C₆₀ arylthio group, a C₁-C₆₀ heteroaryl group, a monovalent non-aromatic condensed polycyclic group, and a monovalent non-aromatic condensed heteropolycyclic group.

2. The organometallic compound of claim 1, wherein
the bond between Y₁ and M and the bond between Y₂ and M are a covalent bond, and the bond between Y₃ and M and the bond between ligand L₂ and M are a coordinate bond, or
the bond between Y₁ and M and the bond between ligand L₂ and M are a covalent bond, and the bond between Y₃ and M and the bond between Y₂ and M are a coordinate bond.

3. The organometallic compound of claim 1 or 2, wherein
a moiety represented by 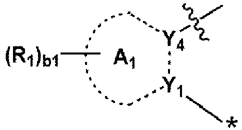 in Formula 2 is identical to a moiety represented by 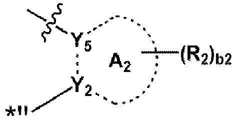 in Formula 2 or
wherein a moiety represented by 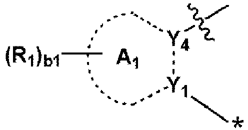 in Formula 2 is different from a moiety represented by 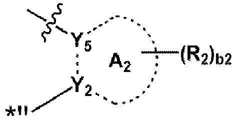 in Formula 2.

4. The organometallic compound of any of claims 1 to 3, wherein
a moiety represented by 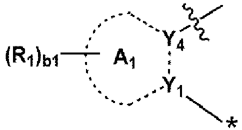 in Formula 2 is represented by one selected from Formulae 3-1 to 3-16 and 3-31 to 3-70,
a moiety represented by 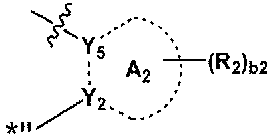 in Formula 2 is represented by one selected from Formulae 4-1 to 4-16 and 4-31 to 4-70, and
a moiety represented by 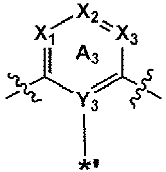 in Formula 2 is represented by one selected from Formulae 5-1 to 5-47: 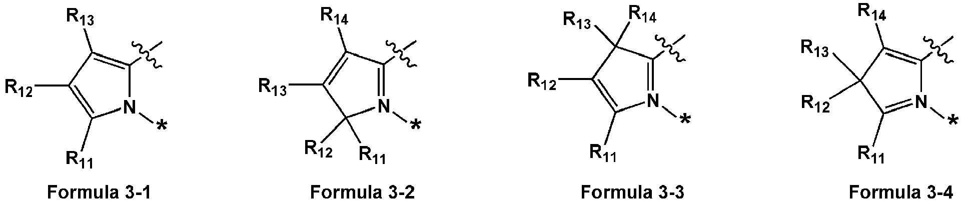 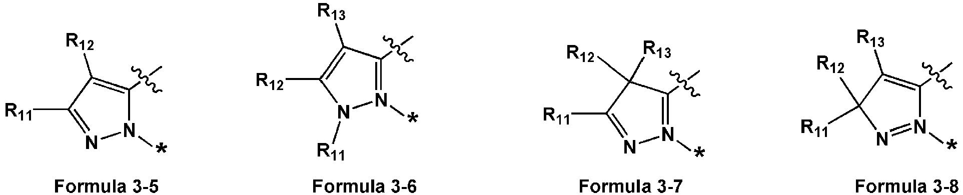 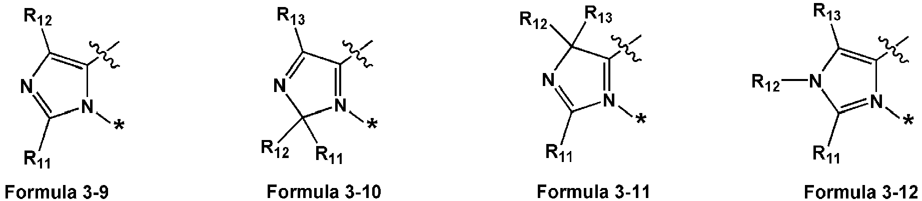 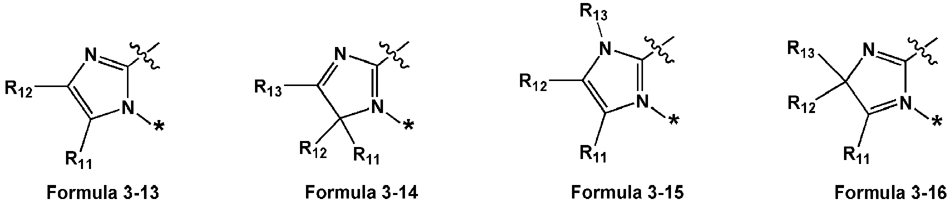 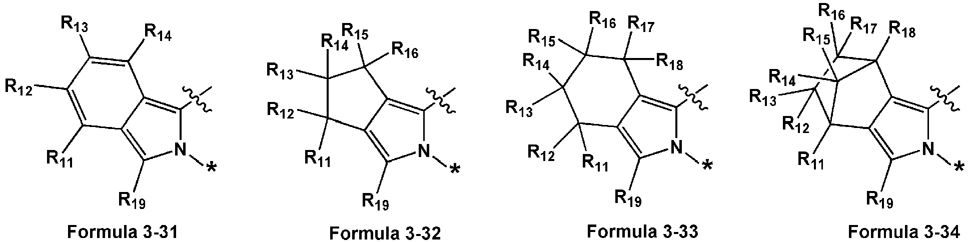 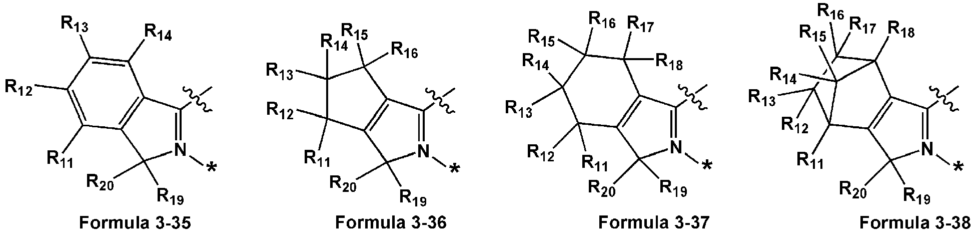 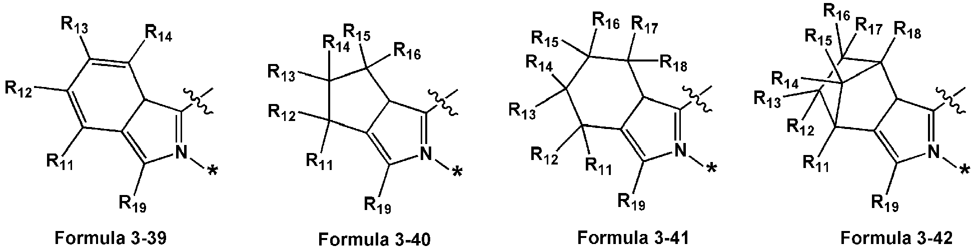 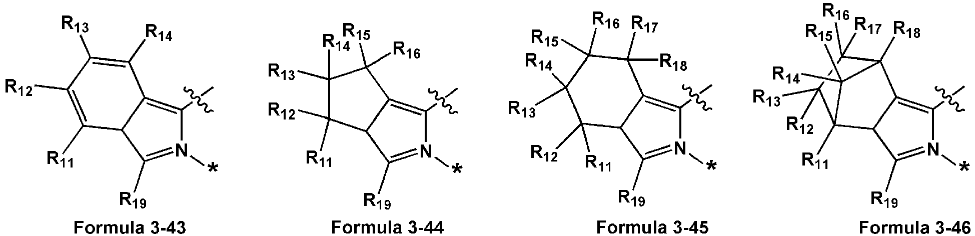 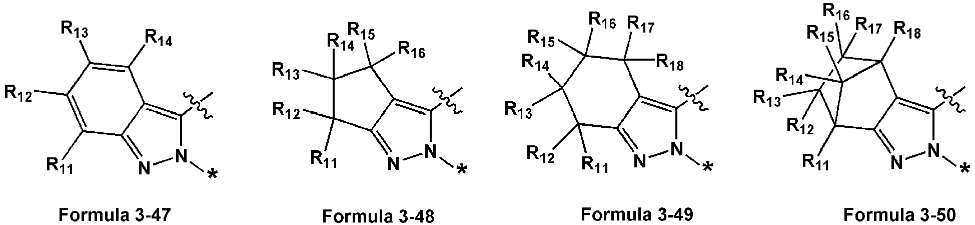 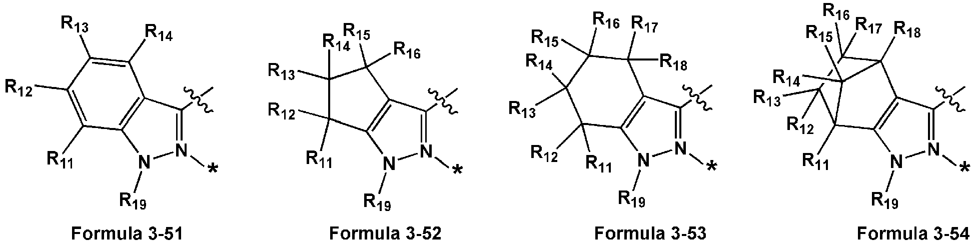 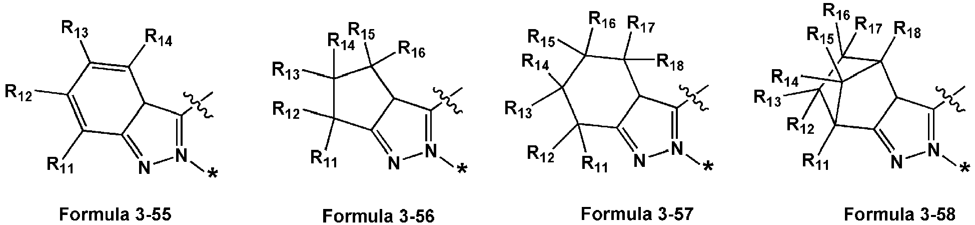 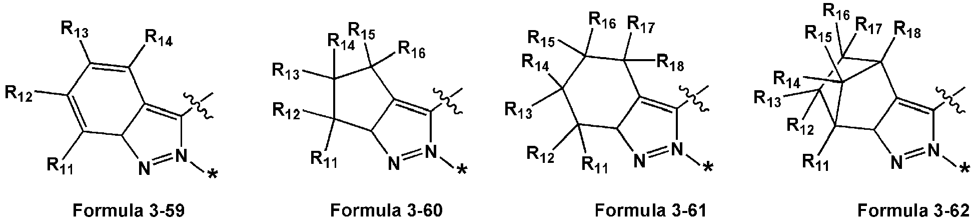 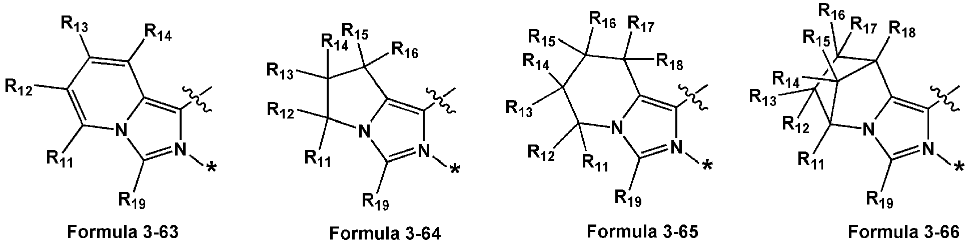 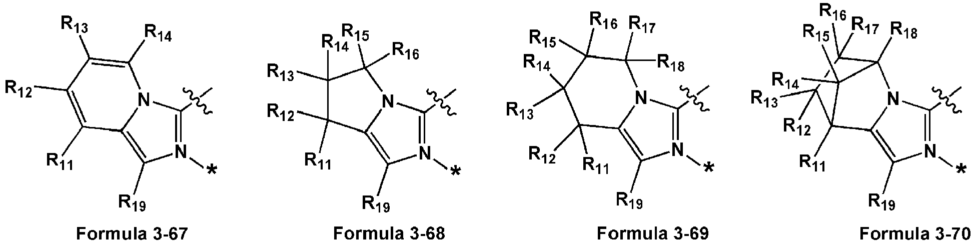 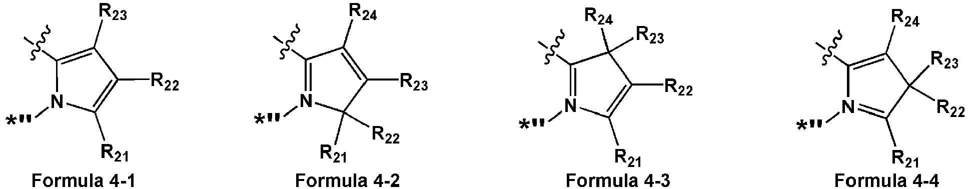 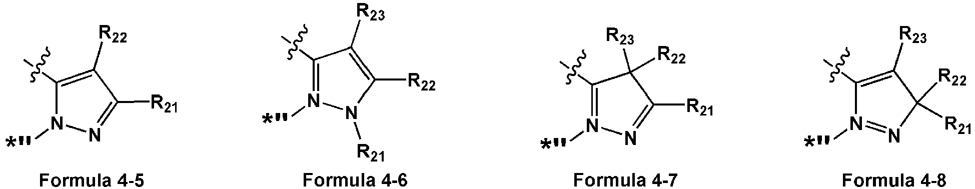 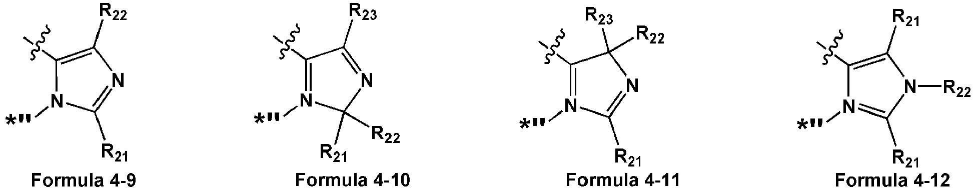 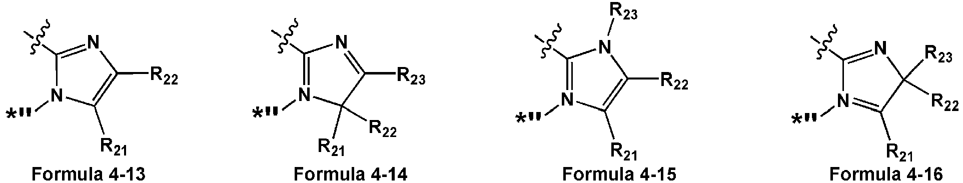 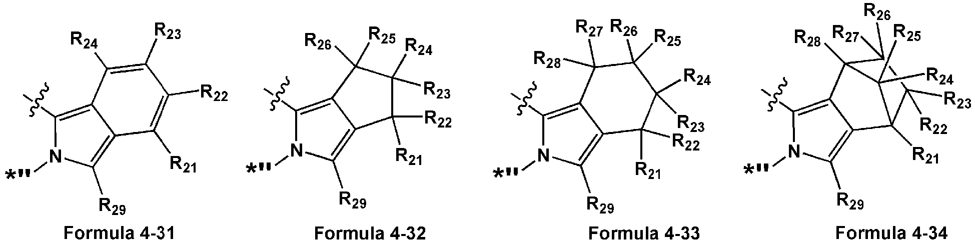 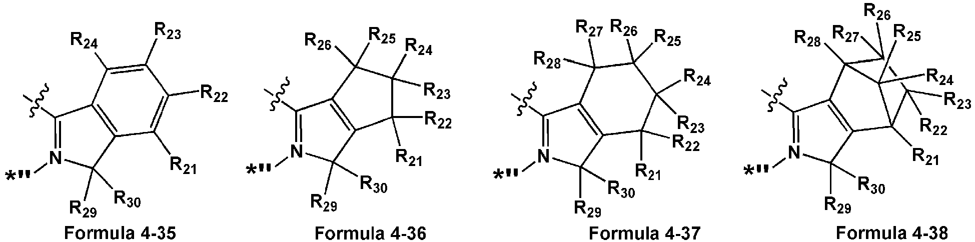 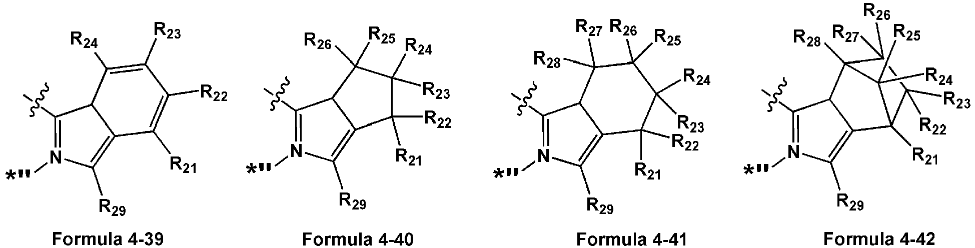 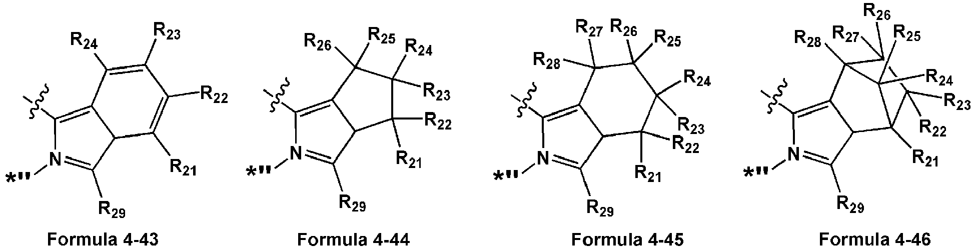 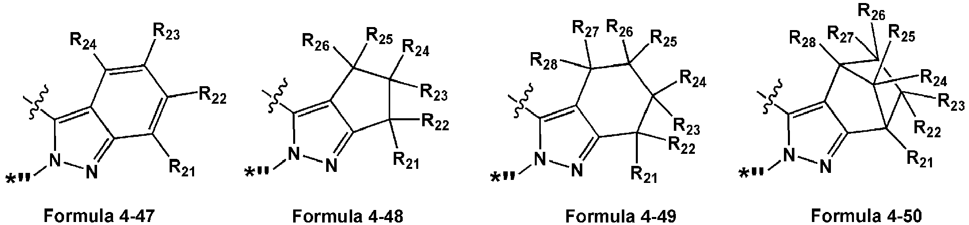 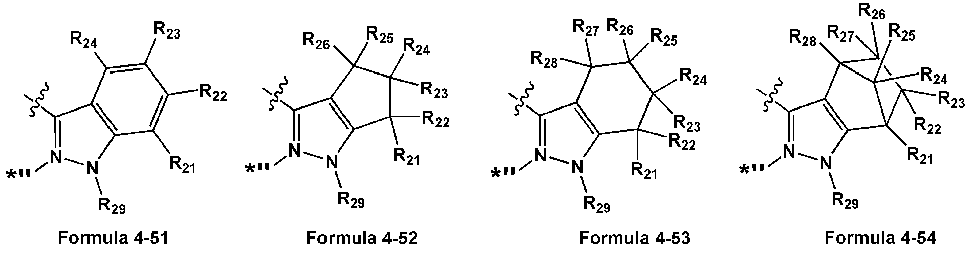 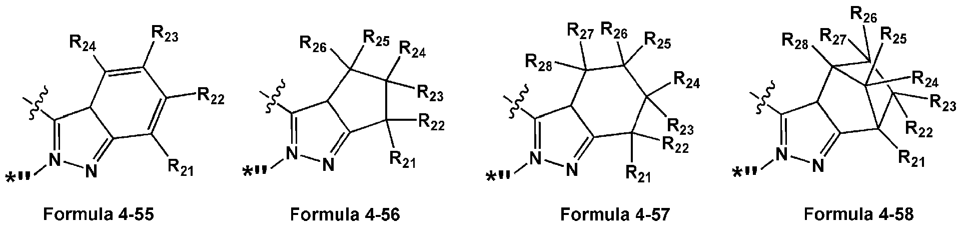 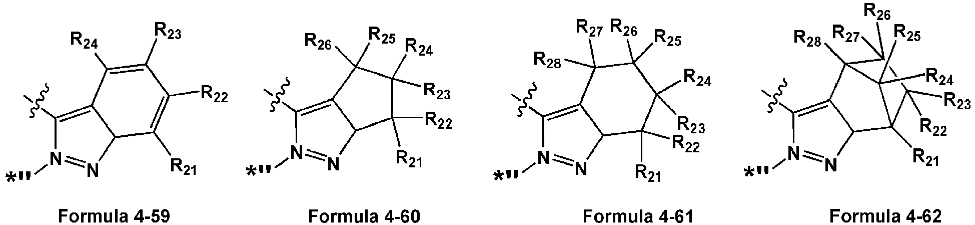 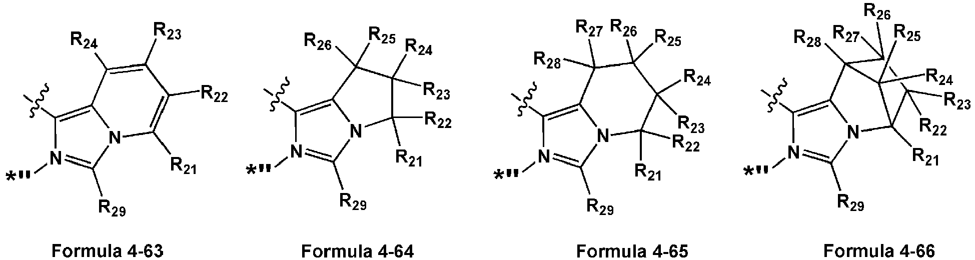 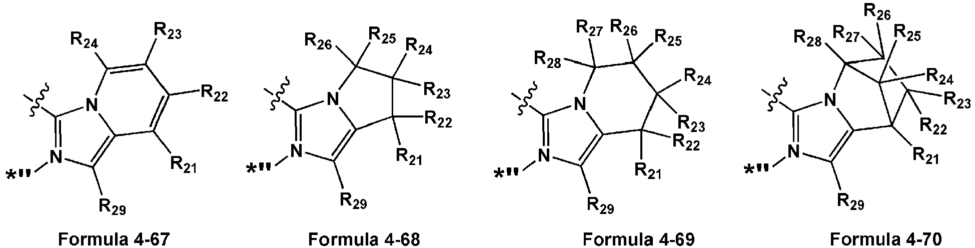 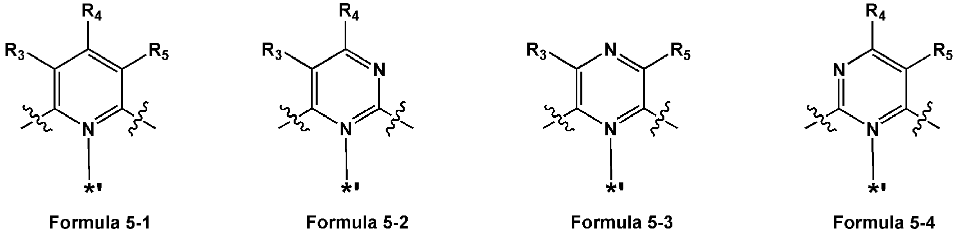 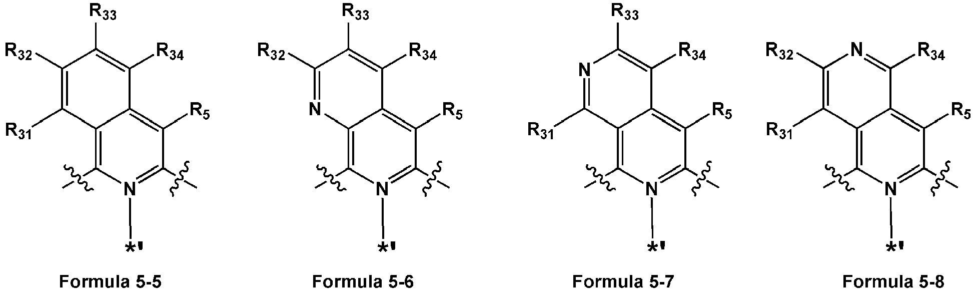 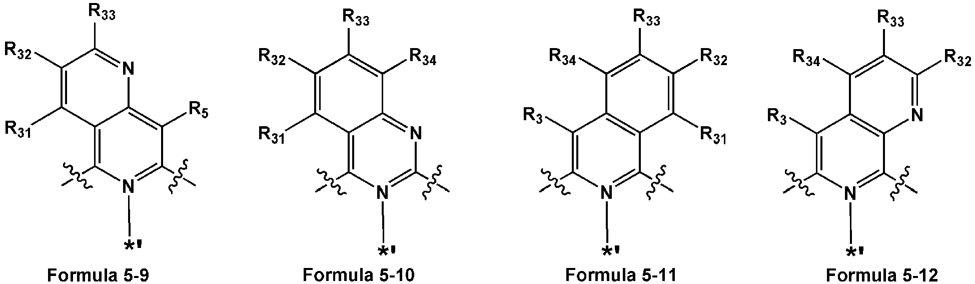 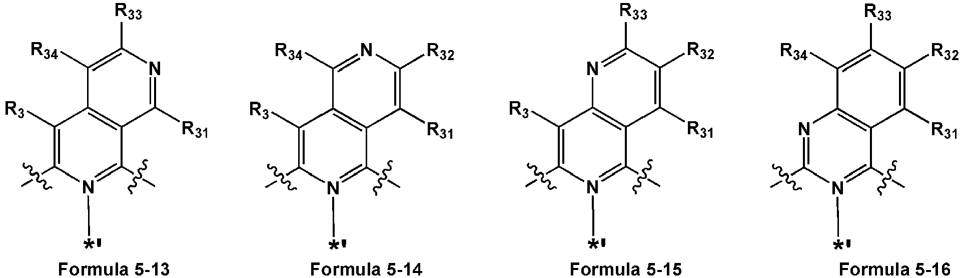 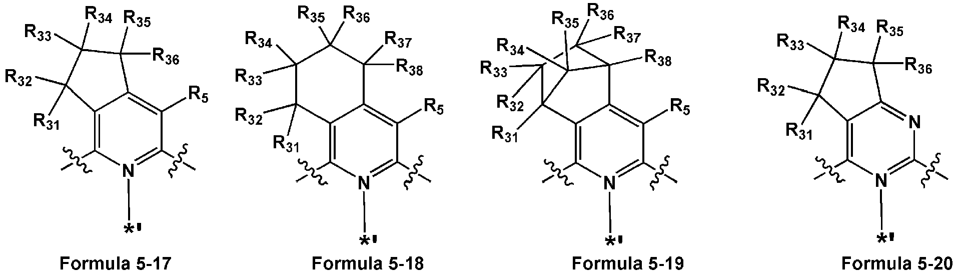 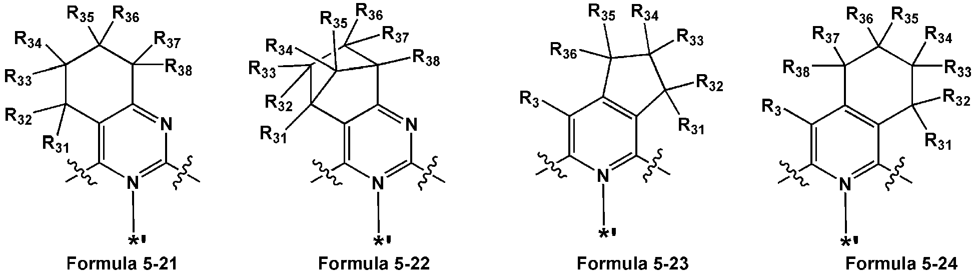 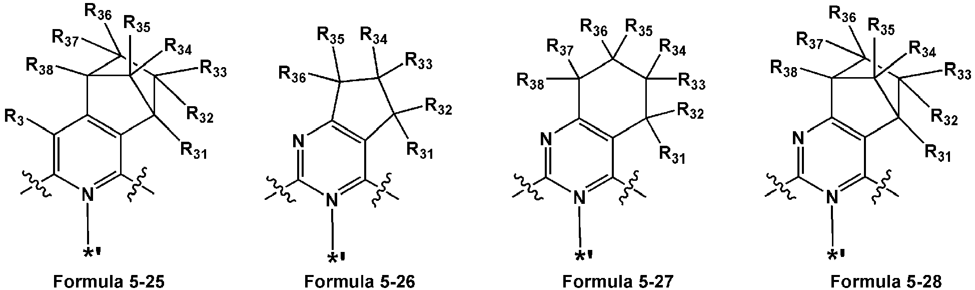 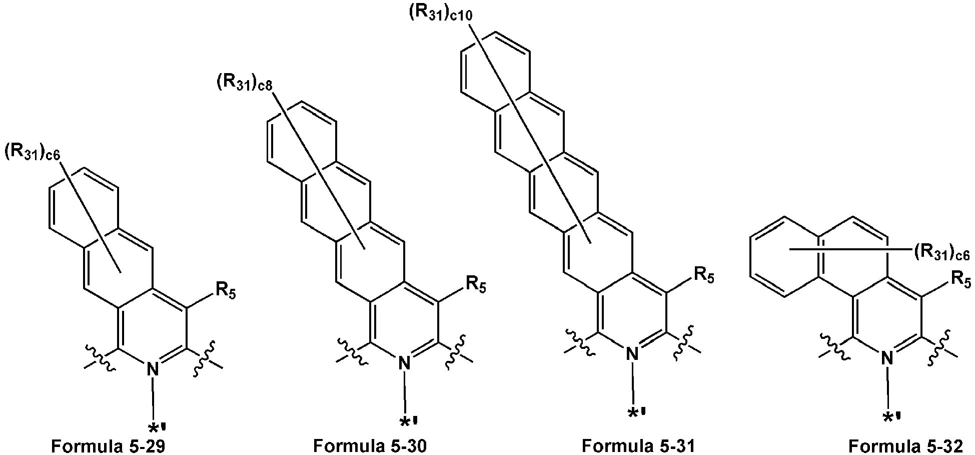 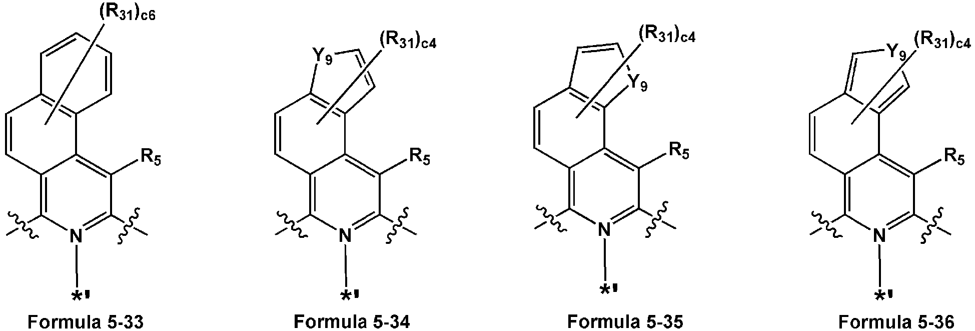 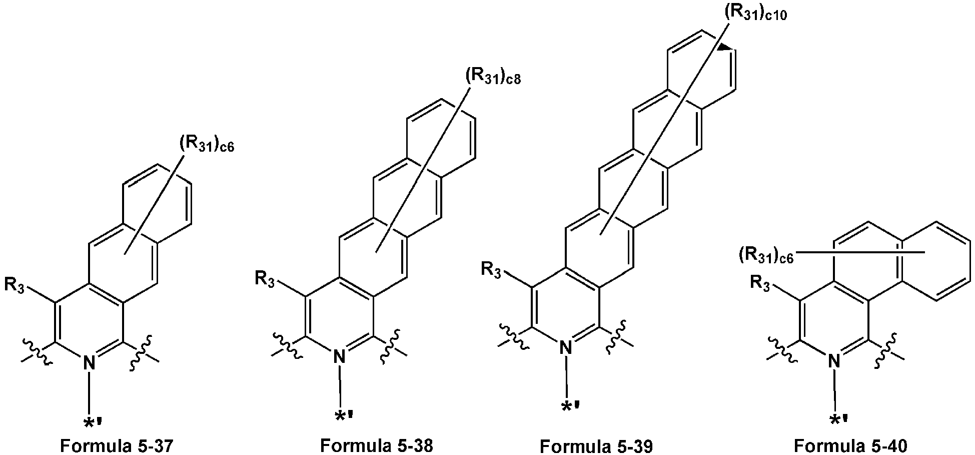 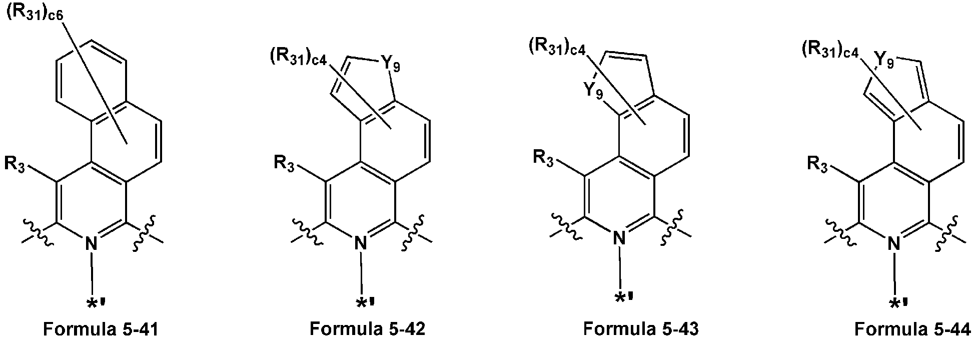 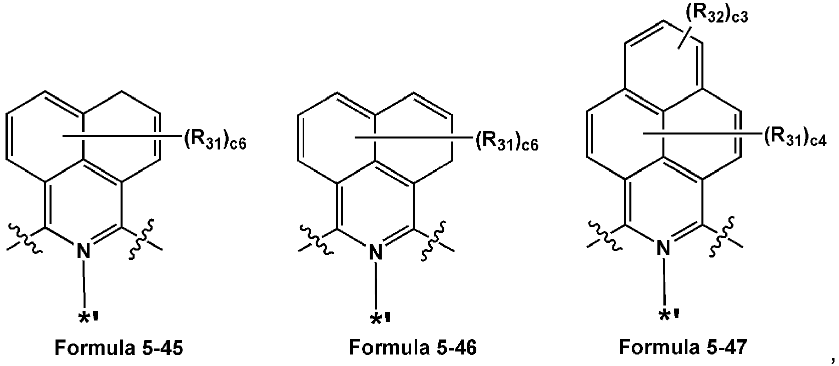
wherein, in Formulae 3-1 to 3-16, 3-31 to 3-70, 4-1 to 4-16, 4-31 to 4-70, and 5-1 to 5-47,
Y₉ is O, S, or N(R₃₉),
R₃ to R₅ are the same as described in claim 1,
R₁₁ to R₂₀ are each independently the same as described in connection with R₁ in claim 1,
R₂₁ to R₃₀ are each independently the same as described in connection with R₂ in claim 1,
R₃₁ to R₃₉ are each independently the same as described in connection with R₃ in claim 1,
c3 is an integer selected from 0 to 3,
c4 is an integer selected from 0 to 4,
c6 is an integer selected from 0 to 6,
c8 is an integer selected from 0 to 8,
c10 is an integer selected from 0 to 10, and
*, *', and *" each indicate a binding site to M in Formula 1, preferably wherein
R₃ to R₅ and R₁₁ to R₃₉ are each independently selected from:
hydrogen, deuterium, -F, -Cl, -Br, -I, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, -SF₅, a C₁-C₂₀ alkyl group, and a C₁-C₂₀ alkoxy group;
a C₁-C₂₀ alkyl group and a C₁-C₂₀ alkoxy group, each substituted with at least one selected from deuterium, -F, -Cl, -Br, -I, -CD₃, -CD₂H, -CDH₂, -CF₃, -CF₂H, -CFH₂, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a C₁-C₁₀ alkyl group, a cyclopentyl group, a cyclohexyl group, a cycloheptyl group, a cyclooctyl group, an adamantanyl group, a norbornanyl group, a norbornenyl group, a cyclopentenyl group, a cyclohexenyl group, a cycloheptenyl group, a phenyl group, a naphthyl group, a pyridinyl group, and a pyrimidinyl group;
a cyclopentyl group, a cyclohexyl group, a cycloheptyl group, a cyclooctyl group, an adamantanyl group, a norbornanyl group, a norbornenyl group, a cyclopentenyl group, a cyclohexenyl group, a cycloheptenyl group, a phenyl group, a naphthyl group, a fluorenyl group, a phenanthrenyl group, an anthracenyl group, a fluoranthenyl group, a triphenylenyl group, a pyrenyl group, a chrysenyl group, a pyrrolyl group, a thiophenyl group, a furanyl group, an imidazolyl group, a pyrazolyl group, a thiazolyl group, an isothiazolyl group, an oxazolyl group, an isoxazolyl group, a pyridinyl group, a pyrazinyl group, a pyrimidinyl group, a pyridazinyl group, an isoindolyl group, an indolyl group, an indazolyl group, a purinyl group, a quinolinyl group, an isoquinolinyl group, a benzoquinolinyl group, a quinoxalinyl group, a quinazolinyl group, a cinnolinyl group, a carbazolyl group, a phenanthrolinyl group, a benzimidazolyl group, a benzofuranyl group, a benzothiophenyl group, an isobenzothiazolyl group, a benzoxazolyl group, an isobenzoxazolyl group, a triazolyl group, a tetrazolyl group, an oxadiazolyl group, a triazinyl group, a dibenzofuranyl group, a dibenzothiophenyl group, a benzocarbazolyl group, a dibenzocarbazolyl group, an imidazopyridinyl group, and an imidazopyrimidinyl group;
a cyclopentyl group, a cyclohexyl group, a cycloheptyl group, a cyclooctyl group, an adamantanyl group, a norbornanyl group, a norbornenyl group, a cyclopentenyl group, a cyclohexenyl group, a cycloheptenyl group, a phenyl group, a naphthyl group, a fluorenyl group, a phenanthrenyl group, an anthracenyl group, a fluoranthenyl group, a triphenylenyl group, a pyrenyl group, a chrysenyl group, a pyrrolyl group, a thiophenyl group, a furanyl group, an imidazolyl group, a pyrazolyl group, a thiazolyl group, an isothiazolyl group, an oxazolyl group, an isoxazolyl group, a pyridinyl group, a pyrazinyl group, a pyrimidinyl group, a pyridazinyl group, an isoindolyl group, an indolyl group, an indazolyl group, a purinyl group, a quinolinyl group, an isoquinolinyl group, a benzoquinolinyl group, a quinoxalinyl group, a quinazolinyl group, a cinnolinyl group, a carbazolyl group, a phenanthrolinyl group, a benzimidazolyl group, a benzofuranyl group, a benzothiophenyl group, an isobenzothiazolyl group, a benzoxazolyl group, an isobenzoxazolyl group, a triazolyl group, a tetrazolyl group, an oxadiazolyl group, a triazinyl group, a dibenzofuranyl group, a dibenzothiophenyl group, a benzocarbazolyl group, a dibenzocarbazolyl group, an imidazopyridinyl group, and an imidazopyrimidinyl group, each substituted with at least one selected from deuterium, -F, -Cl, -Br, -I, -CD₃, -CD₂H, -CDH₂, -CF₃, -CF₂H, -CFH₂, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a C₁-C₂₀ alkyl group, a C₁-C₂₀ alkoxy group, a cyclopentyl group, a cyclohexyl group, a cycloheptyl group, a cyclooctyl group, an adamantanyl group, a norbornanyl group, a norbornenyl group, a cyclopentenyl group, a cyclohexenyl group, a cycloheptenyl group, a phenyl group, a naphthyl group, a fluorenyl group, a phenanthrenyl group, an anthracenyl group, a fluoranthenyl group, a triphenylenyl group, a pyrenyl group, a chrysenyl group, a pyrrolyl group, a thiophenyl group, a furanyl group, an imidazolyl group, a pyrazolyl group, a thiazolyl group, an isothiazolyl group, an oxazolyl group, an isoxazolyl group, a pyridinyl group, a pyrazinyl group, a pyrimidinyl group, a pyridazinyl group, an isoindolyl group, an indolyl group, an indazolyl group, a purinyl group, a quinolinyl group, an isoquinolinyl group, a benzoquinolinyl group, a quinoxalinyl group, a quinazolinyl group, a cinnolinyl group, a carbazolyl group, a phenanthrolinyl group, a benzimidazolyl group, a benzofuranyl group, a benzothiophenyl group, an isobenzothiazolyl group, a benzoxazolyl group, an isobenzoxazolyl group, a triazolyl group, a tetrazolyl group, an oxadiazolyl group, a triazinyl group, a dibenzofuranyl group, a dibenzothiophenyl group, a benzocarbazolyl group, a dibenzocarbazolyl group, an imidazopyridinyl group, and an imidazopyrimidinyl group; and
-N(Q₁)(Q₂), -Si(Q₃)(Q₄)(Q₅), -B(Q₆)(Q₇) and -P(=O)(Q₈)(Q₉),
wherein Q₁ to Q₉ are each independently selected from:
-CH₃, -CD₃, -CD₂H, -CDH₂, -CH₂CH₃, -CH₂CD₃, -CH₂CD₂H, -CH₂CDH₂, -CHDCH₃, -CHDCD₂H, -CHDCDH₂, -CHDCD₃, -CD₂CD₃, -CD₂CD₂H, and -CD₂CDH₂;
an n-propyl group, an isopropyl group, an n-butyl group, an isobutyl group, a sec-butyl group, a tert-butyl group, an n-pentyl group, an isopentyl group, a sec-pentyl group, a tert-pentyl group, a phenyl group, and a naphthyl group; and
an n-propyl group, an iso-propyl group, an n-butyl group, an isobutyl group, a sec-butyl group, a tert-butyl group, an n-pentyl group, an isopentyl group, a sec-pentyl group, a tert-pentyl group, a phenyl group, and a naphthyl group, each substituted with at least one selected from deuterium, a C₁-C₁₀ alkyl group, and a phenyl group, more preferably wherein
R₃ to R₅ and R₁₁ to R₃₉ are each independently selected from:
hydrogen, deuterium, -F, a cyano group, a nitro group, -SF₅, a methyl group, an ethyl group, an n-propyl group, an iso-propyl group, an n-butyl group, an isobutyl group, a sec-butyl group, a tert-butyl group, an n-pentyl group, an isopentyl group, a sec-pentyl group, a tert-pentyl group, an n-hexyl group, an iso-hexyl group, a sec-hexyl group, a tert-hexyl group, an n-heptyl group, an iso-heptyl group, a sec-heptyl group, a tert-heptyl group, an n-octyl group, an iso-octyl group, a sec-octyl group, a tert-octyl group, an n-nonyl group, an iso-nonyl group, a sec-nonyl group, a tert-nonyl group, an n-decyl group, an iso-decyl group, a sec-decyl group, a tert-decyl group, a methoxy group, an ethoxy group, a propoxy group, a butoxy group, a pentoxy group, a cyclopentyl group, a cyclohexyl group, a cycloheptyl group, a cyclooctyl group, an adamantanyl group, a norbornanyl group, a norbornenyl group, a cyclopentenyl group, a cyclohexenyl group, a cycloheptenyl group, a phenyl group, a naphthyl group, a pyridinyl group, a pyrimidinyl group, a dibenzofuranyl group, and a dibenzothiophenyl group;
a methyl group, an ethyl group, an n-propyl group, an iso-propyl group, an n-butyl group, an isobutyl group, a sec-butyl group, a tert-butyl group, an n-pentyl group, an isopentyl group, a sec-pentyl group, a tert-pentyl group, an n-hexyl group, an iso-hexyl group, a sec-hexyl group, a tert-hexyl group, an n-heptyl group, an iso-heptyl group, a sec-heptyl group, a tert-heptyl group, an n-octyl group, an iso-octyl group, a sec-octyl group, a tert-octyl group, an n-nonyl group, an iso-nonyl group, a sec-nonyl group, a tert-nonyl group, an n-decyl group, an iso-decyl group, a sec-decyl group, a tert-decyl group, a methoxy group, an ethoxy group, a propoxy group, a butoxy group, a pentoxy group, a cyclopentyl group, a cyclohexyl group, a cycloheptyl group, a cyclooctyl group, an adamantanyl group, a norbornanyl group, a norbornenyl group, a cyclopentenyl group, a cyclohexenyl group, a cycloheptenyl group, a phenyl group, a naphthyl group, a pyridinyl group, a pyrimidinyl group, a dibenzofuranyl group, and a dibenzothiophenyl group, each substituted with at least one selected from deuterium, -F, -CD₃, -CD₂H, -CDH₂, -CF₃, -CF₂H, -CFH₂, a cyano group, a nitro group, a C₁-C₁₀ alkyl group, a C₁-C₁₀ alkoxy group, a cyclopentyl group, a cyclohexyl group, a cycloheptyl group, a cyclooctyl group, an adamantanyl group, a norbornanyl group, a norbornenyl group, a cyclopentenyl group, a cyclohexenyl group, a cycloheptenyl group, a phenyl group, a naphthyl group, a pyridinyl group, a pyrimidinyl group, a dibenzofuranyl group, and a dibenzothiophenyl group; and
-N(Q₁)(Q₂), -Si(Q₃)(Q₄)(Q₅), -B(Q₆)(Q₇), and -P(=O)(Q₈)(Q₉),
wherein Q₁ to Q₉ are each independently selected from:
-CH₃, -CD₃, -CD₂H, -CDH₂, -CH₂CH₃, -CH₂CD₃, -CH₂CD₂H, -CH₂CDH₂, -CHDCH₃, -CHDCD₂H, -CHDCDH₂, -CHDCD₃, -CD₂CD₃, -CD₂CD₂H, and -CD₂CDH₂;
an n-propyl group, an isopropyl group, an n-butyl group, an isobutyl group, a sec-butyl group, a tert-butyl group, an n-pentyl group, an isopentyl group, a sec-pentyl group, a tert-pentyl group, a phenyl group, and a naphthyl group; and
an n-propyl group, an iso-propyl group, an n-butyl group, an isobutyl group, a sec-butyl group, a tert-butyl group, an n-pentyl group, an isopentyl group, a sec-pentyl group, a tert-pentyl group, a phenyl group, and a naphthyl group, each substituted with at least one selected from deuterium, a C₁-C₁₀ alkyl group, and a phenyl group, and in particular wherein
R₃ to R₅ and R₁₁ to R₃₉ are each independently selected from hydrogen, deuterium, -F, a cyano group, a nitro group, -SF₅, -CH₃, -CD₃, -CD₂H, -CDH₂, -CF₃, -CF₂H, -CFH₂, a group represented by any of Formulae 9-1 to 9-19, a group represented by any of Formulae 10-1 to 10-38, and -Si(Q₃)(Q₄)(Q₅): 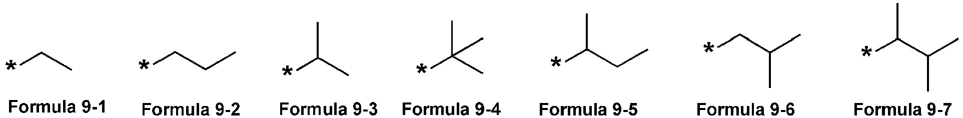 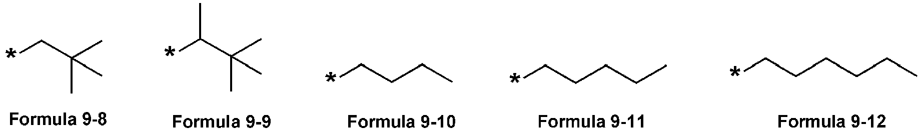 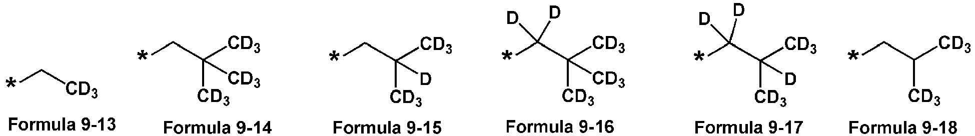 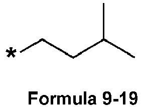 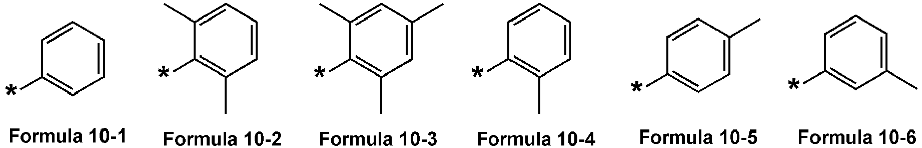 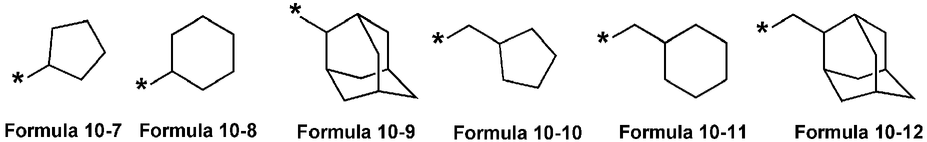 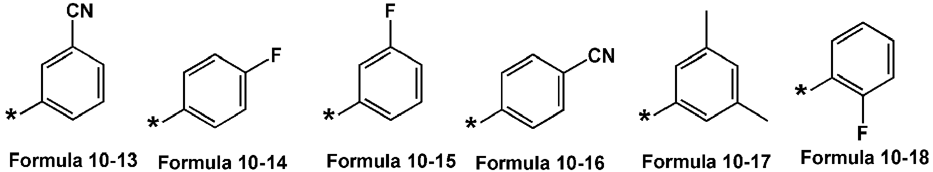 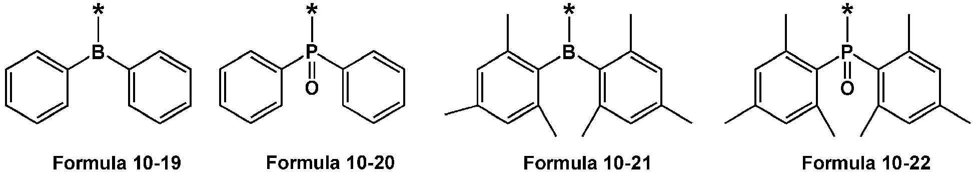 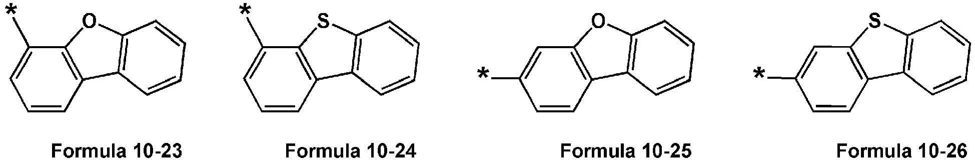 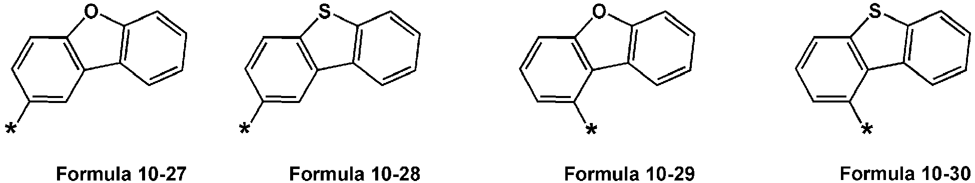 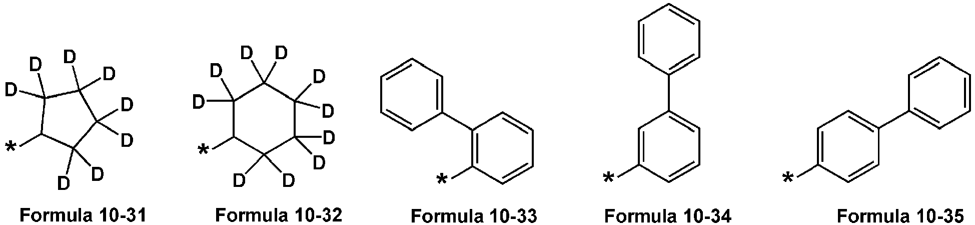 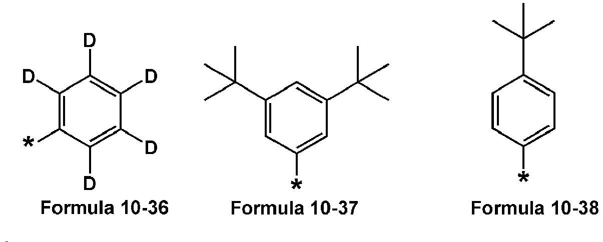
wherein * in Formulae 9-1 to 9-19 and 10-1 to 10-38 indicates a binding site to a neighboring atom.

5. The organometallic compound of claim 4, wherein
a moiety represented by 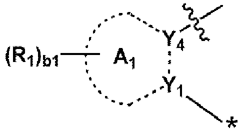 in Formula 2 is represented by one selected from Formulae 3-5 to 3-8, and 3-47 to 3-62,
a moiety represented by 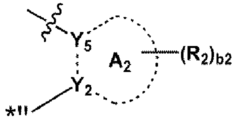 in Formula 2 is represented by one selected from Formulae 4-5 to 4-8, and 4-47 to 4-62.

6. The organometallic compound of claim 4, wherein
a moiety represented by 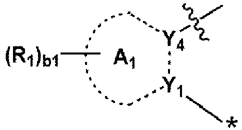 in Formula 2 is represented by one selected from Formulae 3-1, 3-5, 3-9, 3-13, 3-31 to 3-34, and 3-47 to 3-50, and
a moiety represented by 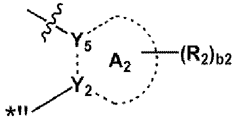 in Formula 2 is represented by one selected from Formulae 4-1, 4-5, 4-9, 4-13, 4-31 to 4-34, and 4-47 to 4-50.

7. The organometallic compound of claim 4, wherein
a moiety represented by 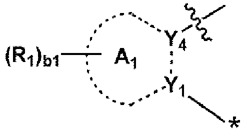 in Formula 2 is represented by one selected from Formulae 3-1, 3-5, 3-9, 3-13, 3-31 to 3-34, and 3-47 to 3-50, and
a moiety represented by 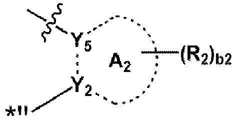 in Formula 2 is represented by one selected from Formulae 4-2 to 4-4, 4-6 to 4-8, 4-10 to 4-12, 4-14 to 4-16, 4-35 to 4-46, and 4-51 to 4-70.

8. The organometallic compound of any of claims 4 to 7, wherein
a moiety represented by 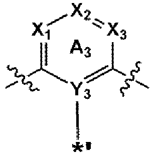 in Formula 2 is represented by one selected from Formulae 5-1 to 5-28, 5-29, and 5-45.

9. The organometallic compound of any of claims 1 to 8, wherein
L₁ in Formula 1 is selected from ligands represented by Formulae 2A-1 to 2E-1 and 2A-2 to 2E-2: 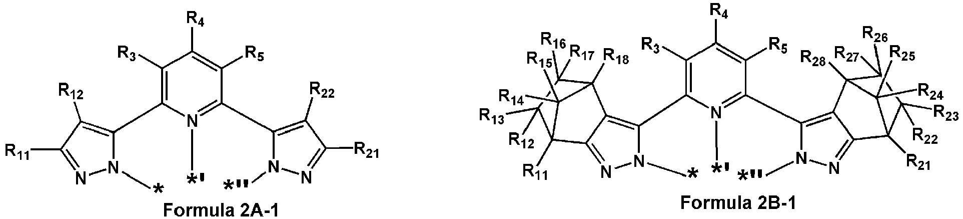 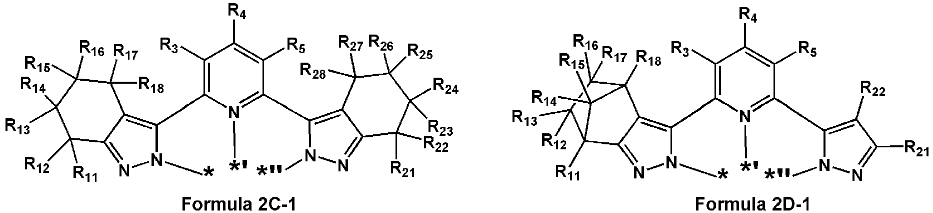 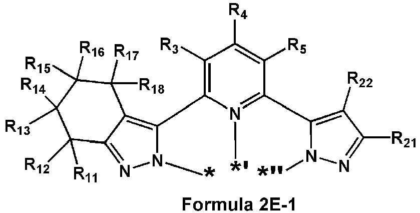 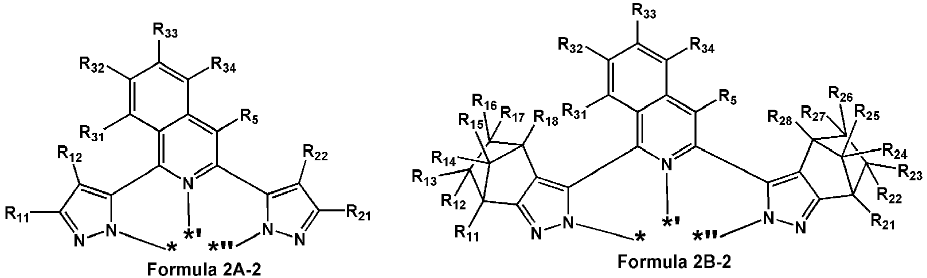 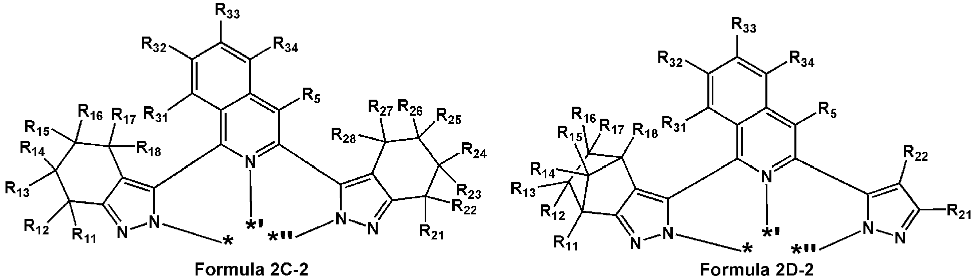 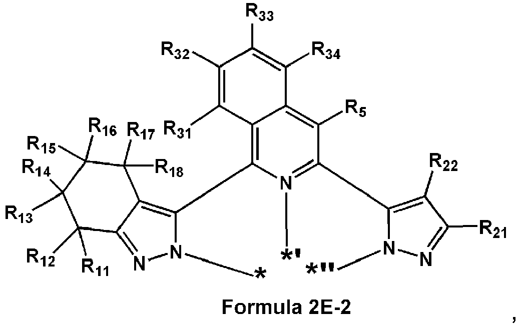
wherein, in Formulae 2A-1 to 2E-1 and 2A-2 to 2E-2,
R₃ to R₅, R₁₁ to R₁₃, R₂₁ to R₂₈, and R₃₁ to R₃₄ are each independently selected from hydrogen, deuterium, -F, a cyano group, a nitro group, -SF₅, -CH₃, -CD₃, -CD₂H, -CDH₂, -CF₃, -CF₂H, -CFH₂, a group represented by any of Formulae 9-1 to 9-19, a group represented by any of Formulae 10-1 to 10-38, and -Si(Q₃)(Q₄)(Q₅): 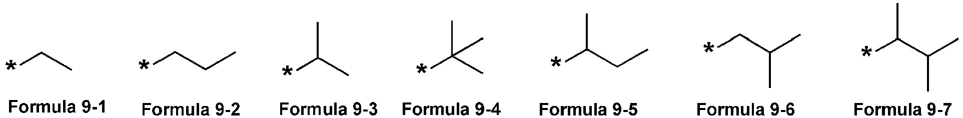 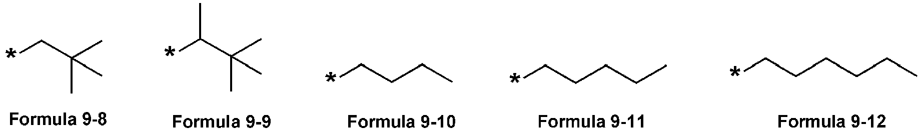 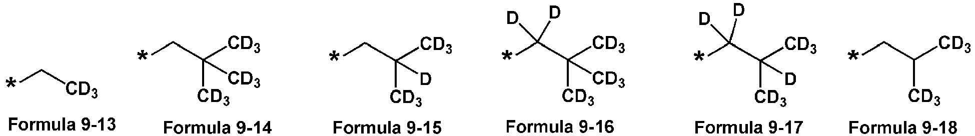 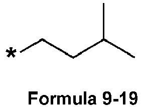 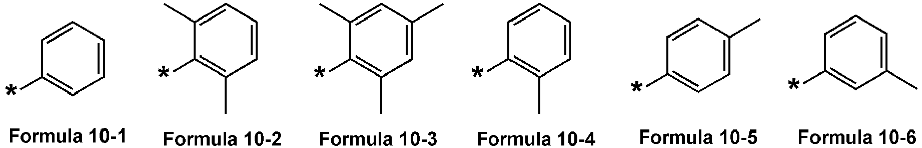 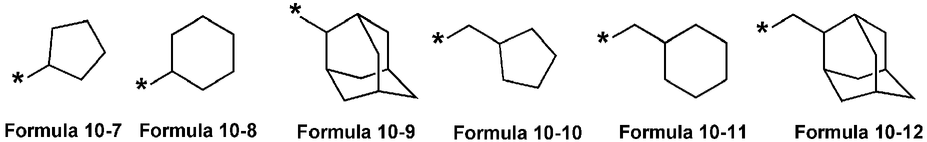 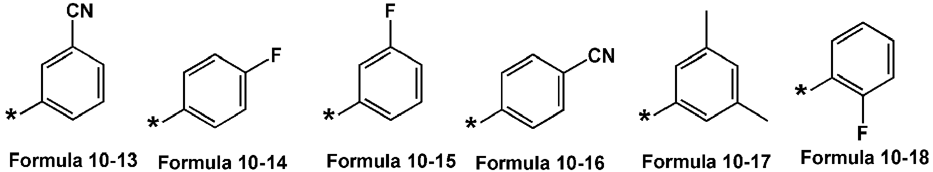 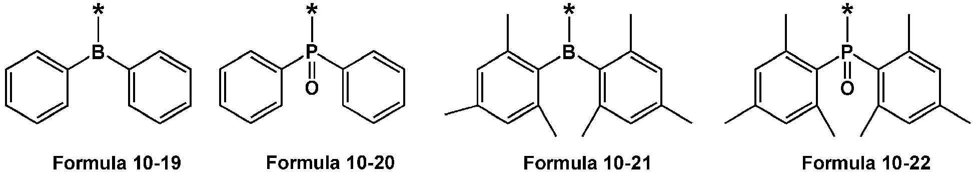 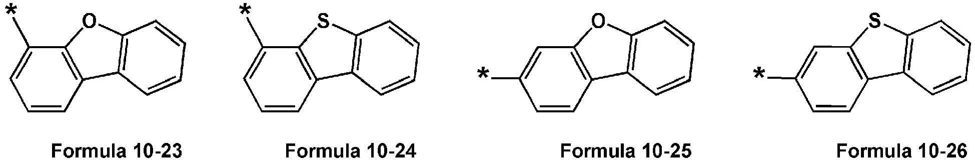 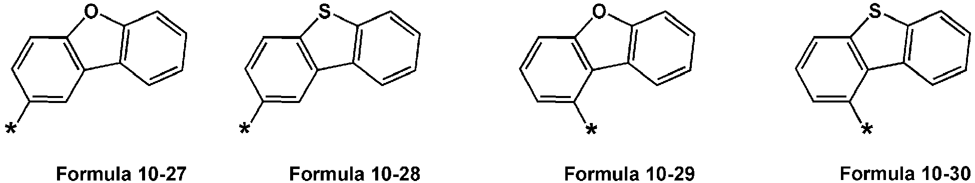 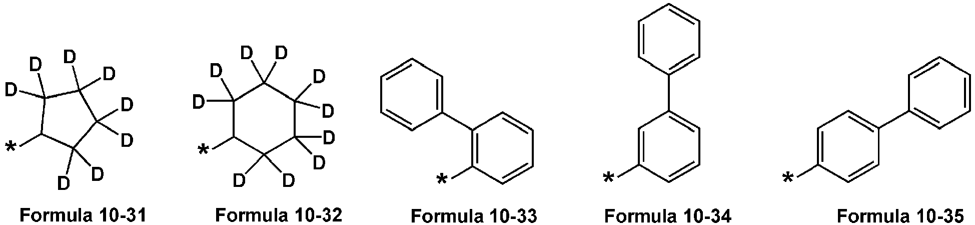 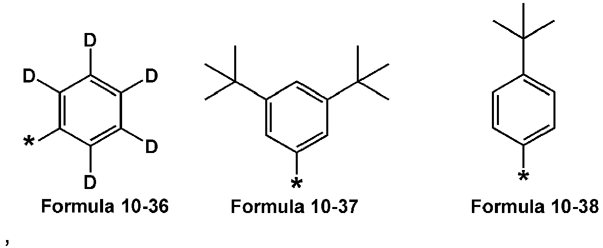
wherein * in Formulae 9-1 to 9-19 and 10-1 to 10-38 indicates a binding site to a neighboring atom.

10. The organometallic compound of any of claims 1 to 9, wherein
L₂ in Formula 1 is selected from ligands represented by Formula 6-1:
*-(̵T₃)ₐ₃-R₆₁ **Formula 6-1**,
wherein, in Formula 6-1,
T₃ is selected from a single bond, *-O-*', *-S-*', *-C(R₆₂)(R₆₃)-*', *-C(R₆₂)=*', *=C(R₆₂)-*', *-C(R₆₂)=C(R₆₃)-*', *-C(=O)-*', *-C(=S)-*', *-C=C-*', and *-N(R₆₂)-*',
a3 is an integer selected from 1 to 5,
R₆₁ is selected from hydrogen, deuterium, -F, -Cl, -Br, -I, -SF₅, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a substituted or unsubstituted C₁-C₆₀ alkyl group, a substituted or unsubstituted C₂-C₆₀ alkenyl group, a substituted or unsubstituted C₂-C₆₀ alkynyl group, a substituted or unsubstituted C₁-C₆₀ alkoxy group, a substituted or unsubstituted C₃-C₁₀ cycloalkyl group, a substituted or unsubstituted C₁-C₁₀ heterocycloalkyl group, a substituted or unsubstituted C₃-C₁₀ cycloalkenyl group, a substituted or unsubstituted C₁-C₁₀ heterocycloalkenyl group, a substituted or unsubstituted C₆-C₆₀ aryl group, a substituted or unsubstituted C₆-C₆₀ aryloxy group, a substituted or unsubstituted C₆-C₆₀ arylthio group, a substituted or unsubstituted C₁-C₆₀ heteroaryl group, a substituted or unsubstituted monovalent non-aromatic condensed polycyclic group, a substituted or unsubstituted monovalent non-aromatic condensed heteropolycyclic group, -N(Q₁)(Q₂), -Si(Q₃)(Q₄)(Q₅), -B(Q₆)(Q₇), -P(=O)(Q₈)(Q₉), and -P(Q₄₁)(Q₄₂)(Q₄₃),
wherein Q₁ to Q₉ and Q₄₁ to Q₄₃ are each independently selected from:
-CH₃, -CD₃, -CD₂H, -CDH₂, -CH₂CH₃, -CH₂CD₃, -CH₂CD₂H, -CH₂CDH₂, -CHDCH₃, -CHDCD₂H, -CHDCDH₂, -CHDCD₃, -CD₂CD₃, -CD₂CD₂H, and -CD₂CDH₂;
an n-propyl group, an isopropyl group, an n-butyl group, an isobutyl group, a sec-butyl group, a tert-butyl group, an n-pentyl group, an isopentyl group, a sec-pentyl group, a tert-pentyl group, a phenyl group, and a naphthyl group; and
an n-propyl group, an iso-propyl group, an n-butyl group, an isobutyl group, a sec-butyl group, a tert-butyl group, an n-pentyl group, an isopentyl group, a sec-pentyl group, a tert-pentyl group, a phenyl group, and a naphthyl group, each substituted with at least one selected from deuterium, a C₁-C₁₀ alkyl group, and a phenyl group,
R₆₂ and R₆₃ are each independently selected from:
hydrogen, deuterium, -F, a cyano group, a nitro group, -SF₅, a methyl group, an ethyl group, an n-propyl group, an iso-propyl group, an n-butyl group, an isobutyl group, a sec-butyl group, a tert-butyl group, an n-pentyl group, an isopentyl group, a sec-pentyl group, a tert-pentyl group, an n-hexyl group, an iso-hexyl group, a sec-hexyl group, a tert-hexyl group, an n-heptyl group, an iso-heptyl group, a sec-heptyl group, a tert-heptyl group, an n-octyl group, an iso-octyl group, a sec-octyl group, a tert-octyl group, an n-nonyl group, an iso-nonyl group, a sec-nonyl group, a tert-nonyl group, an n-decyl group, an iso-decyl group, a sec-decyl group, a tert-decyl group, a methoxy group, an ethoxy group, a propoxy group, a butoxy group, a pentoxy group, a cyclopentyl group, a cyclohexyl group, a cycloheptyl group, a cyclooctyl group, an adamantanyl group, a norbornanyl group, a norbornenyl group, a cyclopentenyl group, a cyclohexenyl group, a cycloheptenyl group, a phenyl group, a naphthyl group, a pyridinyl group, a pyrimidinyl group, a dibenzofuranyl group, and a dibenzothiophenyl group; and
a methyl group, an ethyl group, an n-propyl group, an iso-propyl group, an n-butyl group, an isobutyl group, a sec-butyl group, a tert-butyl group, an n-pentyl group, an isopentyl group, a sec-pentyl group, a tert-pentyl group, an n-hexyl group, an iso-hexyl group, a sec-hexyl group, a tert-hexyl group, an n-heptyl group, an iso-heptyl group, a sec-heptyl group, a tert-heptyl group, an n-octyl group, an iso-octyl group, a sec-octyl group, a tert-octyl group, an n-nonyl group, an iso-nonyl group, a sec-nonyl group, a tert-nonyl group, an n-decyl group, an iso-decyl group, a sec-decyl group, a tert-decyl group, a methoxy group, an ethoxy group, a propoxy group, a butoxy group, a pentoxy group, a cyclopentyl group, a cyclohexyl group, a cycloheptyl group, a cyclooctyl group, an adamantanyl group, a norbornanyl group, a norbornenyl group, a cyclopentenyl group, a cyclohexenyl group, a cycloheptenyl group, a phenyl group, a naphthyl group, a pyridinyl group, a pyrimidinyl group, a dibenzofuranyl group, and a dibenzothiophenyl group, each substituted with at least one selected from deuterium, -F, -CD₃, -CD₂H, -CDH₂, -CF₃, -CF₂H, -CFH₂, a cyano group, a nitro group, a C₁-C₁₀ alkyl group, a C₁-C₁₀ alkoxy group, a cyclopentyl group, a cyclohexyl group, a cycloheptyl group, a cyclooctyl group, an adamantanyl group, a norbornanyl group, a norbornenyl group, a cyclopentenyl group, a cyclohexenyl group, a cycloheptenyl group, a phenyl group, a naphthyl group, a pyridinyl group, a pyrimidinyl group, a dibenzofuranyl group, and a dibenzothiophenyl group,
R₆₂ and R₆₃ are optionally connected to each other to form a substituted or unsubstituted C₅-C₃₀ carbocyclic group or a substituted or unsubstituted C₂-C₃₀ heterocyclic group, and
* indicates a binding site to M in Formula 1.

11. The organometallic compound of any of claims 1 to 10, wherein
L₂ in Formula 1 is selected from ligands represented by Formulae 12-1 to 12-5: 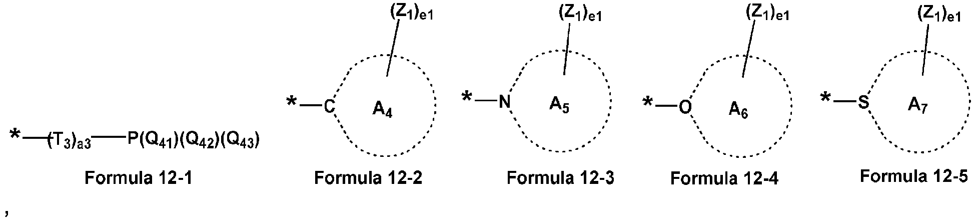 wherein, in Formulae 12-1 to 12-5,
T₃ is selected from a single bond, *-O-*', *-S-*', *-C(R₆₂)(R₆₃)-*', *-C(R₆₂)=*', *=C(R₆₂)-*', *-C(R₆₂)=C(R₆₃)-*', *-C(=O)-*', *-C(=S)-*', *-C=C-*', and *-N(R₆₂)-*',
R₆₂ and R₆₃ are each independently selected from hydrogen, deuterium, -F, -Cl, -Br, -I, -SF₅, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a C₁-C₂₀ alkyl group, a C₁-C₂₀ alkoxy group, a phenyl group, and a naphthyl group,
a3 is an integer selected from 1 to 5,
ring A₄ is selected from a cyclopentene ring, a cyclohexene ring, cycloheptene ring, a benzene ring, an indene ring, a naphthalene ring, an azulene ring, a heptalene ring, an indacene ring, an acenaphthylene ring, a fluorene ring, a spiro-bifluorene ring, a benzofluorene ring, a dibenzofluorene ring, a phenalene ring, a phenanthrene ring, an anthracene ring, a fluoranthene ring, a triphenylene ring, a pyrene ring, a chrysene ring, a naphthacene ring, a picene ring, a perylene ring, a pentacene ring, a hexacene ring, a rubicene ring, a coronene ring, an ovalene ring, a pyrrole ring, a thiophene ring, a furan ring, an imidazole ring, a pyrazole ring, a thiazole ring, an isothiazole ring, an oxazole ring, an isoxazole ring, a pyridine ring, a pyrazine ring, a pyrimidine ring, a pyridazine ring, an iso-indole ring, an indole ring, an indazole ring, a purine ring, a quinoline ring, an isoquinoline ring, a benzoquinoline ring, a quinoxaline ring, a quinazoline ring, a cinnoline ring, a naphthyridine ring, a carbazole ring, a phenanthroline ring, a benzimidazole ring, a benzofuran ring, a benzothiophene ring, a benzothiazole ring, an iso-benzothiazole ring, a benzoxazole ring, an isobenzoxazole ring, a triazole ring, a tetrazole ring, an oxadiazole ring, a thiadiazol ring, a triazine ring, a dibenzofuran ring, a dibenzothiophene ring, a benzocarbazole ring, a dibenzocarbazole ring, an imidazopyridine ring, and an imidazopyrimidine ring,
ring A₅ is selected from a pyrrole ring, an imidazole ring, a pyrazole ring, a thiazole ring, an isothiazole ring, an oxazole ring, an isoxazole ring, a pyridine ring, a pyrazine ring, a pyrimidine ring, a pyridazine ring, an iso-indole ring, an indole ring, an indazole ring, a purine ring, a quinoline ring, an isoquinoline ring, a benzoquinoline ring, a quinoxaline ring, a quinazoline ring, a cinnoline ring, a naphthyridine ring, a carbazole ring, a phenanthroline ring, a benzimidazole ring, a benzofuran ring, a benzothiazole ring, an iso-benzothiazole ring, a benzoxazole ring, an isobenzoxazole ring, a triazole ring, a tetrazole ring, an oxadiazole ring, a thiadiazol ring, a triazine ring, a benzocarbazole ring, a dibenzocarbazole ring, an imidazopyridine ring, and an imidazopyrimidine ring,
ring A₆ is selected from a furan ring, an oxazole ring, an isoxazole ring, a benzofuran ring, a benzoxazole ring, an isobenzoxazole ring, an oxadiazole ring, and a dibenzofuran ring,
ring A₇ is selected from a thiophene ring, a thiazole ring, an isothiazole ring, a benzothiophene ring, a benzothiazole ring, an iso-benzothiazole ring, a thiadiazol ring, and a dibenzothiophene ring,
Z₁ is each independently selected from:
hydrogen, deuterium, -F, a cyano group, a nitro group, -SF₅, a methyl group, an ethyl group, an n-propyl group, an iso-propyl group, an n-butyl group, an isobutyl group, a sec-butyl group, a tert-butyl group, an n-pentyl group, an isopentyl group, a sec-pentyl group, a tert-pentyl group, an n-hexyl group, an iso-hexyl group, a sec-hexyl group, a tert-hexyl group, an n-heptyl group, an iso-heptyl group, a sec-heptyl group, a tert-heptyl group, an n-octyl group, an iso-octyl group, a sec-octyl group, a tert-octyl group, an n-nonyl group, an iso-nonyl group, a sec-nonyl group, a tert-nonyl group, an n-decyl group, an iso-decyl group, a sec-decyl group, a tert-decyl group, a methoxy group, an ethoxy group, a propoxy group, a butoxy group, a pentoxy group, a cyclopentyl group, a cyclohexyl group, a cycloheptyl group, a cyclooctyl group, an adamantanyl group, a norbornanyl group, a norbornenyl group, a cyclopentenyl group, a cyclohexenyl group, a cycloheptenyl group, a phenyl group, a naphthyl group, a pyridinyl group, a pyrimidinyl group, a dibenzofuranyl group, and a dibenzothiophenyl group;
a methyl group, an ethyl group, an n-propyl group, an iso-propyl group, an n-butyl group, an isobutyl group, a sec-butyl group, a tert-butyl group, an n-pentyl group, an isopentyl group, a sec-pentyl group, a tert-pentyl group, an n-hexyl group, an iso-hexyl group, a sec-hexyl group, a tert-hexyl group, an n-heptyl group, an iso-heptyl group, a sec-heptyl group, a tert-heptyl group, an n-octyl group, an iso-octyl group, a sec-octyl group, a tert-octyl group, an n-nonyl group, an iso-nonyl group, a sec-nonyl group, a tert-nonyl group, an n-decyl group, an iso-decyl group, a sec-decyl group, a tert-decyl group, a methoxy group, an ethoxy group, a propoxy group, a butoxy group, a pentoxy group, a cyclopentyl group, a cyclohexyl group, a cycloheptyl group, a cyclooctyl group, an adamantanyl group, a norbornanyl group, a norbornenyl group, a cyclopentenyl group, a cyclohexenyl group, a cycloheptenyl group, a phenyl group, a naphthyl group, a pyridinyl group, a pyrimidinyl group, a dibenzofuranyl group, and a dibenzothiophenyl group, each substituted with at least one selected from deuterium, -F, -CD₃, -CD₂H, -CDH₂, -CF₃, -CF₂H, -CFH₂, a cyano group, a nitro group, a C₁-C₁₀ alkyl group, a C₁-C₁₀ alkoxy group, a cyclopentyl group, a cyclohexyl group, a cycloheptyl group, a cyclooctyl group, an adamantanyl group, a norbornanyl group, a norbornenyl group, a cyclopentenyl group, a cyclohexenyl group, a cycloheptenyl group, a phenyl group, a naphthyl group, a pyridinyl group, a pyrimidinyl group, a dibenzofuranyl group, and a dibenzothiophenyl group; and
-N(Q₃₁)(Q₃₂), -Si(Q₃₃)(Q₃₄)(Q₃₅), -B(Q₃₆)(Q₃₇), and -P(=O)(Q₃₈)(Q₃₉),
wherein Q₃₁ to Q₃₉ are independently selected from:
-CH₃, -CD₃, -CD₂H, -CDH₂, -CH₂CH₃, -CH₂CD₃, -CH₂CD₂H, -CH₂CDH₂, -CHDCH₃, -CHDCD₂H, -CHDCDH₂, -CHDCD₃, -CD₂CD₃, -CD₂CD₂H, and -CD₂CDH₂;
an n-propyl group, an isopropyl group, an n-butyl group, an isobutyl group, a sec-butyl group, a tert-butyl group, an n-pentyl group, an isopentyl group, a sec-pentyl group, a tert-pentyl group, a phenyl group, and a naphthyl group; and
an n-propyl group, an iso-propyl group, an n-butyl group, an isobutyl group, a sec-butyl group, a tert-butyl group, an n-pentyl group, an isopentyl group, a sec-pentyl group, a tert-pentyl group, a phenyl group, and a naphthyl group, each substituted with at least one selected from deuterium, a C₁-C₁₀ alkyl group, and a phenyl group,
neighboring two or more selected from a plurality of Z₁(s) are optionally connected to each other to form a substituted or unsubstituted C₅-C₃₀ carbocyclic group or a substituted or unsubstituted C₂-C₃₀ heterocyclic group,
e1 is an integer selected from 0 to 8, and
* indicates a binding site to M in Formula 1.

12. The organometallic compound of any of claims 1 to 11, wherein
L₂ in Formula 1 is selected from ligands represented by Formulae 13-1 to 13-47 and 14-1 to 14-28: 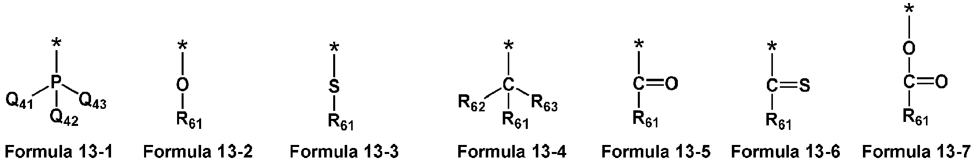 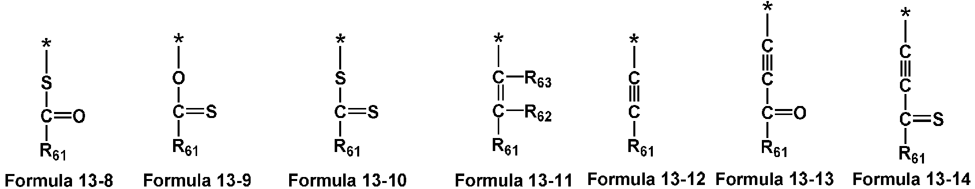 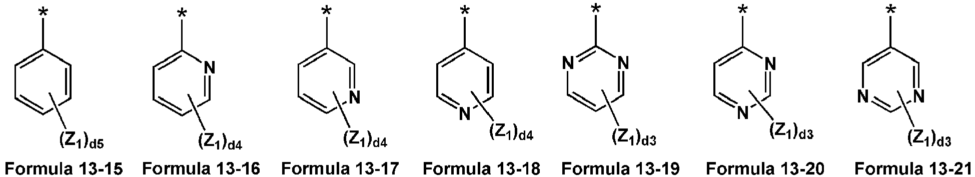 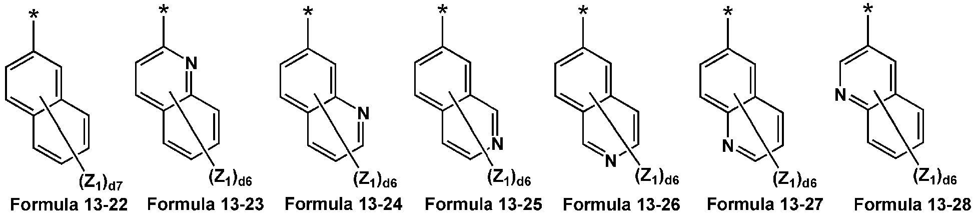 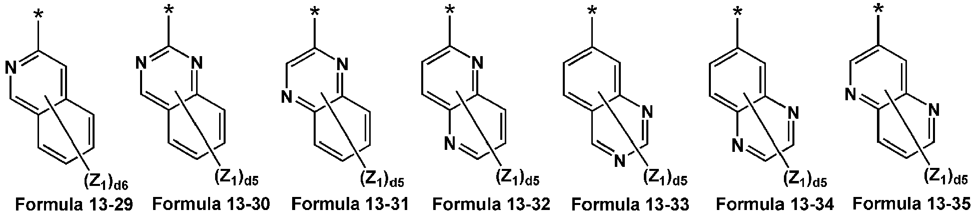 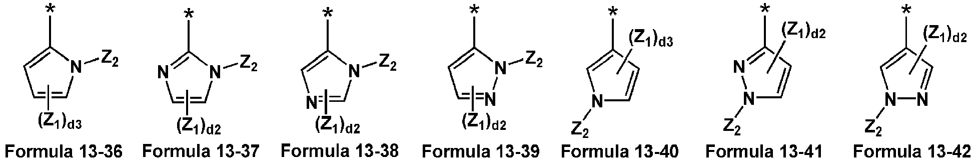 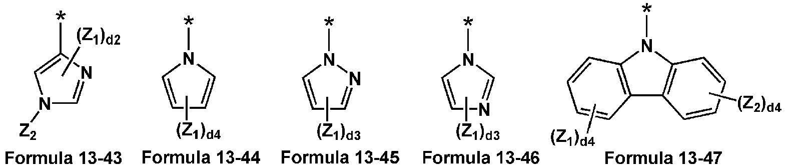 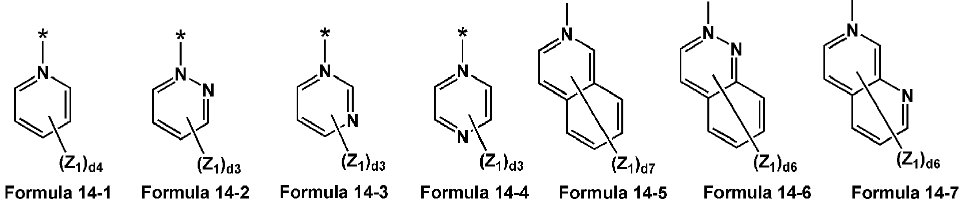 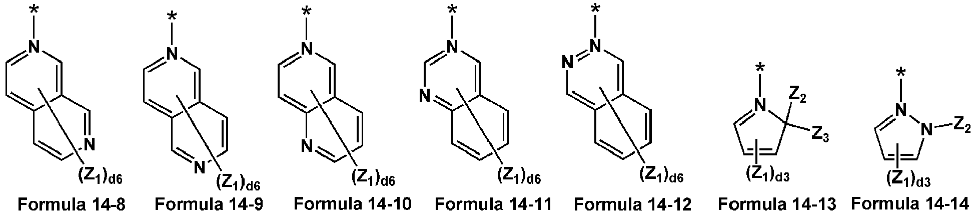 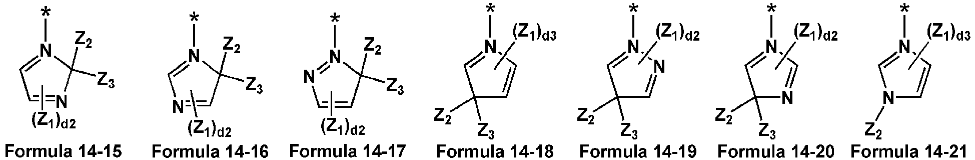 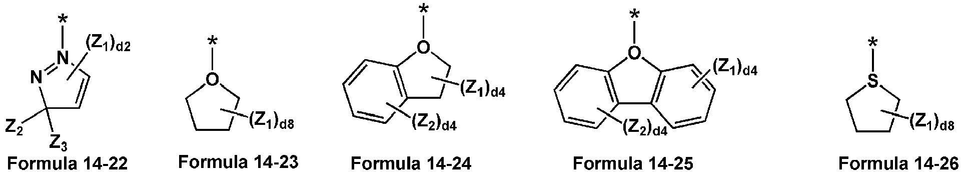 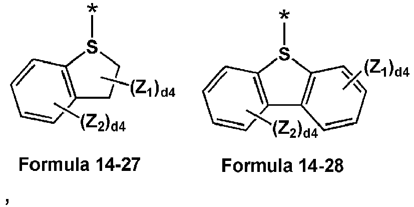
wherein, in Formulae 13-1 to 13-47 and 14-1 to 14-28,
R₆₁ is selected from:
hydrogen, deuterium, -F, -Cl, -Br, -I, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, -SF₅, a C₁-C₂₀ alkyl group, and a C₁-C₂₀ alkoxy group;
a C₁-C₂₀ alkyl group and a C₁-C₂₀ alkoxy group, each substituted with at least one selected from deuterium, -F, -Cl, -Br, -I, -CD₃, -CD₂H, -CDH₂, -CF₃, -CF₂H, -CFH₂, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a C₁-C₁₀ alkyl group, a cyclopentyl group, a cyclohexyl group, a cycloheptyl group, a cyclooctyl group, an adamantanyl group, a norbornanyl group, a norbornenyl group, a cyclopentenyl group, a cyclohexenyl group, a cycloheptenyl group, a phenyl group, a naphthyl group, a pyridinyl group, and a pyrimidinyl group;
a cyclopentyl group, a cyclohexyl group, a cycloheptyl group, a cyclooctyl group, an adamantanyl group, a norbornanyl group, a norbornenyl group, a cyclopentenyl group, a cyclohexenyl group, a cycloheptenyl group, a phenyl group, a naphthyl group, a fluorenyl group, a phenanthrenyl group, an anthracenyl group, a fluoranthenyl group, a triphenylenyl group, a pyrenyl group, a chrysenyl group, a pyrrolyl group, a thiophenyl group, a furanyl group, an imidazolyl group, a pyrazolyl group, a thiazolyl group, an isothiazolyl group, an oxazolyl group, an isoxazolyl group, a pyridinyl group, a pyrazinyl group, a pyrimidinyl group, a pyridazinyl group, an isoindolyl group, an indolyl group, an indazolyl group, a purinyl group, a quinolinyl group, an isoquinolinyl group, a benzoquinolinyl group, a quinoxalinyl group, a quinazolinyl group, a cinnolinyl group, a carbazolyl group, a phenanthrolinyl group, a benzimidazolyl group, a benzofuranyl group, a benzothiophenyl group, an isobenzothiazolyl group, a benzoxazolyl group, an isobenzoxazolyl group, a triazolyl group, a tetrazolyl group, an oxadiazolyl group, a triazinyl group, a dibenzofuranyl group, a dibenzothiophenyl group, a benzocarbazolyl group, a dibenzocarbazolyl group, an imidazopyridinyl group, and an imidazopyrimidinyl group;
a cyclopentyl group, a cyclohexyl group, a cycloheptyl group, a cyclooctyl group, an adamantanyl group, a norbornanyl group, a norbornenyl group, a cyclopentenyl group, a cyclohexenyl group, a cycloheptenyl group, a phenyl group, a naphthyl group, a fluorenyl group, a phenanthrenyl group, an anthracenyl group, a fluoranthenyl group, a triphenylenyl group, a pyrenyl group, a chrysenyl group, a pyrrolyl group, a thiophenyl group, a furanyl group, an imidazolyl group, a pyrazolyl group, a thiazolyl group, an isothiazolyl group, an oxazolyl group, an isoxazolyl group, a pyridinyl group, a pyrazinyl group, a pyrimidinyl group, a pyridazinyl group, an isoindolyl group, an indolyl group, an indazolyl group, a purinyl group, a quinolinyl group, an isoquinolinyl group, a benzoquinolinyl group, a quinoxalinyl group, a quinazolinyl group, a cinnolinyl group, a carbazolyl group, a phenanthrolinyl group, a benzimidazolyl group, a benzofuranyl group, a benzothiophenyl group, an isobenzothiazolyl group, a benzoxazolyl group, an isobenzoxazolyl group, a triazolyl group, a tetrazolyl group, an oxadiazolyl group, a triazinyl group, a dibenzofuranyl group, a dibenzothiophenyl group, a benzocarbazolyl group, a dibenzocarbazolyl group, an imidazopyridinyl group, and an imidazopyrimidinyl group, each substituted with at least one selected from deuterium, -F, -Cl, -Br, -I, -CD₃, -CD₂H, -CDH₂, -CF₃, -CF₂H, -CFH₂, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a C₁-C₂₀ alkyl group, a C₁-C₂₀ alkoxy group, a cyclopentyl group, a cyclohexyl group, a cycloheptyl group, a cyclooctyl group, an adamantanyl group, a norbornanyl group, a norbornenyl group, a cyclopentenyl group, a cyclohexenyl group, a cycloheptenyl group, a phenyl group, a naphthyl group, a fluorenyl group, a phenanthrenyl group, an anthracenyl group, a fluoranthenyl group, a triphenylenyl group, a pyrenyl group, a chrysenyl group, a pyrrolyl group, a thiophenyl group, a furanyl group, an imidazolyl group, a pyrazolyl group, a thiazolyl group, an isothiazolyl group, an oxazolyl group, an isoxazolyl group, a pyridinyl group, a pyrazinyl group, a pyrimidinyl group, a pyridazinyl group, an isoindolyl group, an indolyl group, an indazolyl group, a purinyl group, a quinolinyl group, an isoquinolinyl group, a benzoquinolinyl group, a quinoxalinyl group, a quinazolinyl group, a cinnolinyl group, a carbazolyl group, a phenanthrolinyl group, a benzimidazolyl group, a benzofuranyl group, a benzothiophenyl group, an isobenzothiazolyl group, a benzoxazolyl group, an isobenzoxazolyl group, a triazolyl group, a tetrazolyl group, an oxadiazolyl group, a triazinyl group, a dibenzofuranyl group, a dibenzothiophenyl group, a benzocarbazolyl group, a dibenzocarbazolyl group, an imidazopyridinyl group, and an imidazopyrimidinyl group; and
-N(Q₁)(Q₂), -Si(Q₃)(Q₄)(Q₅), -B(Q₆)(Q₇), -P(=O)(Q₈)(Q₉), and -P(Q₄₁)(Q₄₂)(Q₄₃),
Z₁ to Z₃ are each independently selected from:
hydrogen, deuterium, -F, a cyano group, a nitro group, -SF₅, a methyl group, an ethyl group, an n-propyl group, an iso-propyl group, an n-butyl group, an isobutyl group, a sec-butyl group, a tert-butyl group, an n-pentyl group, an isopentyl group, a sec-pentyl group, a tert-pentyl group, an n-hexyl group, an iso-hexyl group, a sec-hexyl group, a tert-hexyl group, an n-heptyl group, an iso-heptyl group, a sec-heptyl group, a tert-heptyl group, an n-octyl group, an iso-octyl group, a sec-octyl group, a tert-octyl group, an n-nonyl group, an iso-nonyl group, a sec-nonyl group, a tert-nonyl group, an n-decyl group, an iso-decyl group, a sec-decyl group, a tert-decyl group, a methoxy group, an ethoxy group, a propoxy group, a butoxy group, a pentoxy group, a cyclopentyl group, a cyclohexyl group, a cycloheptyl group, a cyclooctyl group, an adamantanyl group, a norbornanyl group, a norbornenyl group, a cyclopentenyl group, a cyclohexenyl group, a cycloheptenyl group, a phenyl group, a naphthyl group, a pyridinyl group, a pyrimidinyl group, a dibenzofuranyl group, and a dibenzothiophenyl group;
a methyl group, an ethyl group, an n-propyl group, an iso-propyl group, an n-butyl group, an isobutyl group, a sec-butyl group, a tert-butyl group, an n-pentyl group, an isopentyl group, a sec-pentyl group, a tert-pentyl group, an n-hexyl group, an iso-hexyl group, a sec-hexyl group, a tert-hexyl group, an n-heptyl group, an iso-heptyl group, a sec-heptyl group, a tert-heptyl group, an n-octyl group, an iso-octyl group, a sec-octyl group, a tert-octyl group, an n-nonyl group, an iso-nonyl group, a sec-nonyl group, a tert-nonyl group, an n-decyl group, an iso-decyl group, a sec-decyl group, a tert-decyl group, a methoxy group, an ethoxy group, a propoxy group, a butoxy group, a pentoxy group, a cyclopentyl group, a cyclohexyl group, a cycloheptyl group, a cyclooctyl group, an adamantanyl group, a norbornanyl group, a norbornenyl group, a cyclopentenyl group, a cyclohexenyl group, a cycloheptenyl group, a phenyl group, a naphthyl group, a pyridinyl group, a pyrimidinyl group, a dibenzofuranyl group, and a dibenzothiophenyl group, each substituted with at least one selected from deuterium, -F, -CD₃, -CD₂H, -CDH₂, -CF₃, -CF₂H, -CFH₂, a cyano group, a nitro group, a C₁-C₁₀ alkyl group, a C₁-C₁₀ alkoxy group, a cyclopentyl group, a cyclohexyl group, a cycloheptyl group, a cyclooctyl group, an adamantanyl group, a norbornanyl group, a norbornenyl group, a cyclopentenyl group, a cyclohexenyl group, a cycloheptenyl group, a phenyl group, a naphthyl group, a pyridinyl group, a pyrimidinyl group, a dibenzofuranyl group, and a dibenzothiophenyl group; and
-N(Q₃₁)(Q₃₂), -Si(Q₃₃)(Q₃₄)(Q₃₅), -B(Q₃₆)(Q₃₇), and -P(=O)(Q₃₈)(Q₃₉),
wherein Q₁ to Q₉, Q₃₁ to Q₃₉, and Q₄₁ to Q₄₃ are each independently selected from:
-CH₃, -CD₃, -CD₂H, -CDH₂, -CH₂CH₃, -CH₂CD₃, -CH₂CD₂H, -CH₂CDH₂, -CHDCH₃, -CHDCD₂H, -CHDCDH₂, -CHDCD₃, -CD₂CD₃, -CD₂CD₂H, and -CD₂CDH₂;
an n-propyl group, an isopropyl group, an n-butyl group, an isobutyl group, a sec-butyl group, a tert-butyl group, an n-pentyl group, an isopentyl group, a sec-pentyl group, a tert-pentyl group, a phenyl group, and a naphthyl group; and
an n-propyl group, an iso-propyl group, an n-butyl group, an isobutyl group, a sec-butyl group, a tert-butyl group, an n-pentyl group, an isopentyl group, a sec-pentyl group, a tert-pentyl group, a phenyl group, and a naphthyl group, each substituted with at least one selected from deuterium, a C₁-C₁₀ alkyl group, and a phenyl group,
d2 is 1 or 2,
d3 is an integer selected from 1 to 3,
d4 is an integer selected from 1 to 4,
d5 is an integer selected from 1 to 5,
d6 is an integer selected from 1 to 6,
d7 is an integer selected from 1 to 7,
d8 is an integer selected from 1 to 8, and
* indicates a binding site to M in Formula 1.

13. The organometallic compound of claim 1, wherein
the organometallic compound is one selected from Compounds 1 to 142:

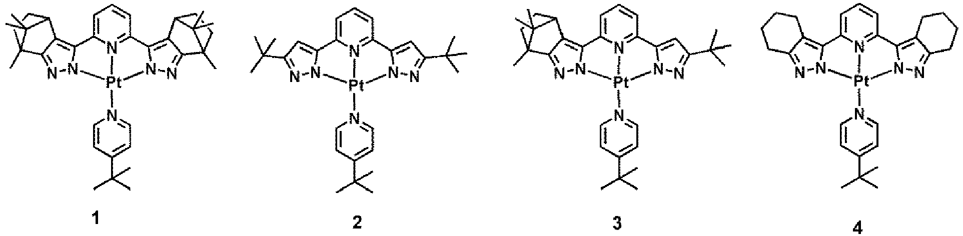

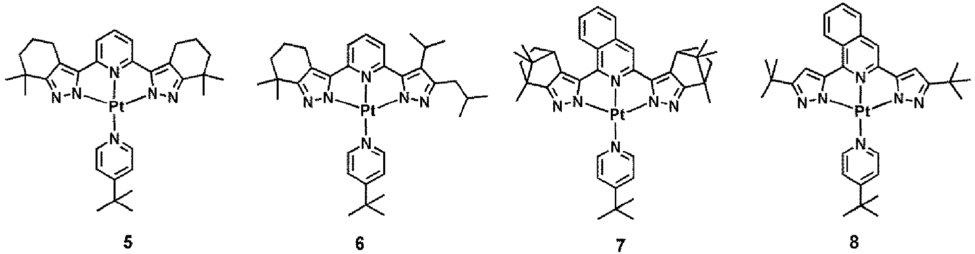

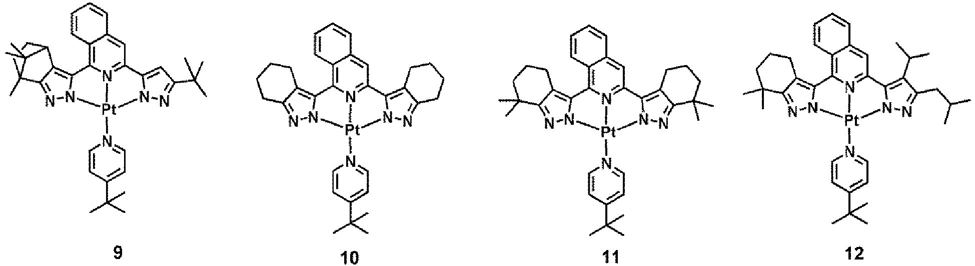

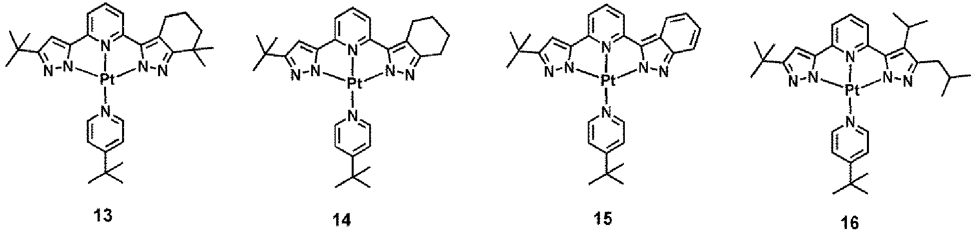

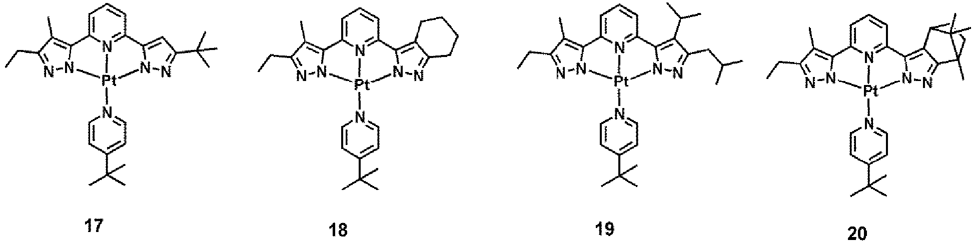

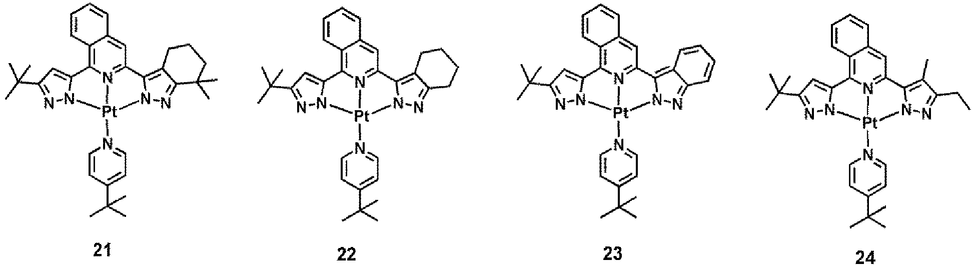

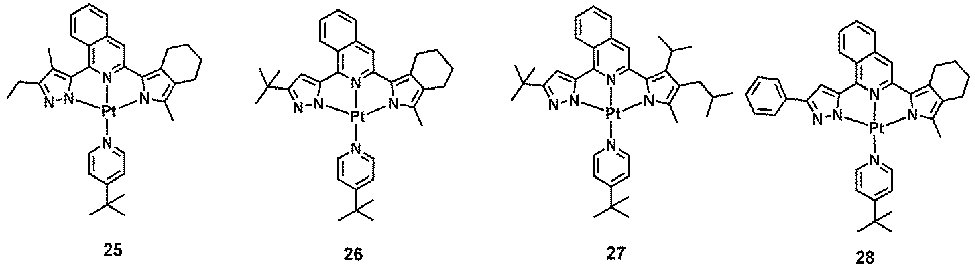

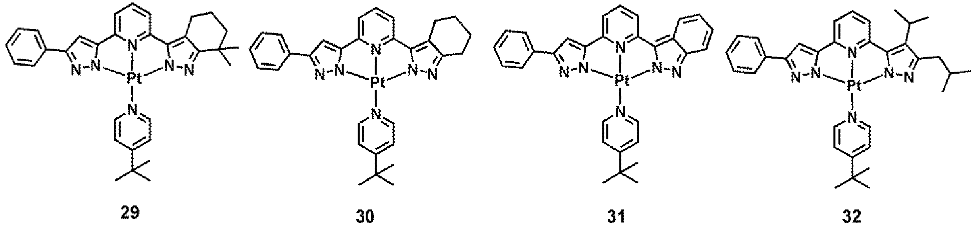

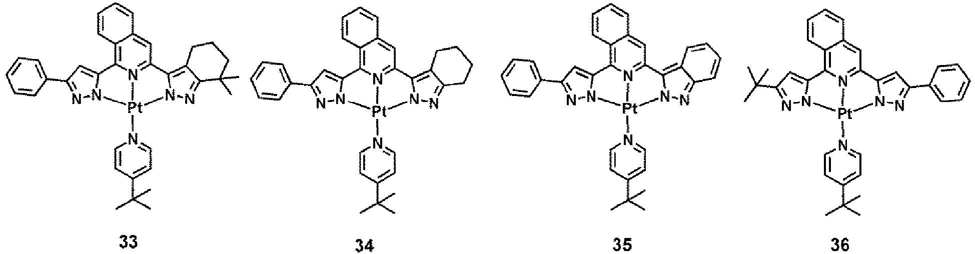

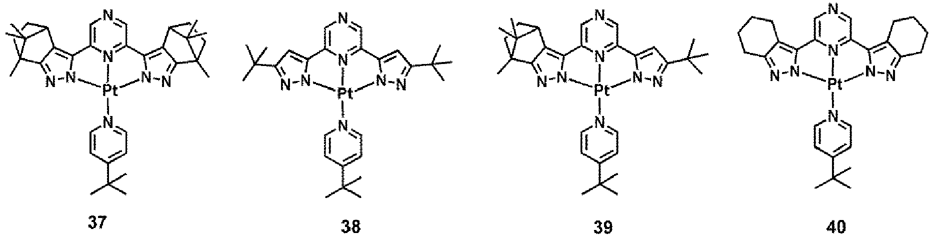

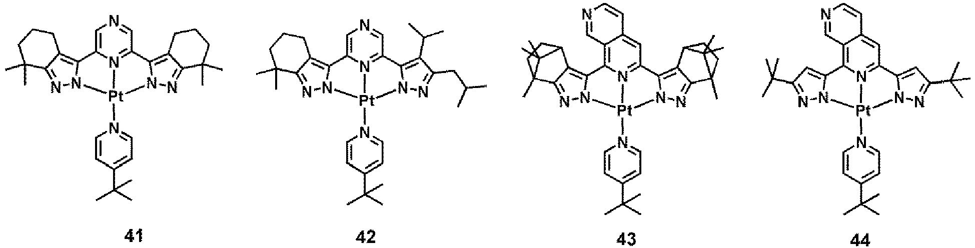

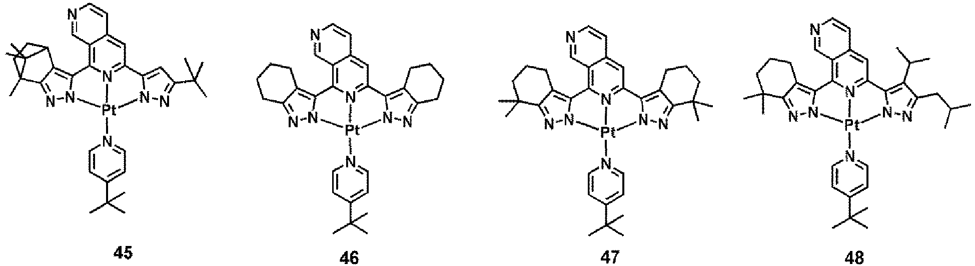

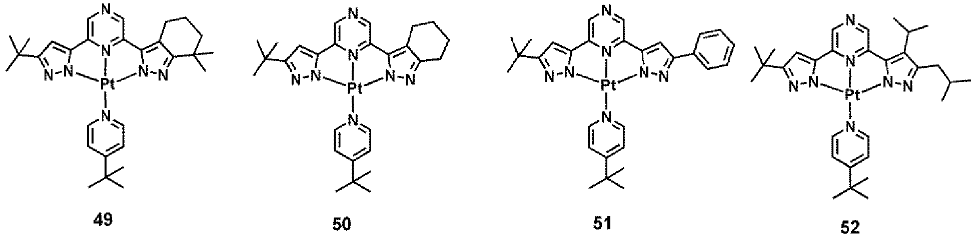

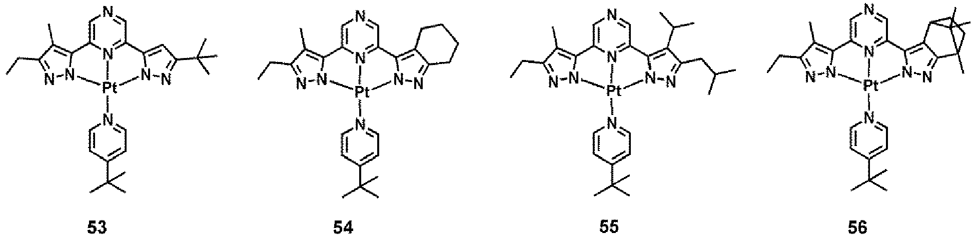

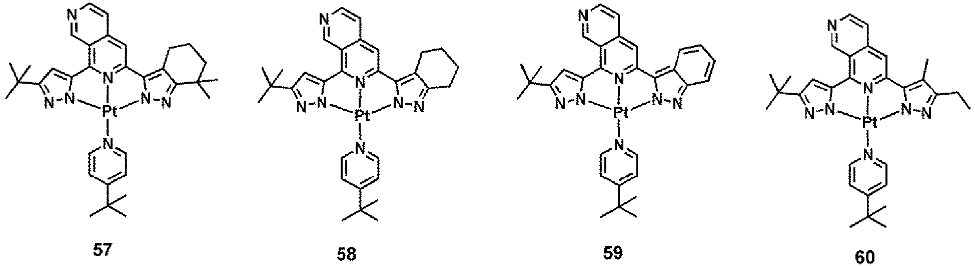

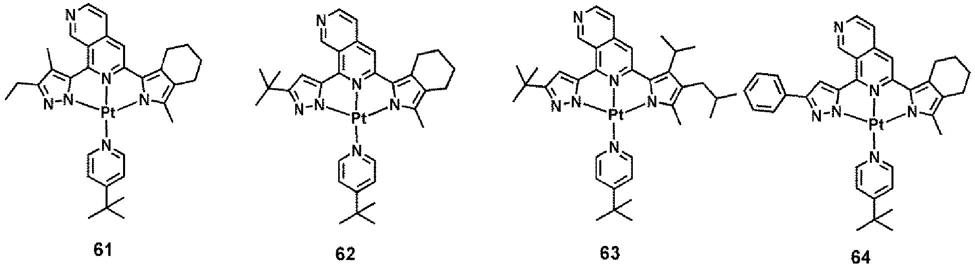

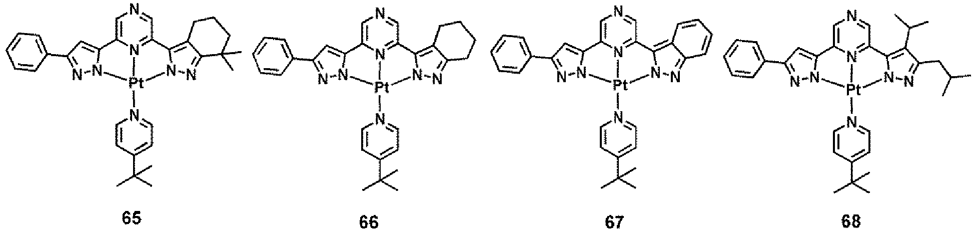

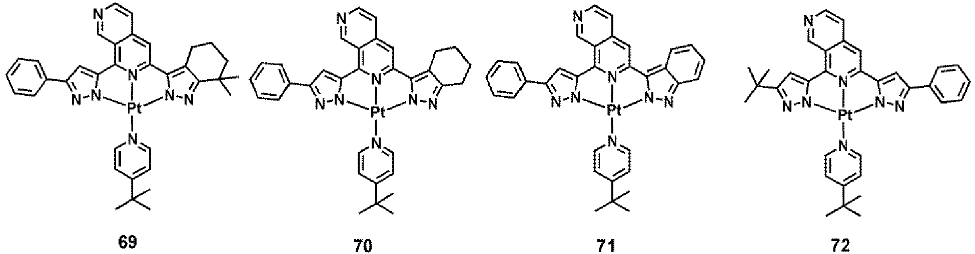

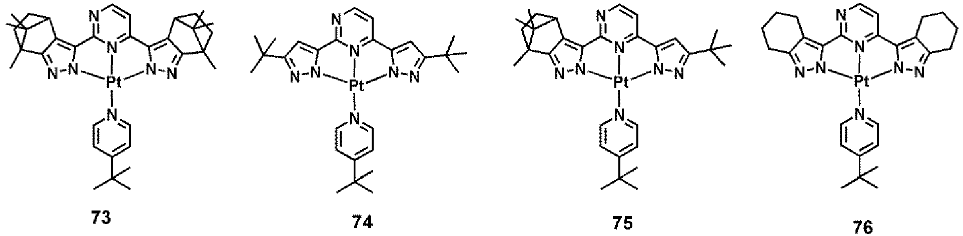

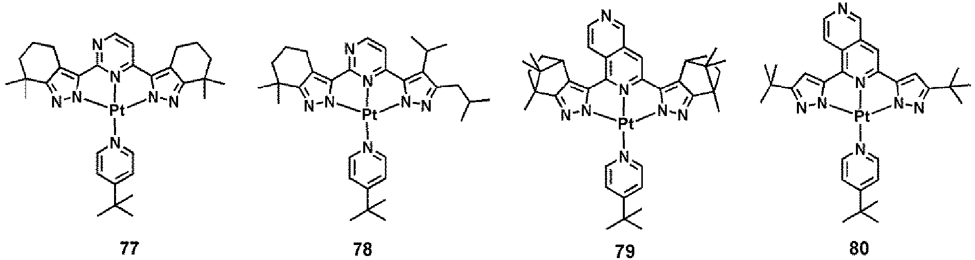

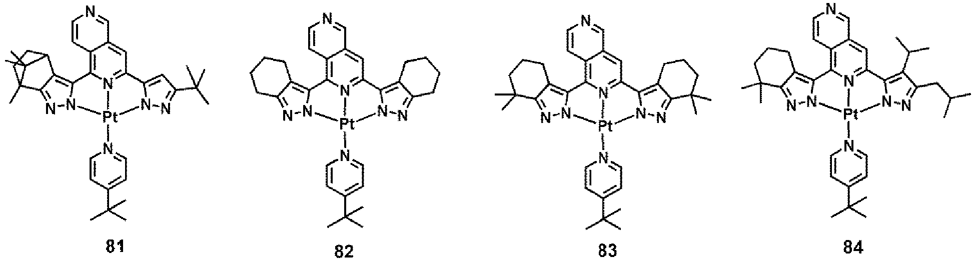

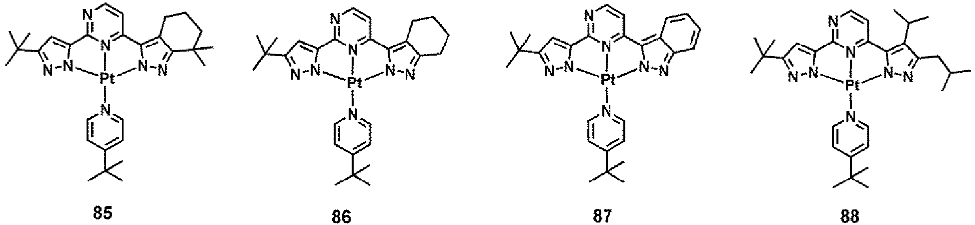

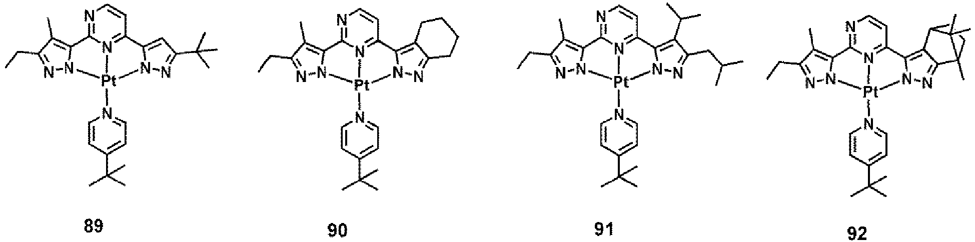

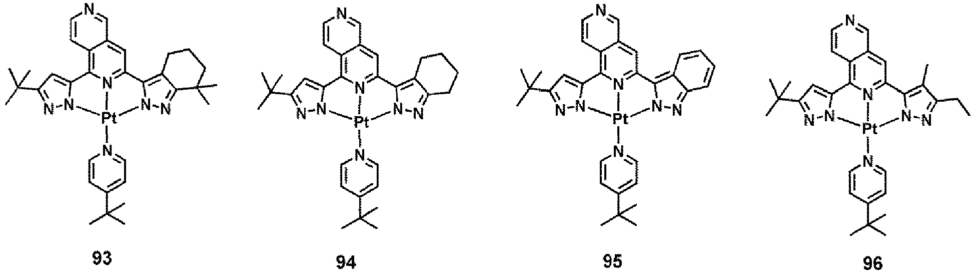

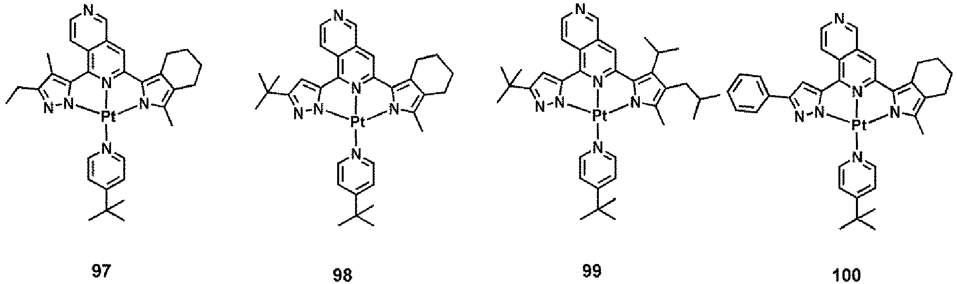

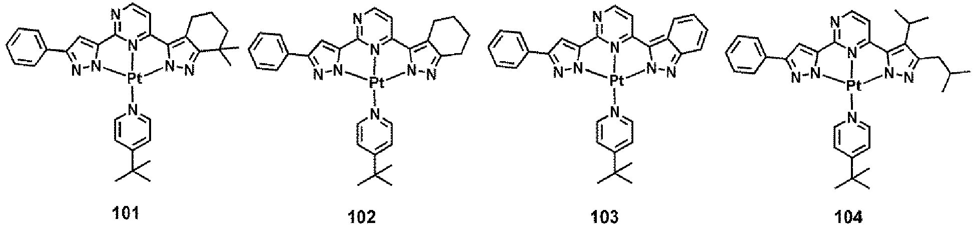

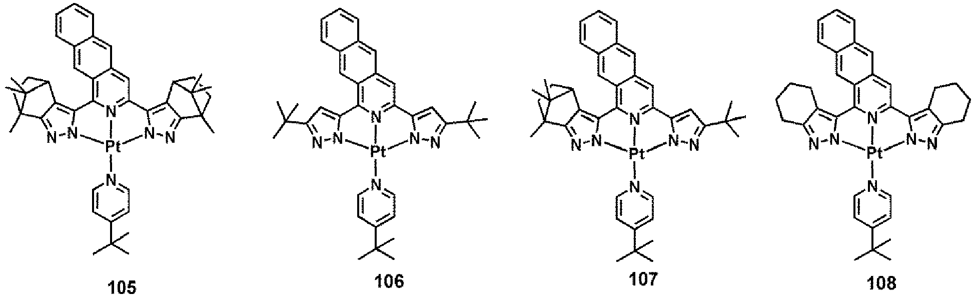

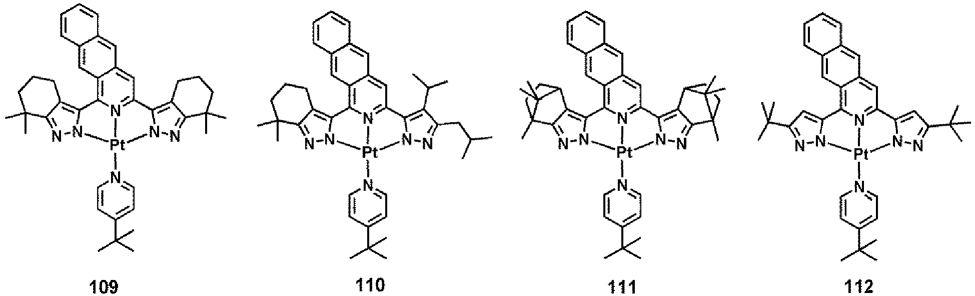

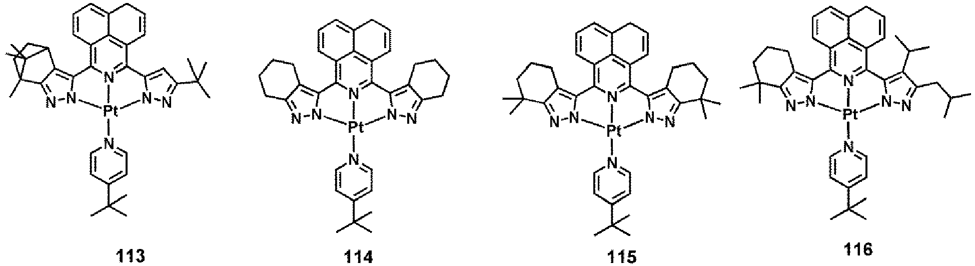

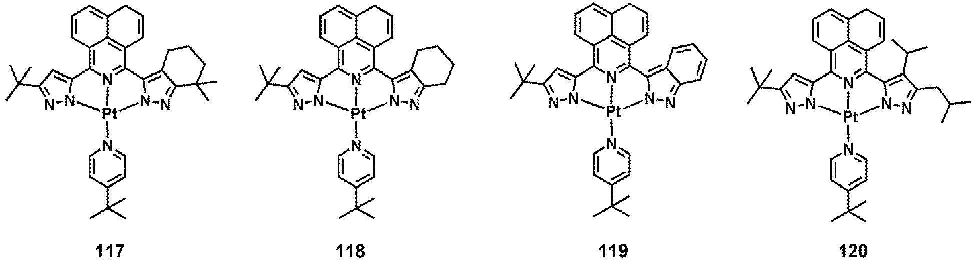

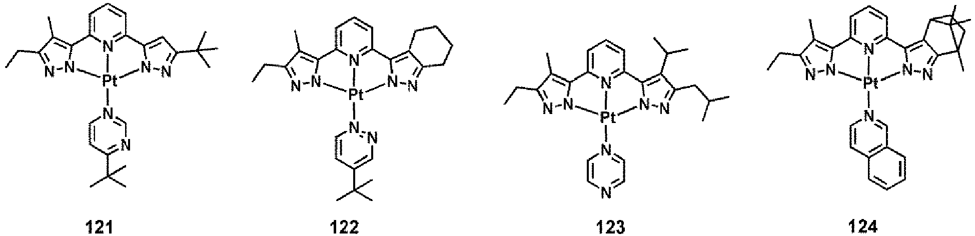

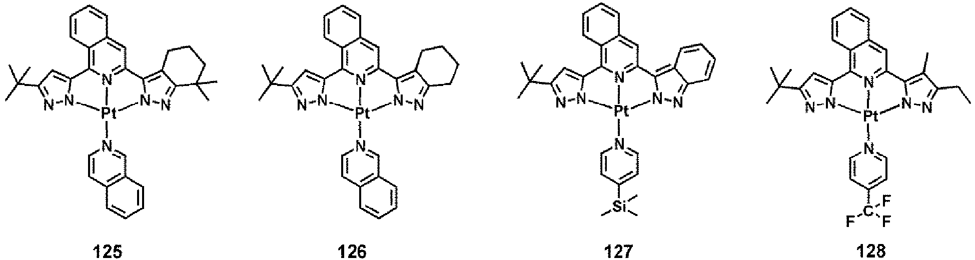

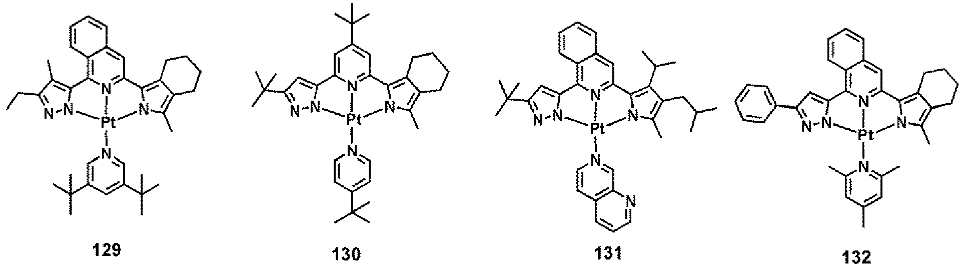

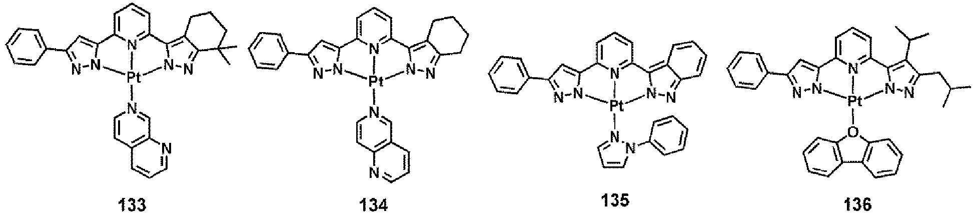

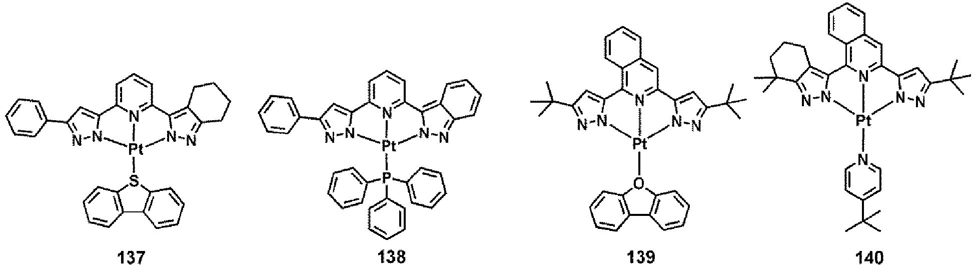

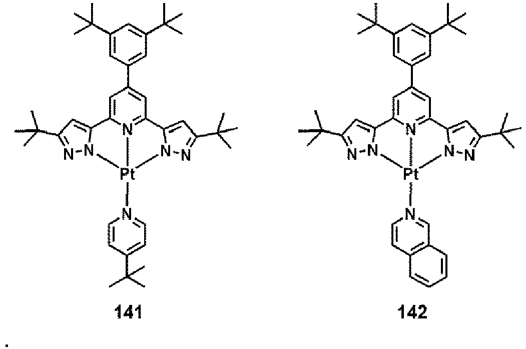

14. An organic light-emitting device comprising:
a first electrode;
a second electrode; and
an organic layer between the first electrode and the second electrode, the organic layer comprising an emission layer,
wherein the organic layer comprises one or more selected from the organometallic compounds of any of claims 1 to 13, preferably wherein
the organometallic compound is included in the emission layer.

15. A composition comprisingone or more selected from the organometallic compounds of any of claims 1 to 13 for use in *in vivo* diagnosis.

16. Use of a composition comprising:
one or more selected from the organometallic compounds of any of claims 1 to 13 for *in vitro* diagnosis.

## Patentansprüche

1. Organometallische Verbindung, dargestellt durch Formel 1:
<Formula 1> M(L₁)(L₂) 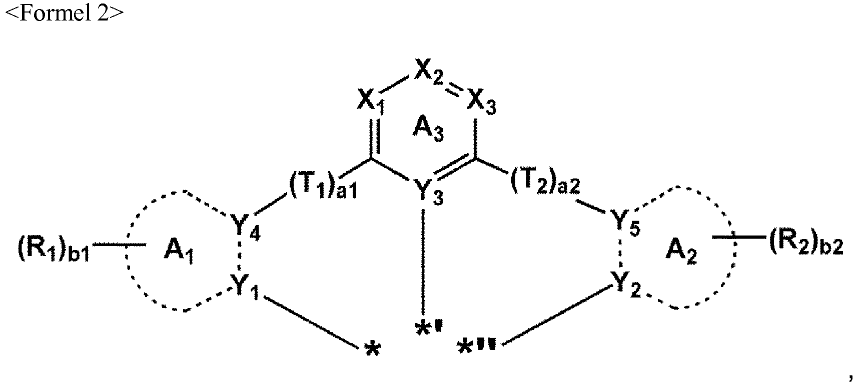
wobei in den Formeln 1 und 2,
M Ruthenium (Ru), Rhodium (Rh), Palladium (pd), Platin (pt) oder Gold (au) ist,
L₁ ausgewählt ist aus dreizahnigen Liganden, dargestellt durch Formel 2, L₂ ausgewählt ist aus einzahnigen organischen Liganden,
*, *' und *" in Formel 2 jeweils eine Bindungsstelle mit M in Formel 1 angeben,
Y₁ bis Y₃ jeweils unabhängig Stickstoff (N) sind,
Y₄ und Y₅ jeweils unabhängig Kohlenstoff (C) sind,
eine Bindung zwischen Y₁ und Y₄ eine Einfachbindung oder eine Doppelbindung ist und eine Bindung zwischen Y₂ und Y₅ eine Einfachbindung oder eine Doppelbindung ist,
eines ausgewählt aus einer Bindung zwischen Y₁ und M, einer Bindung zwischen Y₂ und M und einer Bindung zwischen Ligand L₂ und M eine koordinative Bindung ist und die anderen zwei eine kovalente Bindung sind,
eine Bindung zwischen Y₃ und M eine koordinative Bindung ist,
die Ringe A₁ und A₂ jeweils unabhängig ausgewählt sind aus einem Pyrrol-Ring, einem Pyrazol-Ring und einem Imidazol-Ring und mindestens einer ausgewählt aus den Ringen A₁ und A₂ ein Pyrazol-Ring ist
X₁ N oder C(R₃) ist, X₂ N oder C(R₄) ist, X₃ N oder C(R₅) ist, zwei oder mehrere ausgewählt aus R₃ bis R₅ optional miteinander verbunden sind, um eine substituierte oder unsubstituierte C₅-C₃₀ carbocyclische Gruppe oder eine substituierte oder unsubstituierte C₂-C₃₀ heterocyclische Gruppe zu bilden, und Ring A₃ zwei oder weniger Stickstoffatome als ein ringbildendes Atom hat,
T₁ und T₂ jeweils unabhängig ausgewählt sind aus einer Einfachbindung, *-O-*', *-S-*', *-C(R₆)(R₇)-*', *-C(R₆)=*', *=C(R₆)-*', *-C(R₆)=C(R₇)-*', *-C(=O)-*', *-C(=S)-*', *-C=C-*', *-N(R₆)-*' und *-Si(R₆)(R₇)-*' und R₅ und R₇ optional miteinander verbunden sind, um eine substituierte oder unsubstituierte C₅-C₃₀ carbocyclische Gruppe oder eine substituierte oder unsubstituierte C₂-C₃₀ heterocyclische Gruppe zu bilden,
a1 und a2 jeweils unabhängig eine Ganzzahl ausgewählt aus 1 bis 3 sind,
R₁ bis R₇ jeweils unabhängig ausgewählt sind aus Wasserstoff, Deuterium, -F, -Cl, -Br, -I, -SF₅, einer Hydroxyl-Gruppe, einer Cyano-Gruppe, einer Nitro-Gruppe, einer Amino-Gruppe, einer Amidino-Gruppe, einer Hydrazin-Gruppe, einer Hydrazon-Gruppe, einer Carbonsäure-Gruppe oder einem Salz davon, einer Sulfonsäure-Gruppe oder einem Salz davon, einer Phosphorsäure-Gruppe oder einem Salz davon, einer substituierten oder unsubstituierten C₁-C₆₀-Alkyl-Gruppe, einer substituierten oder unsubstituierten C₂-C₆₀-Alkenyl-Gruppe, einer substituierten oder unsubstituierten C₂-C₆₀-Alkynyl-Gruppe, einer substituierten oder unsubstituierten C₁-C₆₀-Alkoxy-Gruppe, einer substituierten oder unsubstituierten C₃-C₁₀-Cycloalkyl-Gruppe, einer substituierten oder unsubstituierten C₁-C₁₀-Heterocycloalkyl-Gruppe, einer substituierten oder unsubstituierten C₃-C₁₀-Cycloalkenyl-Gruppe, einer substituierten oder unsubstituierten C₁-C₁₀-Heterocycloalkenyl-Gruppe, einer substituierten oder unsubstituierten C₆-C₆₀-Aryl-Gruppe, einer substituierten oder unsubstituierten C₆-C₆₀-Aryloxy-Gruppe, einer substituierten oder unsubstituierten C₆-C₆₀-Arylthio-Gruppe, einer substituierten oder unsubstituierten C₁-C₆₀-Heteroaryl-Gruppe, einer substituierten oder unsubstituierten monovalenten nichtaromatischen kondensierten polycyclischen Gruppe, einer substituierten oder unsubstituierten monovalenten nichtaromatischen kondensierten heteropolycyclischen-Gruppe, -N(Q₁)(Q₂), -Si(Q₃)(Q₄)(Q₅), -B(Q₆)(Q₇) und -P(=O)(Q₈)(Q₉),
b1 und b2 jeweils unabhängig eine Ganzzahl ausgewählt aus 0 bis 3 sind, wobei, wenn b1 zwei oder mehr ist, zwei oder mehrere R₁ identisch miteinander oder verschieden voneinander sind, und wenn b2 zwei oder mehr ist, zwei oder mehrere R₂ identisch miteinander oder verschieden voneinander sind, zwei ausgewählt aus R₁ in der Zahl von b1 optional verbunden sind, um eine substituierte oder unsubstituierte C₅-C₃₀ carbocyclische Gruppe oder eine substituierte oder unsubstituierte C₂-C₃₀ heterocyclische Gruppe zu bilden,
zwei ausgewählt aus R₂ in der Zahl von b2 optional verbunden sind, um eine substituierte oder unsubstituierte C₅-C₃₀ carbocyclische Gruppe oder eine substituierte oder unsubstituierte C₂-C₃₀ heterocyclische Gruppe zu bilden,
eine Gruppierung, dargestellt durch 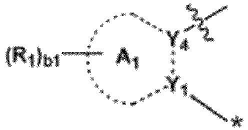 in Formel 2 nicht 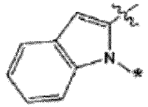 und 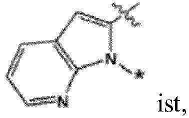 und eine Gruppierung, dargestellt durch 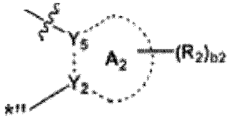 in Formel 2 nicht 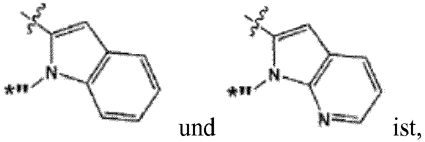 und
mindestens ein Substituent, ausgewählt aus einem Substituenten der substituierten C₅-C₃₀ carbocyclischen Gruppe, der substituierten C₂-C₃₀ heterocyclischen Gruppe, der substituierten C₁-C₆₀-Alkyl-Gruppe, der substituierten C₂-C₆₀-Alkenyl-Gruppe, der substituierten C₂-C₆₀-Alkynyl-Gruppe, der substituierten C₁-C₆₀-Alkoxy-Gruppe, der substituierten C₃-C₁₀-Cycloalkyl-Gruppe, der substituierten C₁-C₁₀-Heterocycloalkyl-Gruppe, der substituierten C₃-C₁₀-Cycloalkenyl-Gruppe, der substituierten C₁-C₁₀-Heterocycloalkenyl-Gruppe, der substituierten C₆-C₆₀-Aryl-Gruppe, der substituierten C₆-C₆₀-Aryloxy-Gruppe, der substituierten C₆-C₆₀-Arylthio-Gruppe, der substituierten C₁-C₆₀-Heteroaryl-Gruppe, der substituierten monovalenten nichtaromatischen kondensierten polycyclischen Gruppe und der substituierten monovalenten nichtaromatischen kondensierten heteropolycyclischen Gruppe, ausgewählt ist aus: Deuterium, -F, -Cl, -Br, -I, -CD₃, -CD₂H, -CDH₂, -CF₃, -CF₂H, -CFH₂, einer Hydroxyl-Gruppe, einer Cyano-Gruppe, einer Nitro-Gruppe, einer Amino-Gruppe, einer Amidino-Gruppe, einer Hydrazin-Gruppe, einer Hydrazon-Gruppe, einer Carbonsäure-Gruppe oder einem Salz davon, einer Sulfonsäure-Gruppe oder einem Salz davon, einer Phosphorsäure-Gruppe oder einem Salz davon, einer C₁-C₆₀-Alkyl-Gruppe, einer C₂-C₆₀-Alkenyl-Gruppe, einer C₂-C₆₀-Alkynyl-Gruppe und einer C₁-C₆₀-Alkoxy-Gruppe;
einer C₁-C₆₀-Alkyl-Gruppe, einer C₂-C₆₀-Alkenyl-Gruppe, einer C₂-C₆₀-Alkynyl-Gruppe und einer C₁-C₆₀-Alkoxy-Gruppe, jeweils substituiert mit mindestens one ausgewählt aus Deuterium, -F, -Cl, -Br, -I, -CD₃, -CD₂H, -CDH₂, -CF₃, -CF₂H, -CFH₂, einer Hydroxyl-Gruppe, einer Cyano-Gruppe, einer Nitro-Gruppe, einer Amino-Gruppe, einer Amidino-Gruppe, einer Hydrazin-Gruppe, einer Hydrazon-Gruppe, einer Carbonsäure-Gruppe oder einem Salz davon, einer Sulfonsäure-Gruppe oder einem Salz davon, einer Phosphorsäure-Gruppe oder einem Salz davon, einer C₃-C₁₀-Cycloalkyl-Gruppe, einer C₁-C₁₀-Heterocycloalkyl-Gruppe, einer C₃-C₁₀-Cycloalkenyl-Gruppe, einer C₁-C₁₀-Heterocycloalkenyl-Gruppe, einer C₆-C₆₀-Aryl-Gruppe, einer C₆-C₆₀-Aryloxy-Gruppe, einer C₆-C₆₀-Arylthio-Gruppe, einer C₁-C₆₀-Heteroaryl-Gruppe, einer monovalenten nichtaromatischen kondensierten polycyclischen Gruppe, einer monovalenten nichtaromatischen kondensierten heteropolycyclischen Gruppe, -N(Q₁₁)(Q₁₂), -Si(Q₁₃)(Q₁₄)(Q₁₅), -B(Q₁₆)(Q₁₇) und -P(=O)(Q₁₈)(Q₁₉);
einer C₃-C₁₀-Cycloalkyl-Gruppe, einer C₁-C₁₀-Heterocycloalkyl-Gruppe, einer C₃-C₁₀-Cycloalkenyl-Gruppe, einer C₁-C₁₀-Heterocycloalkenyl-Gruppe, einer C₆-C₆₀-Aryl-Gruppe, einer C₆-C₆₀-Aryloxy-Gruppe, einer C₆-C₆₀-Arylthio-Gruppe, einer C₁-C₆₀-Heteroaryl-Gruppe, einer monovalenten nichtaromatischen kondensierten polycyclischen Gruppe und einer monovalenten nichtaromatischen kondensierten heteropolycyclischen Gruppe;
einer C₃-C₁₀-Cycloalkyl-Gruppe, einer C₁-C₁₀-Heterocycloalkyl-Gruppe, einer C₃-C₁₀-Cycloalkenyl-Gruppe, einer C₁-C₁₀-Heterocycloalkenyl-Gruppe, einer C₆-C₆₀-Aryl-Gruppe, einer C₆-C₆₀-Aryloxy-Gruppe, einer C₆-C₆₀-Arylthio-Gruppe, einer C₁-C₆₀-Heteroaryl-Gruppe, einer monovalenten nichtaromatischen kondensierten polycyclischen Gruppe und einer monovalenten nichtaromatischen kondensierten heteropolycyclischen Gruppe, jeweils substituiert mit mindestens einem, ausgewählt aus Deuterium, -F, -Cl, -Br, -I, -CD₃, -CD₂H, -CDH₂, -CF₃, -CF₂H, -CFH₂, einer Hydroxyl-Gruppe, einer Cyano-Gruppe, einer Nitro-Gruppe, einer Amino-Gruppe, einer Amidino-Gruppe, einer Hydrazin-Gruppe, einer Hydrazon-Gruppe, einer Carbonsäure-Gruppe oder einem Salz davon, einer Sulfonsäure-Gruppe oder einem Salz davon, einer Phosphorsäure-Gruppe oder einem Salz davon, einer C₁-C₆₀-Alkyl-Gruppe, einer C₂-C₆₀-Alkenyl-Gruppe, einer C₂-C₆₀-Alkynyl-Gruppe, einer C₁-C₆₀-Alkoxy-Gruppe, einer C₃-C₁₀-Cycloalkyl-Gruppe, einer C₁-C₁₀-Heterocycloalkyl-Gruppe, einer C₃-C₁₀-Cycloalkenyl-Gruppe, einer C₁-C₁₀-Heterocycloalkenyl-Gruppe, einer C₆-C₆₀-Aryl-Gruppe, einer C₆-C₆₀-Aryloxy-Gruppe, einer C₆-C₆₀-Arylthio-Gruppe, einer C₁-C₆₀-Heteroaryl-Gruppe, einer monovalenten nichtaromatischen kondensierten polycyclischen Gruppe und einer monovalenten nichtaromatischen kondensierten heteropolycyclischen Gruppe, -N(Q₂₁)(Q₂₂), -Si(Q₂₃)(Q₂₄)(Q₂₅), -B(Q₂₆)(Q₂₇) und -P(=O)(Q₂₈)(Q₂₉); und
-N(Q₃₁)(Q₃₂), -Si(Q₃₃)(Q₃₄)(Q₃₅), -B(Q₃₆)(Q₃₇) und -P(=O)(Q₃₈)(Q₃₉),
wobei Q₁ bis Q₉, Q₁₁ bis Q₁₉, Q₂₁ bis Q₂₉ und Q₃₁ bis Q₃₉ jeweils unabhängig ausgewählt sind aus Wasserstoff, Deuterium, -F, -Cl, -Br, -I, einer Hydroxyl-Gruppe, einer Cyano-Gruppe, einer Nitro-Gruppe, einer Amino-Gruppe, einer Amidino-Gruppe, einer Hydrazin-Gruppe, einer Hydrazon-Gruppe, einer Carbonsäure-Gruppe oder einem Salz davon, einer Sulfonsäure-Gruppe oder einem Salz davon, einer Phosphorsäure-Gruppe oder einem Salz davon, einer C₁-C₆₀-Alkyl-Gruppe, einer C₂-C₆₀-Alkenyl-Gruppe, einer C₂-C₆₀-Alkynyl-Gruppe, einer C₁-C₆₀-Alkoxy-Gruppe, einer C₃-C₁₀-Cycloalkyl-Gruppe, einer C₁-C₁₀-Heterocycloalkyl-Gruppe, einer C₃-C₁₀-Cycloalkenyl-Gruppe, einer C₁-C₁₀-Heterocycloalkenyl-Gruppe, einer C₆-C₆₀-Aryl-Gruppe, einer C₆-C₆₀-Aryl-Gruppe substituiert mit mindestens einem, ausgewählt aus einer C₁-C₆₀-Alkyl-Gruppe und einer C₆-C₆₀-Aryl-Gruppe, einer C₆-C₆₀-Aryloxy-Gruppe, einer C₆-C₆₀-Arylthio-Gruppe, einer C₁-C₆₀-Heteroaryl-Gruppe, einer monovalenten nichtaromatischen kondensierten polycyclischen Gruppe und einer monovalenten nichtaromatischen kondensierten heteropolycyclischen Gruppe.

2. Organometallische Verbindung nach Anspruch 1, wobei
die Bindung zwischen Y₁ und M und die Bindung zwischen Y₂ und M eine kovalente Bindung sind, und die Bindung zwischen Y₃ und M und die Bindung zwischen dem Liganden L₂ und M eine koordinative Bindung sind, oder
die Bindung zwischen Y₁ und M und die Bindung zwischen dem Liganden L₂ und M eine kovalente Bindung sind, und die Bindung zwischen Y₃ und M und die Bindung zwischen Y₂ und M eine koordinative Bindung sind.

3. Organometallische Verbindung nach Anspruch 1 oder 2, wobei
eine Gruppierung, dargestellt durch 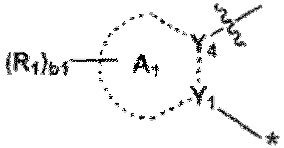 in Formel 2 identisch wie eine Gruppierung ist, die durch 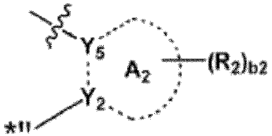 in Formel 2 dargestellt ist, oder wobei eine Gruppierung, dargestellt durch 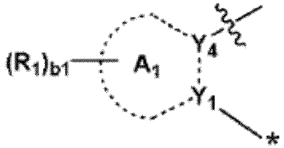 in Formel 2 verschieden von einer Gruppierung ist, die durch 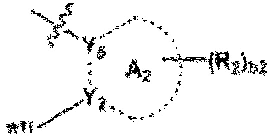 in Formel 2 dargestellt ist.

4. Organometallische Verbindung nach einem der Ansprüche 1 bis 3, wobei
eine Gruppierung, dargestellt durch 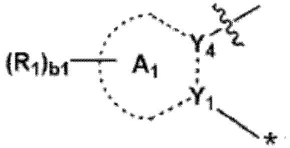 in Formel 2, durch eine dargestellt ist, die aus den Formeln 3-1 bis 3-16 und 3-31 bis 3-70 ausgewählt ist,
eine Gruppierung, dargestellt durch 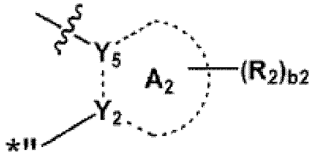 in Formel 2, durch eine dargestellt ist, die aus den Formeln 4-1 bis 4-16 und 4-31 bis 4-70 ausgewählt ist, und
eine Gruppierung, dargestellt durch 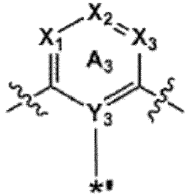 in Formel 2, durch eine dargestellt ist, die aus den Formeln 5-1 bis 5-47 ausgewählt ist: 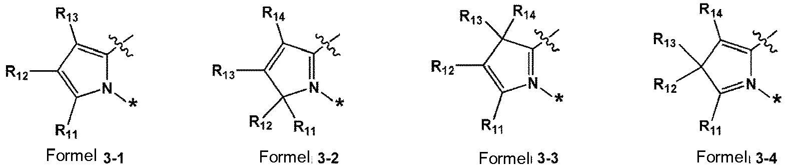 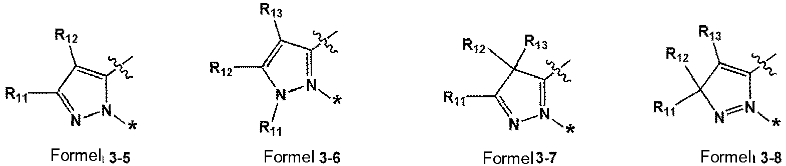 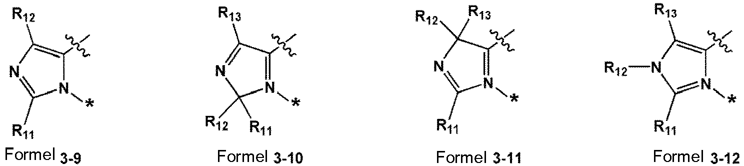 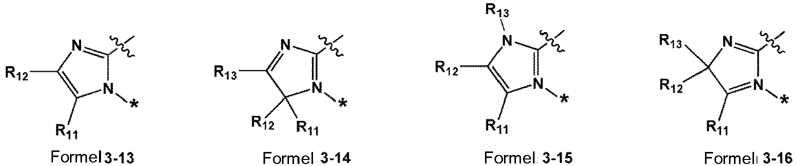 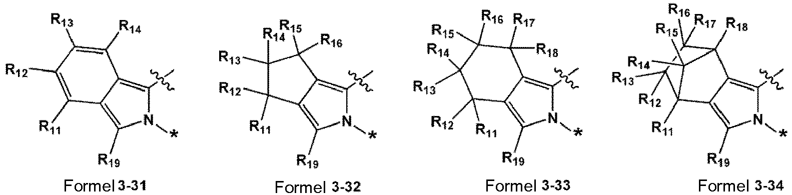 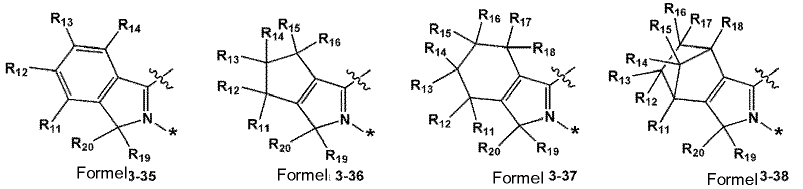 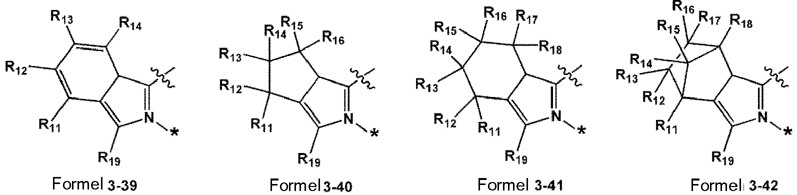 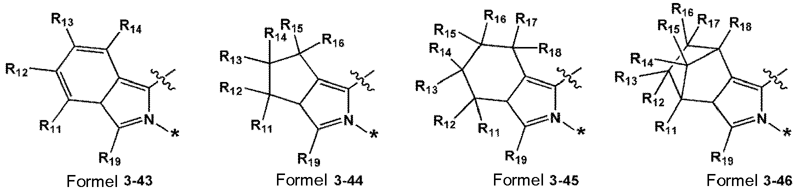 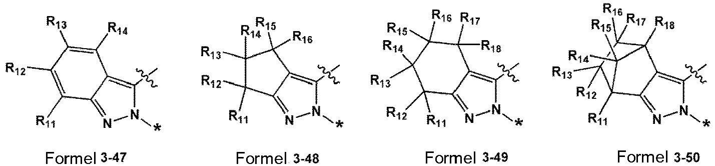 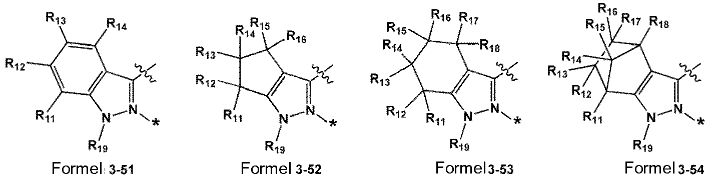 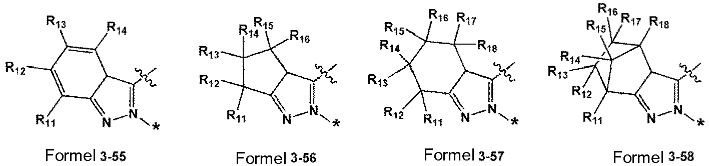 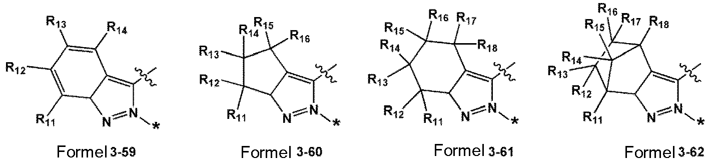 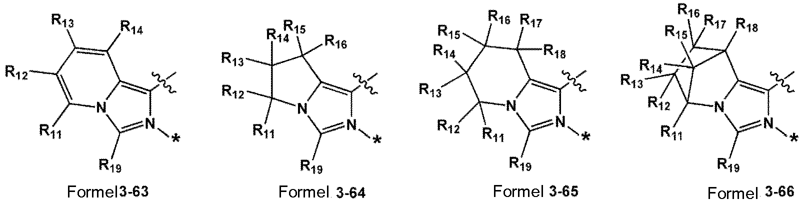 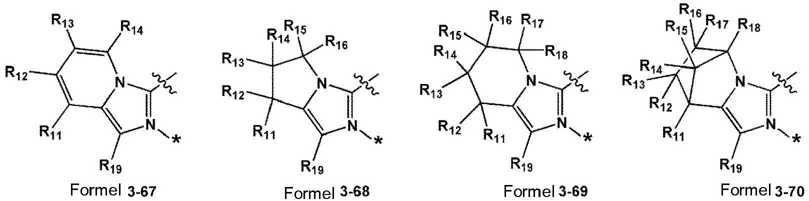 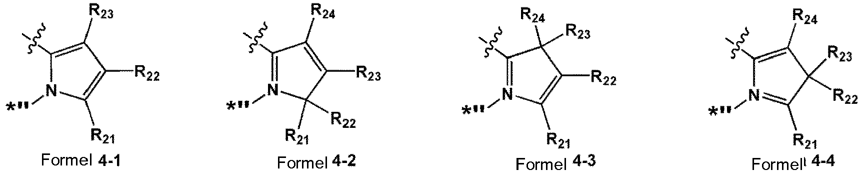 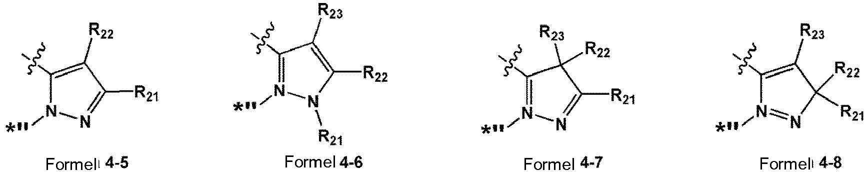 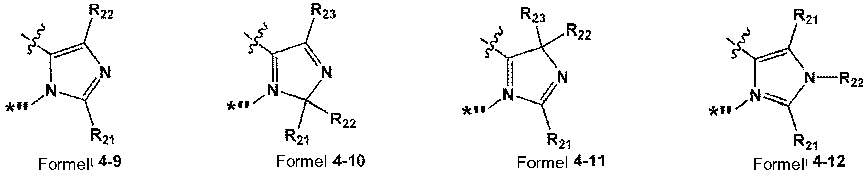 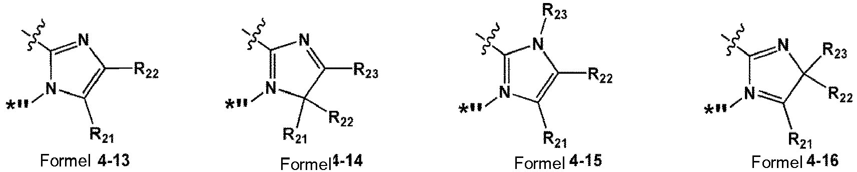 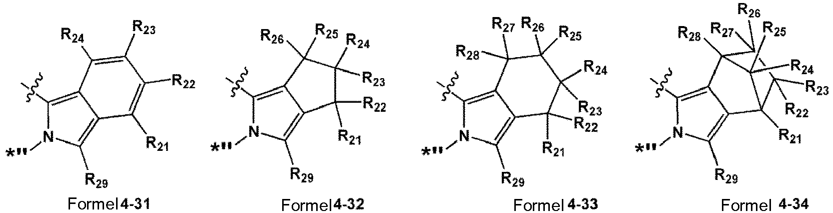 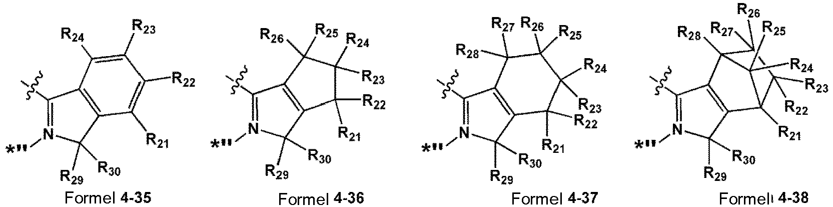 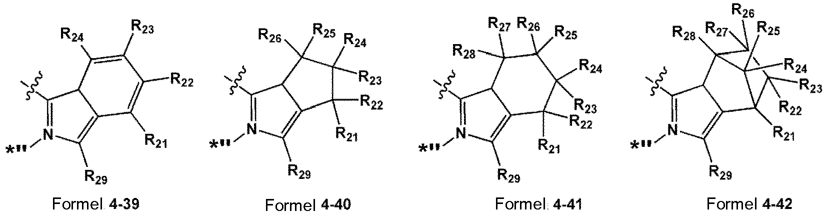 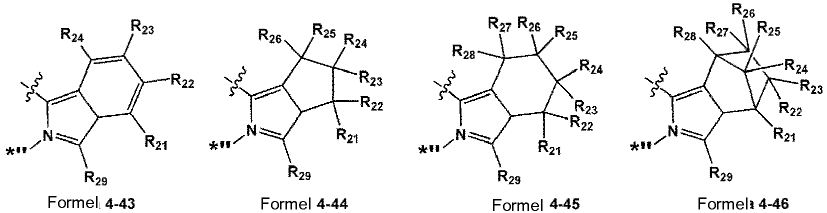 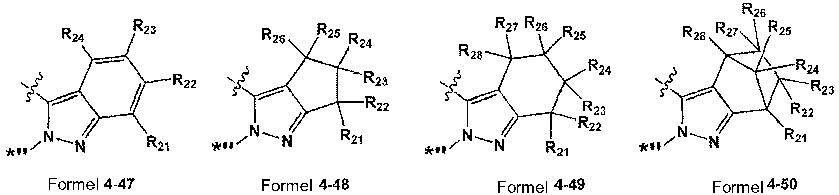 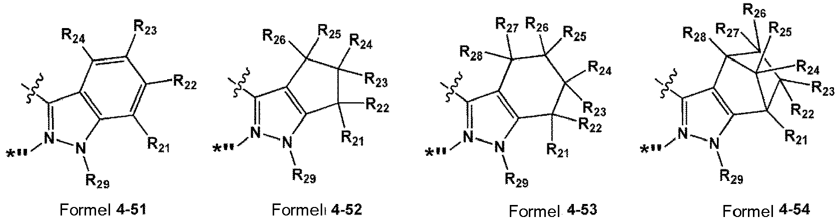 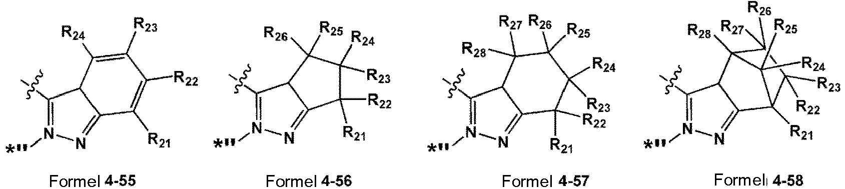 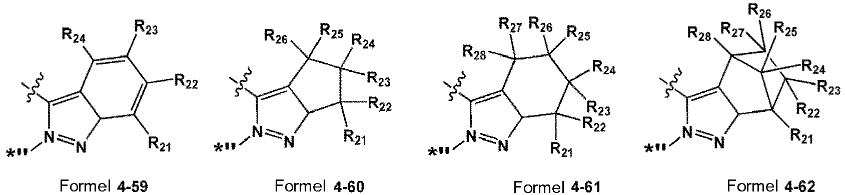 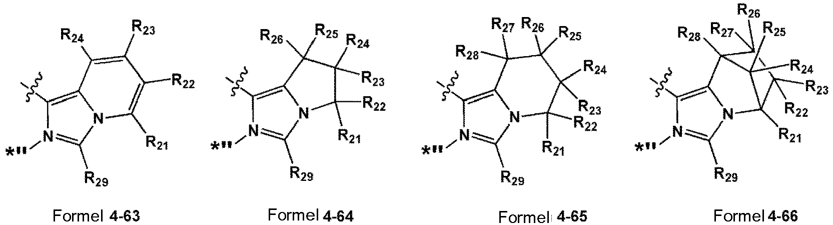 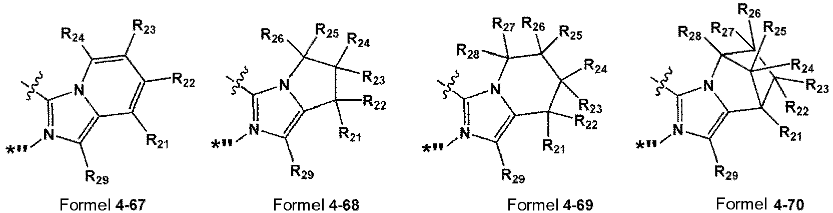 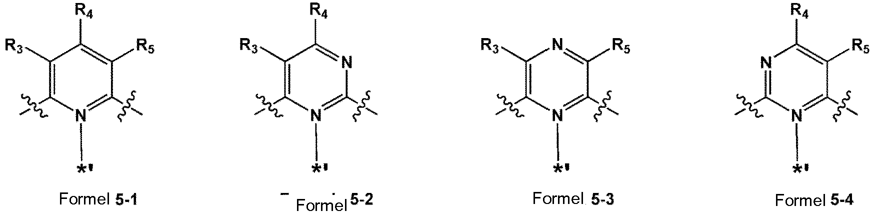 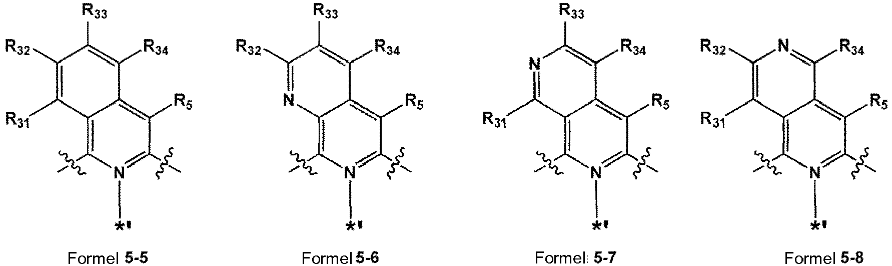 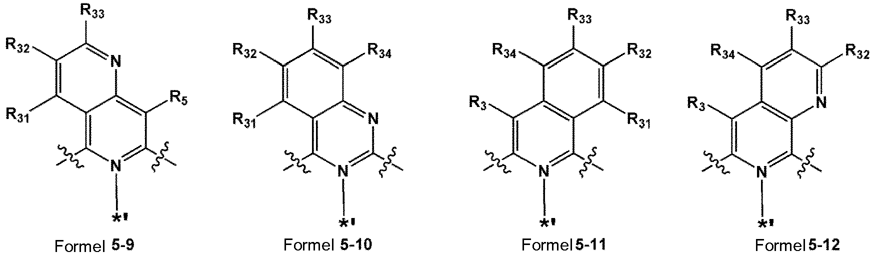 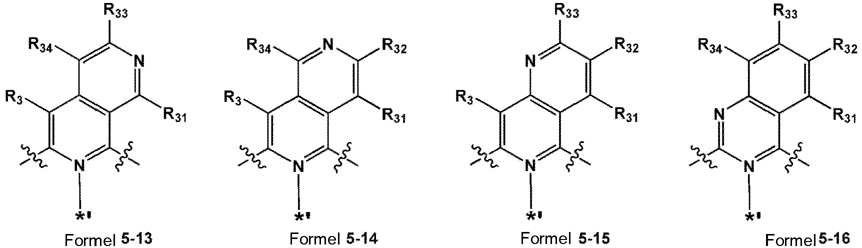 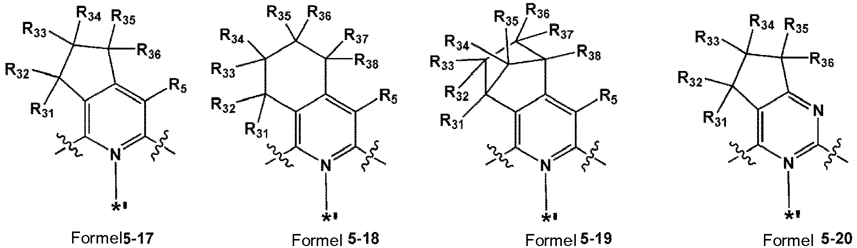 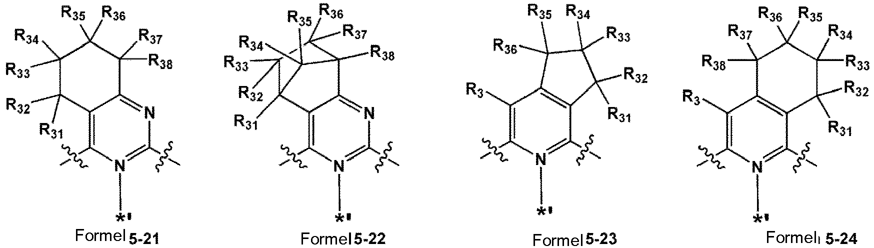 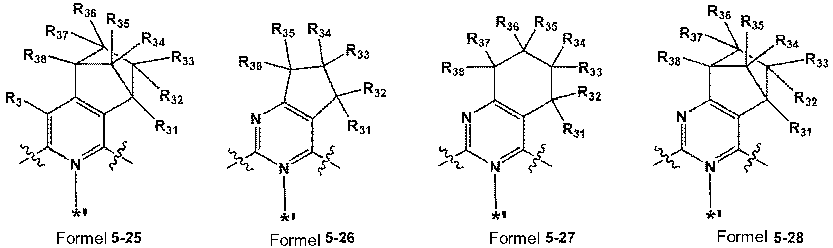 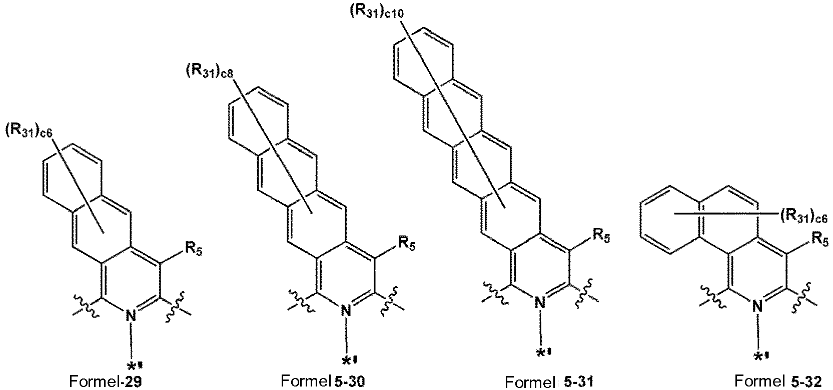 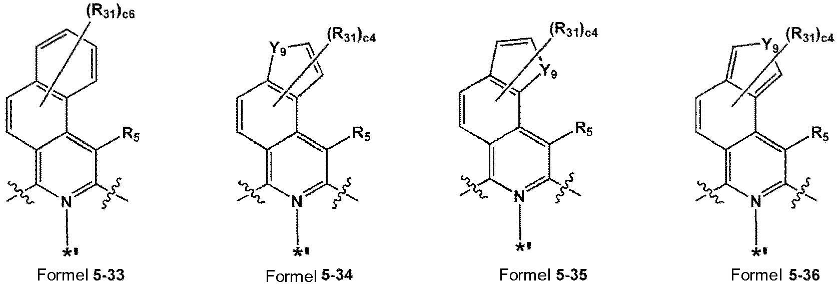 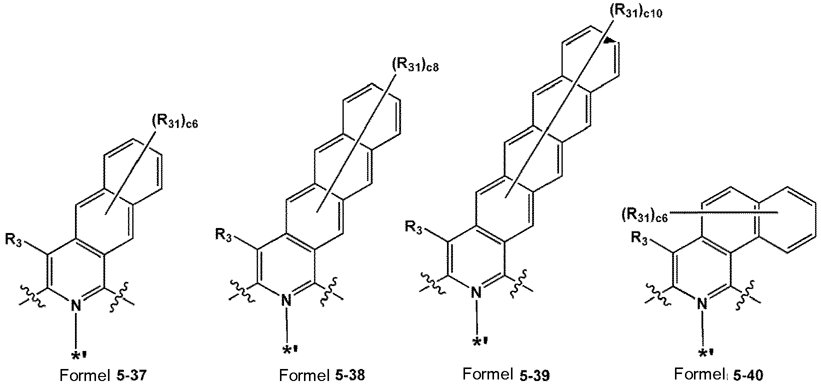 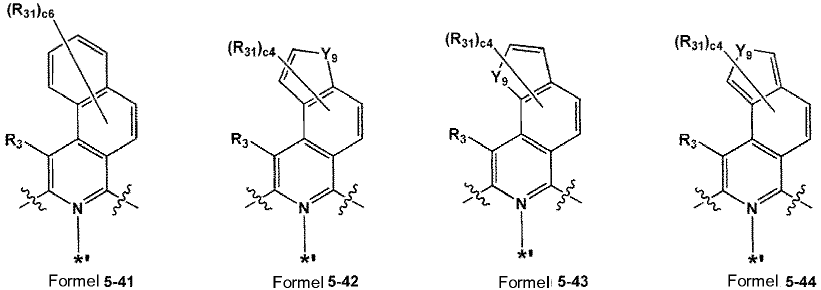 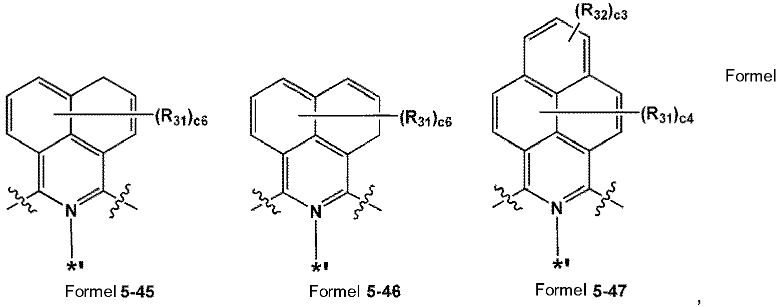
wobei in den Formeln 3-1 bis 3-16, 3-31 bis 3-70, 4-1 bis 4-16, 4-31 bis 4-70 und 5-1 bis 5-47 Y₉ O, S, oder N(R₃₉) ist,
R₃ bis R₅ gleich wie beschrieben in Anspruch 1 sind,
R₁₁ bis R₂₀ jeweils unabhängig gleich wie beschrieben in Verbindung mit R₁ in Anspruch 1 sind,
R₂₁ bis R₃₀ jeweils unabhängig gleich wie beschrieben in Verbindung mit R₂ in Anspruch 1 sind,
R₃₁ bis R₃₉ jeweils unabhängig gleich wie beschrieben in Verbindung mit R₃ in Anspruch 1 sind,
c3 eine Ganzzahl ausgewählt aus 0 bis 3 ist,
c4 eine Ganzzahl ausgewählt aus 0 bis 4 ist,
c6 eine Ganzzahl ausgewählt aus 0 bis 6 ist,
c8 eine Ganzzahl ausgewählt aus 0 bis 8 ist,
c10 eine Ganzzahl ausgewählt aus 0 bis 10 ist und
*, *' und *" jeweils eine Bindungsstelle mit M in Formel 1 angeben, vorzugsweise wobei R₃ bis R₅ und R₁₁ bis R₃₉ jeweils unabhängig ausgewählt sind aus: Wasserstoff, Deuterium, -F, -Cl, -Br, -I, einer Hydroxyl-Gruppe, einer Cyano-Gruppe, einer Nitro-Gruppe, einer Amino-Gruppe, einer Amidino-Gruppe, einer Hydrazin-Gruppe, einer Hydrazon-Gruppe, einer Carbonsäure-Gruppe oder einem Salz davon, einer Sulfonsäure-Gruppe oder einem Salz davon, einer Phosphorsäure-Gruppe oder einem Salz davon, -SF₅, einer C₁-C₂₀-Alkyl-Gruppe und einer C₁-C₂₀-Alkoxy-Gruppe;
einer C₁-C₂₀ Alkyl-Gruppe und einer C₁-C₂₀ Alkoxy-Gruppe, jeweils substituiert mit mindestens einem, ausgewählt aus Deuterium, -F, -Cl, -Br, -I, -CD₃, -CD₂H, -CDH₂, -CF₃, -CF₂H, -CFH₂, einer Hydroxyl-Gruppe, einer Cyano-Gruppe, einer Nitro-Gruppe, einer Amino-Gruppe, einer Amidino-Gruppe, einer Hydrazin-Gruppe, einer Hydrazon-Gruppe, einer Carbonsäure-Gruppe oder einem Salz davon, einer Sulfonsäure-Gruppe oder einem Salz davon, einer Phosphorsäure-Gruppe oder einem Salz davon, einer C₁-C₁₀-Alkyl-Gruppe, einer Cyclopentyl-Gruppe, einer Cyclohexyl-Gruppe, einer Cycloheptyl-Gruppe, einer Cyclooctyl-Gruppe, einer Adamantanyl-Gruppe, einer Norbornanyl-Gruppe, einer Norbornenyl-Gruppe, einer Cyclopentenyl-Gruppe, einer Cyclohexenyl-Gruppe, einer Cycloheptenyl-Gruppe, einer Phenyl-Gruppe, einer Naphthyl-Gruppe, einer Pyridinyl-Gruppe und einer Pyrimidinyl-Gruppe;
einer Cyclopentyl-Gruppe, einer Cyclohexyl-Gruppe, einer Cycloheptyl-Gruppe, einer Cyclooctyl-Gruppe, einer Adamantanyl-Gruppe, einer Norbornanyl-Gruppe, einer Norbornenyl-Gruppe, einer Cyclopentenyl-Gruppe, einer Cyclohexenyl-Gruppe, einer Cycloheptenyl-Gruppe, einer Phenyl-Gruppe, einer Naphthyl-Gruppe, einer Fluorenyl-Gruppe, einer Phenanthrenyl-Gruppe, einer Anthracenyl-Gruppe, einer Fluoranthenyl-Gruppe, einer Triphenylenyl-Gruppe, einer Pyrenyl-Gruppe, einer Chrysenyl-Gruppe, einer Pyrrolyl-Gruppe, einer Thiophenyl-Gruppe, einer Furanyl-Gruppe, einer Imidazolyl-Gruppe, einer Pyrazolyl-Gruppe, einer Thiazolyl-Gruppe, einer
Isothiazolyl-Gruppe, einer Oxazolyl-Gruppe, einer Isoxazolyl-Gruppe, einer Pyridinyl-Gruppe, einer Pyrazinyl-Gruppe, einer Pyrimidinyl-Gruppe, einer Pyridazinyl-Gruppe, einer Isoindolyl-Gruppe, einer Indolyl-Gruppe, einer Indazolyl-Gruppe, einer Purinyl-Gruppe, einer Chinolinyl-Gruppe, einer Isochinolinyl-Gruppe, einer Benzochinolinyl-Gruppe, einer Chinoxalinyl-Gruppe, einer Chinazolinyl-Gruppe, einer Cinnolinyl-Gruppe, einer Carbazolyl-Gruppe, einer Phenanthrolinyl-Gruppe, einer Benzimidazolyl-Gruppe, einer Benzofuranyl-Gruppe, einer Benzothiophenyl-Gruppe, einer Isobenzothiazolyl-Gruppe, einer Benzoxazolyl-Gruppe, einer Isobenzoxazolyl-Gruppe, einer Triazolyl-Gruppe, einer Tetrazolyl-Gruppe, einer Oxadiazolyl-Gruppe, einer Triazinyl-Gruppe, einer Dibenzofuranyl-Gruppe, einer Dibenzothiophenyl-Gruppe, einer Benzocarbazolyl-Gruppe, einer Dibenzocarbazolyl-Gruppe, einer Imidazopyridinyl-Gruppe und einer Imidazopyrimidinyl-Gruppe;
einer Cyclopentyl-Gruppe, einer Cyclohexyl-Gruppe, einer Cycloheptyl-Gruppe, einer Cyclooctyl-Gruppe, einer Adamantanyl-Gruppe, einer Norbornanyl-Gruppe, einer Norbornenyl-Gruppe, einer Cyclopentenyl-Gruppe, einer Cyclohexenyl-Gruppe, einer Cycloheptenyl-Gruppe, einer Phenyl-Gruppe, einer Naphthyl-Gruppe, einer Fluorenyl-Gruppe, einer Phenanthrenyl-Gruppe, einer Anthracenyl-Gruppe, einer Fluoranthenyl-Gruppe, einer Triphenylenyl-Gruppe, einer Pyrenyl-Gruppe, einer Chrysenyl-Gruppe, einer Pyrrolyl-Gruppe, einer Thiophenyl-Gruppe, einer Furanyl-Gruppe, einer Imidazolyl-Gruppe, einer Pyrazolyl-Gruppe, einer Thiazolyl-Gruppe, einer Isothiazolyl-Gruppe, einer Oxazolyl-Gruppe, einer Isoxazolyl-Gruppe, einer Pyridinyl-Gruppe, einer Pyrazinyl-Gruppe, einer Pyrimidinyl-Gruppe, einer Pyridazinyl-Gruppe, einer Isoindolyl-Gruppe, einer Indolyl-Gruppe, einer Indazolyl-Gruppe, einer Purinyl-Gruppe, einer Chinolinyl-Gruppe, einer Isochinolinyl-Gruppe, einer Benzochinolinyl-Gruppe, einer Chinoxalinyl-Gruppe, einer Chinazolinyl-Gruppe, einer Cinnolinyl-Gruppe, einer Carbazolyl-Gruppe, einer Phenanthrolinyl-Gruppe, einer Benzimidazolyl-Gruppe, einer Benzofuranyl-Gruppe, einer Benzothiophenyl-Gruppe, einer Isobenzothiazolyl-Gruppe, einer Benzoxazolyl-Gruppe, einer Isobenzoxazolyl-Gruppe, einer Triazolyl-Gruppe, einer Tetrazolyl-Gruppe, einer Oxadiazolyl-Gruppe, einer Triazinyl-Gruppe, einer Dibenzofuranyl-Gruppe, einer Dibenzothiophenyl-Gruppe, einer Benzocarbazolyl-Gruppe, einer Dibenzocarbazolyl-Gruppe, einer Imidazopyridinyl-Gruppe und einer Imidazopyrimidinyl-Gruppe, jeweils substituiert mit mindestens einem, ausgewählt aus Deuterium, -F, -Cl, -Br, -I, -CD₃, -CD₂H, -CDH₂, -CF₃, -CF₂H, -CFH₂, einer Hydroxyl-Gruppe, einer Cyano-Gruppe, einer Nitro-Gruppe, einer Amino-Gruppe, einer Amidino-Gruppe, einer Hydrazin-Gruppe, einer Hydrazon-Gruppe, einer Carbonsäure-Gruppe oder einem Salz davon, einer Sulfonsäure-Gruppe oder einem Salz davon, einer Phosphorsäure-Gruppe oder einem Salz davon, einer C₁-C₂₀-Alkyl-Gruppe, einer C₁-C₂₀-Alkoxy-Gruppe, einer Cyclopentyl-Gruppe, einer Cyclohexyl-Gruppe, einer Cycloheptyl-Gruppe, einer Cyclooctyl-Gruppe, einer Adamantanyl-Gruppe, einer Norbornanyl-Gruppe, einer Norbornenyl-Gruppe, einer Cyclopentenyl-Gruppe, einer Cyclohexenyl-Gruppe, einer Cycloheptenyl-Gruppe, einer Phenyl-Gruppe, einer Naphthyl-Gruppe, einer Fluorenyl-Gruppe, einer
Phenanthrenyl-Gruppe, einer Anthracenyl-Gruppe, einer Fluoranthenyl-Gruppe, einer Triphenylenyl-Gruppe, einer Pyrenyl-Gruppe, einer Chrysenyl-Gruppe, einer Pyrrolyl-Gruppe, einer Thiophenyl-Gruppe, einer Furanyl-Gruppe, einer Imidazolyl-Gruppe, einer Pyrazolyl-Gruppe, einer Thiazolyl-Gruppe, einer Isothiazolyl-Gruppe, einer Oxazolyl-Gruppe, einer Isoxazolyl-Gruppe, einer Pyridinyl-Gruppe, einer Pyrazinyl-Gruppe, einer Pyrimidinyl-Gruppe, einer Pyridazinyl-Gruppe, einer Isoindolyl-Gruppe, einer Indolyl-Gruppe, einer Indazolyl-Gruppe, einer Purinyl-Gruppe, einer Chinolinyl-Gruppe, einer Isochinolinyl-Gruppe, einer Benzochinolinyl-Gruppe, einer Chinoxalinyl-Gruppe, einer Chinazolinyl-Gruppe, einer Cinnolinyl-Gruppe, einer Carbazolyl-Gruppe, einer Phenanthrolinyl-Gruppe, einer Benzimidazolyl-Gruppe, einer Benzofuranyl-Gruppe, einer Benzothiophenyl-Gruppe, einer Isobenzothiazolyl-Gruppe, einer Benzoxazolyl-Gruppe, einer Isobenzoxazolyl-Gruppe, einer Triazolyl-Gruppe, einer Tetrazolyl-Gruppe, einer Oxadiazolyl-Gruppe, einer Triazinyl-Gruppe, einer Dibenzofuranyl-Gruppe, einer Dibenzothiophenyl-Gruppe, einer Benzocarbazolyl-Gruppe, einer Dibenzocarbazolyl-Gruppe, einer Imidazopyridinyl-Gruppe und einer Imidazopyrimidinyl-Gruppe; und
-N(Q₁)(Q₂), -Si(Q₃)(Q₄)(Q₅), -B(Q₆)(Q₇) und -P(=O)(Q₈)(Q₉), wobei Q₁ bis Q₉ jeweils unabhängig ausgewählt sind aus:
-CH₃, -CD₃, -CD₂H, -CDH₂, -CH₂CH₃, -CH₂CD₃, -CH₂CD₂H, -CH₂CDH₂, -CHDCH₃, -CHDCD₂H, -CHDCDH₂, -CHDCD₃, -CD₂CD₃, -CD₂CD₂H und -CD₂CDH₂;
einer n-Propyl-Gruppe, einer Isopropyl-Gruppe, einer n-Butyl-Gruppe, einer Isobutyl-Gruppe, einer sec-Butyl-Gruppe, einer tert-Butyl-Gruppe, einer n-Pentyl-Gruppe, einer Isopentyl-Gruppe, einer sec-Pentyl-Gruppe, einer tert-Pentyl-Gruppe, einer Phenyl-Gruppe und einer Naphthyl-Gruppe; und
einer n-Propyl-Gruppe, einer iso-Propyl-Gruppe, einer n-Butyl-Gruppe, einer Isobutyl-Gruppe, einer sec-Butyl-Gruppe, einer tert-Butyl-Gruppe, einer n-Pentyl-Gruppe, einer Isopentyl-Gruppe, einer sec-Pentyl-Gruppe, einer tert-Pentyl-Gruppe, einer Phenyl-Gruppe und einer Naphthyl-Gruppe, jeweils substituiert mit mindestens einem, ausgewählt aus Deuterium, einer C₁-C₁₀-Alkyl-Gruppe und einer Phenyl-Gruppe, bevorzugter wobei
R₃ bis R₅ und R₁₁ bis R₃₉ jeweils unabhängig ausgewählt sind aus: Wasserstoff, Deuterium, -F, einer Cyano-Gruppe, einer Nitro-Gruppe, -SF₅, einer Methyl-Gruppe, einer Ethyl-Gruppe, einer n-Propyl-Gruppe, einer iso-Propyl-Gruppe, einer n-Butyl-Gruppe, einer Isobutyl-Gruppe, einer sec-Butyl-Gruppe, einer tert-Butyl-Gruppe, einer n-Pentyl-Gruppe, einer Isopentyl-Gruppe, einer sec-Pentyl-Gruppe, einer tert-Pentyl-Gruppe, einer n-Hexyl-Gruppe, einer iso-Hexyl-Gruppe, einer sec-Hexyl-Gruppe, einer tert-Hexyl-Gruppe, einer n-Heptyl-Gruppe, einer iso-Heptyl-Gruppe, einer sec-Heptyl-Gruppe, einer tert-Heptyl-Gruppe, einer n-Octyl-Gruppe, einer iso-Octyl-Gruppe, einer sec-Octyl-Gruppe, einer tert-Octyl-Gruppe, einer n-Nonyl-Gruppe, einer iso-Nonyl-Gruppe, einer sec-Nonyl-Gruppe, einer tert-Nonyl-Gruppe, einer n-Decyl-Gruppe, einer iso-Decyl-Gruppe, einer sec-Decyl-Gruppe, einer tert-Decyl-Gruppe, einer Methoxy-Gruppe, einer Ethoxy-Gruppe, einer Propoxy-Gruppe, einer Butoxy-Gruppe, einer Pentoxy-Gruppe, einer Cyclopentyl-Gruppe, einer Cyclohexyl-Gruppe, einer Cycloheptyl-Gruppe, einer Cyclooctyl-Gruppe, einer Adamantanyl-Gruppe, einer Norbornanyl-Gruppe, einer Norbornenyl-Gruppe, einer Cyclopentenyl-Gruppe, einer Cyclohexenyl-Gruppe, einer Cycloheptenyl-Gruppe, einer Phenyl-Gruppe, einer Naphthyl-Gruppe, einer Pyridinyl-Gruppe, einer Pyrimidinyl-Gruppe, einer Dibenzofuranyl-Gruppe und einer Dibenzothiophenyl-Gruppe;
einer Methyl-Gruppe, einer Ethyl-Gruppe, einer n-Propyl-Gruppe, einer iso-Propyl-Gruppe, einer n-Butyl-Gruppe, einer Isobutyl-Gruppe, einer sec-Butyl-Gruppe, einer tert-Butyl-Gruppe, einer n-Pentyl-Gruppe, einer Isopentyl-Gruppe, einer sec-Pentyl-Gruppe, einer tert-Pentyl-Gruppe, einer n-Hexyl-Gruppe, einer iso-Hexyl-Gruppe, einer sec-Hexyl-Gruppe, einer tert-Hexyl-Gruppe, einer n-Heptyl-Gruppe, einer iso-Heptyl-Gruppe, einer sec-Heptyl-Gruppe, einer tert-Heptyl-Gruppe, einer n-Octyl-Gruppe, einer iso-Octyl-Gruppe, einer sec-Octyl-Gruppe, einer tert-Octyl-Gruppe, einer n-Nonyl-Gruppe, einer iso-Nonyl-Gruppe, einer sec-Nonyl-Gruppe, einer tert-Nonyl-Gruppe, einer n-Decyl-Gruppe, einer iso-Decyl-Gruppe, einer sec-Decyl-Gruppe, einer tert-Decyl-Gruppe, einer Methoxy-Gruppe, einer Ethoxy-Gruppe, einer Propoxy-Gruppe, einer Butoxy-Gruppe, einer Pentoxy-Gruppe, einer Cyclopentyl-Gruppe, einer Cyclohexyl-Gruppe, einer Cycloheptyl-Gruppe, einer Cyclooctyl-Gruppe, einer Adamantanyl-Gruppe, einer Norbornanyl-Gruppe, einer Norbornenyl-Gruppe, einer Cyclopentenyl-Gruppe, einer Cyclohexenyl-Gruppe, einer Cycloheptenyl-Gruppe, einer Phenyl-Gruppe, einer Naphthyl-Gruppe, einer Pyridinyl-Gruppe, einer Pyrimidinyl-Gruppe, einer Dibenzofuranyl-Gruppe und einer Dibenzothiophenyl-Gruppe, jeweils substituiert mit mindestens einem, ausgewählt aus Deuterium, -F, -CD₃, -CD₂H, -CDH₂, -CF₃, -CF₂H, -CFH₂, einer Cyano-Gruppe, einer Nitro-Gruppe, einer C₁-C₁₀-Alkyl-Gruppe, einer C₁-C₁₀-Alkoxy-Gruppe, einer Cyclopentyl-Gruppe, einer Cyclohexyl-Gruppe, einer Cycloheptyl-Gruppe, einer Cyclooctyl-Gruppe, einer Adamantanyl-Gruppe, einer Norbornanyl-Gruppe, einer Norbornenyl-Gruppe, einer Cyclopentenyl-Gruppe, einer Cyclohexenyl-Gruppe, einer Cycloheptenyl-Gruppe, einer Phenyl-Gruppe, einer Naphthyl-Gruppe, einer Pyridinyl-Gruppe, einer Pyrimidinyl-Gruppe, einer Dibenzofuranyl-Gruppe und einer Dibenzothiophenyl-Gruppe; und
-N(Q₁)(Q₂), -Si(Q₃)(Q₄)(Q₅), -B(Q₆)(Q₇) und -P(=O)(Q₈)(Q₉), wobei Q₁ bis Q₉ jeweils unabhängig ausgewählt sind aus:
-CH₃, -CD₃, -CD₂H, -CDH₂, -CH₂CH₃, -CH₂CD₃, -CH₂CD₂H, -CH₂CDH₂, -CHDCH₃, -CHDCD₂H, -CHDCDH₂, -CHDCD₃, -CD₂CD₃, -CD₂CD₂H und -CD₂CDH₂;
einer n-Propyl-Gruppe, einer Isopropyl-Gruppe, einer n-Butyl-Gruppe, einer Isobutyl-Gruppe, einer sec-Butyl-Gruppe, einer tert-Butyl-Gruppe, einer n-Pentyl-Gruppe, einer Isopentyl-Gruppe, einer sec-Pentyl-Gruppe, einer tert-Pentyl-Gruppe, einer Phenyl-Gruppe und einer Naphthyl-Gruppe; und
einer n-Propyl-Gruppe, einer iso-Propyl-Gruppe, einer n-Butyl-Gruppe, einer Isobutyl-Gruppe, einer
sec-Butyl-Gruppe, einer tert-Butyl-Gruppe, einer n-Pentyl-Gruppe, einer Isopentyl-Gruppe, einer sec-Pentyl-Gruppe, einer tert-Pentyl-Gruppe, einer Phenyl-Gruppe und einer Naphthyl-Gruppe, jeweils substituiert mit mindestens einem, ausgewählt aus Deuterium, einer C₁-C₁₀-Alkyl-Gruppe und einer Phenyl-Gruppe, und besonders wobei
R₃ bis R₅ und R₁₁ bis R₃₉ jeweils unabhängig ausgewählt sind aus Wasserstoff, Deuterium, -F, einer Cyano-Gruppe, einer Nitro-Gruppe, -SF₅, -CH₃, -CD₃, -CD₂H, -CDH₂, -CF₃, -CF₂H, -CFH₂, einer Gruppe, die durch eine der Formeln 9-1 bis 9-19 dargestellt ist, einer Gruppe, die durch eine der Formeln 10-1 bis 10-38 dargestellt ist, und -Si(Q₃)(Q₄)(Q₅): 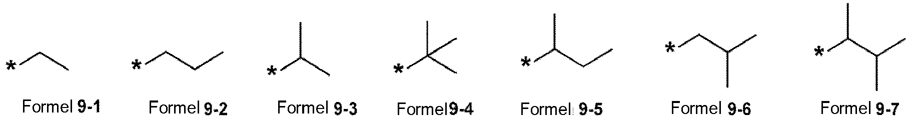 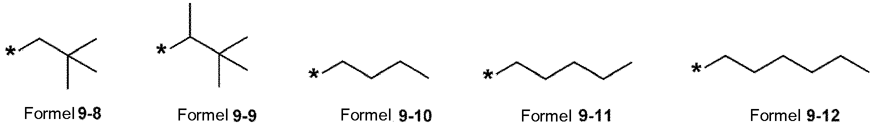 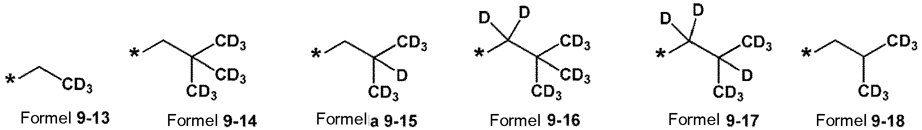 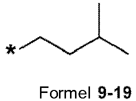 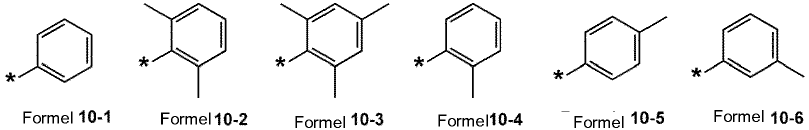 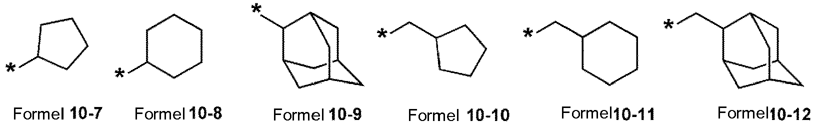 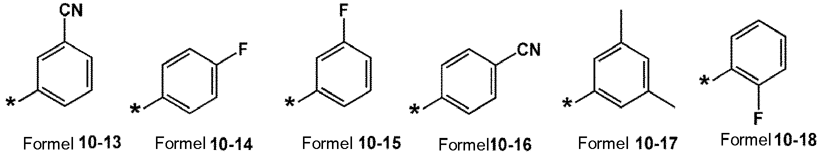 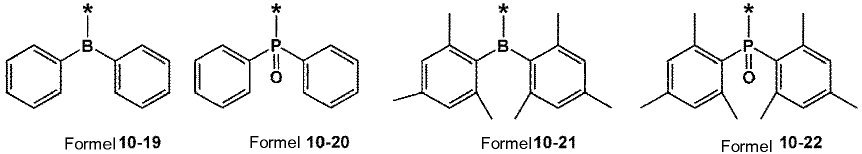 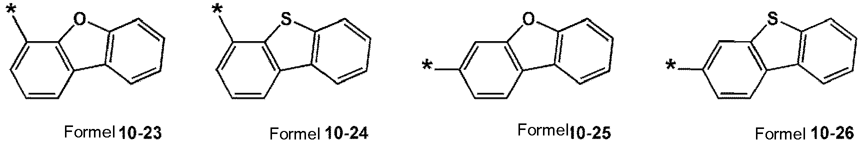 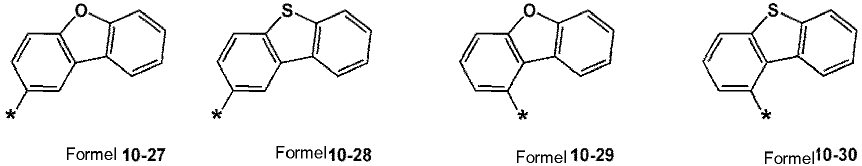 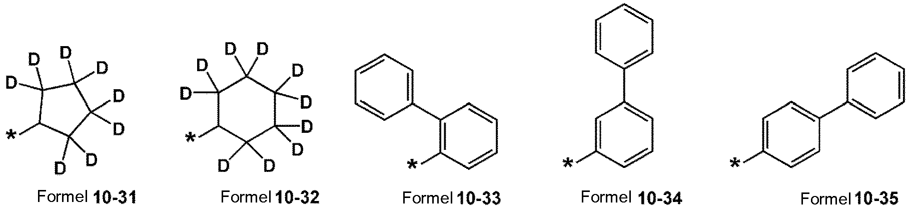 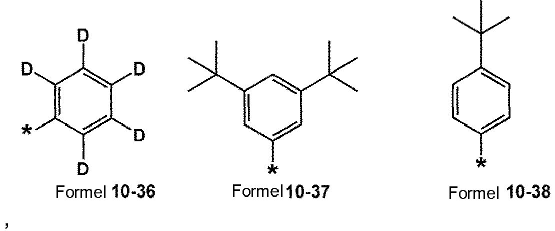
wobei * in den Formeln 9-1 bis 9-19 und 10-1 bis 10-38 eine Bindungsstelle mit einem benachbarten Atom angibt.

5. Organometallische Verbindung nach Anspruch 4, wobei
eine Gruppierung, dargestellt durch 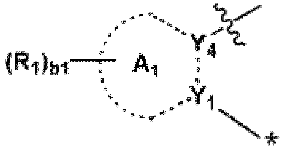 in Formel 2, durch eine dargestellt ist, die aus den Formeln 3-5 bis 3-8 und 3-47 bis 3-62 ausgewählt ist,
eine Gruppierung, dargestellt durch 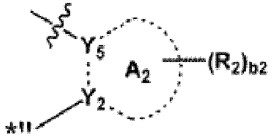 in Formel 2, durch eine dargestellt ist, die aus den Formeln 4-5 bis 4-8 und 4-47 bis 4-62 ausgewählt ist.

6. Organometallische Verbindung nach Anspruch 4, wobei
eine Gruppierung, dargestellt durch 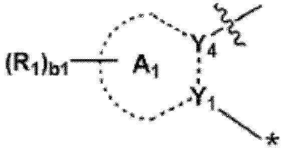 in Formel 2, durch eine dargestellt ist, die aus den Formeln 3-1, 3-5, 3-9, 3-13, 3-31 bis 3-34 und 3-47 bis 3-50 ausgewählt ist, und
eine Gruppierung, dargestellt durch 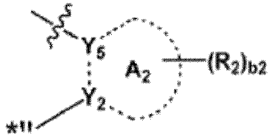 in Formel 2, durch eine dargestellt ist, die aus den Formeln 4-1, 4-5, 4-9, 4-13, 4-31 bis 4-34 und 4-47 bis 4-50 ausgewählt ist.

7. Organometallische Verbindung nach Anspruch 4, wobei
eine Gruppierung, dargestellt durch 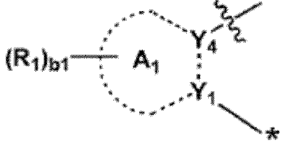 in Formel 2, durch eine dargestellt ist, die aus den Formeln 3-1, 3-5, 3-9, 3-13, 3-31 bis 3-34 und 3-47 bis 3-50 ausgewählt ist, und
eine Gruppierung, dargestellt durch 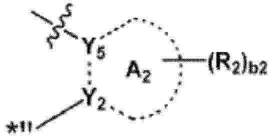 in Formel 2, durch eine dargestellt ist, die aus den Formeln 4-2 bis 4-4, 4-6 bis 4-8, 4-10 bis 4-12, 4-14 bis 4-16, 4-35 bis 4-46 und 4-51 bis 4-70 ausgewählt ist.

8. Organometallische Verbindung nach einem der Ansprüche 4 bis 7, wobei
eine Gruppierung, dargestellt durch 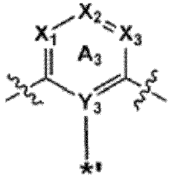 in Formel 2, durch eine dargestellt ist, die aus den Formeln 5-1 bis 5-28, 5-29 und 5-45 ausgewählt ist.

9. Organometallische Verbindung nach einem der Ansprüche 1 bis 8, wobei L₁ in Formel 1 ausgewählt ist aus Liganden, die durch die Formeln 2A-1 bis 2E-1 und 2A-2 bis 2E-2 dargestellt sind: 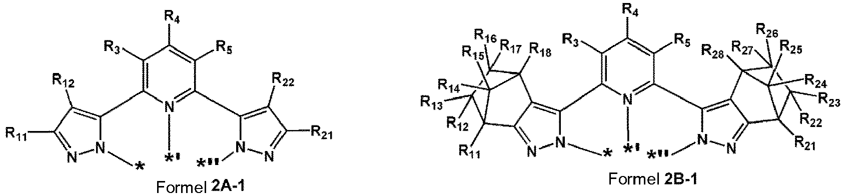 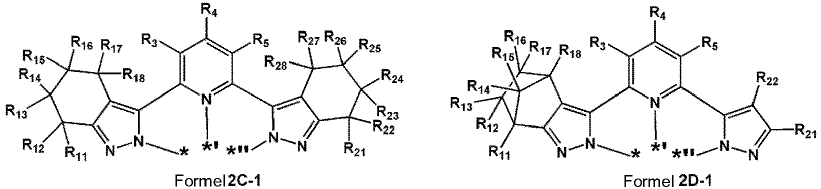 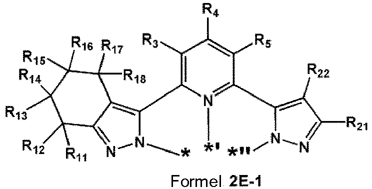 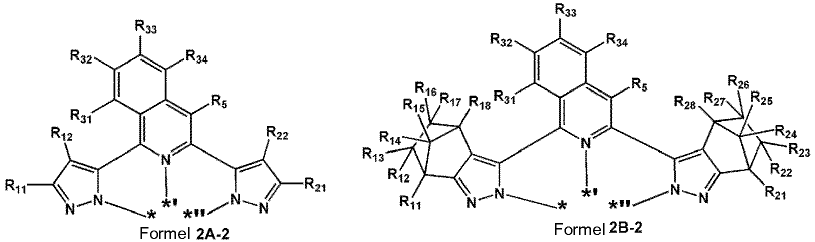 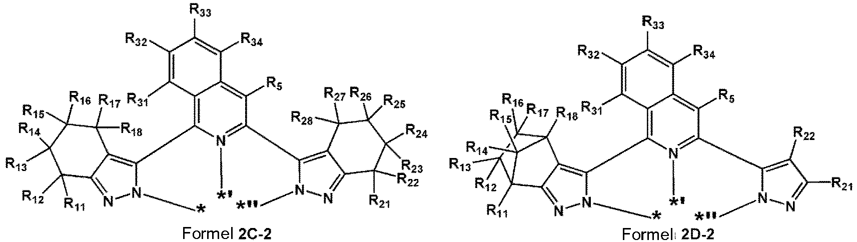 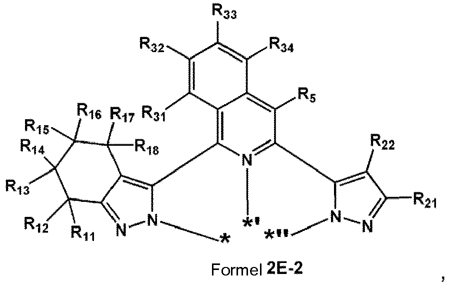
wobei in den Formeln 2A-1 bis 2E-1 und 2A-2 bis 2E-2
R₃ bis R₅, R₁₁ bis R₁₈, R₂₁ bis R₂₈ und R₃₁ bis R₃₄ jeweils unabhängig ausgewählt sind aus Wasserstoff, Deuterium, -F, einer Cyano-Gruppe, einer Nitro-Gruppe, -SF₅, -CH₃, -CD₃, -CD₂H, -CDH₂, -CF₃, -CF₂H, -CFH₂, einer Gruppe, die durch eine der Formeln 9-1 bis 9-19 dargestellt ist, einer Gruppe, die durch eine der Formeln 10-1 bis 10-38 und -Si(Q₃)(Q₄)(Q₅) dargestellt ist: 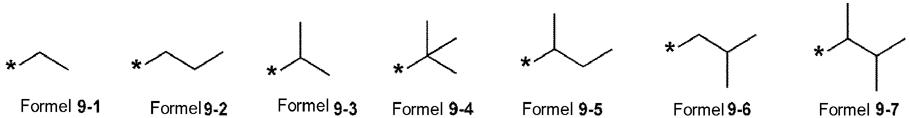 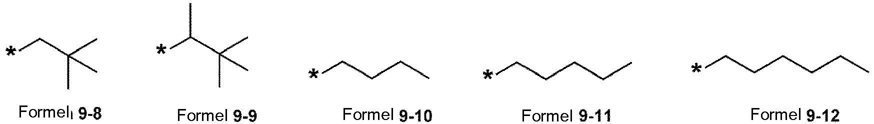 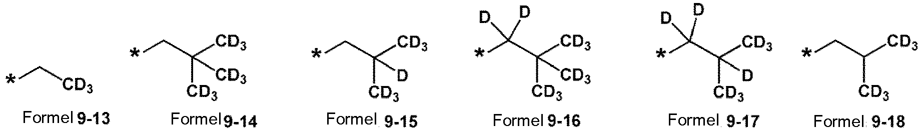 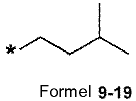 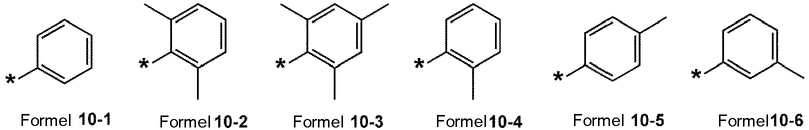 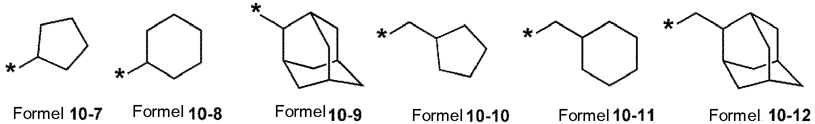 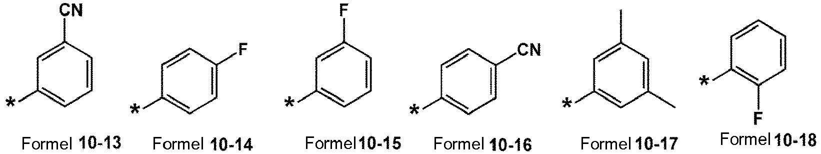 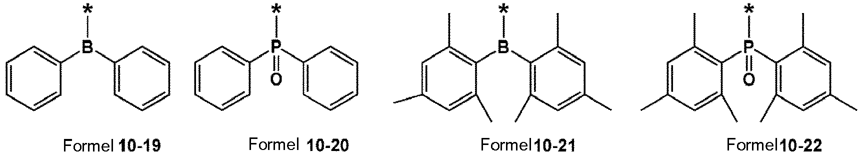 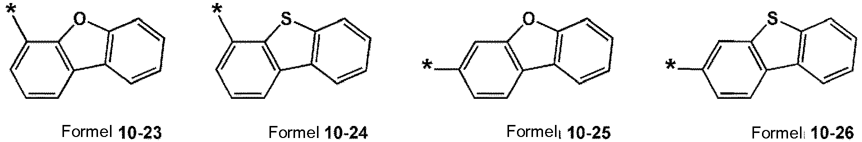 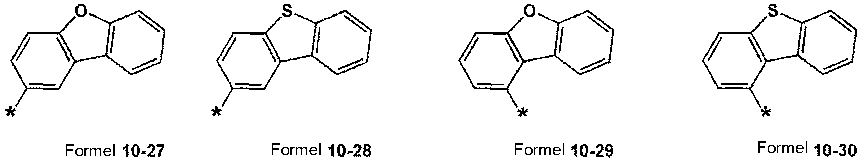 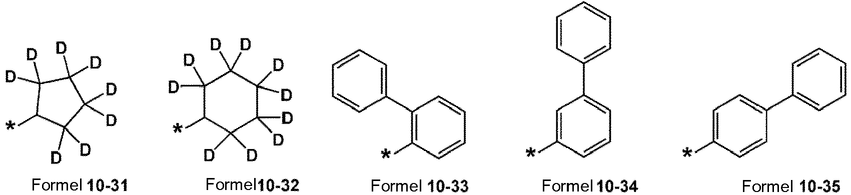 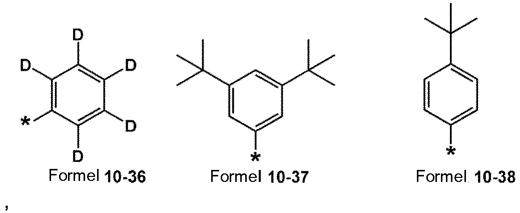
wobei * in den Formeln 9-1 bis 9-19 und 10-1 bis 10-38 eine Bindungsstelle mit einem benachbarten Atom angibt.

10. Organometallische Verbindung nach einem der Ansprüche 1 bis 9, wobei L₂ in Formel 1 ausgewählt ist aus Liganden, die durch Formel 6-1 dargestellt sind:
***(̵T₃)ₐ₃-R₆₁** Formel **6-1**,
wobei in Formel 6-1
T₃ ausgewählt ist aus einer Einfachbindung, *-O-*', *-S-*', *-C(R₆₂)(R₆₃)-*', *-C(R₆₂)=*', *=C(R₆₂)-*', *-C(R₆₂)=C(R₆₃)-*', *-C(=O)-*', *-C(=S)-*', *-C≡C-*' und *-N(R₆₂)-*', a3 eine Ganzzahl ausgewählt aus 1 bis 5 ist,
R₆₁ ausgewählt ist aus Wasserstoff, Deuterium, -F, -Cl, -Br, -I, -SF₅, einer Hydroxyl-Gruppe, einer Cyano-Gruppe, einer Nitro-Gruppe, einer Amino-Gruppe, einer Amidino-Gruppe, einer Hydrazin-Gruppe, einer Hydrazon-Gruppe, einer Carbonsäure-Gruppe oder einem Salz davon, einer Sulfonsäure-Gruppe oder einem Salz davon, einer Phosphorsäure-Gruppe oder einem Salz davon, einer substituierten oder unsubstituierten C₁-C₆₀-Alkyl-Gruppe, einer substituierten oder unsubstituierten C₂-C₆₀-Alkenyl-Gruppe, einer substituierten oder unsubstituierten C₂-C₆₀-Alkynyl-Gruppe, einer substituierten oder unsubstituierten C₁-C₆₀-Alkoxy-Gruppe, einer substituierten oder unsubstituierten C₃-C₁₀-Cycloalkyl-Gruppe, einer substituierten oder unsubstituierten C₁-C₁₀-Heterocycloalkyl-Gruppe, einer substituierten oder unsubstituierten C₃-C₁₀-Cycloalkenyl-Gruppe, einer substituierten oder unsubstituierten C₁-C₁₀-Heterocycloalkenyl-Gruppe, einer substituierten oder unsubstituierten C₆-C₆₀-Aryl-Gruppe, einer substituierten oder unsubstituierten C₆-C₆₀-Aryloxy-Gruppe, einer substituierten oder unsubstituierten C₆-C₆₀-Arylthio-Gruppe, einer substituierten oder unsubstituierten C₁-C₆₀-Heteroaryl-Gruppe, einer substituierten oder unsubstituierten monovalenten nichtaromatischen kondensierten polycyclischen Gruppe, einer substituierten oder unsubstituierten monovalenten nichtaromatischen kondensierten heteropolycyclischen-Gruppe, -N(Q₁)(Q₂), -Si(Q₃)(Q₄)(Q₅), -B(Q₆)(Q₇), -P(=O)(Q₈)(Q₉) und -P(Q₄₁)(Q₄₂)(Q₄₃),
wobei Q₁ bis Q₉ und Q₄₁ bis Q₄₃ jeweils unabhängig ausgewählt sind aus:
-CH₃, -CD₃, -CD₂H, -CDH₂, -CH₂CH₃, -CH₂CD₃, -CH₂CD₂H, -CH₂CDH₂, -CHDCH₃, -CHDCD₂H, -CHDCDH₂, -CHDCD₃, -CD₂CD₃, -CD₂CD₂H und -CD₂CDH₂;
einer n-Propyl-Gruppe, einer Isopropyl-Gruppe, einer n-Butyl-Gruppe, einer Isobutyl-Gruppe, einer sec-Butyl-Gruppe, einer tert-Butyl-Gruppe, einer n-Pentyl-Gruppe, einer Isopentyl-Gruppe, einer sec-Pentyl-Gruppe, einer tert-Pentyl-Gruppe, einer Phenyl-Gruppe und einer Naphthyl-Gruppe; und
einer n-Propyl-Gruppe, einer iso-Propyl-Gruppe, einer n-Butyl-Gruppe, einer Isobutyl-Gruppe, einer sec-Butyl-Gruppe, einer tert-Butyl-Gruppe, einer n-Pentyl-Gruppe, einer Isopentyl-Gruppe, einer sec-Pentyl-Gruppe, einer tert-Pentyl-Gruppe, einer Phenyl-Gruppe und einer Naphthyl-Gruppe, jeweils substituiert mit mindestens einem, ausgewählt aus Deuterium, einer C₁-C₁₀ Alkyl-Gruppe und einer Phenyl-Gruppe,
R₆₂ und R₆₃ jeweils unabhängig ausgewählt sind aus:
Wasserstoff, Deuterium, -F, einer Cyano-Gruppe, einer Nitro-Gruppe, -SF₅, einer Methyl-Gruppe, einer Ethyl-Gruppe, einer n-Propyl-Gruppe, einer iso-Propyl-Gruppe, einer n-Butyl-Gruppe, einer Isobutyl-Gruppe, einer sec-Butyl-Gruppe, einer tert-Butyl-Gruppe, einer n-Pentyl-Gruppe, einer Isopentyl-Gruppe, einer sec-Pentyl-Gruppe, einer tert-Pentyl-Gruppe, einer n-Hexyl-Gruppe, einer iso-Hexyl-Gruppe, einer sec-Hexyl-Gruppe, einer tert-Hexyl-Gruppe, einer n-Heptyl-Gruppe, einer iso-Heptyl-Gruppe, einer sec-Heptyl-Gruppe, einer tert-Heptyl-Gruppe, einer n-Octyl-Gruppe, einer iso-Octyl-Gruppe, einer sec-Octyl-Gruppe, einer tert-Octyl-Gruppe, einer n-Nonyl-Gruppe, einer iso-Nonyl-Gruppe, einer sec-Nonyl-Gruppe, einer tert-Nonyl-Gruppe, einer n-Decyl-Gruppe, einer iso-Decyl-Gruppe, einer sec-Decyl-Gruppe, einer tert-Decyl-Gruppe, einer Methoxy-Gruppe, einer Ethoxy-Gruppe, einer Propoxy-Gruppe, einer Butoxy-Gruppe, einer Pentoxy-Gruppe, einer Cyclopentyl-Gruppe, einer Cyclohexyl-Gruppe, einer Cycloheptyl-Gruppe, einer Cyclooctyl-Gruppe, einer Adamantanyl-Gruppe, einer Norbornanyl-Gruppe, einer Norbornenyl-Gruppe, einer Cyclopentenyl-Gruppe, einer Cyclohexenyl-Gruppe, einer Cycloheptenyl-Gruppe, einer Phenyl-Gruppe, einer Naphthyl-Gruppe, einer Pyridinyl-Gruppe, einer Pyrimidinyl-Gruppe, einer Dibenzofuranyl-Gruppe und einer Dibenzothiophenyl-Gruppe; und
einer Methyl-Gruppe, einer Ethyl-Gruppe, einer n-Propyl-Gruppe, einer iso-Propyl-Gruppe, einer n-Butyl-Gruppe, einer Isobutyl-Gruppe, einer sec-Butyl-Gruppe, einer tert-Butyl-Gruppe, einer n-Pentyl-Gruppe, einer Isopentyl-Gruppe, einer sec-Pentyl-Gruppe, einer tert-Pentyl-Gruppe, einer n-Hexyl-Gruppe, einer iso-Hexyl-Gruppe, einer sec-Hexyl-Gruppe, einer tert-Hexyl-Gruppe, einer n-Heptyl-Gruppe, einer iso-Heptyl-Gruppe, einer sec-Heptyl-Gruppe, einer tert-Heptyl-Gruppe, einer n-Octyl-Gruppe, einer iso-Octyl-Gruppe, einer sec-Octyl-Gruppe, einer tert-Octyl-Gruppe, einer n-Nonyl-Gruppe, einer iso-Nonyl-Gruppe, einer sec-Nonyl-Gruppe, einer tert-Nonyl-Gruppe, einer n-Decyl-Gruppe, einer iso-Decyl-Gruppe, einer sec-Decyl-Gruppe, einer tert-Decyl-Gruppe, einer Methoxy-Gruppe, einer Ethoxy-Gruppe, einer Propoxy-Gruppe, einer Butoxy-Gruppe, einer Pentoxy-Gruppe, einer Cyclopentyl-Gruppe, einer Cyclohexyl-Gruppe, einer Cycloheptyl-Gruppe, einer Cyclooctyl-Gruppe, einer Adamantanyl-Gruppe, einer Norbornanyl-Gruppe, einer Norbornenyl-Gruppe, einer Cyclopentenyl-Gruppe, einer Cyclohexenyl-Gruppe, einer Cycloheptenyl-Gruppe, einer Phenyl-Gruppe, einer Naphthyl-Gruppe, einer Pyridinyl-Gruppe, einer Pyrimidinyl-Gruppe, einer Dibenzofuranyl-Gruppe und einer Dibenzothiophenyl-Gruppe, jeweils substituiert mit mindestens einem, ausgewählt aus Deuterium, -F, -CD₃, -CD₂H, -CDH₂, -CF₃, -CF₂H, -CFH₂, einer Cyano-Gruppe, einer Nitro-Gruppe, einer C₁-C₁₀ Alkyl-Gruppe, einer C₁-C₁₀ Alkoxy-Gruppe, einer Cyclopentyl-Gruppe, einer Cyclohexyl-Gruppe, einer Cycloheptyl-Gruppe, einer Cyclooctyl-Gruppe, einer Adamantanyl-Gruppe, einer Norbornanyl-Gruppe, einer Norbornenyl-Gruppe, einer Cyclopentenyl-Gruppe, einer Cyclohexenyl-Gruppe, einer Cycloheptenyl-Gruppe, einer Phenyl-Gruppe, einer Naphthyl-Gruppe, einer Pyridinyl-Gruppe, einer Pyrimidinyl-Gruppe, einer Dibenzofuranyl-Gruppe und einer Dibenzothiophenyl-Gruppe, R₆₂ und R₆₃ optional miteinander verbunden sind, um eine substituierte oder unsubstituierte C₅-C₃₀ carbocyclische-Gruppe oder eine substituierte oder unsubstituierte C₂-C₃₀ heterocyclische-Gruppe zu bilden, und
* eine Bindungsstelle mit M in Formel 1 angibt.

11. Organometallische Verbindung nach einem der Ansprüche 1 bis 10, wobei L₂ in Formel 1 ausgewählt ist aus Liganden, die durch die Formeln 12-1 bis 12-5 dargestellt sind: 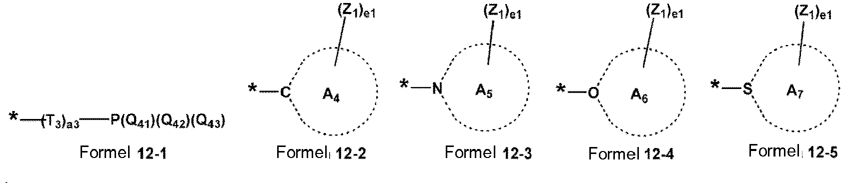
wobei in den Formeln 12-1 bis 12-5
T₃ ausgewählt ist aus einer Einfachbindung, *-O-*', *-S-*', *-C(R₆₂)(R₆₃)-*', *-C(R₆₂)=*', *=C(R₆₂)-*', *-C(R₆₂)=C(R₆₃)-*', *-C(=O)-*', *-C(=S)-*', *-C≡C-*' und *-N(R₆₂)-*',
R₆₂ und R₆₃ jeweils unabhängig ausgewählt sind aus Wasserstoff, Deuterium, -F, -Cl, -Br, -I, -SF₅, einer Hydroxyl-Gruppe, einer Cyano-Gruppe, einer Nitro-Gruppe, einer Amino-Gruppe, einer Amidino-Gruppe, einer Hydrazin-Gruppe, einer Hydrazon-Gruppe, einer Carbonsäure-Gruppe oder einem Salz davon, einer Sulfonsäure-Gruppe oder einem Salz davon, einer Phosphorsäure-Gruppe oder einem Salz davon, einer C₁-C₂₀-Alkyl-Gruppe, einer C₁-C₂₀-Alkoxy-Gruppe, einer Phenyl-Gruppe und einer Naphthyl-Gruppe,
a3 eine Ganzzahl ausgewählt aus 1 bis 5 ist,
Ring A4 ausgewählt ist aus einem Cyclopenten-Ring, einem Cyclohexen-Ring, Cyclohepten-Ring, einem Benzol-Ring, einem Inden-Ring, einem Naphthalen-Ring, einem Azulen-Ring, einem Heptalen-Ring, einem Indacen-Ring, einem Acenaphthylen-Ring, einem Fluoren-Ring, einem spiro-Bifluoren-Ring, einem Benzofluoren-Ring, einem Dibenzofluoren-Ring, einem Phenalen-Ring, einem Phenanthren-Ring, einem Anthracen-Ring, einem Fluoranthen-Ring, einem Triphenylen-Ring, einem Pyren-Ring, einem Chrysen-Ring, einem Naphthacen-Ring, einem Picen-Ring, einem Perylen-Ring, einem Pentacen-Ring, einem Hexacen-Ring, einem Rubicen-Ring, einem Coronen-Ring, einem Ovalen-Ring, einem Pyrrol-Ring, einem Thiophen-Ring, einem Furan-Ring, einem Imidazol-Ring, einem Pyrazol-Ring, einem Thiazol-Ring, einem Isothiazol-Ring, einem Oxazol-Ring, einem Isoxazol-Ring, einem Pyridin-Ring, einem Pyrazin-Ring, einem Pyrimidin-Ring, einem Pyridazin-Ring, einem iso-Indol-Ring, einem Indol-Ring, einem Indazol-Ring, einem Purin-Ring, einem Chinolin-Ring, einem Isochinolin-Ring, einem Benzochinolin-Ring, einem Chinoxalin-Ring, einem Chinazolin-Ring, einem Cinnolin-Ring, einem Naphthyridin-Ring, einem Carbazol-Ring, einem Phenanthrolin-Ring, einem Benzimidazol-Ring, einem Benzofuran-Ring, einem Benzothiophen-Ring, einem Benzothiazol-Ring, einem iso-Benzothiazol-Ring, einem Benzoxazol-Ring, einem Isobenzoxazol-Ring, einem Triazol-Ring, einem Tetrazol-Ring, einem Oxadiazol-Ring, einem Thiadiazol-Ring, einem Triazin-Ring, einem Dibenzofuran-Ring, einem Dibenzothiophen-Ring, einem Benzocarbazol-Ring, einem Dibenzocarbazol-Ring, einem Imidazopyridin-Ring und einem Imidazopyrimidin-Ring,
Ring A₅ ausgewählt ist aus einem Pyrrol-Ring, einem Imidazol-Ring, einem Pyrazol-Ring, einem Thiazol-Ring, einem Isothiazol-Ring, einem Oxazol-Ring, einem Isoxazol-Ring, einem Pyridin-Ring, einem Pyrazin-Ring, einem Pyrimidin-Ring, einem Pyridazin-Ring, einem iso-Indol-Ring, einem Indol-Ring, einem Indazol-Ring, einem Purin-Ring, einem Chinolin-Ring, einem Isochinolin-Ring, einem Benzochinolin-Ring, einem Chinoxalin-Ring, einem Chinazolin-Ring, einem Cinnolin-Ring, einem Naphthyridin-Ring, einem Carbazol-Ring, einem Phenanthrolin-Ring, einem Benzimidazol-Ring, einem Benzofuran-Ring, einem Benzothiazol-Ring, einem iso-Benzothiazol-Ring, einem Benzoxazol-Ring, einem Isobenzoxazol-Ring, einem Triazol-Ring, einem Tetrazol-Ring, einem Oxadiazol-Ring, einem Thiadiazol-Ring, einem Triazin-Ring, einem Benzocarbazol-Ring, einem Dibenzocarbazol-Ring, einem Imidazopyridin-Ring und einem Imidazopyrimidin-Ring,
Ring A₆ ausgewählt ist aus einem Furan-Ring, einem Oxazol-Ring, einem Isoxazol-Ring, einem Benzofuran-Ring, einem Benzoxazol-Ring, einem Isobenzoxazol-Ring, einem Oxadiazol-Ring und einem Dibenzofuran-Ring,
Ring A₇ ausgewählt ist aus einem Thiophen-Ring, einem Thiazol-Ring, einem Isothiazol-Ring, einem Benzothiophen-Ring, einem Benzothiazol-Ring, einem iso-Benzothiazol-Ring, einem Thiadiazol-Ring und einem Dibenzothiophen-Ring,
Z₁ is jeweils unabhängig ausgewählt aus:
Wasserstoff, Deuterium, -F, einer Cyano-Gruppe, einer Nitro-Gruppe, -SF₅, einer Methyl-Gruppe, einer Ethyl-Gruppe, einer n-Propyl-Gruppe, einer iso-Propyl-Gruppe, einer n-Butyl-Gruppe, einer Isobutyl-Gruppe, einer sec-Butyl-Gruppe, einer tert-Butyl-Gruppe, einer n-Pentyl-Gruppe, einer Isopentyl-Gruppe, einer sec-Pentyl-Gruppe, einer tert-Pentyl-Gruppe, einer n-Hexyl-Gruppe, einer iso-Hexyl-Gruppe, einer sec-Hexyl-Gruppe, einer tert-Hexyl-Gruppe, einer n-Heptyl-Gruppe, einer iso-Heptyl-Gruppe, einer sec-Heptyl-Gruppe, einer tert-Heptyl-Gruppe, einer n-Octyl-Gruppe, einer iso-Octyl-Gruppe, einer sec-Octyl-Gruppe, einer tert-Octyl-Gruppe, einer n-Nonyl-Gruppe, einer iso-Nonyl-Gruppe, einer sec-Nonyl-Gruppe, einer tert-Nonyl-Gruppe, einer n-Decyl-Gruppe, einer iso-Decyl-Gruppe, einer sec-Decyl-Gruppe, einer tert-Decyl-Gruppe, einer Methoxy-Gruppe, einer Ethoxy-Gruppe, einer Propoxy-Gruppe, einer Butoxy-Gruppe, einer Pentoxy-Gruppe, einer Cyclopentyl-Gruppe, einer Cyclohexyl-Gruppe, einer Cycloheptyl-Gruppe, einer Cyclooctyl-Gruppe, einer Adamantanyl-Gruppe, einer Norbornanyl-Gruppe, einer Norbornenyl-Gruppe, einer Cyclopentenyl-Gruppe, einer Cyclohexenyl-Gruppe, einer Cycloheptenyl-Gruppe, einer Phenyl-Gruppe, einer Naphthyl-Gruppe, einer Pyridinyl-Gruppe, einer Pyrimidinyl-Gruppe, einer Dibenzofuranyl-Gruppe und einer Dibenzothiophenyl-Gruppe;
einer Methyl-Gruppe, einer Ethyl-Gruppe, einer n-Propyl-Gruppe, einer iso-Propyl-Gruppe, einer n-Butyl-Gruppe, einer Isobutyl-Gruppe, einer sec-Butyl-Gruppe, einer tert-Butyl-Gruppe, einer n-Pentyl-Gruppe, einer Isopentyl-Gruppe, einer sec-Pentyl-Gruppe, einer tert-Pentyl-Gruppe, einer n-Hexyl-Gruppe, einer iso-Hexyl-Gruppe, einer sec-Hexyl-Gruppe, einer tert-Hexyl-Gruppe, einer n-Heptyl-Gruppe, einer iso-Heptyl-Gruppe, einer sec-Heptyl-Gruppe, einer tert-Heptyl-Gruppe, einer n-Octyl-Gruppe, einer iso-Octyl-Gruppe, einer sec-Octyl-Gruppe, einer tert-Octyl-Gruppe, einer n-Nonyl-Gruppe, einer iso-Nonyl-Gruppe, einer sec-Nonyl-Gruppe, einer tert-Nonyl-Gruppe, einer n-Decyl-Gruppe, einer iso-Decyl-Gruppe, einer sec-Decyl-Gruppe, einer tert-Decyl-Gruppe, einer Methoxy-Gruppe, einer Ethoxy-Gruppe, einer Propoxy-Gruppe, einer Butoxy-Gruppe, einer Pentoxy-Gruppe, einer Cyclopentyl-Gruppe, einer Cyclohexyl-Gruppe, einer Cycloheptyl-Gruppe, einer Cyclooctyl-Gruppe, einer Adamantanyl-Gruppe, einer Norbornanyl-Gruppe, einer Norbornenyl-Gruppe, einer Cyclopentenyl-Gruppe, einer Cyclohexenyl-Gruppe, einer Cycloheptenyl-Gruppe, einer Phenyl-Gruppe, einer Naphthyl-Gruppe, einer Pyridinyl-Gruppe, einer Pyrimidinyl-Gruppe, einer Dibenzofuranyl-Gruppe und einer Dibenzothiophenyl-Gruppe, jeweils substituiert mit mindestens einem, ausgewählt aus Deuterium, -F, -CD₃, -CD₂H, -CDH₂, -CF₃, -CF₂H, -CFH₂, einer Cyano-Gruppe, einer Nitro-Gruppe, einer C₁-C₁₀ Alkyl-Gruppe, einer C₁-C₁₀ Alkoxy-Gruppe, einer Cyclopentyl-Gruppe, einer Cyclohexyl-Gruppe, einer Cycloheptyl-Gruppe, einer Cyclooctyl-Gruppe, einer Adamantanyl-Gruppe, einer Norbornanyl-Gruppe, einer Norbornenyl-Gruppe, einer Cyclopentenyl-Gruppe, einer Cyclohexenyl-Gruppe, einer Cycloheptenyl-Gruppe, einer Phenyl-Gruppe, einer Naphthyl-Gruppe, einer Pyridinyl-Gruppe, einer Pyrimidinyl-Gruppe, einer Dibenzofuranyl-Gruppe und einer Dibenzothiophenyl-Gruppe; und
-N(Q₃₁)(Q₃₂), -Si(Q₃₃)(Q₃₄)(Q₃₅), -B(Q₃₆)(Q₃₇) und -P(=O)(Q₃₈)(Q₃₉), wobei Q₃₁ bis Q₃₉ unabhängig ausgewählt sind aus:
-CH₃, -CD₃, -CD₂H, -CDH₂, -CH₂CH₃, -CH₂CD₃, -CH₂CD₂H, -CH₂CDH₂, -CHDCH₃, -CHDCD₂H, -CHDCDH₂, -CHDCD₃, -CD₂CD₃, -CD₂CD₂H und -CD₂CDH₂;
einer n-Propyl-Gruppe, einer Isopropyl-Gruppe, einer n-Butyl-Gruppe, einer Isobutyl-Gruppe, einer sec-Butyl-Gruppe, einer tert-Butyl-Gruppe, einer n-Pentyl-Gruppe, einer Isopentyl-Gruppe, einer sec-Pentyl-Gruppe, einer tert-Pentyl-Gruppe, einer Phenyl-Gruppe und einer Naphthyl-Gruppe; und
einer n-Propyl-Gruppe, einer iso-Propyl-Gruppe, einer n-Butyl-Gruppe, einer Isobutyl-Gruppe, einer sec-Butyl-Gruppe, einer tert-Butyl-Gruppe, einer n-Pentyl-Gruppe, einer Isopentyl-Gruppe, einer sec-Pentyl-Gruppe, einer tert-Pentyl-Gruppe, einer Phenyl-Gruppe und einer Naphthyl-Gruppe, jeweils substituiert mit mindestens einem, ausgewählt aus Deuterium, einer C₁-C₁₀ Alkyl-Gruppe und einer Phenyl-Gruppe,
benachbarte zwei oder mehrere, ausgewählt aus einer Vielzahl von Z₁ optional miteinander verbunden sind, um eine substituierte oder unsubstituierte C₅-C₃₀ carbocyclische Gruppe oder eine substituierte oder unsubstituierte C₂-C₃₀ heterocyclische Gruppe zu bilden, el eine Ganzzahl ausgewählt aus 0 bis 8 ist und * eine Bindungsstelle mit M in Formel 1 angibt.

12. Organometallische Verbindung nach einem der Ansprüche 1 bis 11, wobei L₂ in Formel 1 ausgewählt ist aus den Liganden, die durch die Formeln 13-1 bis 13-47 und 14-1 bis 14-28 dargestellt sind: 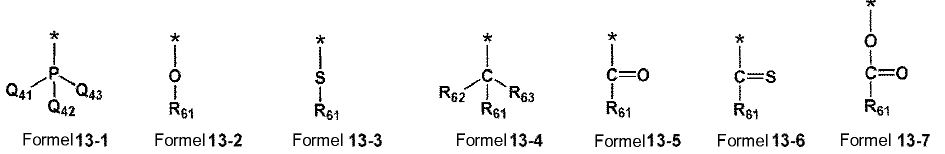 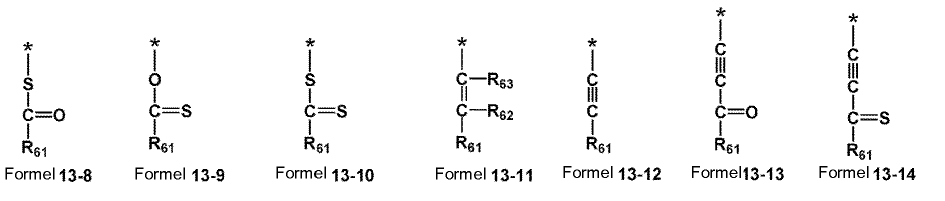 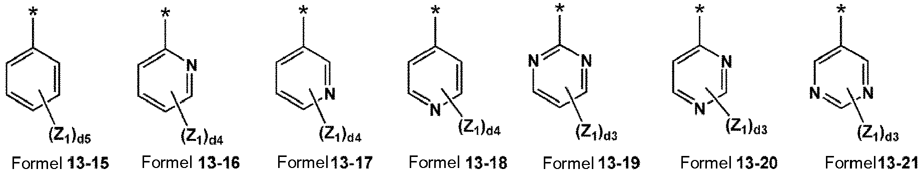 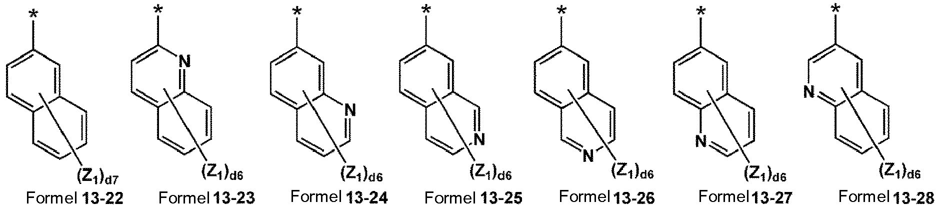 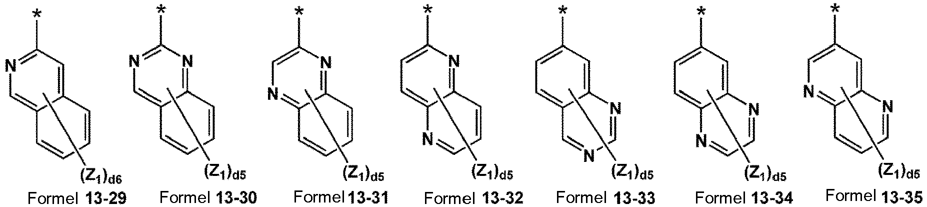 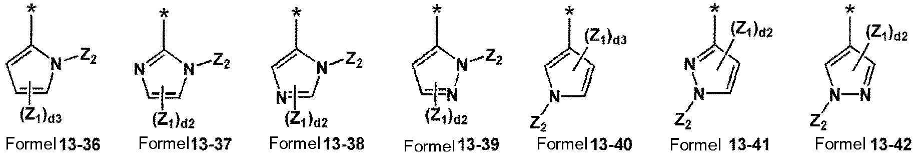 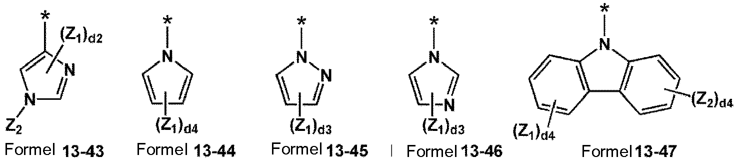 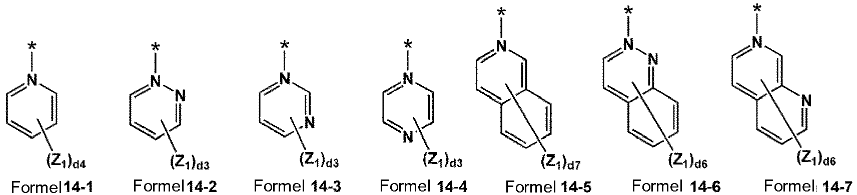 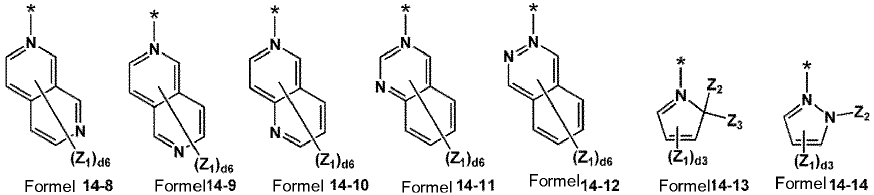 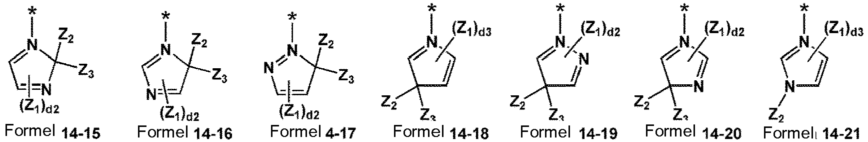 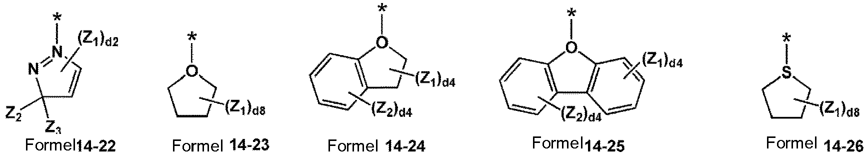 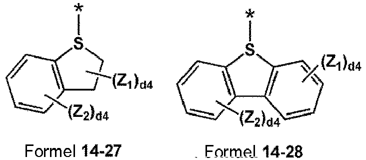 ,
wobei in den Formeln 13-1 bis 13-47 und 14-1 bis 14-28
R₆₁ ausgewählt ist aus:
Wasserstoff, Deuterium, -F, -Cl, -Br, -I, einer Hydroxyl-Gruppe, einer Cyano-Gruppe, einer Nitro-Gruppe, einer Amino-Gruppe, einer Amidino-Gruppe, einer Hydrazin-Gruppe, einer Hydrazon-Gruppe, einer Carbonsäure-Gruppe oder einem Salz davon, einer Sulfonsäure-Gruppe oder einem Salz davon, einer Phosphorsäure-Gruppe oder einem Salz davon, -SF₅, einer C₁-C₂₀-Alkyl-Gruppe und einer C₁-C₂₀-Alkoxy-Gruppe;
einer C₁-C₂₀-Alkyl-Gruppe und einer C₁-C₂₀-Alkoxy-Gruppe, jeweils substituiert mit mindestens einem, ausgewählt aus Deuterium, -F, -Cl, -Br, -I, -CD₃, -CD₂H, -CDH₂, -CF₃, -CF₂H, -CFH₂, einer Hydroxyl-Gruppe, einer Cyano-Gruppe, einer Nitro-Gruppe, einer Amino-Gruppe, einer Amidino-Gruppe, einer Hydrazin-Gruppe, einer Hydrazon-Gruppe, einer Carbonsäure-Gruppe oder einem Salz davon, einer Sulfonsäure-Gruppe oder einem Salz davon, einer Phosphorsäure-Gruppe oder einem Salz davon, einer C₁-C₁₀-Alkyl-Gruppe, einer Cyclopentyl-Gruppe, einer Cyclohexyl-Gruppe, einer Cycloheptyl-Gruppe, einer Cyclooctyl-Gruppe, einer Adamantanyl-Gruppe, einer Norbornanyl-Gruppe, einer Norbornenyl-Gruppe, einer Cyclopentenyl-Gruppe, einer Cyclohexenyl-Gruppe, einer Cycloheptenyl-Gruppe, einer Phenyl-Gruppe, einer Naphthyl-Gruppe, einer Pyridinyl-Gruppe und einer Pyrimidinyl-Gruppe;
einer Cyclopentyl-Gruppe, einer Cyclohexyl-Gruppe, einer Cycloheptyl-Gruppe, einer Cyclooctyl-Gruppe, einer Adamantanyl-Gruppe, einer Norbornanyl-Gruppe, einer Norbornenyl-Gruppe, einer Cyclopentenyl-Gruppe, einer Cyclohexenyl-Gruppe, einer Cycloheptenyl-Gruppe, einer Phenyl-Gruppe, einer Naphthyl-Gruppe, einer Fluorenyl-Gruppe, einer Phenanthrenyl-Gruppe, einer Anthracenyl-Gruppe, einer Fluoranthenyl-Gruppe, einer Triphenylenyl-Gruppe, einer Pyrenyl-Gruppe, einer Chrysenyl-Gruppe, einer Pyrrolyl-Gruppe, einer Thiophenyl-Gruppe, einer Furanyl-Gruppe, einer Imidazolyl-Gruppe, einer Pyrazolyl-Gruppe, einer Thiazolyl-Gruppe, einer Isothiazolyl-Gruppe, einer Oxazolyl-Gruppe, einer Isoxazolyl-Gruppe, einer Pyridinyl-Gruppe, einer Pyrazinyl-Gruppe, einer Pyrimidinyl-Gruppe, einer Pyridazinyl-Gruppe, einer Isoindolyl-Gruppe, einer Indolyl-Gruppe, einer Indazolyl-Gruppe, einer Purinyl-Gruppe, einer Chinolinyl-Gruppe, einer Isochinolinyl-Gruppe, einer Benzochinolinyl-Gruppe, einer Chinoxalinyl-Gruppe, einer Chinazolinyl-Gruppe, einer Cinnolinyl-Gruppe, einer Carbazolyl-Gruppe, einer Phenanthrolinyl-Gruppe, einer Benzimidazolyl-Gruppe, einer Benzofuranyl-Gruppe, einer Benzothiophenyl-Gruppe, einer Isobenzothiazolyl-Gruppe, einer Benzoxazolyl-Gruppe, einer Isobenzoxazolyl-Gruppe, einer Triazolyl-Gruppe, einer Tetrazolyl-Gruppe, einer Oxadiazolyl-Gruppe, einer Triazinyl-Gruppe, einer Dibenzofuranyl-Gruppe, einer Dibenzothiophenyl-Gruppe, einer Benzocarbazolyl-Gruppe, einer Dibenzocarbazolyl-Gruppe, einer Imidazopyridinyl-Gruppe und einer Imidazopyrimidinyl-Gruppe;
einer Cyclopentyl-Gruppe, einer Cyclohexyl-Gruppe, einer Cycloheptyl-Gruppe, einer Cyclooctyl-Gruppe, einer Adamantanyl-Gruppe, einer Norbornanyl-Gruppe, einer Norbornenyl-Gruppe, einer Cyclopentenyl-Gruppe, einer Cyclohexenyl-Gruppe, einer Cycloheptenyl-Gruppe, einer Phenyl-Gruppe, einer Naphthyl-Gruppe, einer Fluorenyl-Gruppe, einer Phenanthrenyl-Gruppe, einer Anthracenyl-Gruppe, einer Fluoranthenyl-Gruppe, einer Triphenylenyl-Gruppe, einer Pyrenyl-Gruppe, einer Chrysenyl-Gruppe, einer Pyrrolyl-Gruppe, einer Thiophenyl-Gruppe, einer Furanyl-Gruppe, einer Imidazolyl-Gruppe, einer Pyrazolyl-Gruppe, einer Thiazolyl-Gruppe, einer Isothiazolyl-Gruppe, einer Oxazolyl-Gruppe, einer Isoxazolyl-Gruppe, einer Pyridinyl-Gruppe, einer Pyrazinyl-Gruppe, einer Pyrimidinyl-Gruppe, einer Pyridazinyl-Gruppe, einer Isoindolyl-Gruppe, einer Indolyl-Gruppe, einer Indazolyl-Gruppe, einer Purinyl-Gruppe, einer Chinolinyl-Gruppe, einer Isochinolinyl-Gruppe, einer Benzochinolinyl-Gruppe, einer Chinoxalinyl-Gruppe, einer Chinazolinyl-Gruppe, einer Cinnolinyl-Gruppe, einer Carbazolyl-Gruppe, einer Phenanthrolinyl-Gruppe, einer Benzimidazolyl-Gruppe, einer Benzofuranyl-Gruppe, einer Benzothiophenyl-Gruppe, einer Isobenzothiazolyl-Gruppe, einer Benzoxazolyl-Gruppe, einer Isobenzoxazolyl-Gruppe, einer Triazolyl-Gruppe, einer Tetrazolyl-Gruppe, einer Oxadiazolyl-Gruppe, einer Triazinyl-Gruppe, einer Dibenzofuranyl-Gruppe, einer Dibenzothiophenyl-Gruppe, einer Benzocarbazolyl-Gruppe, einer Dibenzocarbazolyl-Gruppe, einer Imidazopyridinyl-Gruppe und einer Imidazopyrimidinyl-Gruppe, jeweils substituiert mit mindestens einem, ausgewählt aus Deuterium, -F, -Cl, -Br, -I, -CD₃, -CD₂H, -CDH₂, -CF₃, -CF₂H, -CFH₂, einer Hydroxyl-Gruppe, einer Cyano-Gruppe, einer Nitro-Gruppe, einer Amino-Gruppe, einer Amidino-Gruppe, einer Hydrazin-Gruppe, einer Hydrazon-Gruppe, einer Carbonsäure-Gruppe oder einem Salz davon, einer Sulfonsäure-Gruppe oder einem Salz davon, einer Phosphorsäure-Gruppe oder einem Salz davon, einer C₁-C₂₀-Alkyl-Gruppe, einer C₁-C₂₀-Alkoxy-Gruppe, einer Cyclopentyl-Gruppe, einer Cyclohexyl-Gruppe, einer Cycloheptyl-Gruppe, einer Cyclooctyl-Gruppe, einer Adamantanyl-Gruppe, einer Norbornanyl-Gruppe, einer Norbornenyl-Gruppe, einer Cyclopentenyl-Gruppe, einer Cyclohexenyl-Gruppe, einer Cycloheptenyl-Gruppe, einer Phenyl-Gruppe, einer Naphthyl-Gruppe, einer Fluorenyl-Gruppe, einer Phenanthrenyl-Gruppe, einer Anthracenyl-Gruppe, einer Fluoranthenyl-Gruppe, einer Triphenylenyl-Gruppe, einer Pyrenyl-Gruppe, einer Chrysenyl-Gruppe, einer Pyrrolyl-Gruppe, einer Thiophenyl-Gruppe, einer Furanyl-Gruppe, einer Imidazolyl-Gruppe, einer Pyrazolyl-Gruppe, einer Thiazolyl-Gruppe, einer Isothiazolyl-Gruppe, einer Oxazolyl-Gruppe, einer Isoxazolyl-Gruppe, einer Pyridinyl-Gruppe, einer Pyrazinyl-Gruppe, einer Pyrimidinyl-Gruppe, einer Pyridazinyl-Gruppe, einer Isoindolyl-Gruppe, einer Indolyl-Gruppe, einer Indazolyl-Gruppe, einer Purinyl-Gruppe, einer Chinolinyl-Gruppe, einer Isochinolinyl-Gruppe, einer Benzochinolinyl-Gruppe, einer Chinoxalinyl-Gruppe, einer Chinazolinyl-Gruppe, einer Cinnolinyl-Gruppe, einer Carbazolyl-Gruppe, einer Phenanthrolinyl-Gruppe, einer Benzimidazolyl-Gruppe, einer Benzofuranyl-Gruppe, einer Benzothiophenyl-Gruppe, einer Isobenzothiazolyl-Gruppe, einer Benzoxazolyl-Gruppe, einer Isobenzoxazolyl-Gruppe, einer Triazolyl-Gruppe, einer Tetrazolyl-Gruppe, einer Oxadiazolyl-Gruppe, einer Triazinyl-Gruppe, einer Dibenzofuranyl-Gruppe, einer Dibenzothiophenyl-Gruppe, einer Benzocarbazolyl-Gruppe, einer Dibenzocarbazolyl-Gruppe, einer Imidazopyridinyl-Gruppe und einer Imidazopyrimidinyl-Gruppe; und
-N(Q₁)(Q₂), -Si(Q₃)(Q₄)(Q₅), -B(Q₆)(Q₇), -P(=O)(Q₈)(Q₉) UND -P(Q₄₁)(Q₄₂)(Q₄₃),
Z₁ bis Z₃ jeweils unabhängig ausgewählt sind aus:
Wasserstoff, Deuterium, -F, einer Cyano-Gruppe, einer Nitro-Gruppe, -SF₅, einer Methyl-Gruppe, einer Ethyl-Gruppe, einer n-Propyl-Gruppe, einer iso-Propyl-Gruppe, einer n-Butyl-Gruppe, einer Isobutyl-Gruppe, einer sec-Butyl-Gruppe, einer tert-Butyl-Gruppe, einer n-Pentyl-Gruppe, einer Isopentyl-Gruppe, einer sec-Pentyl-Gruppe, einer tert-Pentyl-Gruppe, einer n-Hexyl-Gruppe, einer iso-Hexyl-Gruppe, einer sec-Hexyl-Gruppe, einer tert-Hexyl-Gruppe, einer n-Heptyl-Gruppe, einer iso-Heptyl-Gruppe, einer sec-Heptyl-Gruppe, einer tert-Heptyl-Gruppe, einer n-Octyl-Gruppe, einer iso-Octyl-Gruppe, einer sec-Octyl-Gruppe, einer tert-Octyl-Gruppe, einer n-Nonyl-Gruppe, einer iso-Nonyl-Gruppe, einer sec-Nonyl-Gruppe, einer tert-Nonyl-Gruppe, einer n-Decyl-Gruppe, einer iso-Decyl-Gruppe, einer sec-Decyl-Gruppe, einer tert-Decyl-Gruppe, einer Methoxy-Gruppe, einer Ethoxy-Gruppe, einer Propoxy-Gruppe, einer Butoxy-Gruppe, einer Pentoxy-Gruppe, einer Cyclopentyl-Gruppe, einer Cyclohexyl-Gruppe, einer Cycloheptyl-Gruppe, einer Cyclooctyl-Gruppe, einer Adamantanyl-Gruppe, einer Norbornanyl-Gruppe, einer Norbornenyl-Gruppe, einer Cyclopentenyl-Gruppe, einer Cyclohexenyl-Gruppe, einer Cycloheptenyl-Gruppe, einer Phenyl-Gruppe, einer Naphthyl-Gruppe, einer Pyridinyl-Gruppe, einer Pyrimidinyl-Gruppe, einer Dibenzofuranyl-Gruppe und einer Dibenzothiophenyl-Gruppe;
einer Methyl-Gruppe, einer Ethyl-Gruppe, einer n-Propyl-Gruppe, einer iso-Propyl-Gruppe, einer n-Butyl-Gruppe, einer Isobutyl-Gruppe, einer sec-Butyl-Gruppe, einer tert-Butyl-Gruppe, einer n-Pentyl-Gruppe, einer Isopentyl-Gruppe, einer sec-Pentyl-Gruppe, einer tert-Pentyl-Gruppe, einer n-Hexyl-Gruppe, einer iso-Hexyl-Gruppe, einer sec-Hexyl-Gruppe, einer tert-Hexyl-Gruppe, einer n-Heptyl-Gruppe, einer iso-Heptyl-Gruppe, einer sec-Heptyl-Gruppe, einer tert-Heptyl-Gruppe, einer n-Octyl-Gruppe, einer iso-Octyl-Gruppe, einer sec-Octyl-Gruppe, einer tert-Octyl-Gruppe, einer n-Nonyl-Gruppe, einer iso-Nonyl-Gruppe, einer sec-Nonyl-Gruppe, einer tert-Nonyl-Gruppe, einer n-Decyl-Gruppe, einer iso-Decyl-Gruppe, einer sec-Decyl-Gruppe, einer tert-Decyl-Gruppe, einer Methoxy-Gruppe, einer Ethoxy-Gruppe, einer Propoxy-Gruppe, einer Butoxy-Gruppe, einer Pentoxy-Gruppe, einer Cyclopentyl-Gruppe, einer Cyclohexyl-Gruppe, einer Cycloheptyl-Gruppe, einer Cyclooctyl-Gruppe, einer Adamantanyl-Gruppe, einer Norbornanyl-Gruppe, einer Norbornenyl-Gruppe, einer Cyclopentenyl-Gruppe, einer Cyclohexenyl-Gruppe, einer Cycloheptenyl-Gruppe, einer Phenyl-Gruppe, einer Naphthyl-Gruppe, einer Pyridinyl-Gruppe, einer Pyrimidinyl-Gruppe, einer Dibenzofuranyl-Gruppe und einer Dibenzothiophenyl-Gruppe, jeweils substituiert mit mindestens einem, ausgewählt aus Deuterium, -F, -CD₃, -CD₂H, -CDH₂, -CF₃, -CF₂H, -CFH₂, einer Cyano-Gruppe, einer Nitro-Gruppe, einer C₁-C₁₀-Alkyl-Gruppe, einer C₁-C₁₀-Alkoxy-Gruppe, einer Cyclopentyl-Gruppe, einer Cyclohexyl-Gruppe, einer Cycloheptyl-Gruppe, einer Cyclooctyl-Gruppe, einer Adamantanyl-Gruppe, einer Norbornanyl-Gruppe, einer Norbornenyl-Gruppe, einer Cyclopentenyl-Gruppe, einer Cyclohexenyl-Gruppe, einer Cycloheptenyl-Gruppe, einer Phenyl-Gruppe, einer Naphthyl-Gruppe, einer Pyridinyl-Gruppe, einer Pyrimidinyl-Gruppe, einer Dibenzofuranyl-Gruppe und einer Dibenzothiophenyl-Gruppe; und
-N(Q₃₁)(Q₃₂), -Si(Q₃₃)(Q₃₄)(Q₃₅), -B(Q₃₆)(Q₃₇) und -P(=O)(Q₃₈)(Q₃₉),
wobei Q₁ bis Q₉, Q₃₁ bis Q₃₉ und Q₄₁ bis Q₄₃ jeweils unabhängig ausgewählt sind aus:
-CH₃, -CD₃, -CD₂H, -CDH₂, -CH₂CH₃, -CH₂CD₃, -CH₂CD₂H, -CH₂CDH₂, -CHDCH₃, -CHDCD₂H, -CHDCDH₂, -CHDCD₃, -CD₂CD₃, -CD₂CD₂H UND -CD₂CDH₂;
einer n-Propyl-Gruppe, einer Isopropyl-Gruppe, einer n-Butyl-Gruppe, einer Isobutyl-Gruppe, einer sec-Butyl-Gruppe, einer tert-Butyl-Gruppe, einer n-Pentyl-Gruppe, einer Isopentyl-Gruppe, einer sec-Pentyl-Gruppe, einer tert-Pentyl-Gruppe, einer Phenyl-Gruppe und einer Naphthyl-Gruppe; und
einer n-Propyl-Gruppe, einer iso-Propyl-Gruppe, einer n-Butyl-Gruppe, einer Isobutyl-Gruppe, einer sec-Butyl-Gruppe, einer tert-Butyl-Gruppe, einer n-Pentyl-Gruppe, einer Isopentyl-Gruppe, einer sec-Pentyl-Gruppe, einer tert-Pentyl-Gruppe, einer Phenyl-Gruppe und einer Naphthyl-Gruppe, jeweils substituiert mit mindestens einem, ausgewählt aus Deuterium, einer C₁-C₁₀-Alkyl-Gruppe und einer Phenyl-Gruppe,
d2 1 oder 2 ist,
d3 eine Ganzzahl ausgewählt aus 1 bis 3 ist,
d4 eine Ganzzahl ausgewählt aus 1 bis 4 ist,
d5 eine Ganzzahl ausgewählt aus 1 bis 5 ist,
d6 eine Ganzzahl ausgewählt aus 1 bis 6 ist,
d7 eine Ganzzahl ausgewählt aus 1 bis 7 ist,
d8 eine Ganzzahl ausgewählt aus 1 bis 8 ist, und
* eine Bindungsstelle bis M in Formel 1 ist.

13. Organometallische Verbindung nach Anspruch 1, wobei
die organometallische Verbindung eine ist, die aus den verbindungen 1 bis 142 ausgewählt ist:

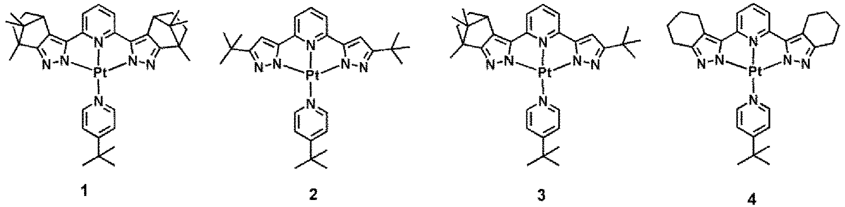

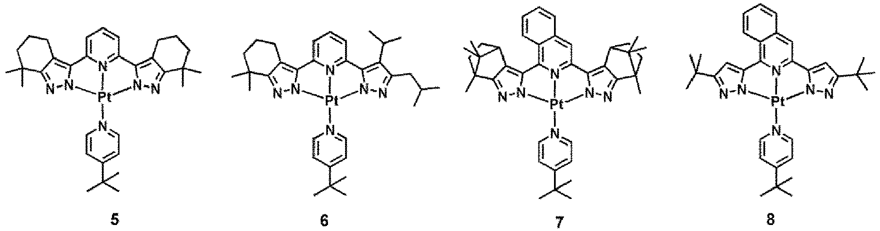

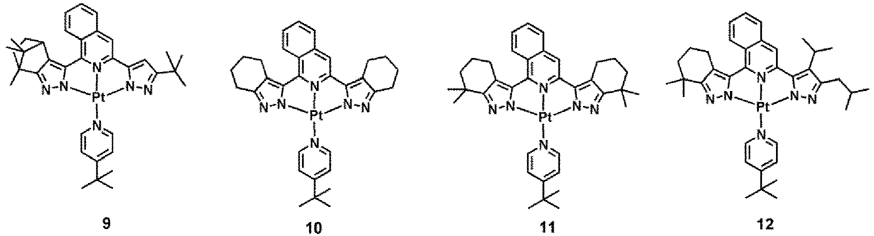

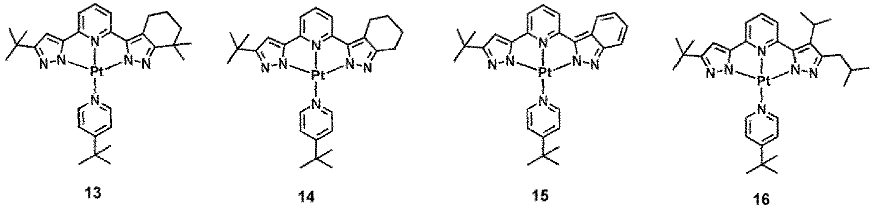

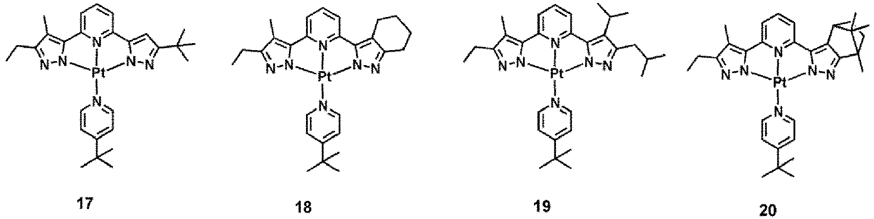

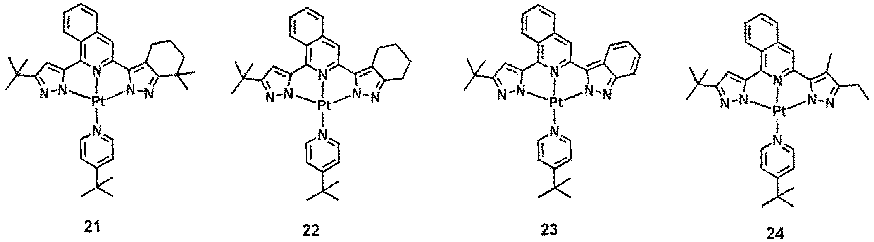

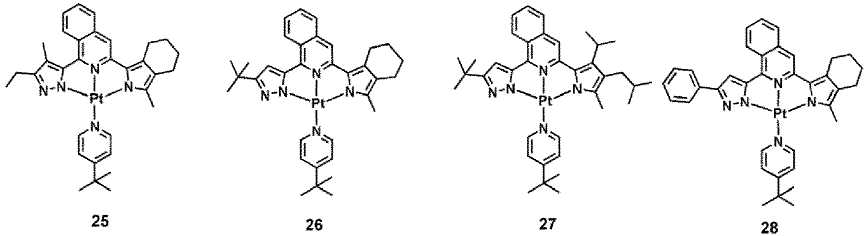

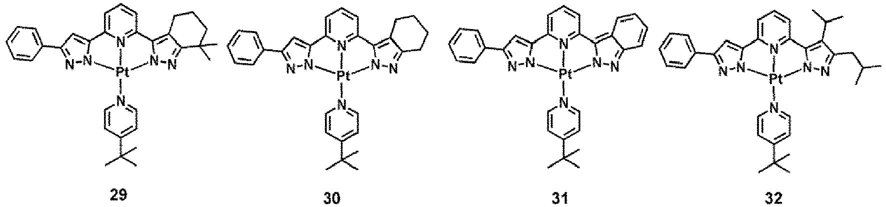

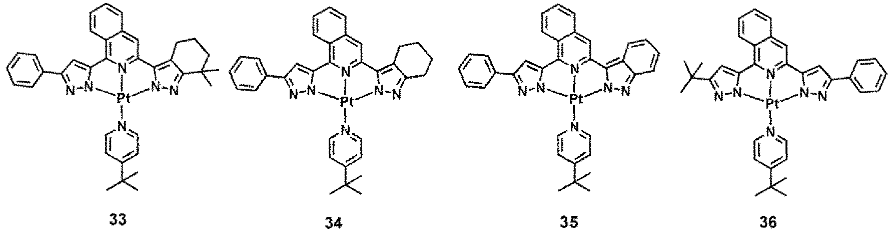

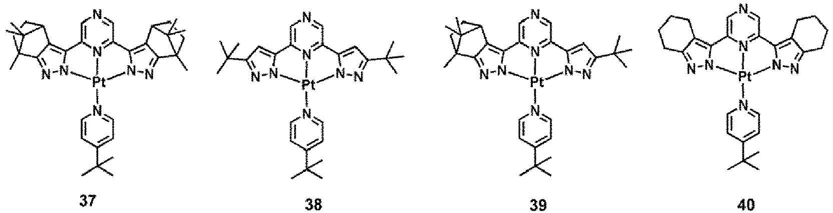

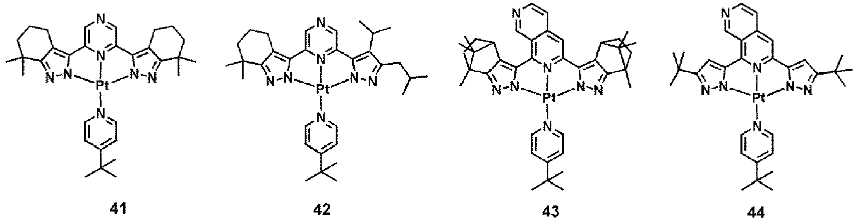

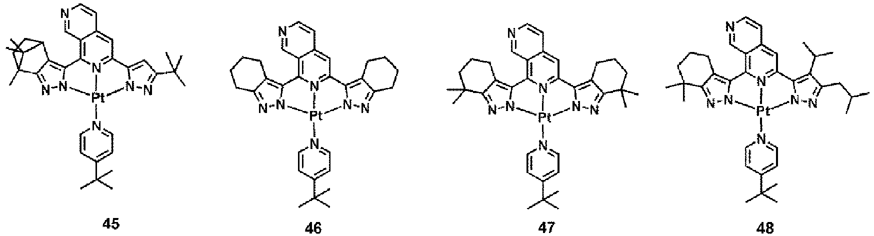

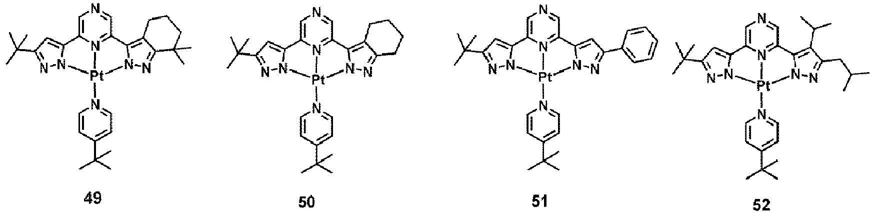

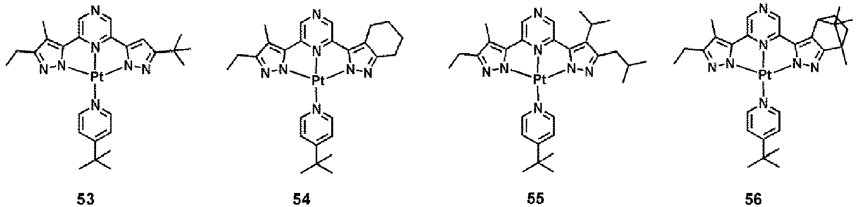

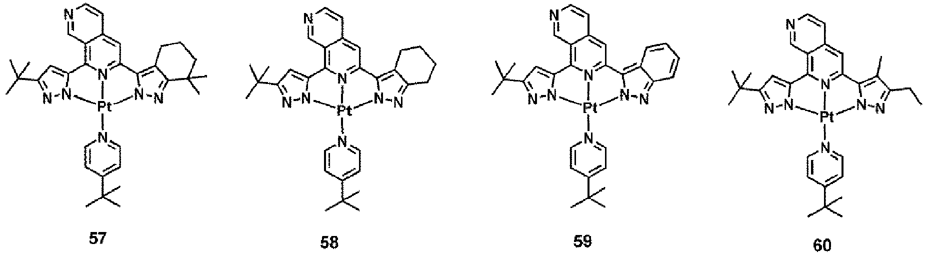

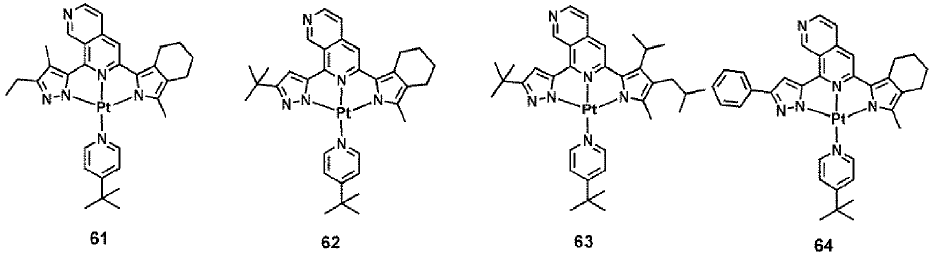

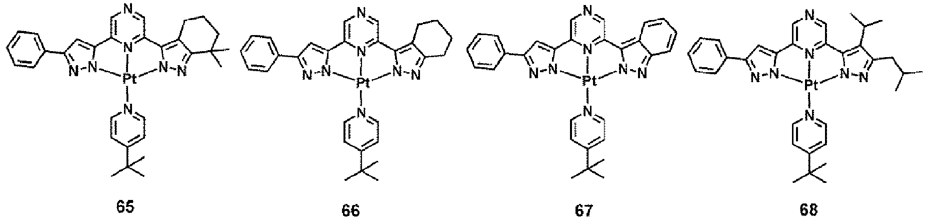

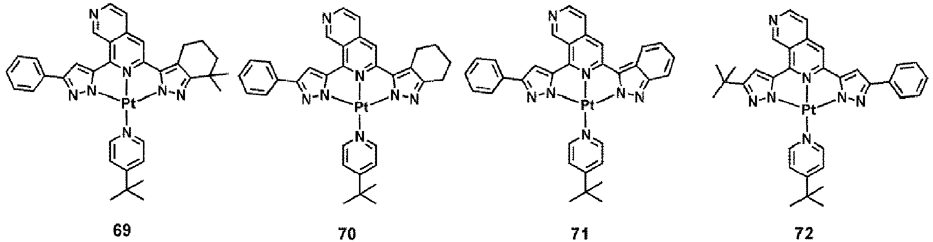

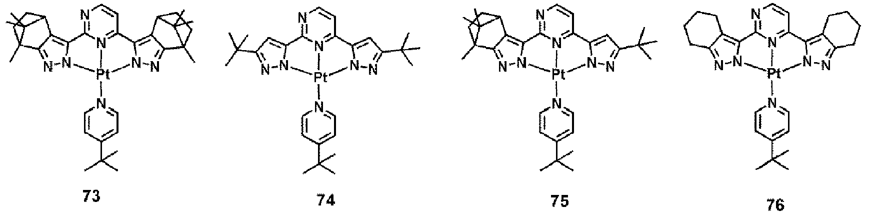

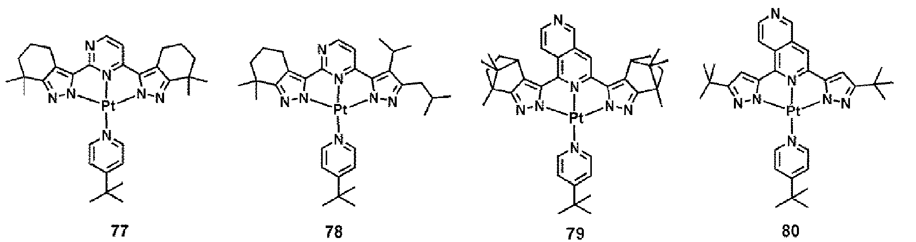

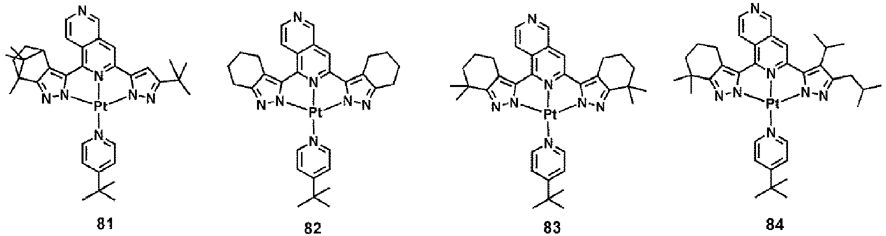

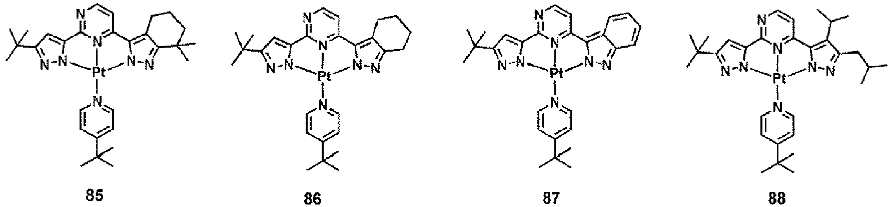

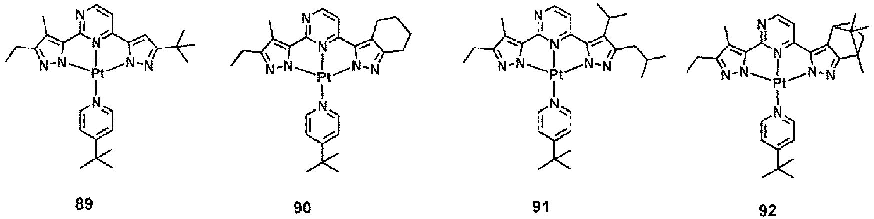

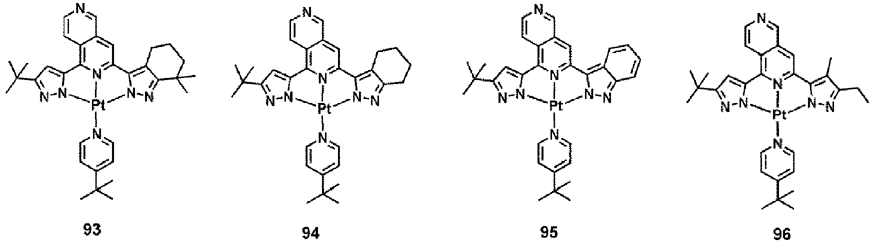

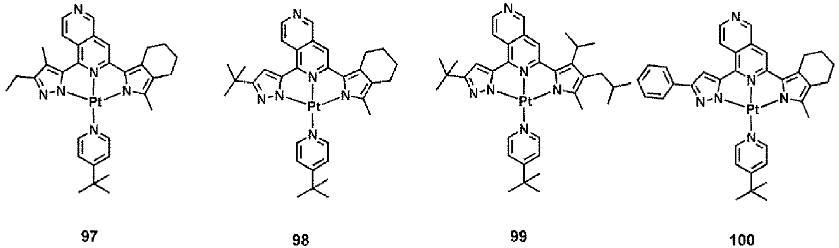

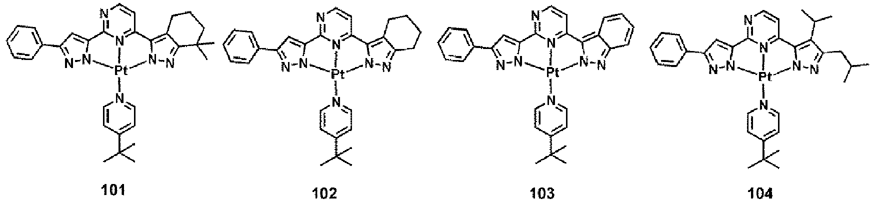

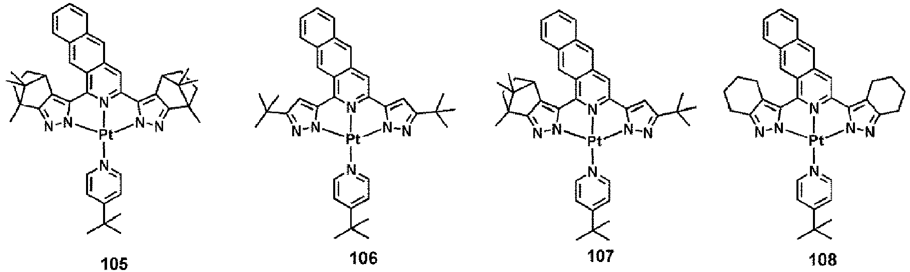

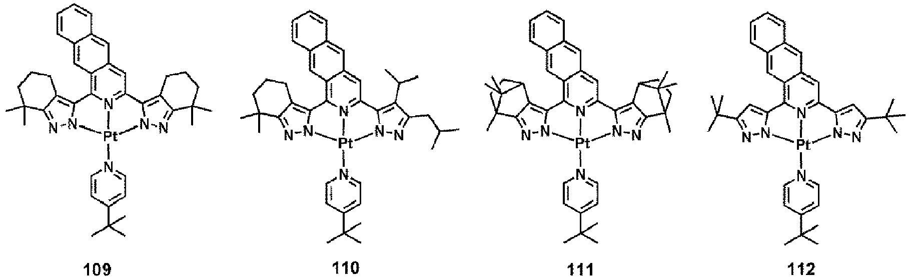

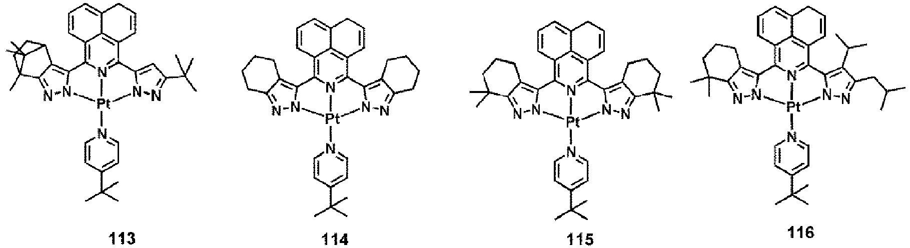

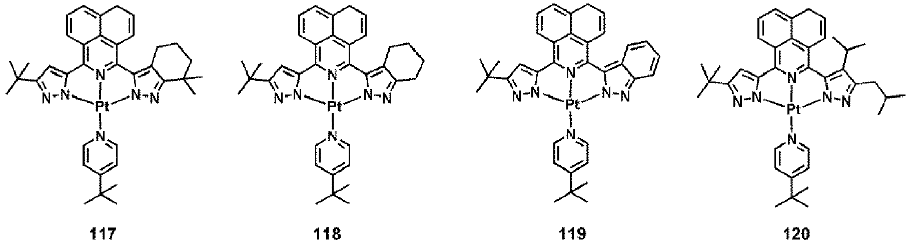

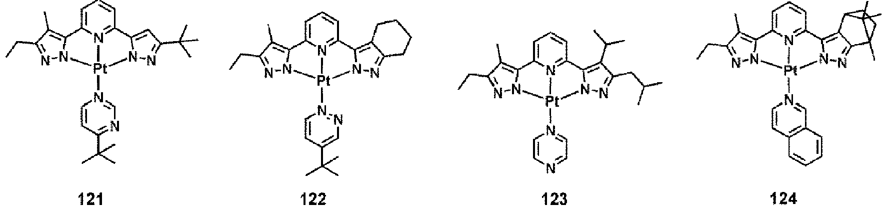

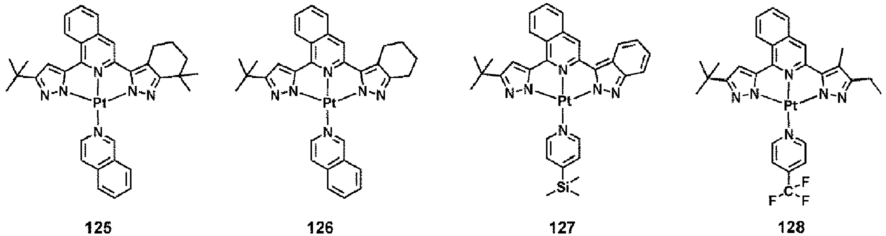

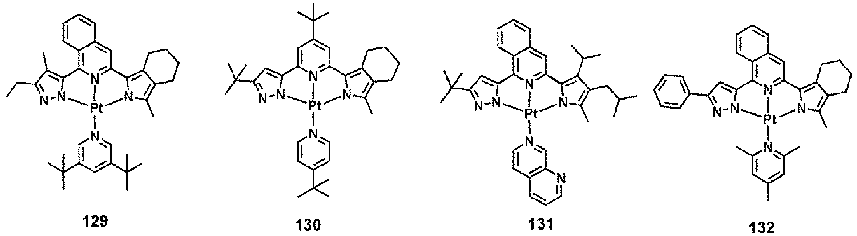

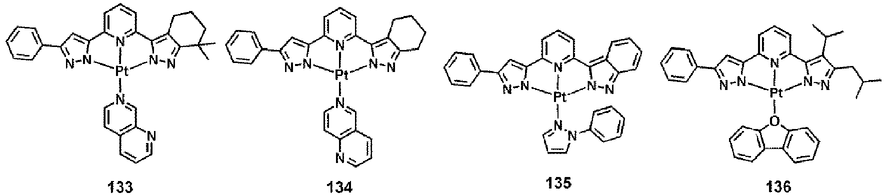

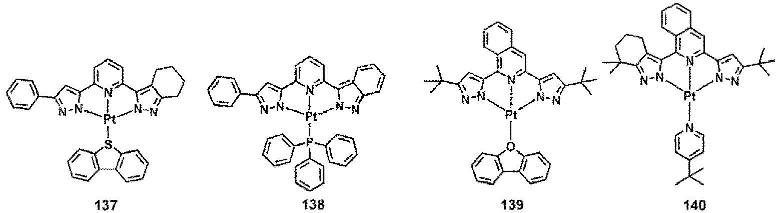

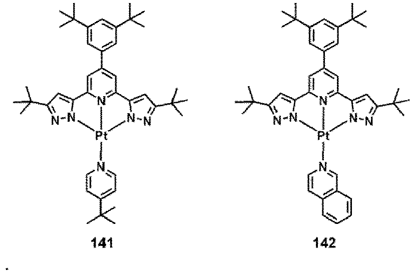

14. Organische lichtemittierende Vorrichtung, umfassend:
eine erste Elektrode;
eine zweite Elektrode; und
eine organische Schicht zwischen der ersten Elektrode und der zweiten Elektrode, wobei die organische Schicht eine Emissionsschicht umfasst,
wobei die organische Schicht eine oder mehrere ausgewählt aus den organometallischen Verbindungen nach einem der Ansprüche 1 bis 13 umfasst, vorzugsweise wobei die organometallische Verbindung in der Emissionsschicht enthalten ist.

15. Zusammensetzung, umfassend eine oder mehrere Zusammensetzungen, ausgewählt aus den organometallischen Verbindungen nach einem der Ansprüche 1 bis 13 zur Verwendung bei *in*-*vivo*-Diagnose.

16. Verwendung einer Zusammensetzung, umfassend:
eine oder mehrere, ausgewählt aus den organometallischen Verbindungen nach einem der Ansprüche 1 bis 13 für *in*-*vivo*-Diagnose.

## Revendications

1. Composé organométallique représenté par la formule 1 :
<Formule 1> M(L₁)(L₂) 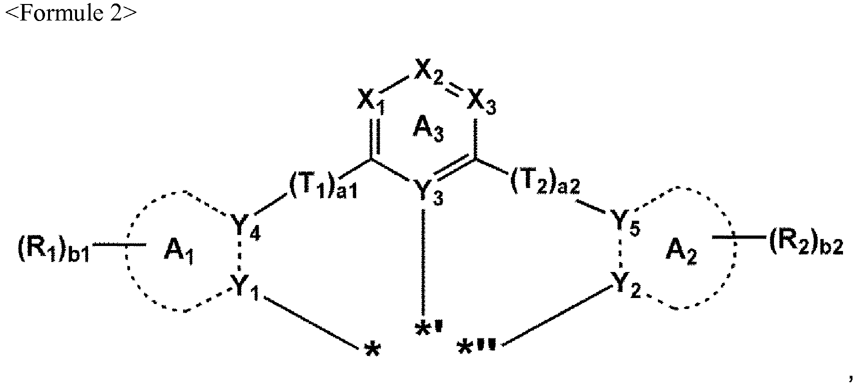
dans lesquelles, dans les formules 1 et 2,
M représente un atome de ruthénium (Ru), de rhodium (Rh), de palladium (Pd), de platine (Pt) ou d'or (Au),
L₁ est choisi parmi les ligands tridentés représentés par la formule 2,
L₂ est choisi parmi les ligands organiques monodentés,
*, *', et *" dans la formule 2 indiquent chacun un site de liaison à M dans la formule 1,
Y₁ à Y₃ représentent chacun indépendamment un atome d'azote (N),
Y₄ et Y₅ représentent chacun indépendamment un atome de carbone (C),
une liaison entre Y₁ et Y₄ est une liaison simple ou une double liaison, et une liaison entre Y₂ et Y₅ est une liaison simple ou une double liaison,
l'une choisie parmi une liaison entre Y₁ et M, une liaison entre Y₂ et M, et une liaison entre le ligand L₂ et M représente une liaison de coordination, et les deux autres représentent une liaison covalente,
une liaison entre Y₃ et M représente une liaison de coordination,
les noyaux A₁ et A₂ sont chacun indépendamment choisis parmi un noyau pyrrole, un noyau pyrazole et un noyau imidazole, et au moins l'un choisi parmi les atomes A₁ et A₂ représente un noyau pyrazole,
X₁ représente un atome N ou un groupe C(R₃), X₂ représente un atome N ou un groupe C(R₄), X₃ représente un atome N ou un groupe C(R₅), deux groupes ou plus parmi R₃ à R₅ sont éventuellement reliés les uns aux autres pour former un groupe carbocyclique en C₅ à C₃₀ substitué ou non substitué ou un groupe hétérocyclique en C₂ à C₃₀ substitué ou non substitué, et le noyau A₃ comporte deux atomes d'azote ou moins en tant qu'atome formant noyau,
T₁ et T₂ sont chacun indépendamment choisis parmi une liaison simple, *-O-*', *-S-*', *-C(R₆)(R₇)-*', *-C(R₆)=*', *=C(R₆)-*', *-C(R₆)=C(R₇)-*', *-C(=O)-*', *-C(=S)-*', *-C≡C-*', *-N(R₆)-*', et *-Si(R₆)(R₇)-*', et R₆ et R₇ sont éventuellement reliés l'un à l'autre pour former un groupe carbocyclique en C₅ à C₃₀ substitué ou non substitué ou un groupe hétérocyclique en C₂ à C₃₀ substitué ou non substitué,
a1 et a2 représentent chacun indépendamment un entier choisi parmi 1 à 3,
R₁ à R₇ sont chacun indépendamment choisis parmi un atome d'hydrogène, un atome de deutérium, -F, -Cl, -Br, -I, un groupe -SF₅, un groupe hydroxy, un groupe cyano, un groupe nitro, un groupe amino, un groupe amidino, un groupe hydrazine, un groupe hydrazone, un groupe acide carboxylique ou un sel de celui-ci, un groupe acide sulfonique ou un sel de celui-ci, un groupe acide phosphorique ou un sel de celui-ci, un groupe alkyle en C₁ à C₆₀ substitué ou non substitué, un groupe alcényle en C₂ à C₆₀ substitué ou non substitué, un groupe alcynyle en C₂ à C₆₀ substitué ou non substitué, un groupe alcoxy en C₁ à C₆₀ substitué ou non substitué, un groupe cycloalkyle en C₃ à C₁₀ substitué ou non substitué, un groupe hétérocycloalkyle en C₁ à C₁₀ substitué ou non substitué, un groupe cycloalcényle en C₃ à C₁₀ substitué ou non substitué, un groupe hétérocycloalcényle en C₁ à C₁₀ substitué ou non substitué, un groupe aryle en C₆ à C₆₀ substitué ou non substitué, un groupe aryloxy en C₆ à C₆₀ substitué ou non substitué, un groupe arylthio en C₆ à C₆₀ substitué ou non substitué, un groupe hétéroaryle en C₁ à C₆₀ substitué ou non substitué, un groupe polycyclique condensé non aromatique monovalent substitué ou non substitué, un groupe hétéropolycyclique condensé non aromatique monovalent substitué ou non substitué, -N(Q₁)(Q₂), -Si(Q₃)(Q₄)(Q₅), -B(Q₆)(Q₇), et -P(=O)(Q₈)(Q₉),
bl et b2 sont chacun indépendamment un entier choisi parmi 0 à 3, lorsque bl est supérieur ou égal à deux, deux groupes R₁ ou plus sont identiques ou différents les uns des autres, et lorsque b2 est supérieur ou égal à deux, deux groupes R₂ ou plus sont identiques ou différents les uns des autres,
deux groupes choisis parmi les groupes R₁ dans le nombre de bl sont éventuellement reliés pour former un groupe carbocyclique en C₅ à C₃₀ substitué ou non substitué ou un groupe hétérocyclique en C₂ à C₃₀ substitué ou non substitué,
deux groupes choisis parmi les groupes R₂ dans le nombre de b2 sont éventuellement reliés pour former un groupe carbocyclique en C₅ à C₃₀ substitué ou non substitué ou un groupe hétérocyclique en C₂ à C₃₀ substitué ou non substitué,
un groupement représenté par 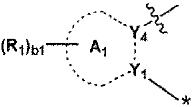 dans la formule 2 n'est pas 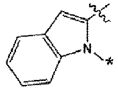 et 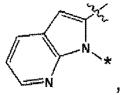 et un groupement représenté par 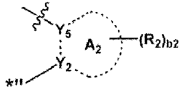 dans la formule 2 n'est pas 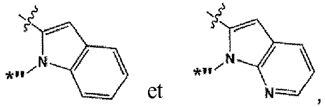 et
au moins un substituent choisi parmi un ou plusieurs substituants du groupe carbocyclique en C₅ à C₃₀ substitué, du groupe hétérocyclique en C₂ à C₃₀ substitué, du groupe alkyle en C₁ à C₆₀ substitué, du groupe alcényle en C₂ à C₆₀ substitué, du groupe alcynyle en C₂ à C₆₀ substitué, du groupe alcoxy en C₁ à C₆₀ substitué, du groupe cycloalkyle en C₃ à C₁₀ substitué, du groupe hétérocycloalkyle en C₁ à C₁₀ substitué, du groupe cycloalcényle en C₃ à C₁₀ substitué, du groupe hétérocycloalcényle en C₁ à C₁₀ substitué, du groupe aryle en C₆ à C₆₀ substitué, du groupe aryloxy en C₆ à C₆₀ substitué, du groupe arylthio en C₆ à C₆₀ substitué, du groupe hétéroaryle en C₁ à C₆₀ substitué, du groupe polycyclique condensé non aromatique monovalent substitué, et du groupe hétéropolycyclique condensé non aromatique monovalent substitué est choisi parmi :
un atome de deutérium, -F, -Cl, -Br, -I, un groupe -CD₃, -CD₂H, -CDH₂, -CF₃, -CF₂H, -CFH₂, un groupe hydroxy, un groupe cyano, un groupe nitro, un groupe amino, un groupe amidino, un groupe hydrazine, un groupe hydrazone, un groupe acide carboxylique ou un sel de celui-ci, un groupe acide sulfonique ou un sel de celui-ci, un groupe acide phosphorique ou un sel de celui-ci, un groupe alkyle en C₁ à C₆₀, un groupe alcényle en C₂ à C₆₀, un groupe alcynyle en C₂ à C₆₀, et un groupe alcoxy en C₁ à C₆₀ ;
un groupe alkyle en C₁ à C₆₀, un groupe alcényle en C₂ à C₆₀, un groupe alcynyle en C₂ à C₆₀, et un groupe alcoxy en C₁ à C₆₀, chacun substitué par au moins un élément choisi parmi un atome de deutérium, -F, -Cl, -Br, -I, un groupe -CD₃, -CD₂H, -CDH₂, -CF₃, -CF₂H, -CFH₂, un groupe hydroxy, un groupe cyano, un groupe nitro, un groupe amino, un groupe amidino, un groupe hydrazine, un groupe hydrazone, un groupe acide carboxylique ou un sel de celui-ci, un groupe acide sulfonique ou un sel de celui-ci, un groupe acide phosphorique ou un sel de celui-ci, un groupe cycloalkyle en C₃ à C₁₀, un groupe hétérocycloalkyle en C₁ à C₁₀, un groupe cycloalcényle en C₃ à C₁₀, un groupe hétérocycloalcényle en C₁ à C₁₀, un groupe aryle en C₆ à C₆₀, un groupe aryloxy en C₆ à C₆₀, un groupe arylthio en C₆ à C₆₀, un groupe hétéroaryle en C₁ à C₆₀, un groupe polycyclique condensé non aromatique monovalent, un groupe hétéropolycyclique condensé non aromatique monovalent, -N(Q₁₁)(Q₁₂), -Si(Q₁₃)(Q₁₄)(Q₁₅), -B(Q₁₆)(Q₁₇), et -P(=O)(Q₁₈)(Q₁₉) ;
un groupe cycloalkyle en C₃ à C₁₀, un groupe hétérocycloalkyle en C₁ à C₁₀, un groupe cycloalcényle en C₃ à C₁₀, un groupe hétérocycloalcényle en C₁ à C₁₀, un groupe aryle en C₆ à C₆₀, un groupe aryloxy en C₆ à C₆₀, un groupe arylthio en C₆ à C₆₀, un groupe hétéroaryle en C₁ à C₆₀, un groupe polycyclique condensé non aromatique monovalent, et un groupe hétéropolycyclique condensé non aromatique monovalent ;
un groupe cycloalkyle en C₃ à C₁₀, un groupe hétérocycloalkyle en C₁ à C₁₀, un groupe cycloalcényle en C₃ à C₁₀, un groupe hétérocycloalcényle en C₁ à C₁₀, un groupe aryle en C₆ à C₆₀, un groupe aryloxy en C₆ à C₆₀, un groupe arylthio en C₆ à C₆₀, un groupe hétéroaryle en C₁ à C₆₀, un groupe polycyclique condensé non aromatique monovalent, et un groupe hétéropolycyclique condensé non aromatique monovalent, chacun substitué par au moins un élément choisi parmi un atome de deutérium, -F, -Cl, -Br, -I, un groupe -CD₃, -CD₂H, -CDH₂, -CF₃, -CF₂H, -CFH₂, un groupe hydroxy, un groupe cyano, un groupe nitro, un groupe amino, un groupe amidino, un groupe hydrazine, un groupe hydrazone, un groupe acide carboxylique ou un sel de celui-ci, un groupe acide sulfonique ou un sel de celui-ci, un groupe acide phosphorique ou un sel de celui-ci, un groupe alkyle en C₁ à C₆₀, un groupe alcényle en C₂ à C₆₀, un groupe alcynyle en C₂ à C₆₀, un groupe alcoxy en C₁ à C₆₀, un groupe cycloalkyle en C₃ à C₁₀, un groupe hétérocycloalkyle en C₁ à C₁₀, un groupe cycloalcényle en C₃ à C₁₀, un groupe hétérocycloalcényle en C₁ à C₁₀, un groupe aryle en C₆ à C₆₀, un groupe aryloxy en C₆ à C₆₀, un groupe arylthio en C₆ à C₆₀, un groupe hétéroaryle en C₁ à C₆₀, un groupe polycyclique condensé non aromatique monovalent, un groupe hétéropolycyclique condensé non aromatique monovalent, -N(Q₂₁)(Q₂₂), -Si(Q₂₃)(Q₂₄)(Q₂₅), -B(Q₂₆)(Q₂₇), et -P(=O)(Q₂₈)(Q₂₉) ; et
-N(Q₃₁)(Q₃₂), -Si(Q₃₃)(Q₃₄)(Q₃₅), -B(Q₃₆)(Q₃₇), et -P(=O)(Q₃₈)(Q₃₉),
dans lesquels Q₁ à Q₉, Q₁₁ à Q₁₉, Q₂₁ à Q₂₉, et Q₃₁ à Q₃₉ sont chacun indépendamment choisis parmi un atome d'hydrogène, un atome de deutérium, -F, -Cl, -Br, -I, un groupe hydroxy, un groupe cyano, un groupe nitro, un groupe amino, un groupe amidino, un groupe hydrazine, un groupe hydrazone, un groupe acide carboxylique ou un sel de celui-ci, un groupe acide sulfonique ou un sel de celui-ci, un groupe acide phosphorique ou un sel de celui-ci, un groupe alkyle en C₁ à C₆₀, un groupe alcényle en C₂ à C₆₀, un groupe alcynyle en C₂ à C₆₀, un groupe alcoxy en C₁ à C₆₀, un groupe cycloalkyle en C₃ à C₁₀, un groupe hétérocycloalkyle en C₁ à C₁₀, un groupe cycloalcényle en C₃ à C₁₀, un groupe hétérocycloalcényle en C₁ à C₁₀, un groupe aryle en C₆ à C₆₀, un groupe aryle en C₆ à C₆₀ substitué par au moins un groupe alkyle en C₁ à C₆₀ et un groupe aryle en C₆ à C₆₀, un groupe aryloxy en C₆ à C₆₀, un groupe arylthio en C₆ à C₆₀, un groupe hétéroaryle en C₁ à C₆₀, un groupe polycyclique condensé non aromatique monovalent, et un groupe hétéropolycyclique condensé non aromatique monovalent.

2. Composé organométallique selon la revendication 1, ladite liaison entre Y₁ et M et ladite liaison entre Y₂ et M étant une liaison covalente, et ladite liaison entre Y₃ et M et ladite liaison entre L₂ ligand et M étant une liaison de coordination, ou
ladite liaison entre Y₁ et M et ladite liaison entre L₂ ligand et M étant une liaison covalente, et ladite liaison entre Y₃ et M et ladite liaison entre Y₂ et M étant une liaison de coordination.

3. Composé organométallique selon la revendication 1 ou 2,
ledit groupement représenté par 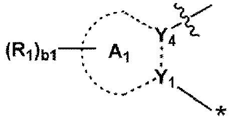 dans la formule 2 étant identique à un groupement représenté par 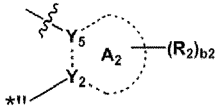 dans la formule 2 ou
dans lequel
ledit groupement représenté par 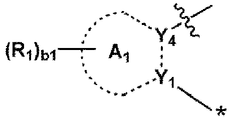 dans la formule 2 étant différent d'un groupement représenté par 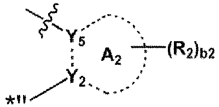 dans la formule 2.

4. Composé organométallique selon l'une quelconque des revendications 1 à 3,
un groupement représenté par 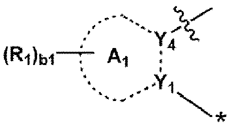 dans la formule 2 étant représenté par un groupement choisi parmi les formules 3-1 à 3-16 et 3-31 à 3-70,
un groupement représenté par 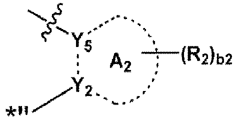 dans la formule 2 étant représenté par un groupement choisi parmi les formules 4-1 à 4-16 et 4-31 à 4-70, et
un groupement représenté par 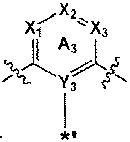 dans la formule 2 étant représenté par un groupement choisi parmi les formule 5-1 à 5-47, 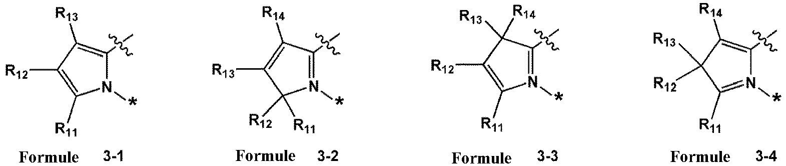 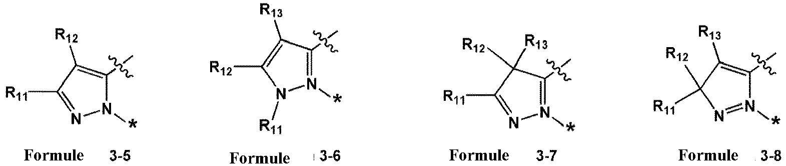 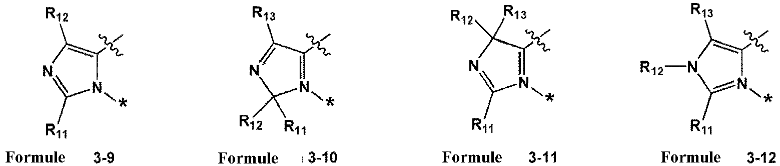 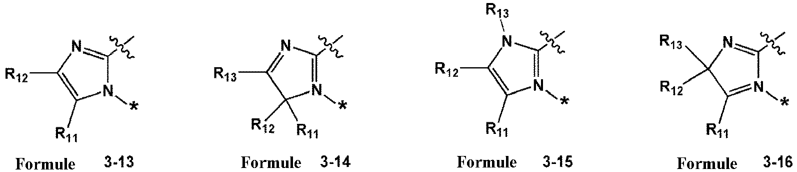 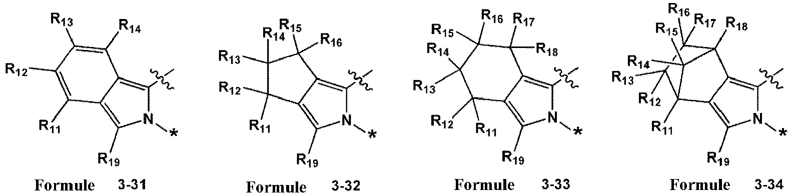 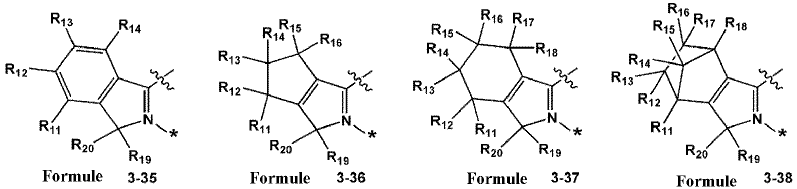 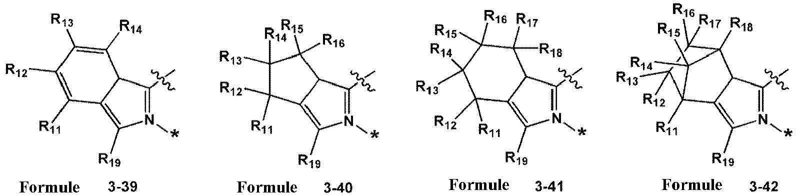 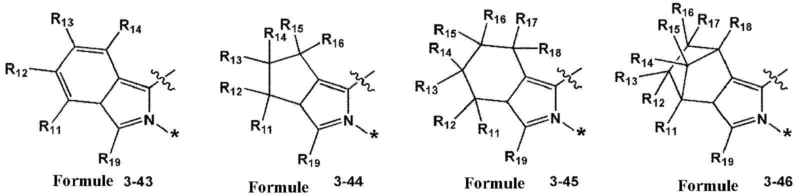 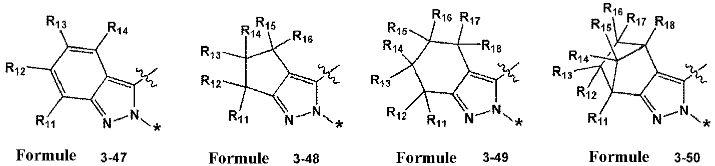 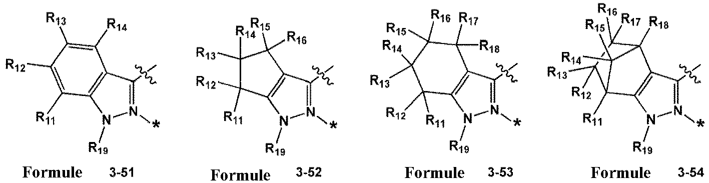 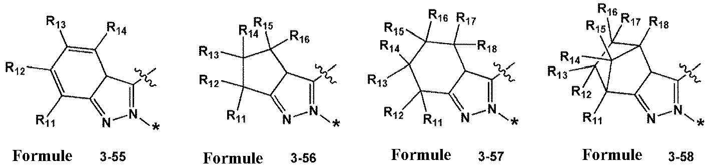 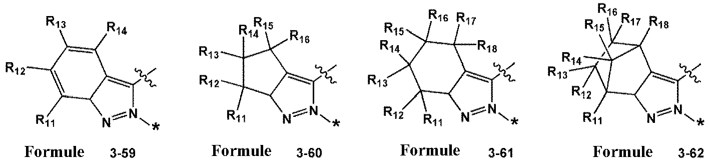 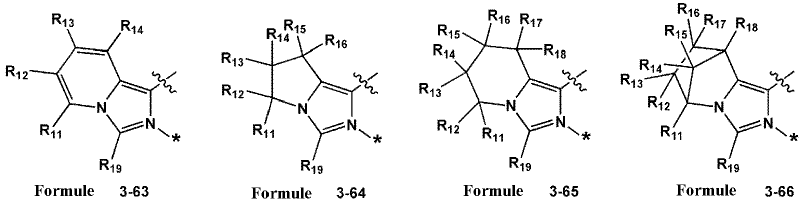 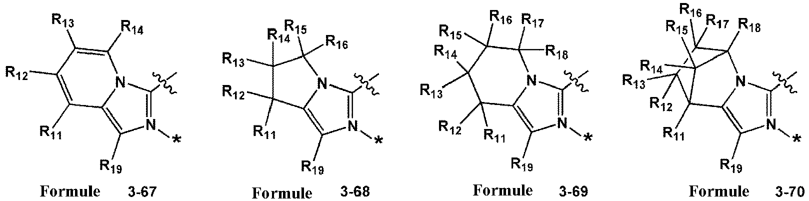 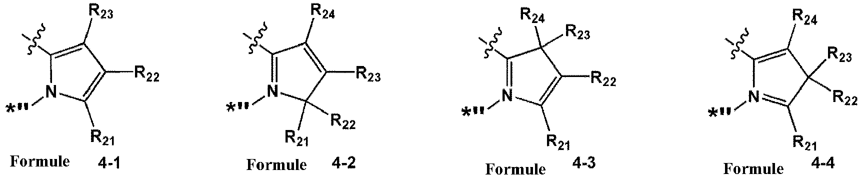 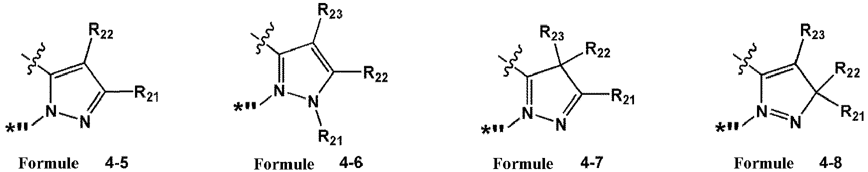 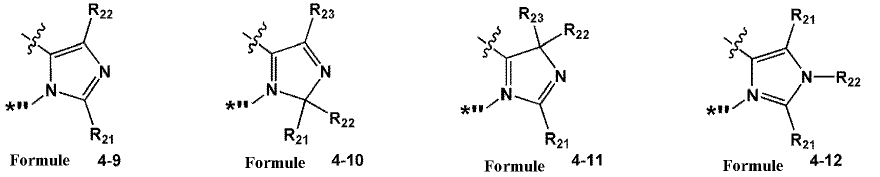 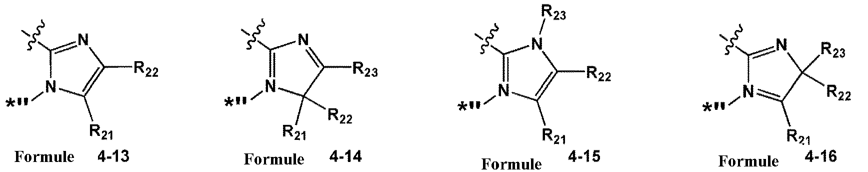 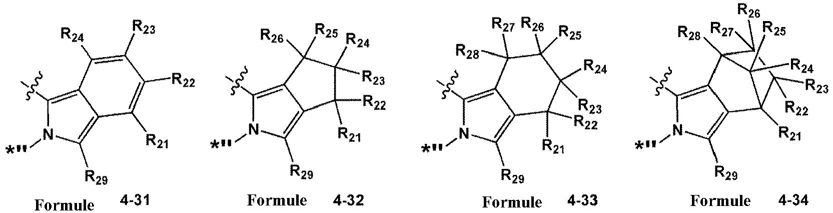 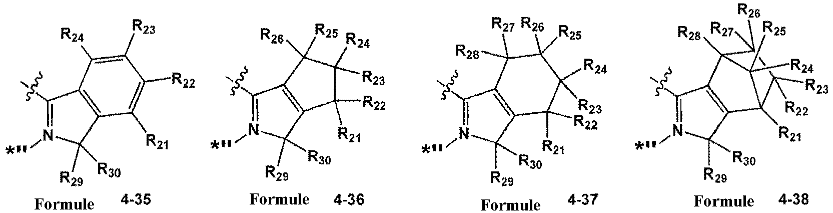 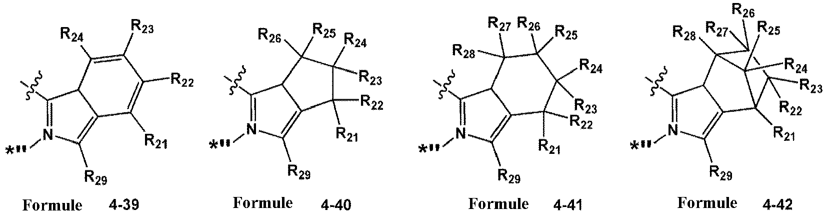 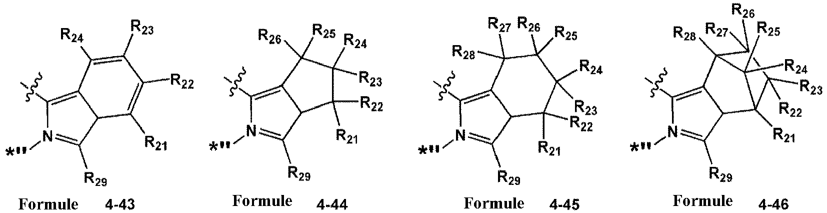 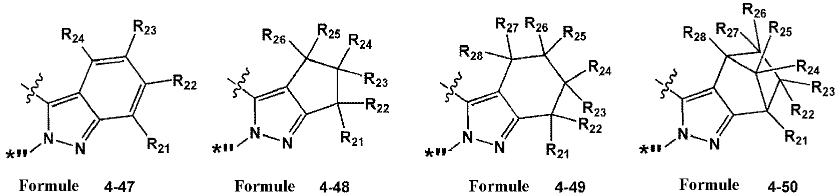 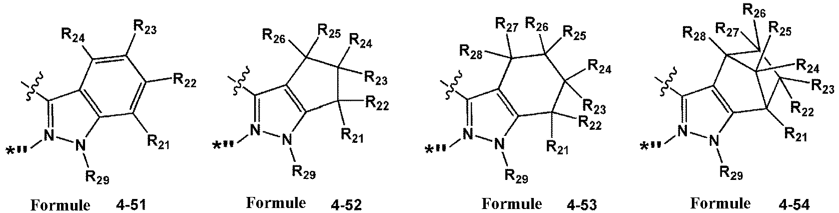 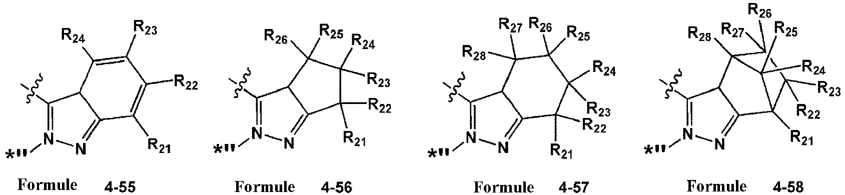 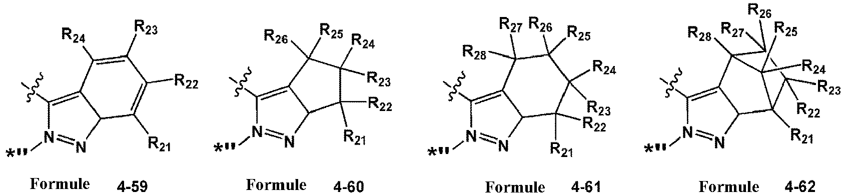 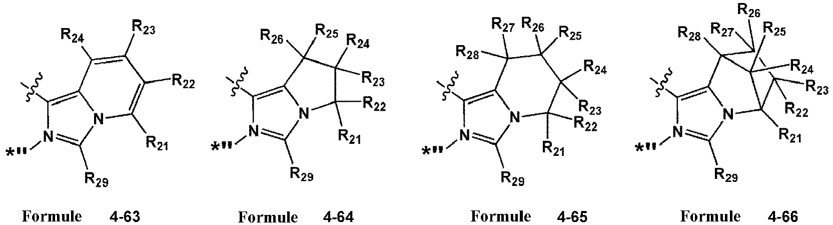 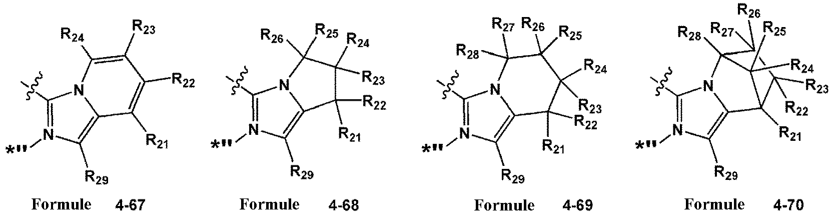 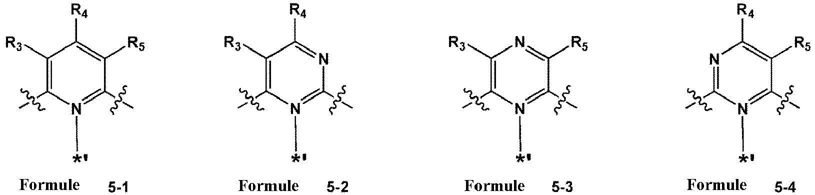 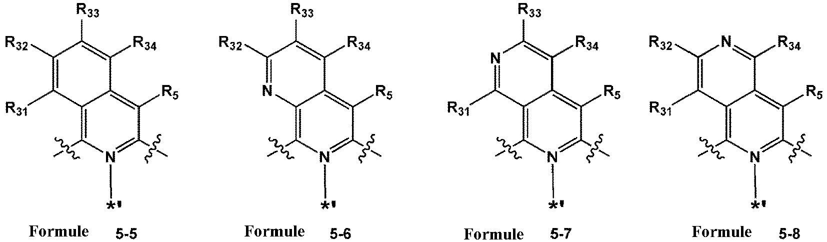 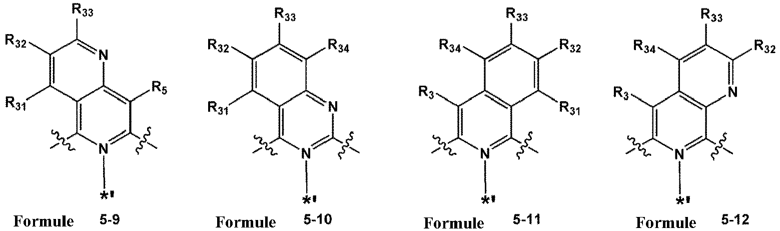 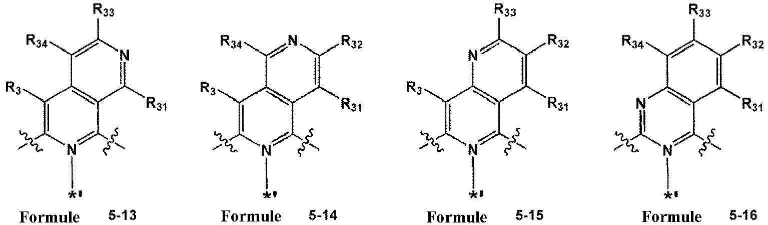 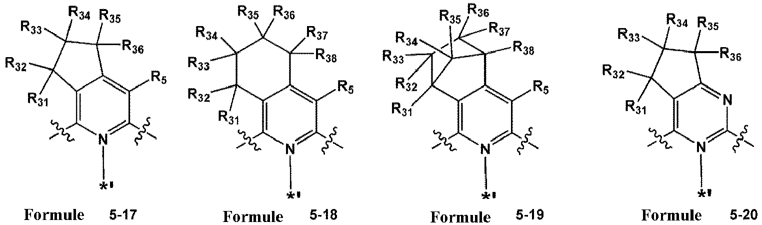 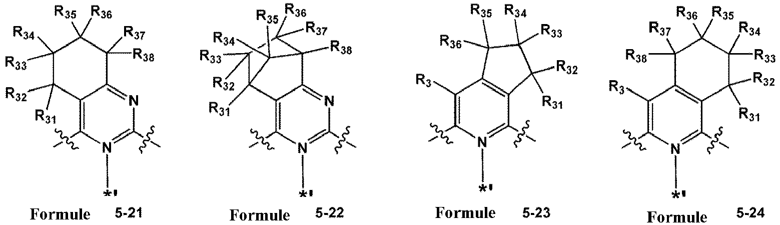 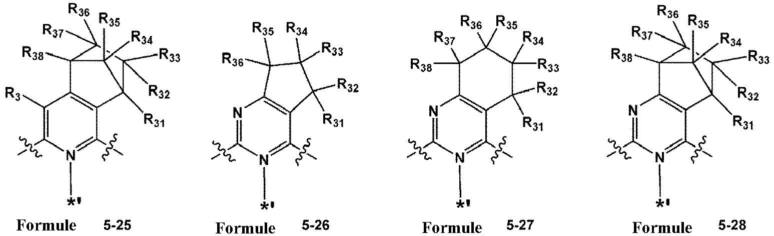 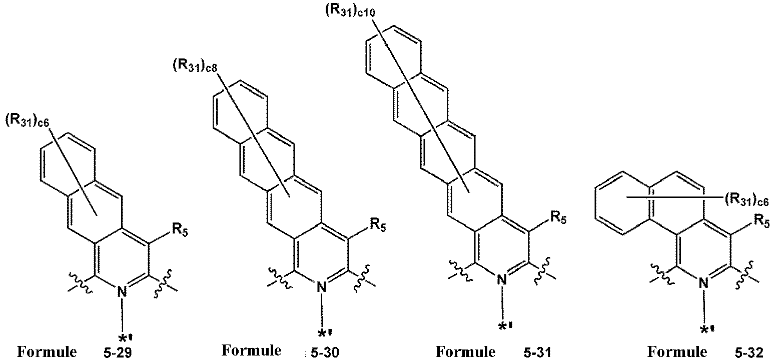 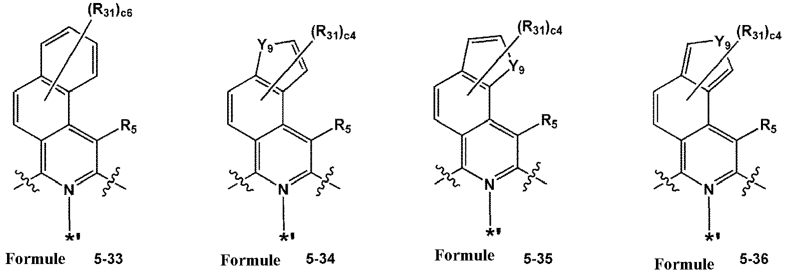 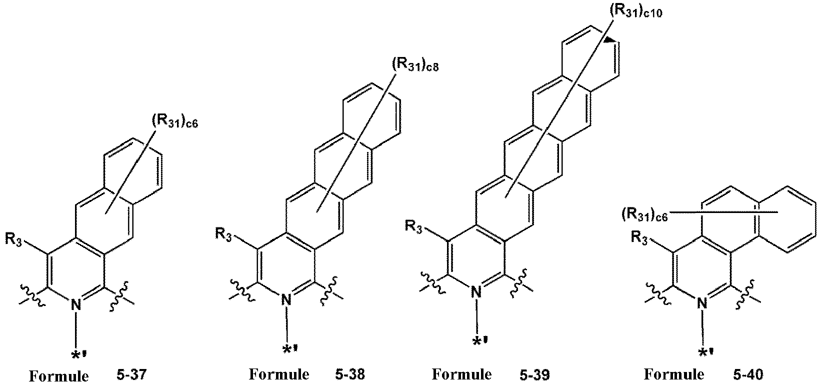 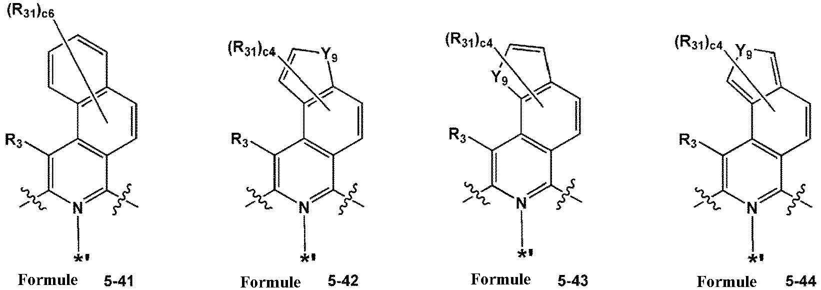 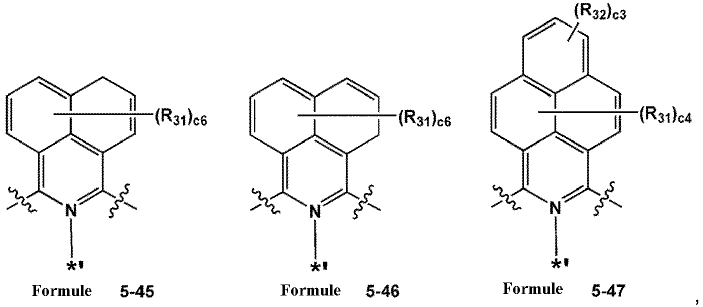
dans lesquelles, dans les formules 3-1 à 3-16, 3-31 à 3-70, 4-1 à 4-16, 4-31 à 4-70, et 5-1 à 5-47,
Y₉ représente un atomes O, un atome S, ou un groupe N(R₃₉),
R₃ à R₅ sont identiques à ceux décrits dans la revendication 1,
R₁₁ à R₂₀ sont chacun identiques à ceux décrits en relation avec R₁ dans la revendication 1,
R₂₁ à R₃₀ sont chacun identiques à ceux décrits en relation avec R₂ dans la revendication 1,
R₃₁ à R₃₉ sont chacun identiques à ceux décrits en relation avec R₃ dans la revendication 1,
c3 représente un entier choisi parmi 0 à 3,
c4 représente un entier choisi parmi 0 à 4,
c6 représente un entier choisi parmi 0 à 6,
c8 représente un entier choisi parmi 0 à 8,
c10 représente un entier choisi parmi 0 à 10, et
*, *', et *" indiquent chacun un site de liaison à M dans la formule 1, de préférence dans laquelle
R₃ à R₅ et R₁₁ à R₃₉ sont chacun indépendamment choisis parmi :
un atome d'hydrogène, un atome de deutérium, -F, -Cl, -Br, -I, un groupe hydroxy, un groupe cyano, un groupe nitro, un groupe amino, un groupe amidino, un groupe hydrazine, un groupe hydrazone, un groupe acide carboxylique ou un sel de celui-ci, un groupe acide sulfonique ou un sel de celui-ci un groupe acide phosphorique ou un sel de celui-ci, un groupe -SF₅, un groupe alkyle en C₁ à C₂₀, et un groupe alcoxy en C₁ à C₂₀ ;
un groupe alkyle en C₁ à C₂₀, et un groupe alcoxy en C₁ à C₂₀, chacun substitué par au moins un élément choisi parmi un atome de deutérium, -F, -Cl, -Br, -I, -CD₃, -CD₂H, -CDH₂, -CF₃, -CF₂H, -CFH₂, un groupe hydroxy, un groupe cyano, un groupe nitro, un groupe amino, un groupe amidino, un groupe hydrazine, un groupe hydrazone, un groupe acide carboxylique ou un sel de celui-ci, un groupe acide sulfonique ou un sel de celui-ci, un groupe acide phosphorique ou un sel de celui-ci, un groupe alkyle en C₁ à C₁₀, un groupe cyclopentyle, un groupe cyclohexyle, un groupe cycloheptyle, un groupe cyclooctyle, un groupe adamantanyle, un groupe norbornanyle, un groupe norbornényle, un groupe cyclopentényle, un groupe cyclohexényle, un groupe cycloheptényle, un groupe phényle, un groupe naphtyle, un groupe pyridinyle, et un groupe pyrimidinyle ;
un groupe cyclopentyle, un groupe cyclohexyle, un groupe cycloheptyle, un groupe cyclooctyle, un groupe adamantanyle, un groupe norbornanyle, un groupe norbornényle, un groupe cyclopentényle, un groupe cyclohexényle, un groupe cycloheptényle, un groupe phényle, un groupe naphtyle, un groupe fluorényle, un groupe phénanthrényle, un groupe anthracényle, un groupe fluoranthényle, un groupe triphénylényle, un groupe pyrényle, un groupe chrysényle, un groupe pyrrolyle, un groupe thiophényle, un groupe furanyle, un groupe imidazolyle, un groupe pyrazolyle, un groupe thiazolyle, un group isothiazolyle, un groupe oxazolyle, un groupe isoxazolyle, un groupe pyridinyle, un groupe pyrazinyle, un groupe pyrimidinyle, un groupe pyridazinyle, un groupe isoindolyle, un groupe indolyle, un groupe indazolyle, un groupe purinyle, un groupe quinolinyle, un groupe isoquinolinyle, un groupe benzoquinolinyle, un groupe quinoxalinyle, un groupe quinazolinyle, un groupe cinnolinyle, un groupe carbazolyle, un groupe phénanthrolinyle, un groupe benzimidazolyle, un groupe benzofuranyle, un groupe benzothiophényle, un groupe isobenzothiazolyle, un groupe benzoxazolyle, un groupe isobenzoxazolyle, un groupe triazolyle, un groupe tétrazolyle, un groupe oxadiazolyle, un groupe triazinyle, un groupe dibenzofuranyle, un groupe dibenzothiophényle, un groupe benzocarbazolyle, un groupe dibenzocarbazolyle, un groupe imidazopyridinyle, et un groupe imidazopyrimidinyle ;
un groupe cyclopentyle, un groupe cyclohexyle, un groupe cycloheptyle, un groupe cyclooctyle, un groupe adamantanyle, un groupe norbornanyle, un groupe norbornényle, un groupe cyclopentényle, un groupe cyclohexényle, un groupe cycloheptényle, un groupe phényle, un groupe naphtyle, un groupe fluorényle, un groupe phénanthrényle, un groupe anthracényle, un groupe fluoranthényle, un groupe triphénylényle, un groupe pyrényle, un groupe chrysényle, un groupe pyrrolyle, un groupe thiophényle, un groupe furanyle, un groupe imidazolyle, un groupe pyrazolyle, un groupe thiazolyle, un groupe isothiazolyle, un groupe oxazolyle, un groupe isoxazolyle, un groupe pyridinyle, un groupe pyrazinyle, un groupe pyrimidinyle, un groupe pyridazinyle, un groupe isoindolyle, un groupe indolyle, un groupe indazolyle, un groupe purinyle, un groupe quinolinyle, un groupe isoquinolinyle, un groupe benzoquinolinyle, un groupe quinoxalinyle, un groupe quinazolinyle, un groupe cinnolinyle, un groupe carbazolyle, un groupe phénanthrolinyle, un groupe benzimidazolyle, un groupe benzofuranyle, un groupe benzothiophényle, un groupe isobenzothiazolyle, un groupe benzoxazolyle, un groupe isobenzoxazolyle, un groupe triazolyle, un groupe tétrazolyle, un groupe oxadiazolyle, un groupe triazinyle, un groupe dibenzofuranyle, un groupe dibenzothiophényle, un groupe benzocarbazolyle, un groupe dibenzocarbazolyle, un groupe imidazopyridinyle, et un groupe imidazopyrimidinyle, chacun substitué par au moins un élément choisi parmi un atome de deutérium, -F, -Cl, -Br, -I, -CD₃, -CD₂H, -CDH₂, -CF₃, -CF₂H, -CFH₂, un groupe hydroxy, un groupe cyano, un groupe nitro, un groupe amino, un groupe amidino, un groupe hydrazine, un groupe hydrazone, un groupe acide carboxylique ou un sel de celui-ci, un groupe acide sulfonique ou un sel de celui-ci, un groupe acide phosphorique ou un sel de celui-ci, un groupe alkyle en C₁ à C₂₀, un groupe alcoxy en C₁ à C₂₀, un groupe cyclopentyle, un groupe cyclohexyle, un groupe cycloheptyle, un groupe cyclooctyle, un groupe adamantanyle, un groupe norbornanyle, un groupe norbornényle, un groupe cyclopentényle, un groupe cyclohexényle, un groupe cycloheptényle, un groupe phényle, un groupe naphtyle, un groupe fluorényle, un groupe phénanthrényle, un groupe anthracényle, un groupe fluoranthényle, un groupe triphénylényle, un groupe pyrényle, un groupe chrysényle, un groupe pyrrolyle, un groupe thiophényle, un groupe furanyle, un groupe imidazolyle, un groupe pyrazolyle, un groupe thiazolyle, un group isothiazolyle, un groupe oxazolyle, un groupe isoxazolyle, un groupe pyridinyle, un groupe pyrazinyle, un groupe pyrimidinyle, un groupe pyridazinyle, un groupe isoindolyle, un groupe indolyle, un groupe indazolyle, un groupe purinyle, un groupe quinolinyle, un groupe isoquinolinyle, un groupe benzoquinolinyle, un groupe quinoxalinyle, un groupe quinazolinyle, un groupe cinnolinyle, un groupe carbazolyle, un groupe phénanthrolinyle, un groupe benzimidazolyle, un groupe benzofuranyle, un groupe benzothiophényle, un groupe isobenzothiazolyle, un groupe benzoxazolyle, un groupe isobenzoxazolyle, un groupe triazolyle, un groupe tétrazolyle, un groupe oxadiazolyle, un groupe triazinyle, un groupe dibenzofuranyle, un groupe dibenzothiophényle, un groupe benzocarbazolyle, un groupe dibenzocarbazolyle, un groupe imidazopyridinyle, et un groupe imidazopyrimidinyle ; et
-N(Q₁)(Q₂), -Si(Q₃)(Q₄)(Q₅), -B(Q₆)(Q₇) et -P(=O)(Q₈)(Q₉),
dans lesquels Q₁ à Q₉ sont chacun indépendamment choisis parmi :
-CH₃, -CD₃, -CD₂H, -CDH₂, -CH₂CH₃, -CH₂CD₃, -CH₂CD₂H, -CH₂CDH₂, -CHDCH₃, -CHDCD₂H, -CHDCDH₂, -CHDCD₃, -CD₂CD₃, -CD₂CD₂H, et -CD₂CDH₂ ;
un groupe n-propyle, un groupe isopropyle, un groupe n-butyle, un groupe isobutyle, un groupe sec-butyle, un groupe tert-butyle, un groupe n-pentyle, un groupe isopentyle, un groupe sec-pentyle, un groupe tert-pentyle, un groupe phényle, et un groupe naphtyle ; et
un groupe n-propyle, un groupe isopropyle, un groupe n-butyle, un groupe isobutyle, un groupe sec-butyle, un groupe tert-butyle, un groupe n-pentyle, un groupe isopentyle, un groupe sec-pentyle, un groupe tert-pentyle, un groupe phényle, et un groupe naphtyle, chacun substitué par au moins un élément choisi parmi un atome de deutérium, un groupe alkyle en C₁ à C₁₀, et un groupe phényle, plus préférablement dans lequel
R₃ à R₅ et R₁₁ à R₃₉ sont chacun indépendamment choisis parmi :
un atome d'hydrogène, un atome de deutérium, -F, un groupe cyano, un groupe nitro, -SF₅, un groupe méthyle, un groupe éthyle, un groupe n-propyle, un groupe isopropyle, un groupe n-butyle, un groupe isobutyle, un groupe sec-butyle, un groupe tert-butyle, un groupe n-pentyle, un groupe isopentyle, un groupe sec-pentyle, un groupe tert-pentyle, un groupe n-hexyle, un groupe isohexyle, un groupe sec-hexyle, un groupe tert-hexyle, un groupe n-heptyle, un groupe isoheptyle, un groupe sec-heptyle, un groupe tert-heptyle, un groupe n-octyle, un groupe isooctyle, un groupe sec-octyle, un groupe tert-octyle, un groupe n-nonyle, un groupe isononyle, un groupe sec-nonyle, un groupe tert-nonyle, un groupe n-décyle, un groupe isodécyle, un groupe sec-décyle, un groupe tert-décyle, un groupe méthoxy, un groupe éthoxy, un groupe propoxy, un groupe butoxy, un groupe pentoxy, un groupe cyclopentyle, un groupe cyclohexyle, un groupe cycloheptyle, un groupe cyclooctyle, un groupe adamantanyle, un groupe norbornanyle, un groupe norbornényle, un groupe cyclopentényle, un groupe cyclohexényle, un groupe cycloheptényle, un groupe phényle, un groupe naphtyle, un groupe pyridinyle, un groupe pyrimidinyle, un groupe dibenzofuranyle, et un groupe dibenzothiophényle ;
un groupe méthyle, un groupe éthyle, un groupe n-propyle, un groupe isopropyle, un groupe n-butyle, un groupe isobutyle, un groupe sec-butyle, un groupe tert-butyle, un groupe n-pentyle, un groupe isopentyle, un groupe sec-pentyle, un groupe tert-pentyle, un groupe n-hexyle, un groupe isohexyle, un groupe sec-hexyle, un groupe tert-hexyle, un groupe n-heptyle, un groupe iso-heptyle, un groupe sec-heptyle, un groupe tert-heptyle, un groupe n-octyle, un groupe isooctyle, un groupe sec-octyle, un groupe tert-octyle, un groupe n-nonyle, un groupe isononyle, un groupe sec-nonyle, un groupe tert-nonyle, un groupe n-décyle, un groupe isodécyle, un groupe sec-décyle, un groupe tert-décyle, un groupe méthoxy, un groupe éthoxy, un groupe propoxy, un groupe butoxy, un groupe pentoxy, un groupe cyclopentyle, un groupe cyclohexyle, un groupe cycloheptyle, un groupe cyclooctyle, un groupe adamantanyle, un groupe norbornanyle, un groupe norbornényle, un groupe cyclopentényle, un groupe cyclohexényle, un groupe cycloheptényle, un groupe phényle, un groupe naphtyle, un groupe pyridinyle, un groupe pyrimidinyle, un groupe dibenzofuranyle, et un groupe dibenzothiophényle, chacun substitué par au moins un élément choisi parmi un atome de deutérium, -F, -CD₃, -CD₂H, -CDH₂, -CF₃, -CF₂H, -CFH₂, un groupe cyano, un groupe nitro, un groupe alkyle en C₁ à C₁₀, un groupe alcoxy en C₁ à C₁₀, un groupe cyclopentyle, un groupe cyclohexyle, un groupe cycloheptyle, un groupe cyclooctyle, un groupe adamantanyle, un groupe norbornanyle, un groupe norbornényle, un groupe cyclopentényle, un groupe cyclohexényle, un groupe cycloheptényle, un groupe phényle, un groupe naphtyle, un groupe pyridinyle, un groupe pyrimidinyle, un groupe dibenzofuranyle, et un groupe dibenzothiophényle ; et
-N(Q₁)(Q₂), -Si(Q₃)(Q₄)(Q₅), -B(Q₆)(Q₇), et -P(=O)(Q₈)(Q₉),
dans lesquels Q₁ à Q₉ sont chacun indépendamment choisis parmi :
-CH₃, -CD₃, -CD₂H, -CDH₂, -CH₂CH₃, -CH₂CD₃, -CH₂CD₂H, -CH₂CDH₂, -CHDCH₃, -CHDCD₂H, -CHDCDH₂, -CHDCD₃, -CD₂CD₃, -CD₂CD₂H, et -CD₂CDH₂ ;
un groupe n-propyle, un groupe isopropyle, un groupe n-butyle, un groupe isobutyle, un groupe sec-butyle, un groupe tert-butyle, un groupe n-pentyle, un groupe isopentyle, un groupe sec-pentyle, un groupe tert-pentyle, un groupe phényle, et un groupe naphtyle ; et
un groupe n-propyle, un groupe isopropyle, un groupe n-butyle, un groupe isobutyle, un groupe sec-butyle, un groupe tert-butyle, un groupe n-pentyle, un groupe isopentyle, un groupe sec-pentyle, un groupe tert-pentyle, un groupe phényle, et un groupe naphtyle, chacun substitué par au moins un élément choisi parmi un atome de deutérium, un groupe alkyle en C₁ à C₁₀, et un groupe phényle, et en particulier dans lequel
R₃ à R₅ et R₁₁ à R₃₉ sont chacun indépendamment choisis parmi un atome d'hydrogène, un atome de deutérium, -F, un groupe cyano, un groupe nitro, -SF₅, -CH₃, -CD₃, -CD₂H, -CDH₂, -CF₃, -CF₂H, -CFH₂, un groupe représenté par l'une quelconque des formules 9-1 à 9-19, un groupe représenté par l'une quelconque des formules 10-1 à 10-38, et -Si(Q₃)(Q₄)(Q₅) : 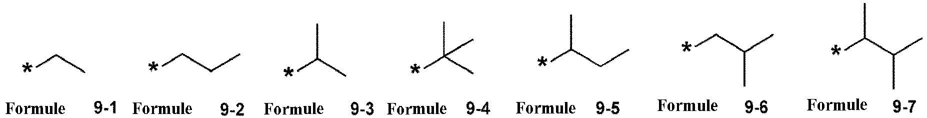 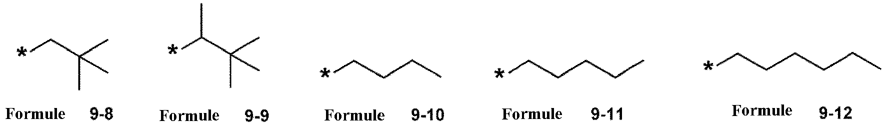 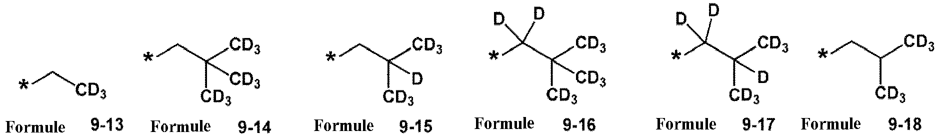 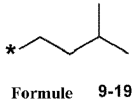 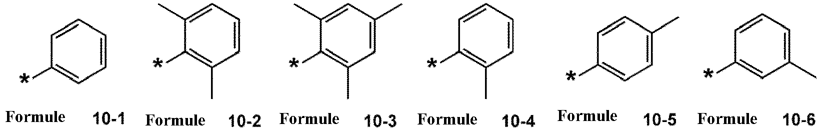 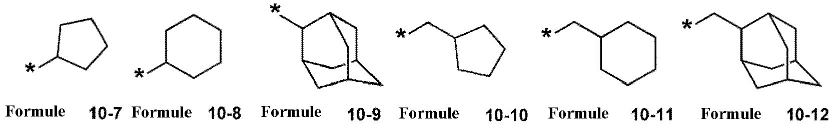 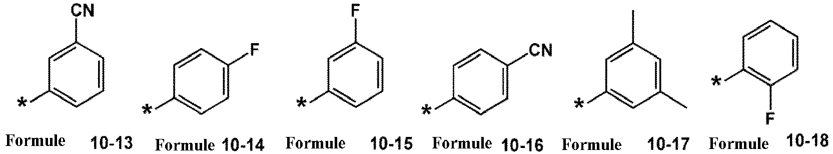 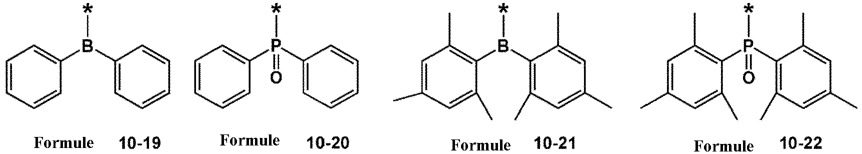 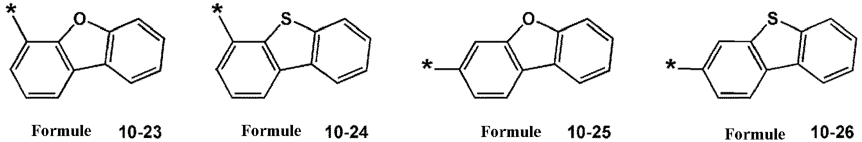 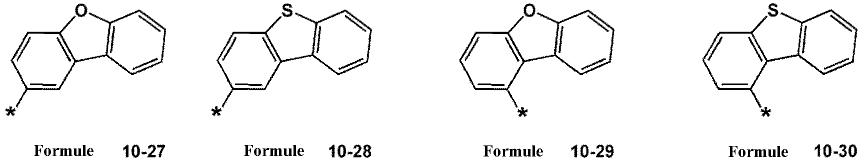 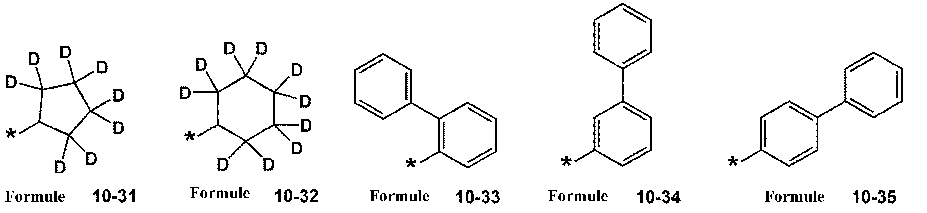 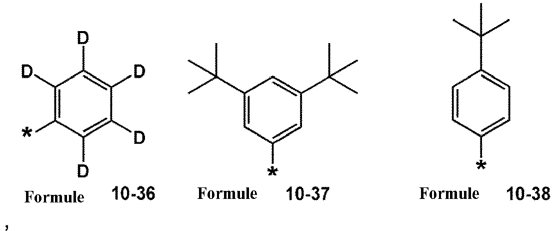 dans lesquelles * dans les formules 9-1 à 9-19 et 10-1 à 10-38 indique un site de liaison à un atome voisin.

5. Composé organométallique selon la revendication 4,
un groupement représenté par 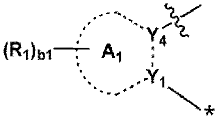 dans la formule 2 étant représenté par un groupement choisi parmi les formules 3-5 à 3-8, et 3-47 à 3-62,
un groupement représenté par 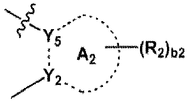 dans la formule 2 étant représenté par un groupement choisi parmi les formules 4-5 à 4-8, et 4-47 à 4-62.

6. Composé organométallique selon la revendication 4,
un groupement représenté par 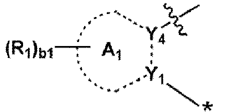 dans la formule 2 étant représenté par un groupement choisi parmi les formules 3-1,3-5, 3-9, 3-13, 3-31 à 3-34, et 3-47 à 3-50, et un groupement représenté par 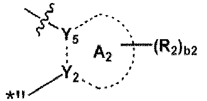 dans la formule 2 étant représenté par
un groupement choisi parmi les formules 4-1,4-5, 4-9, 4-13, 4-31 à 4-34, et 4-47 à 4-50.

7. Composé organométallique selon la revendication 4,
un groupement représenté par 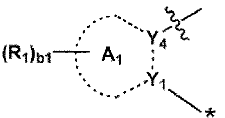 dans la formule 2 étant représenté par un groupement choisi parmi les formules 3-1,3-5, 3-9, 3-13, 3-31 à 3-34, et 3-47 à 3-50, et un groupement représenté par 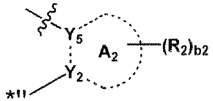 dans la formule 2 étant représenté par un groupement choisi parmi les formules 4-2 to 4-4, 4-6 à 4-8, 4-10 à 4-12, 4-14 à 4-16, 4-35 à 4-46, et 4-51 à 4-70.

8. Composé organométallique selon l'une quelconque des revendications 4 à 7,
un groupement représenté par 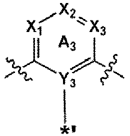 dans la formule 2 étant représenté par un groupement choisi parmi les formules 5-1 à 5-28, 5-29, et 5-45.

9. Composé organométallique selon l'une quelconque des revendications 1 à 8, L₁ dans la formule 1 étant choisi parmi les ligands représentés par les formules 2A-1 à 2E-1 et 2A-2 à 2E-2 : 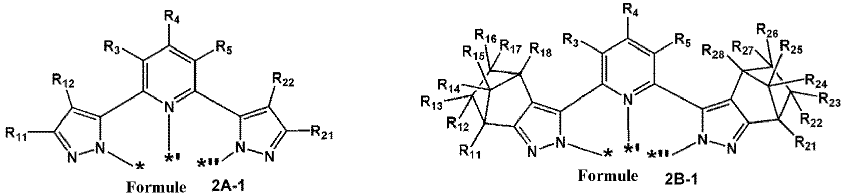 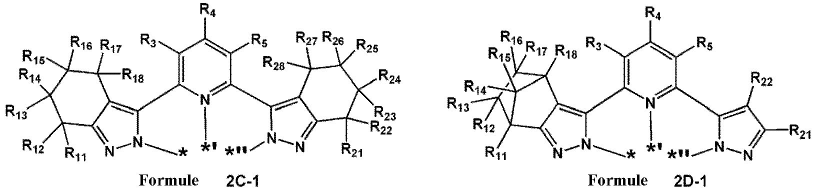 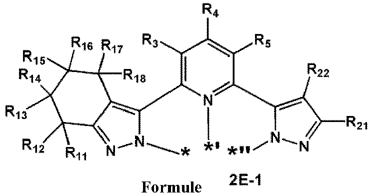 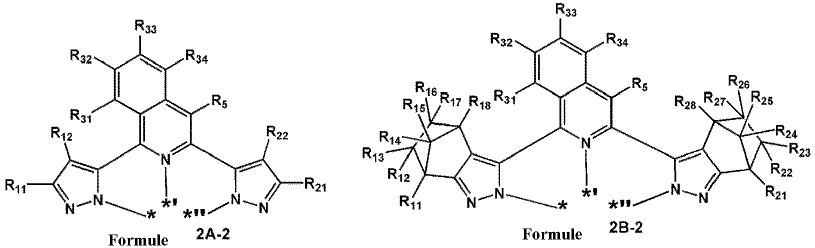 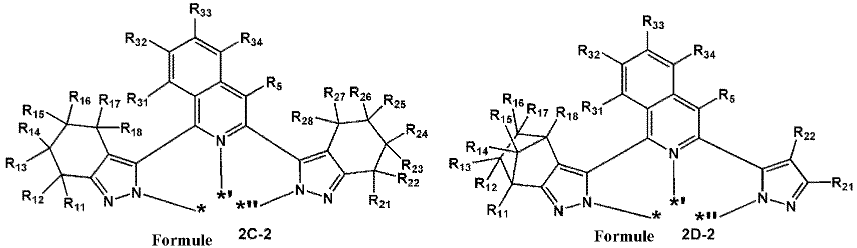 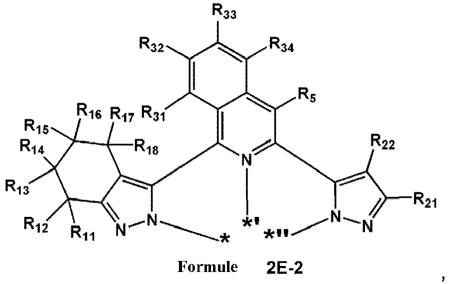
dans lesquelles, dans les formules 2A-1 à 2E-1 et 2A-2 à 2E-2,
R₃ à R₅, R₁₁ à R₁₈, R₂₁ à R₂₈, et R₃₁ à R₃₄ sont chacun indépendamment choisis parmi un atome d'hydrogène, un atome de deutérium, -F, un groupe cyano, un groupe nitro, -SF₅, -CH₃, -CD₃, -CD₂H, -CDH₂, -CF₃, -CF₂H, -CFH₂, un groupe représenté par l'une quelconque des formules 9-1 à 9-19, un groupe représenté par l'une quelconque des formules 10-1 à 10-38, et -Si(Q₃)(Q₄)(Q₅) : 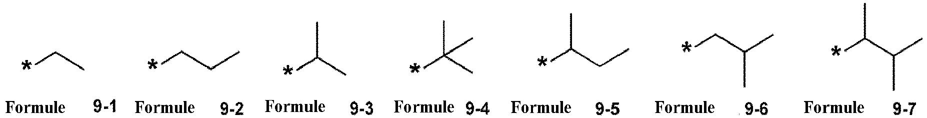 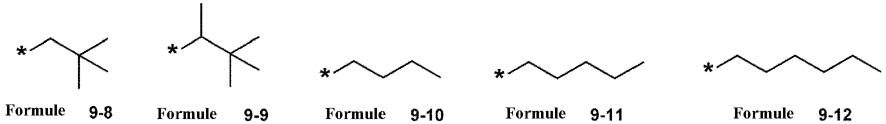 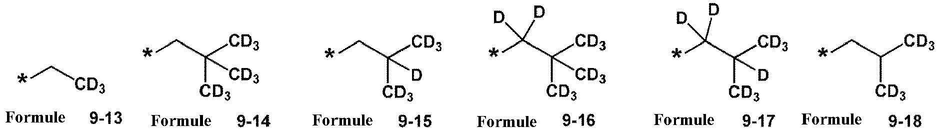 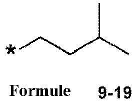 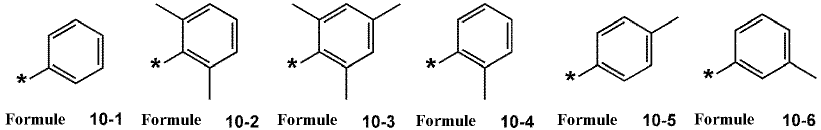 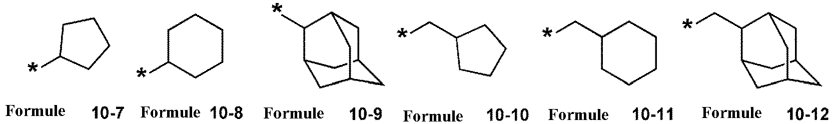 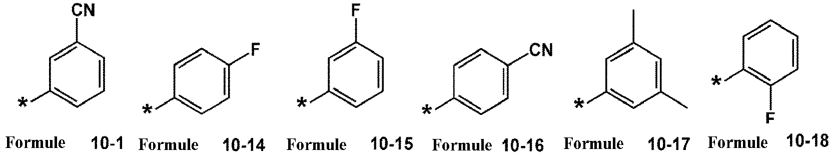 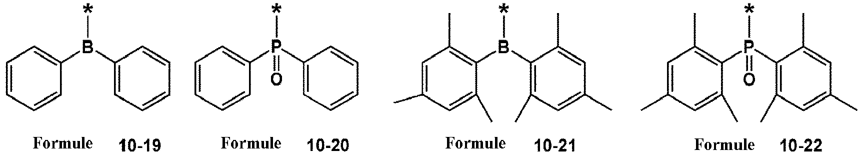 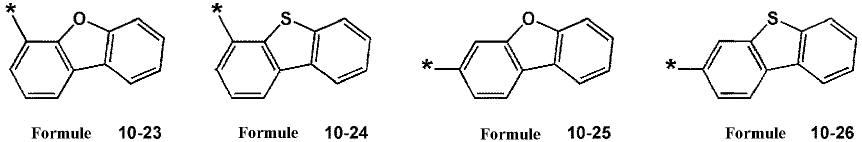 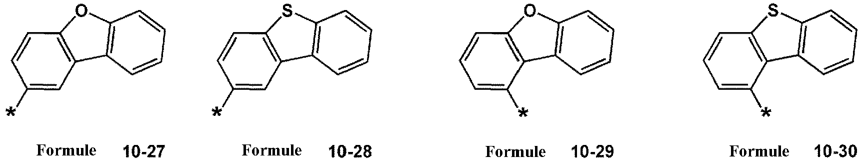 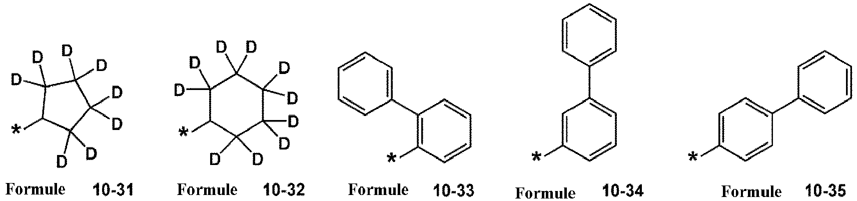 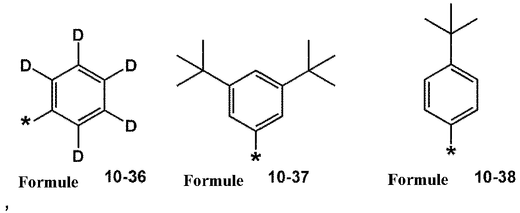
dans lesquelles, * dans les formules 9-1 à 9-19 et 10-1 à 10-38 indique un site de liaison à un atome voisin.

10. Composé organométallique selon l'une quelconque des revendications 1 à 9, L₂ dans la formule 1 étant choisi parmi les ligands représentés par la formule 6-1 :
***(̵T₃)ₐ₃-R₆₁** **Formule 6-1**,
dans laquelle, dans la formule 6-1,
T₃ est choisi parmi une liaison simple, *-O-*', *-S-*', *-C(R₆₂)(R₆₃)-*', *-C(R₆₂)=*', *=C(R₆₂₎-*', *-C(R₆₂)=C(R₆₃)-*', *-C(=O)-*', *-C(=S)-*', *-C≡C-*', et *-N(R₆₂)-*',
a3 représente un entier choisi parmi 1 à 5,
R₆₁ est choisi parmi un atome d'hydrogène, un atome de deutérium, -F, -Cl, -Br, -I, -SF₅, un groupe hydroxy, un groupe cyano, un groupe nitro, un groupe amino, un groupe amidino, un groupe hydrazine, un groupe hydrazone, un groupe acide carboxylique ou un sel de celui-ci, un groupe acide sulfonique ou un sel de celui-ci, un groupe acide phosphorique ou un sel de celui-ci, un groupe alkyle en C₁ à C₆₀ substitué ou non substitué, un groupe alcényle en C₂ à C₆₀ substitué ou non substitué, un groupe alcynyle en C₂ à C₆₀ substitué ou non substitué, un groupe alcoxy en C₁ à C₆₀ substitué ou non substitué, un groupe cycloalkyle en C₃ à C₁₀ substitué ou non substitué, un groupe hétérocycloalkyle en C₁ à C₁₀ substitué ou non substitué, un groupe cycloalcényle en C₃ à C₁₀ substitué ou non substitué, un groupe hétérocycloalcényle en C₁ à C₁₀ substitué ou non substitué, un groupe aryle en C₆ à C₆₀ substitué ou non substitué, un groupe aryloxy en C₆ à C₆₀ substitué ou non substitué, un groupe arylthio en C₆ à C₆₀ substitué ou non substitué, un groupe hétéroaryle en C₁ à C₆₀ substitué ou non substitué, un groupe polycyclique condensé non aromatique monovalent substitué ou non substitué, un groupe hétéropolycyclique condensé non aromatique monovalent substitué ou non substitué, -N(Q₁)(Q₂), -Si(Q₃)(Q₄)(Q₅), -B(Q₆)(Q₇), -P(=O)(Q₈)(Q₉), et -P(Q₄₁)(Q₄₂)(Q₄₃),
dans lesquels Q₁ à Q₉ et Q₄₁ à Q₄₃ sont chacun indépendamment choisis parmi :
-CH₃, -CD₃, -CD₂H, -CDH₂, -CH₂CH₃, -CH₂CD₃, -CH₂CD₂H, -CH₂CDH₂, -CHDCH₃, -CHDCD₂H, -CHDCDH₂, -CHDCD₃, -CD₂CD₃, -CD₂CD₂H, et -CD₂CDH₂ ;
un groupe n-propyle, un groupe isopropyle, un groupe n-butyle, un groupe isobutyle, un groupe sec-butyle, un groupe tert-butyle, un groupe n-pentyle, un groupe isopentyle, un groupe sec-pentyle, un groupe tert-pentyle, un groupe phényle, et un groupe naphtyle ; et
un groupe n-propyle, un groupe isopropyle, un groupe n-butyle, un groupe isobutyle, un groupe sec-butyle, un groupe tert-butyle, un groupe n-pentyle, un groupe isopentyle, un groupe sec-pentyle, un groupe tert-pentyle, un groupe phényle, et un groupe naphtyle, chacun substitué par au moins un élément choisi parmi un atome de deutérium, un groupe alkyle en C₁ à C₁₀, et un groupe phényle ;
R₆₂ et R₆₃ sont chacun indépendamment choisis parmi :
un atome d'hydrogène, un atome de deutérium, -F, un groupe cyano, un groupe nitro, -SF₅, un groupe méthyle, un groupe éthyle, un groupe n-propyle, un groupe isopropyle, un groupe n-butyle, un groupe isobutyle, un groupe sec-butyle, un groupe tert-butyle, un groupe n-pentyle, un groupe isopentyle, un groupe sec-pentyle, un groupe tert-pentyle, un groupe n-hexyle, un groupe isohexyle, un groupe sec-hexyle, un groupe tert-hexyle, un groupe n-heptyle, un groupe isoheptyle, un groupe sec-heptyle, un groupe tert-heptyle, un groupe n-octyle, un groupe isooctyle, un groupe sec-octyle, un groupe tert-octyle, un groupe n-nonyle, un groupe isononyle, un groupe sec-nonyle, un groupe tert-nonyle, un groupe n-décyle, un groupe isodécyle, un groupe sec-décyle, un groupe tert-décyle, un groupe méthoxy, un groupe éthoxy, un groupe propoxy, un groupe butoxy, un groupe pentoxy, un groupe cyclopentyle, un groupe cyclohexyle, un groupe cycloheptyle, un groupe cyclooctyle, un groupe adamantanyle, un groupe norbornanyle, un groupe norbornényle, un groupe cyclopentényle, un groupe cyclohexényle, un groupe cycloheptényle, un groupe phényle, un groupe naphtyle, un groupe pyridinyle, un groupe pyrimidinyle, un groupe dibenzofuranyle, et un groupe dibenzothiophényle ;
un groupe méthyle, un groupe éthyle, un groupe n-propyle, un groupe isopropyle, un groupe n-butyle, un groupe isobutyle, un groupe sec-butyle, un groupe tert-butyle, un groupe n-pentyle, un groupe isopentyle, un groupe sec-pentyle, un groupe tert-pentyle, un groupe n-hexyle, un groupe isohexyle, un groupe sec-hexyle, un groupe tert-hexyle, un groupe n-heptyle, un groupe isoheptyle, un groupe sec-heptyle, un groupe tert-heptyle, un groupe n-octyle, un groupe isooctyle, un groupe sec-octyle, un groupe tert-octyle, un groupe n-nonyle, un groupe isononyle, un groupe sec-nonyle, un groupe tert-nonyle, un groupe n-décyle, un groupe isodécyle, un groupe sec-décyle, un groupe tert-décyle, un groupe méthoxy, un groupe éthoxy, un groupe propoxy, un groupe butoxy, un groupe pentoxy, un groupe cyclopentyle, un groupe cyclohexyle, un groupe cycloheptyle, un groupe cyclooctyle, un groupe adamantanyle, un groupe norbornanyle, un groupe norbornényle, un groupe cyclopentényle, un groupe cyclohexényle, un groupe cycloheptényle, un groupe phényle, un groupe naphtyle, un groupe pyridinyle, un groupe pyrimidinyle, un groupe dibenzofuranyle, et un groupe dibenzothiophényle, chacun substitué par au moins un élément choisi parmi un atome de deutérium, -F, -CD₃, -CD₂H, -CDH₂, -CF₃, -CF₂H, -CFH₂, un groupe cyano, un groupe nitro, un groupe alkyle en C₁ à C₁₀, un groupe alcoxy en C₁ à C₁₀, un groupe cyclopentyle, un groupe cyclohexyle, un groupe cycloheptyle, un groupe cyclooctyle, un groupe adamantanyle, un groupe norbornanyle, un groupe norbornényle, un groupe cyclopentényle, un groupe cyclohexényle, un groupe cycloheptényle, un groupe phényle, un groupe naphtyle, un groupe pyridinyle, un groupe pyrimidinyle, un groupe dibenzofuranyle, et un groupe dibenzothiophényle ; et
R₆₂ et R₆₃ sont éventuellement reliés l'un à l'autre pour former un groupe carbocyclique en C₅ à C₃₀ substitué ou non substitué ou un groupe hétérocyclique en C₂ à C₃₀ substitué ou non substitué, et
* indique un site de liaison à M dans la formule 1.

11. Composé organique selon l'une quelconque des revendications 1 à 10, L₂ dans la formule 1 étant choisi parmi les ligands représentés par les formules 12-1 à 12-5 : 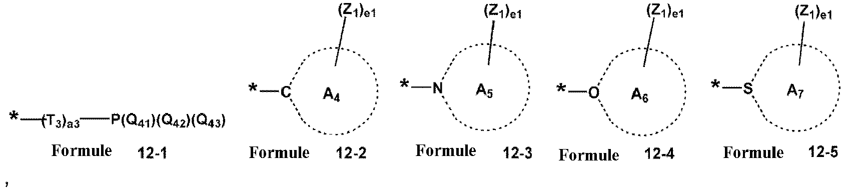
dans lesquelles, dans les formules 12-1 à 12-5,
T₃ est choisi parmi une liaison simple, *-O-*', *-S-*', *-C(R₆₂)(R₆₃)-*', *-C(R₆₂)=*', *=C(R₆₂)-*', *-C(R₆₂)=C(R₆₃)-*', *-C(=O)-*', *-C(=S)-*', *-C≡C-*', et *-N(R₆₂)-*',
R₆₂ et R₆₃ sont chacun indépendamment choisis parmi un atome d'hydrogène, un atome de deutérium, -F, -Cl, -Br, -I, -SF₅, un groupe hydroxy, un groupe cyano, un groupe nitro, un groupe amino, un groupe amidino, un groupe hydrazine, un groupe hydrazone, un groupe acide carboxylique ou un sel de celui-ci, un groupe acide sulfonique ou un sel de celui-ci, un groupe acide phosphorique ou un sel de celui-ci, un groupe alkyle en C₁ à C₂₀, un groupe alcoxy en C₁ à C₂₀, un groupe phényle, et un groupe naphtyle,
a3 représente un entier choisi parmi 1 à 5,
le noyau A4 est choisi parmi un noyau cyclopentène, un noyau cyclohexène, un noyau cycloheptène, un noyau benzène, un noyau indène, un noyau naphtalène, un noyau azulène, un noyau heptalène, un noyau indacène, un noyau acénaphtylène, un noyau fluorène, un noyau spiro-bifluorène, un noyau benzofluorène, un noyau dibenzofluorène, un noyau phénalène, un noyau phénanthrène, un noyau anthracène, un noyau fluoranthène, un noyau triphénylène, un noyau pyrène, un noyau chrysène, un noyau naphtacène, un noyau picène, un noyau pérylène, un noyau pentacène, un noyau hexacène, un noyau rubicène, un noyau coronène, un noyau ovalène, un noyau pyrrole, un noyau thiophène, un noyau furane, un noyau imidazole, un noyau pyrazole, un noyau thiazole, un noyau isothiazole, un noyau oxazole, un noyau isoxazole, un noyau pyridine, un noyau pyrazine, un noyau pyrimidine, un noyau pyridazine, un noyau isoindole, un noyau indole, un noyau indazole, un noyau purine, un noyau quinoline, un noyau isoquinoline, un noyau benzoquinoline, un noyau quinoxaline, un noyau quinazoline, un noyau cinnoline, un noyau naphtyridine, un noyau carbazole, un noyau phénanthroline, un noyau benzimidazole, un noyau benzofurane, un noyau benzothiophène, un noyau benzothiazole, un noyau isobenzothiazole, un noyau benzoxazole, un noyau isobenzoxazole, un noyau triazole, a tétrazole ring, un noyau oxadiazole, un noyau thiadiazol, un noyau triazine, un noyau dibenzofurane, un noyau dibenzothiophène, un noyau benzocarbazole, un noyau dibenzocarbazole, un noyau imidazopyridine, et un noyau imidazopyrimidine,
le noyau A₅ est choisi parmi un noyau pyrrole, un noyau imidazole, un noyau pyrazole, un noyau thiazole, un noyau isothiazole, un noyau oxazole, un noyau isoxazole, un noyau pyridine, un noyau pyrazine, un noyau pyrimidine, un noyau pyridazine, un noyau isoindole, un noyau indole, un noyau indazole, un noyau purine, un noyau quinoline, un noyau isoquinoline, un noyau benzoquinoline, un noyau quinoxaline, un noyau quinazoline, un noyau cinnoline, un noyau naphtyridine, un noyau carbazole, un noyau phénanthroline, un noyau benzimidazole, un noyau benzofurane, un noyau benzothiazole, un noyau isobenzothiazole, un noyau benzoxazole, un noyau isobenzoxazole, un noyau triazole, un noyau tétrazole, un noyau oxadiazole, un noyau thiadiazol, un noyau triazine, un noyau benzocarbazole, un noyau dibenzocarbazole, un noyau imidazopyridine, et un noyau imidazopyrimidine,
le noyau A₆ est choisi parmi un noyau furane, un noyau oxazole, un noyau isoxazole, un noyau benzofurane, un noyau benzoxazole, un noyau isobenzoxazole, un noyau oxadiazole, et un noyau dibenzofurane,
le noyau A₇ est choisi parmi un noyau thiophène, un noyau thiazole, un noyau isothiazole, un noyau benzothiophène, un noyau benzothiazole, un noyau isobenzothiazole, un noyau thiadiazol, et un noyau dibenzothiophène,
Z₁ est chacun indépendamment choisi parmi :
un atome d'hydrogène, un atome de deutérium, -F, un groupe cyano, un groupe nitro, -SF₅, un groupe méthyle, un groupe éthyle, un groupe n-propyle, un groupe isopropyle, un groupe n-butyle, un groupe isobutyle, un groupe sec-butyle, un groupe tert-butyle, un groupe n-pentyle, un groupe isopentyle, un groupe sec-pentyle, un groupe tert-pentyle, un groupe n-hexyle, un groupe isohexyle, un groupe sec-hexyle, un groupe tert-hexyle, un groupe n-heptyle, un groupe isoheptyle, un groupe sec-heptyle, un groupe tert-heptyle, un groupe n-octyle, un groupe isooctyle, un groupe sec-octyle, un groupe tert-octyle, un groupe n-nonyle, un groupe isononyle, un groupe sec-nonyle, un groupe tert-nonyle, un groupe n-décyle, un groupe isodécyle, un groupe sec-décyle, un groupe tert-décyle, un groupe méthoxy, un groupe éthoxy, un groupe propoxy, un groupe butoxy, un groupe pentoxy, un groupe cyclopentyle, un groupe cyclohexyle, un groupe cycloheptyle, un groupe cyclooctyle, un groupe adamantanyle, un groupe norbornanyle, un groupe norbornényle, un groupe cyclopentényle, un groupe cyclohexényle, un groupe cycloheptényle, un groupe phényle, un groupe naphtyle, un groupe pyridinyle, un groupe pyrimidinyle, un groupe dibenzofuranyle, et un groupe dibenzothiophényle ;
un groupe méthyle, un groupe éthyle, un groupe n-propyle, un groupe isopropyle, un groupe n-butyle, un groupe isobutyle, un groupe sec-butyle, un groupe tert-butyle, un groupe n-pentyle, un groupe isopentyle, un groupe sec-pentyle, un groupe tert-pentyle, un groupe n-hexyle, un groupe isohexyle, un groupe sec-hexyle, un groupe tert-hexyle, un groupe n-heptyle, un groupe isoheptyle, un groupe sec-heptyle, un groupe tert-heptyle, un groupe n-octyle, un groupe isooctyle, un groupe sec-octyle, un groupe tert-octyle, un groupe n-nonyle, un groupe isononyle, un groupe sec-nonyle, un groupe tert-nonyle, un groupe n-décyle, un groupe isodécyle, un groupe sec-décyle, un groupe tert-décyle, un groupe méthoxy, un groupe éthoxy, un groupe propoxy, un groupe butoxy, un groupe pentoxy, un groupe cyclopentyle, un groupe cyclohexyle, un groupe cycloheptyle, un groupe cyclooctyle, un groupe adamantanyle, un groupe norbornanyle, un groupe norbornényle, un groupe cyclopentényle, un groupe cyclohexényle, un groupe cycloheptényle, un groupe phényle, un groupe naphtyle, un groupe pyridinyle, un groupe pyrimidinyle, un groupe dibenzofuranyle, et un groupe dibenzothiophényle, chacun substitué par au moins un élément choisi parmi un atome de deutérium, -F, -CD₃, -CD₂H, -CDH₂, -CF₃, -CF₂H, -CFH₂, un groupe cyano, un groupe nitro, un groupe alkyle en C₁ à C₁₀, un groupe alcoxy en C₁ à C₁₀, un groupe cyclopentyle, un groupe cyclohexyle, un groupe cycloheptyle, un groupe cyclooctyle, un groupe adamantanyle, un groupe norbornanyle, un groupe norbornényle, un groupe cyclopentényle, un groupe cyclohexényle, un groupe cycloheptényle, un groupe phényle, un groupe naphtyle, un groupe pyridinyle, un groupe pyrimidinyle, un groupe dibenzofuranyle, et un groupe dibenzothiophényle ; et
-N(Q₃₁)(Q₃₂), -Si(Q₃₃)(Q₃₄)(Q₃₅), -B(Q₃₆)(Q₃₇), et -P(=O)(Q₃₈)(Q₃₉),
dans lesquels Q₃₁ à Q₃₉ sont indépendamment choisis parmi :
-CH₃, -CD₃, -CD₂H, -CDH₂, -CH₂CH₃, -CH₂CD₃, -CH₂CD₂H, -CH₂CDH₂, -CHDCH₃, -CHDCD₂H, -CHDCDH₂, -CHDCD₃, -CD₂CD₃, -CD₂CD₂H, et -CD₂CDH₂ ;
un groupe n-propyle, un groupe isopropyle, un groupe n-butyle, un groupe isobutyle, un groupe sec-butyle, un groupe tert-butyle, un groupe n-pentyle, un groupe isopentyle, un groupe sec-pentyle, un groupe tert-pentyle, un groupe phényle, et un groupe naphtyle ; et
un groupe n-propyle, un groupe isopropyle, un groupe n-butyle, un groupe isobutyle, un groupe sec-butyle, un groupe tert-butyle, un groupe n-pentyle, un groupe isopentyle, un groupe sec-pentyle, un groupe tert-pentyle, un groupe phényle, et un groupe naphtyle, chacun substitué par au moins un élément choisi parmi l'atome de deutérium, un groupe alkyle en C₁ à C₁₀, et un groupe phényle,
deux éléments voisins ou plus choisis parmi une pluralité de groupes Z₁ sont éventuellement reliés les uns aux autres pour former un groupe carbocyclique en C₅ à C₃₀ substitué ou non substitué ou un groupe hétérocyclique en C₂ à C₃₀ substitué ou non substitué,
e1 représente un entier choisi parmi 0 à 8, et
* indique un site de liaison à M dans la formule 1.

12. Composé organométallique selon l'une quelconque des revendications 1 à 11, L₂ dans la formule 1 est choisi parmi les ligands représentés par les formules 13-1 à 13-47 et 14-1 à 14-28 : 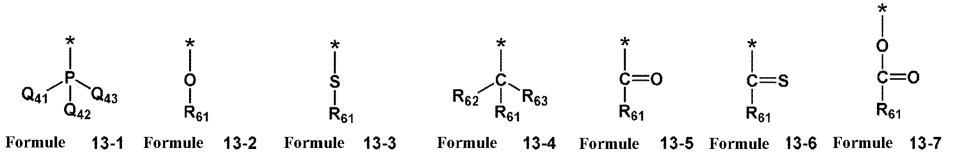 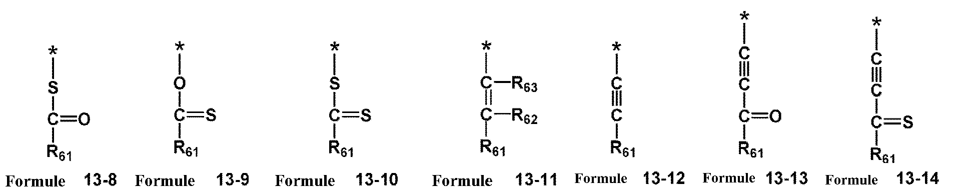 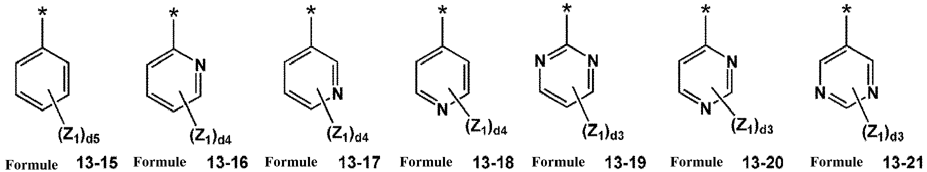 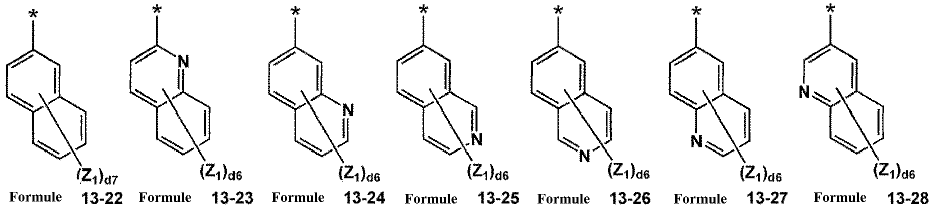 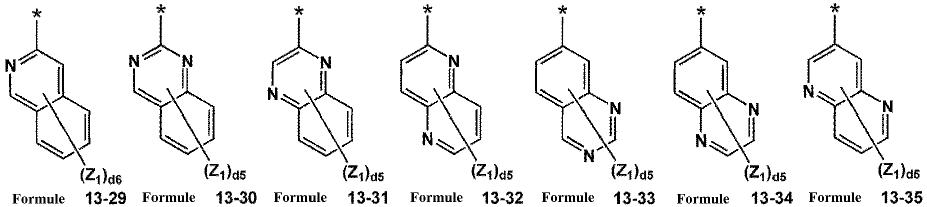 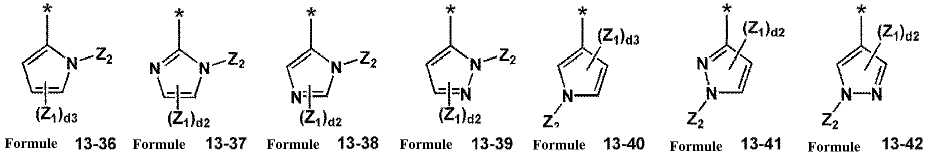 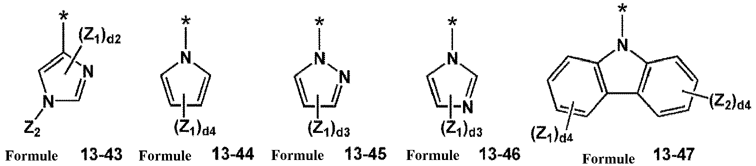 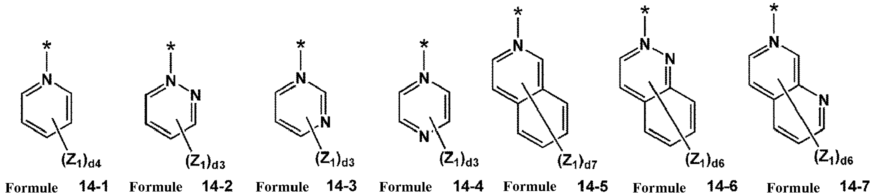 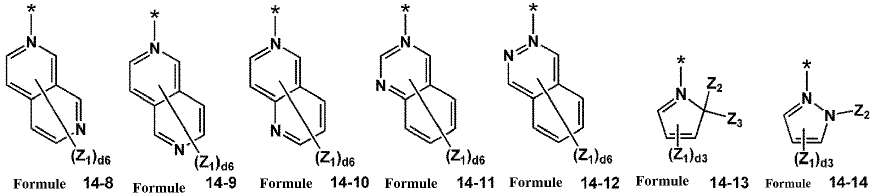 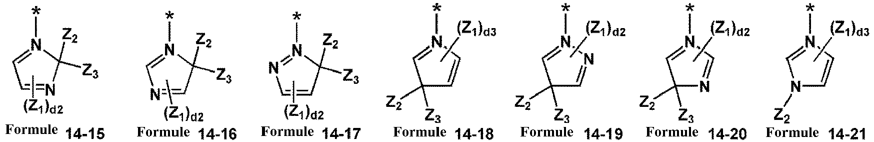 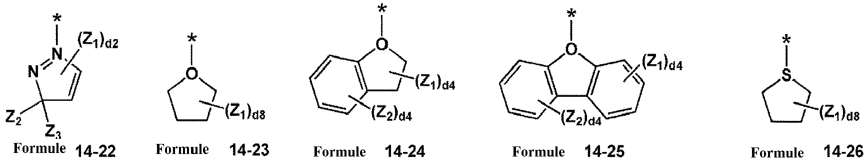 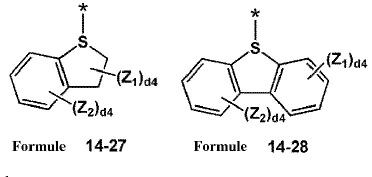
dans lesquelles, dans les formules 13-1 à 13-47 et 14-1 à 14-28,
R₆₁ est choisi parmi :
un atome d'hydrogène, un atome de deutérium, -F, -Cl, -Br, -I, un groupe hydroxy, un groupe cyano, un groupe nitro, un groupe amino, un groupe amidino, un groupe hydrazine, un groupe hydrazone, un groupe acide carboxylique ou un sel de celui-ci, un groupe acide sulfonique ou un sel de celui-ci, un groupe acide phosphorique ou un sel de celui-ci, -SF₅, un groupe alkyle en C₁ à C₂₀, et un groupe alcoxy en C₁ à C₂₀ ;
un groupe alkyle en C₁ à C₂₀, et un groupe alcoxy en C₁ à C₂₀, chacun substitué par au moins un élément choisi parmi un atome de deutérium, -F, -Cl, -Br, -I, -CD₃, -CD₂H, -CDH₂, -CF₃, -CF₂H, -CFH₂, un groupe hydroxy, un groupe cyano, un groupe nitro, un groupe amino, un groupe amidino, un groupe hydrazine, un groupe hydrazone, un groupe acide carboxylique ou un sel de celui-ci, un groupe acide sulfonique ou un sel de celui-ci, un groupe acide phosphorique ou un sel de celui-ci, un groupe alkyle en C₁ à C₁₀, un groupe cyclopentyle, un groupe cyclohexyle, un groupe cycloheptyle, un groupe cyclooctyle, un groupe adamantanyle, un groupe norbornanyle, un groupe norbornényle, un groupe cyclopentényle, un groupe cyclohexényle, un groupe cycloheptényle, un groupe phényle, un groupe naphtyle, un groupe pyridinyle, et un groupe pyrimidinyle ;
un groupe cyclopentyle, un groupe cyclohexyle, un groupe cycloheptyle, un groupe cyclooctyle, un groupe adamantanyle, un groupe norbornanyle, un groupe norbornényle, un groupe cyclopentényle, un groupe cyclohexényle, un groupe cycloheptényle, un groupe phényle, un groupe naphtyle, un groupe fluorényle, un groupe phénanthrényle, un groupe anthracényle, un groupe fluoranthényle, un groupe triphénylényle, un groupe pyrényle, un groupe chrysényle, un groupe pyrrolyle, un groupe thiophényle, un groupe furanyle, un groupe imidazolyle, un groupe pyrazolyle, un groupe thiazolyle, un groupe isothiazolyle, un groupe oxazolyle, un groupe isoxazolyle, un groupe pyridinyle, un groupe pyrazinyle, un groupe pyrimidinyle, un groupe pyridazinyle, un groupe isoindolyle, un groupe indolyle, un groupe indazolyle, un groupe purinyle, un groupe quinolinyle, un groupe isoquinolinyle, un groupe benzoquinolinyle, un groupe quinoxalinyle, un groupe quinazolinyle, un groupe cinnolinyle, un groupe carbazolyle, un groupe phénanthrolinyle, un groupe benzimidazolyle, un groupe benzofuranyle, un groupe benzothiophényle, un groupe isobenzothiazolyle, un groupe benzoxazolyle, un groupe isobenzoxazolyle, un groupe triazolyle, un groupe tétrazolyle, un groupe oxadiazolyle, un groupe triazinyle, un groupe dibenzofuranyle, un groupe dibenzothiophényle, un groupe benzocarbazolyle, un groupe dibenzocarbazolyle, un groupe imidazopyridinyle, et un groupe imidazopyrimidinyle ;
un groupe cyclopentyle, un groupe cyclohexyle, un groupe cycloheptyle, un groupe cyclooctyle, un groupe adamantanyle, un groupe norbornanyle, un groupe norbornényle, un groupe cyclopentényle, un groupe cyclohexényle, un groupe cycloheptényle, un groupe phényle, un groupe naphtyle, un groupe fluorényle, un groupe phénanthrényle, un groupe anthracényle, un groupe fluoranthényle, un groupe triphénylényle, un groupe pyrényle, un groupe chrysényle, un groupe pyrrolyle, un groupe thiophényle, un groupe furanyle, un groupe imidazolyle, un groupe pyrazolyle, un groupe thiazolyle, un groupe isothiazolyle, un groupe oxazolyle, un groupe isoxazolyle, un groupe pyridinyle, un groupe pyrazinyle, un groupe pyrimidinyle, un groupe pyridazinyle, un groupe isoindolyle, un groupe indolyle, un groupe indazolyle, un groupe purinyle, un groupe quinolinyle, un groupe isoquinolinyle, un groupe benzoquinolinyle, un groupe quinoxalinyle, un groupe quinazolinyle, un groupe cinnolinyle, un groupe carbazolyle, un groupe phénanthrolinyle, un groupe benzimidazolyle, un groupe benzofuranyle, un groupe benzothiophényle, un groupe isobenzothiazolyle, un groupe benzoxazolyle, un groupe isobenzoxazolyle, un groupe triazolyle, un groupe tétrazolyle, un groupe oxadiazolyle, un groupe triazinyle, un groupe dibenzofuranyle, un groupe dibenzothiophényle, un groupe benzocarbazolyle, un groupe dibenzocarbazolyle, un groupe imidazopyridinyle, et un groupe imidazopyrimidinyle, chacun substitué par au moins un élément choisi parmi un atome de deutérium, -F, -Cl, -Br, -I, -CD₃, -CD₂H, -CDH₂, -CF₃, -CF₂H, -CFH₂, un groupe hydroxy, un groupe cyano, un groupe nitro, un groupe amino, un groupe amidino, un groupe hydrazine, un groupe hydrazone, un groupe acide carboxylique ou un sel de celui-ci, un groupe acide sulfonique ou un sel de celui-ci, un groupe acide phosphorique ou un sel de celui-ci, un groupe alkyle en C₁ à C₂₀, un groupe alcoxy en C₁ à C₂₀, un groupe cyclopentyle, un groupe cyclohexyle, un groupe cycloheptyle, un groupe cyclooctyle, un groupe adamantanyle, un groupe norbornanyle, un groupe norbornényle, un groupe cyclopentényle, un groupe cyclohexényle, un groupe cycloheptényle, un groupe phényle, un groupe naphtyle, un groupe fluorényle, un groupe phénanthrényle, un groupe anthracényle, un groupe fluoranthényle, un groupe triphénylényle, un groupe pyrényle, un groupe chrysényle, un groupe pyrrolyle, un groupe thiophényle, un groupe furanyle, un groupe imidazolyle, un groupe pyrazolyle, un groupe thiazolyle, un groupe isothiazolyle, un groupe oxazolyle, un groupe isoxazolyle, un groupe pyridinyle, un groupe pyrazinyle, un groupe pyrimidinyle, un groupe pyridazinyle, un groupe isoindolyle, un groupe indolyle, un groupe indazolyle, un groupe purinyle, un groupe quinolinyle, un groupe isoquinolinyle, un groupe benzoquinolinyle, un groupe quinoxalinyle, un groupe quinazolinyle, un groupe cinnolinyle, un groupe carbazolyle, un groupe phénanthrolinyle, un groupe benzimidazolyle, un groupe benzofuranyle, un groupe benzothiophényle, un groupe isobenzothiazolyle, un groupe benzoxazolyle, un groupe isobenzoxazolyle, un groupe triazolyle, un groupe tétrazolyle, un groupe oxadiazolyle, un groupe triazinyle, un groupe dibenzofuranyle, un groupe dibenzothiophényle, un groupe benzocarbazolyle, un groupe dibenzocarbazolyle, un groupe imidazopyridinyle, et un groupe imidazopyrimidinyle ; et
-N(Q₁)(Q₂), -Si(Q₃)(Q₄)(Q₅), -B(Q₆)(Q₇), -P(=O)(Q₈)(Q₉), et -P(Q₄₁)(Q₄₂)(Q₄₃),
Z₁ à Z₃ sont chacun indépendamment choisis parmi :
un atome d'hydrogène, un atome de deutérium, -F, un groupe cyano, un groupe nitro, -SF₅, un groupe méthyle, un groupe éthyle, un groupe n-propyle, un groupe isopropyle, un groupe n-butyle, un groupe isobutyle, un groupe sec-butyle, un groupe tert-butyle, un groupe n-pentyle, un groupe isopentyle, un groupe sec-pentyle, un groupe tert-pentyle, un groupe n-hexyle, un groupe isohexyle, un groupe sec-hexyle, un groupe tert-hexyle, un groupe n-heptyle, un groupe isoheptyle, un groupe sec-heptyle, un groupe tert-heptyle, un groupe n-octyle, un groupe isooctyle, un groupe sec-octyle, un groupe tert-octyle, un groupe n-nonyle, un groupe isononyle, un groupe sec-nonyle, un groupe tert-nonyle, un groupe n-décyle, un groupe isodécyle, un groupe sec-décyle, un groupe tert-décyle, un groupe méthoxy, un groupe éthoxy, un groupe propoxy, un groupe butoxy, un groupe pentoxy, un groupe cyclopentyle, un groupe cyclohexyle, un groupe cycloheptyle, un groupe cyclooctyle, un groupe adamantanyle, un groupe norbornanyle, un groupe norbornényle, un groupe cyclopentényle, un groupe cyclohexényle, un groupe cycloheptényle, un groupe phényle, un groupe naphtyle, un groupe pyridinyle, un groupe pyrimidinyle, un groupe dibenzofuranyle, et un groupe dibenzothiophényle
un groupe méthyle, un groupe éthyle, un groupe n-propyle, un groupe isopropyle, un groupe n-butyle, un groupe isobutyle, un groupe sec-butyle, un groupe tert-butyle, un groupe n-pentyle, un groupe isopentyle, un groupe sec-pentyle, un groupe tert-pentyle, un groupe n-hexyle, un groupe isohexyle, un groupe sec-hexyle, un groupe tert-hexyle, un groupe n-heptyle, un groupe isoheptyle, un groupe sec-heptyle, un groupe tert-heptyle, un groupe n-octyle, un groupe isooctyle, un groupe sec-octyle, un groupe tert-octyle, un groupe n-nonyle, un groupe isononyle, un groupe sec-nonyle, un groupe tert-nonyle, un groupe n-décyle, un groupe isodécyle, un groupe sec-décyle, un groupe tert-décyle, un groupe méthoxy, un groupe éthoxy, un groupe propoxy, un groupe butoxy, un groupe pentoxy, un groupe cyclopentyle, un groupe cyclohexyle, un groupe cycloheptyle, un groupe cyclooctyle, un groupe adamantanyle, un groupe norbornanyle, un groupe norbornényle, un groupe cyclopentényle, un groupe cyclohexényle, un groupe cycloheptényle, un groupe phényle, un groupe naphtyle, un groupe pyridinyle, un groupe pyrimidinyle, un groupe dibenzofuranyle, et un groupe dibenzothiophényle, chacun substitué par au moins un élément choisi parmi un atome de deutérium, -F, -CD₃, -CD₂H, -CDH₂, -CF₃, -CF₂H, -CFH₂, un groupe cyano, un groupe nitro, un groupe alkyle en C₁ à C₁₀, un groupe alcoxy en C₁ à C₁₀, un groupe cyclopentyle, un groupe cyclohexyle, un groupe cycloheptyle, un groupe cyclooctyle, un groupe adamantanyle, un groupe norbornanyle, un groupe norbornényle, un groupe cyclopentényle, un groupe cyclohexényle, un groupe cycloheptényle, un groupe phényle, un groupe naphtyle, un groupe pyridinyle, un groupe pyrimidinyle, un groupe dibenzofuranyle, et un groupe dibenzothiophényle ; et
-N(Q₃₁)(Q₃₂), -Si(Q₃₃)(Q₃₄)(Q₃₅), -B(Q₃₆)(Q₃₇), et -P(=O)(Q₃₈)(Q₃₉),
dans lesquels Q₁ à Q₉, Q₃₁ à Q₃₉, et Q₄₁ à Q₄₃ sont chacun indépendamment choisis parmi :
-CH₃, -CD₃, -CD₂H, -CDH₂, -CH₂CH₃, -CH₂CD₃, -CH₂CD₂H, -CH₂CDH₂, -CHDCH₃, -CHDCD₂H, -CHDCDH₂, -CHDCD₃, -CD₂CD₃, -CD₂CD₂H, et -CD₂CDH₂ ;
un groupe n-propyle, un groupe isopropyle, un groupe n-butyle, un groupe isobutyle, un groupe sec-butyle, un groupe tert-butyle, un groupe n-pentyle, un groupe isopentyle, un groupe sec-pentyle, un groupe tert-pentyle, un groupe phényle, et un groupe naphtyle ; et
un groupe n-propyle, un groupe isopropyle, un groupe n-butyle, un groupe isobutyle, un groupe sec-butyle, un groupe tert-butyle, un groupe n-pentyle, un groupe isopentyle, un groupe sec-pentyle, un groupe tert-pentyle, un groupe phényle, et un groupe naphtyle, chacun substitué par au moins un élément choisi parmi un atome de deutérium, un groupe alkyle en C₁ à C₁₀, et un groupe phényle,
d2 vaut 1 ou 2,
d3 représente un entier choisi parmi 1 à 3,
d4 représente un entier choisi parmi 1 à 4,
d5 représente un entier choisi parmi 1 à 5,
d6 représente un entier choisi parmi 1 à 6,
d7 représente un entier choisi parmi 1 à 7,
d8 représente un entier choisi parmi 1 à 8, et
* indique un site de liaison à M dans la formule 1.

13. Composé organométallique selon la revendication 1,
ledit composé organométallique étant l'un choisi parmi les composés 1 à 142 :

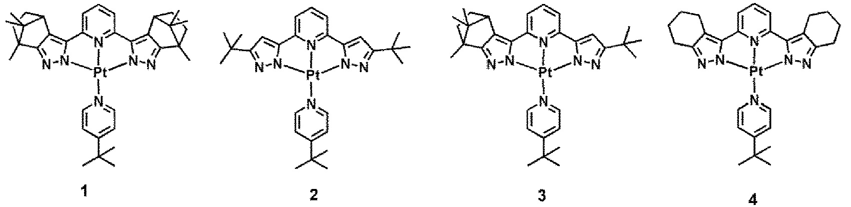

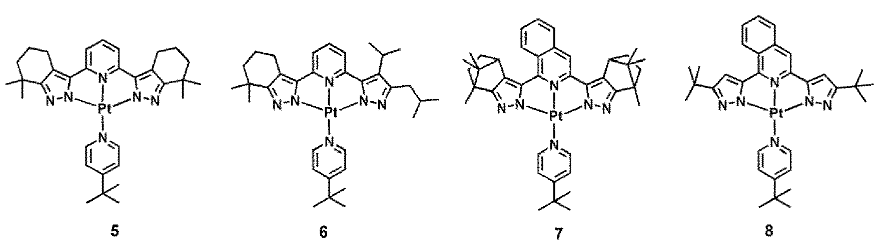

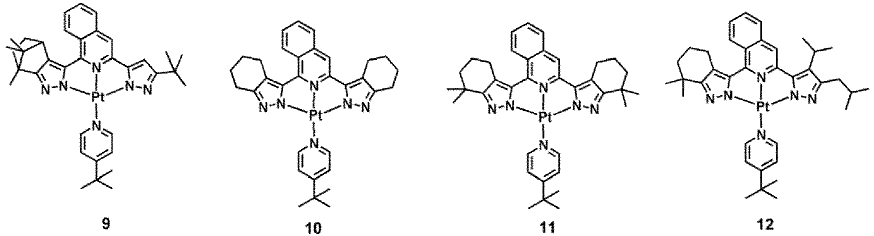

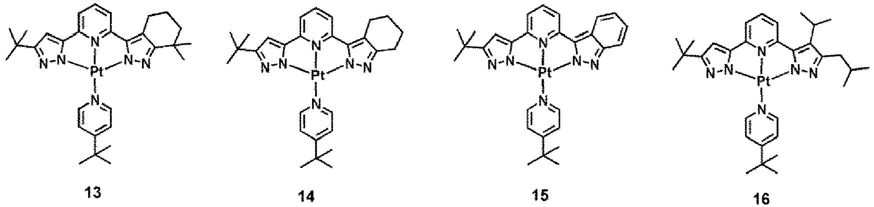

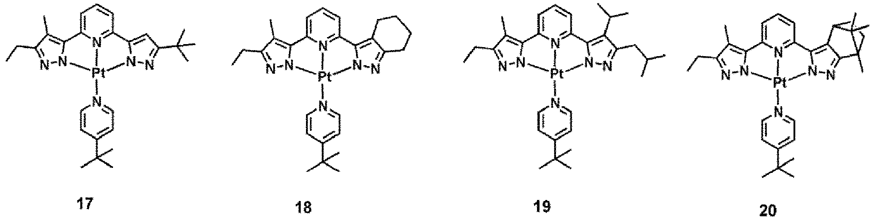

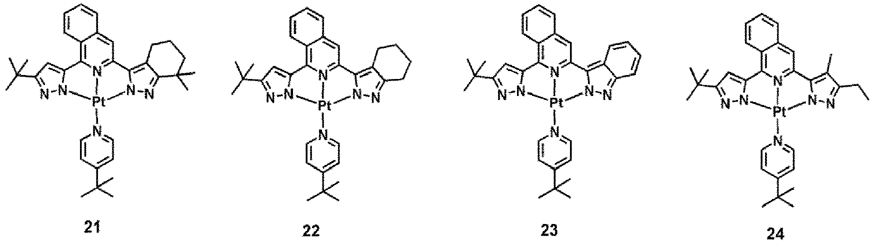

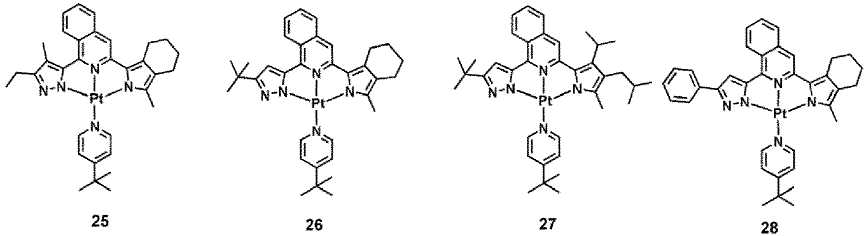

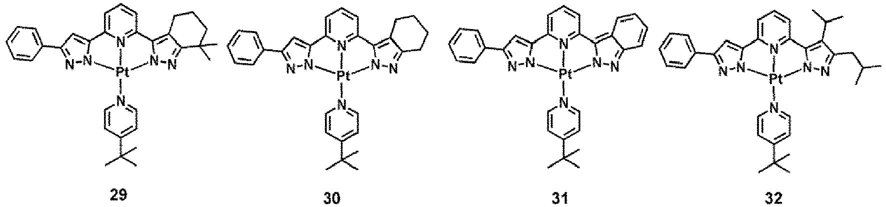

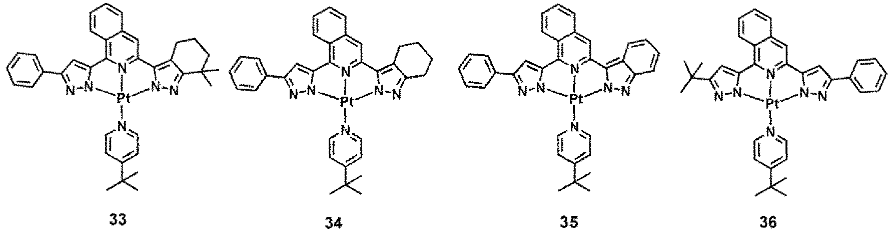

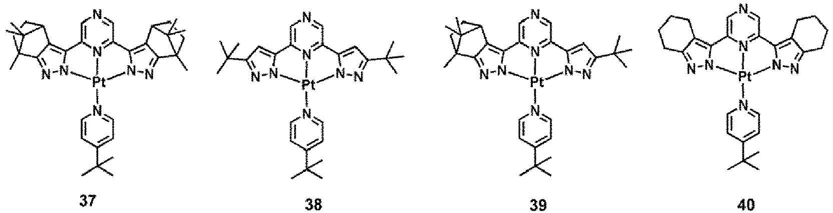

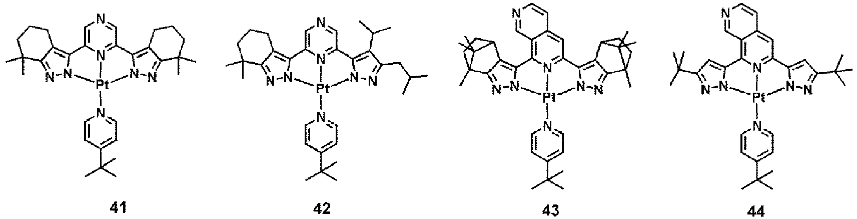

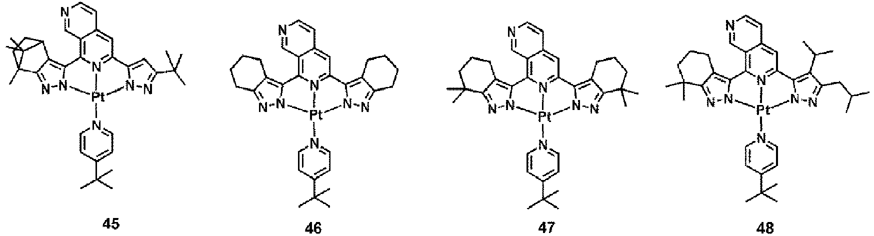

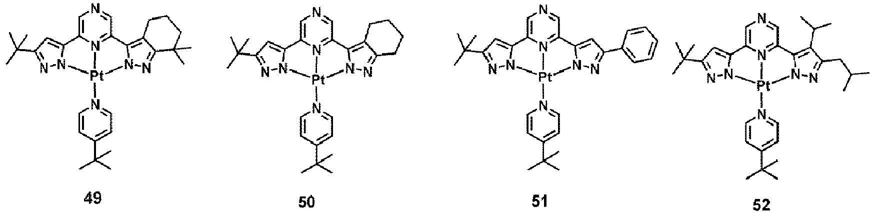

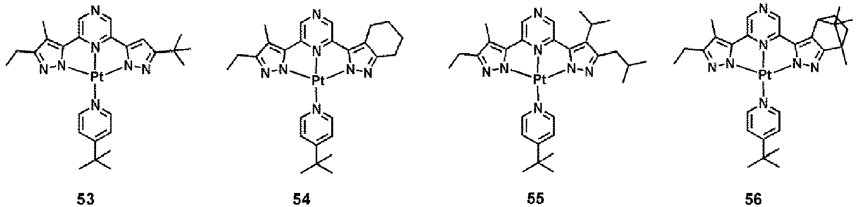

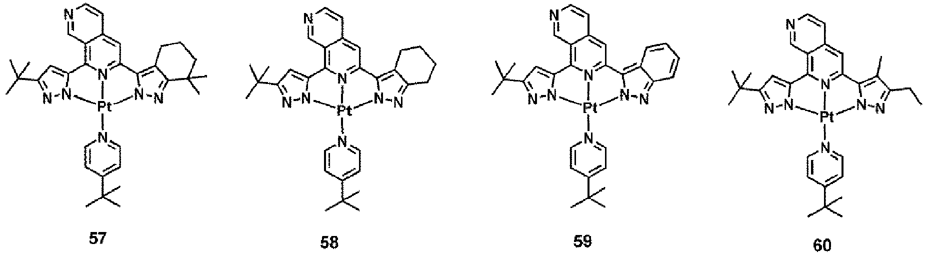

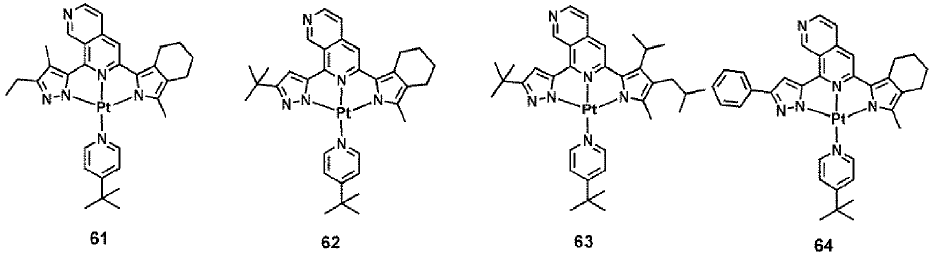

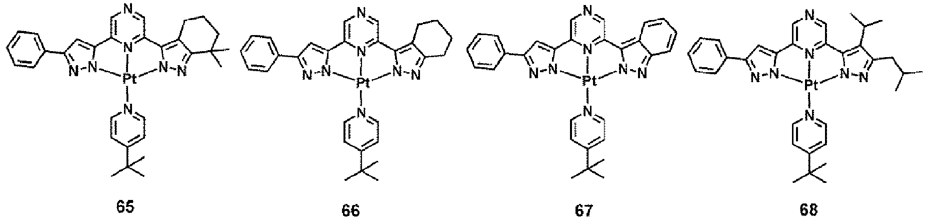

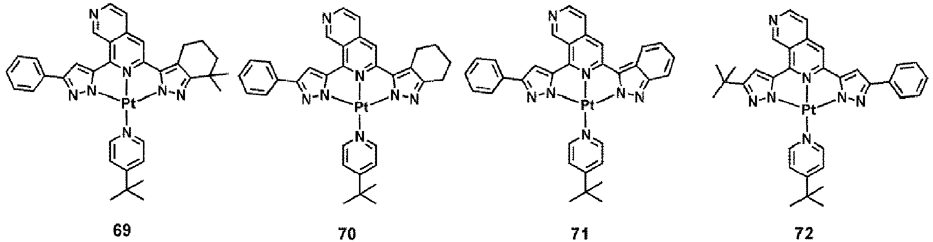

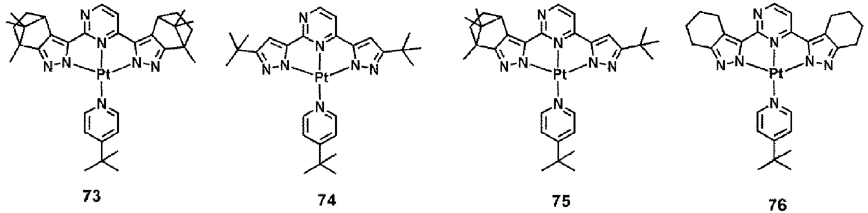

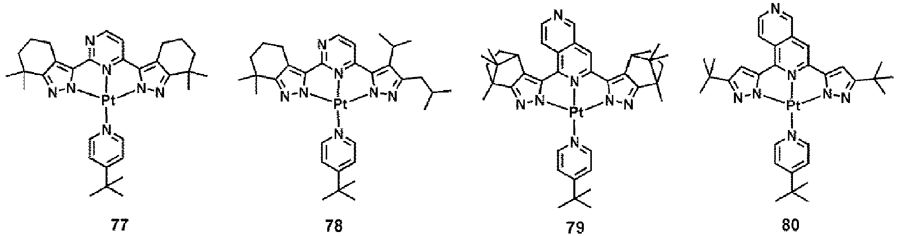

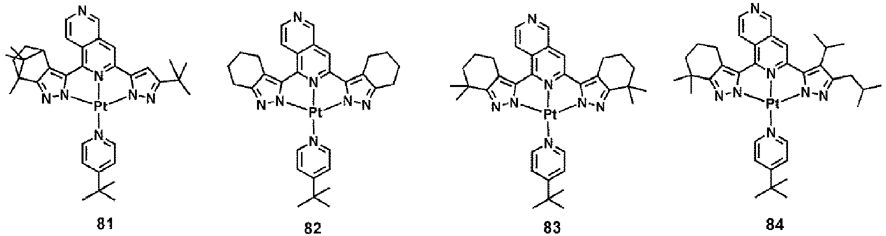

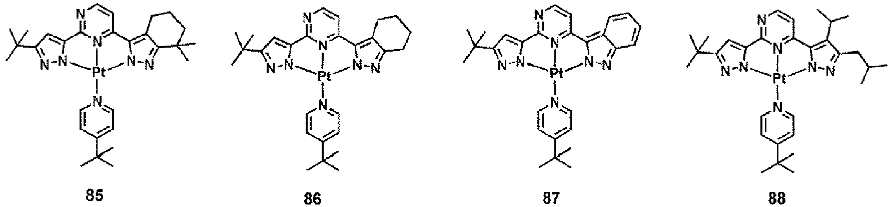

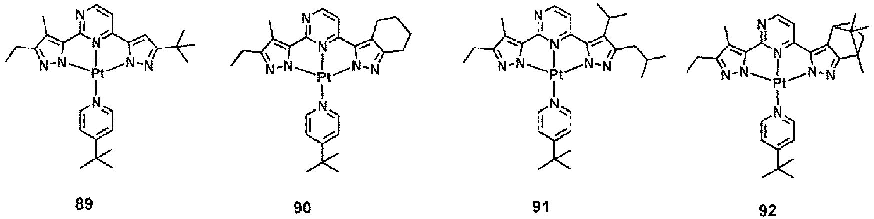

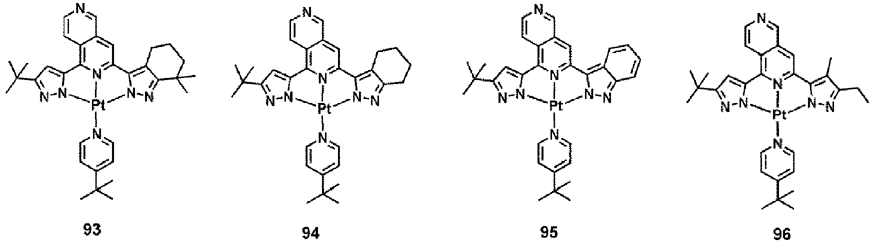

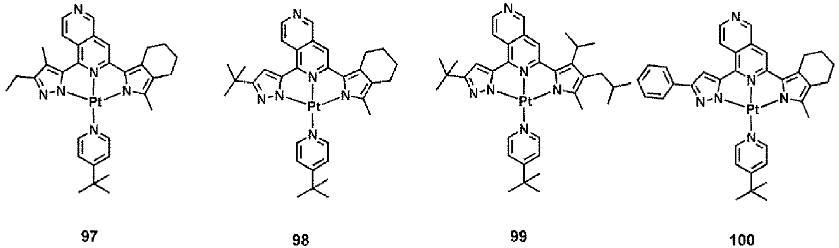

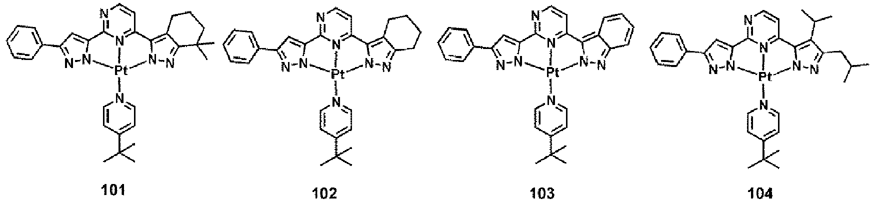

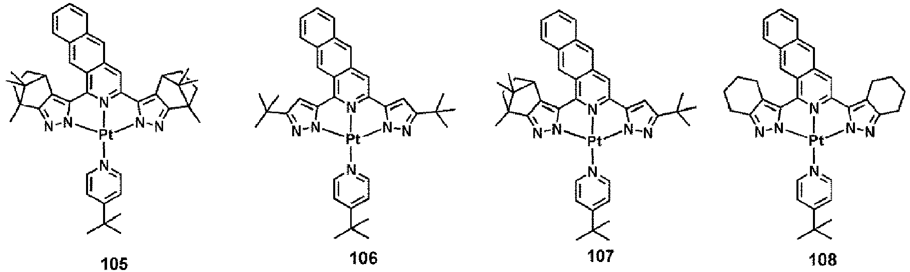

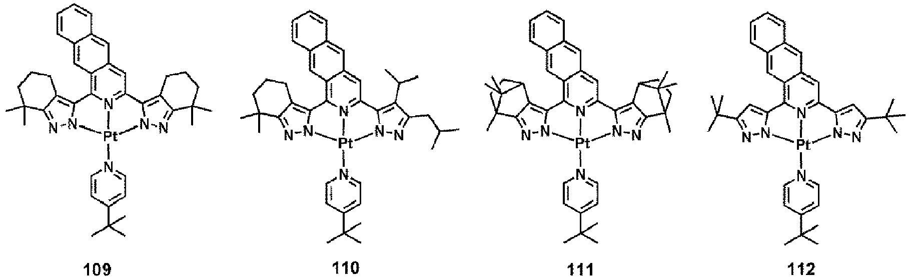

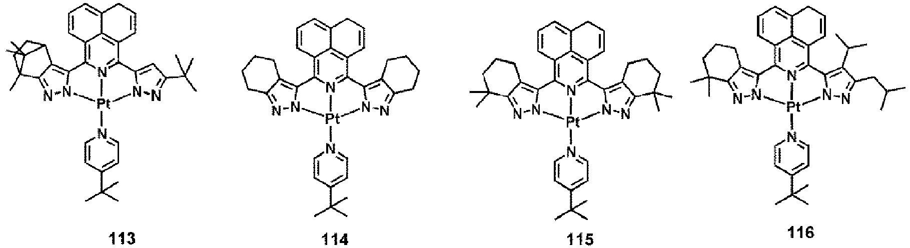

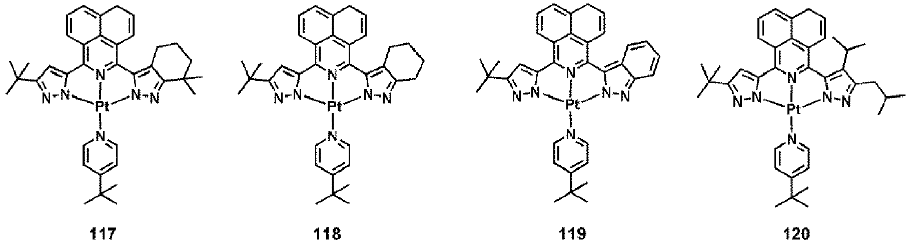

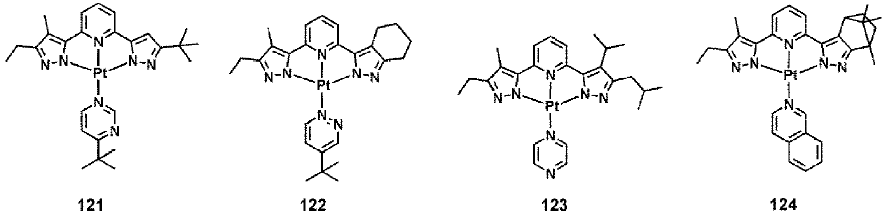

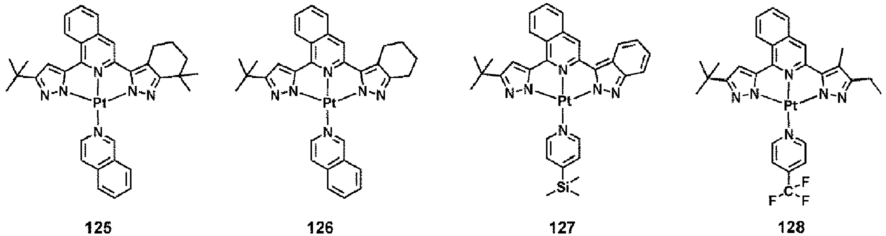

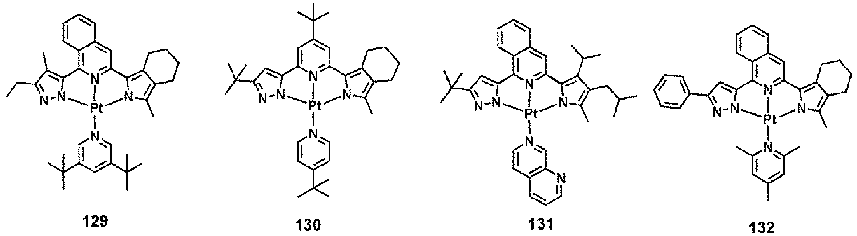

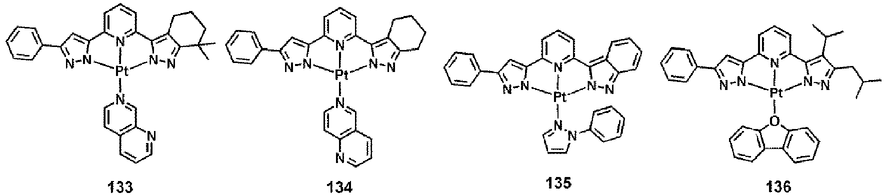

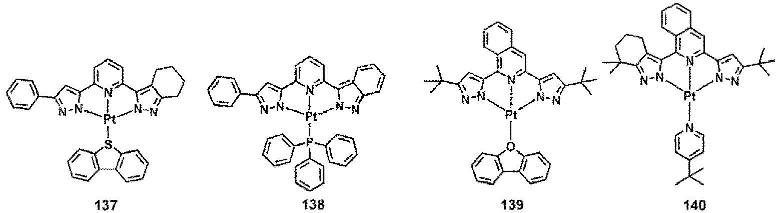

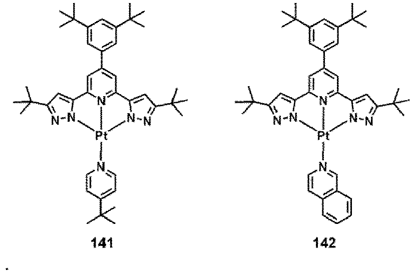

14. Dispositif électroluminescent organique comprenant :
une première électrode ;
une seconde électrode ; et
une couche organique entre la première électrode et la seconde électrode, la couche organique comprenant une couche d'émission,
ladite couche organique comprenant un ou plusieurs composés choisis parmi les composés organométalliques selon l'une quelconque des revendications 1 à 13, de préférence ledit composé organométallique étant inclus dans la couche d'émission.

15. Composition comprenant un ou plusieurs composés choisis parmi les composés organométalliques selon l'une quelconque des revendications 1 à 13 pour utilisation en diagnostic *in vivo.*

16. Utilisation d'une composition comprenant :
un ou plusieurs composés choisis parmi les composés organométalliques selon l'une quelconque des revendications 1 à 13 pour diagnostic *in vitro.*
